(19) Europäisches Patentamt
European Patent Office
Office européen des brevets

(11) EP 4 053 124 A1

(12) EUROPEAN PATENT APPLICATION
published in accordance with Art. 153(4) EPC

(43) Date of publication:
**07.09.2022 Bulletin 2022/36**

(21) Application number: **20881171.1**

(22) Date of filing: **23.10.2020**

(51) International Patent Classification (IPC):
***C07D 409/04*** (2006.01) ***C07D 409/14*** (2006.01)
***C07D 407/04*** (2006.01) ***C07D 333/00*** (2006.01)
***C07D 231/56*** (2006.01) ***C07D 493/00*** (2006.01)
***C07D 487/00*** (2006.01) ***A61K 31/381*** (2006.01)
***A61K 31/403*** (2006.01) ***A61K 31/343*** (2006.01)
***A61K 31/357*** (2006.01) ***A61P 35/00*** (2006.01)
***A61P 37/00*** (2006.01)

(52) Cooperative Patent Classification (CPC):
**A61K 31/343; A61K 31/357; A61K 31/381; A61K 31/403; A61K 31/404; A61K 31/416; A61K 31/4184; A61K 31/4355; A61K 31/4365; A61K 31/437; A61P 31/14; A61P 31/18; A61P 31/20; A61P 35/00; A61P 37/00;** (Cont.)

(86) International application number:
**PCT/CN2020/123404**

(87) International publication number:
**WO 2021/083060 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.10.2019 CN 201911031481**
**25.03.2020 CN 202010218170**

(71) Applicant: **Shanghai Institute of Materia Medica, Chinese Academy of Sciences**
**Pudong, Shanghai 201203 (CN)**

(72) Inventors:
- **DUAN, Wenhu**
**Shanghai 201203 (CN)**
- **GENG, Meiyu**
**Shanghai 201203 (CN)**
- **ZHAN, Zhengsheng**
**Shanghai 201203 (CN)**
- **XIE, Zuoquan**
**Shanghai 201203 (CN)**
- **ZHAO, Kaiyan**
**Shanghai 201203 (CN)**
- **GUO, Yuting**
**Shanghai 201203 (CN)**
- **WANG, Xiyuan**
**Shanghai 201203 (CN)**
- **ZHANG, Yan**
**Shanghai 201203 (CN)**
- **ZHOU, Xiaoqian**
**Shanghai 201203 (CN)**
- **DING, Jian**
**Shanghai 201203 (CN)**

(74) Representative: **Mewburn Ellis LLP**
**Aurora Building**
**Counterslip**
**Bristol BS1 6BX (GB)**

# (54) FIVE-MEMBERED HETEROCYCLIC OXOCARBOXYLIC ACID COMPOUND AND MEDICAL USE THEREOF

(57) The present invention relates to a five-membered heterocyclic oxocarboxylic acid compound and the medical use thereof. Specifically, the present invention relates to a compound as represented by formula (I) and a pharmaceutically acceptable salt, prodrug, hydrate, solvate or crystal form thereof, and also relates to a method for preparing the compound, a pharmaceutical composition containing the compound, and the medical use thereof as a secretion regulator of type I interferon, especially as a STING agonist, and in the preparation of a drug for preventing and/or treating diseases related to type I interferon.



Processed by Luminess, 75001 PARIS (FR)

(Cont. next page)

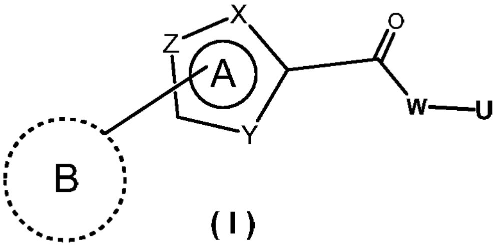

(I)

(52) Cooperative Patent Classification (CPC): (Cont.)
**C07D 231/56; C07D 307/54; C07D 307/78; C07D 333/00; C07D 333/24; C07D 333/60; C07D 333/76; C07D 405/04; C07D 407/04; C07D 409/04; C07D 409/14; C07D 471/04; C07D 487/00; C07D 493/00; C07D 495/04**

## Description

## TECHNICAL FIELD

The present invention belongs to the field of medicinal chemistry and pharmacotherapy, and specifically relates to a five-membered heterocyclic oxocarboxylic acid compound, and also relates to a preparation method of the compound, the pharmaceutically acceptable salt, the prodrug, the hydrate or the solvate thereof and also relates to a pharmaceutical composition of the compound and a use as a secretion regulator of type I interferon, especially as a STING agonist, and a use in the preparation of a medicament for the prevention and/or treatment of type I interferon-related diseases.

## BACKGROUND OF THE INVENTION

In recent years, tumor immunotherapy has become an important means of emerging anti-tumor drug treatement. Taking PD-1/PD-L1 as an example, antagonizing the immunosuppressive signaling pathway of PD-1/PD-L1, releasing an anti-tumor immune response, and exerting an anti-tumor effect through the immune system, which has become a hot spot in tumor treatment today. So far, clinical trials have shown that PD-1/PD-L1 inhibitors have therapeutic effects in more than 10 types of tumors, with an average effective rate of about 20%, and higher safety than conventional chemotherapy drugs. The successful clinical trials of PD-1/PD-L1 inhibitors have greatly promoted the development of tumor immunotherapy. However, PD-1/PD-L1 inhibitors also face the problem of low efficacy. The existence of complex and multiple immunosuppressive factors in the tumor microenvironment hinders the efficacy of PD-1/PD-L1 inhibitors and makes the treatment ineffective, resulting in the lack of efficient anti-tumor drugs for most tumor patients, especially those with advanced tumors. Therefore, the development of new safe and effective tumor immunotherapy drugs has become the focus of current anti-tumor drug research.

Innate immunity plays a very important role in the anti-tumor immune response, and is an important direction for the development of anti-tumor drug therapy in recent years. Innate immune ligands interact with receptor molecules, and are involved in the recognition of molecular patterns of exogenous pathogenic microorganisms and endogenous abnormal damage, respectively called Pathogen Associated Molecular Patterns (PAMP) and Damage Associated Molecular Patterns (DAMP), which recognize receptors on the cell surface or in cells, including TLRs, RLRs, NLRs and cGAS, activate inflammatory pathways such as NF-kB and interferon, and recruit immune cells to produce immune response, and can activate anti-tumor immune response in the tumor, prevent tumor development and play an anti-tumor role.

Different innate immune receptors respond to different ligands, such as Toll-like receptors (TLRs) are a large family of molecules, 11 members have been found, which can exist on the cell surface or endosomes, among which TLR1, TLR2, TLR4, TLR5 and TLR6 exist on the cell membrane surface and sense extracellular ligands. For example, the well-known TLR4 can sense LPS to initiate downstream signaling pathways; while TLR3, TLR7, TLR8 and TLR9 exist in Endosome, which can sense RNA or CpG molecules, for example, TLR9 binds to ligand CpG, and TLR3 binds to dsRNA to initiate downstream signaling pathways. RIG-like receptors (RLRs) mainly sense the cytoplasmic RNA pathway, and the involved molecules include RIG-1, MDA5 and MAVS. NOD-like receptors (NLRs) mainly sense glycoside molecules on the cell surface. cGAS mainly senses cytoplasmic double-stranded DNA (dsDNA), and these dsDNA originate from exogenously entered viruses or from broken dsDNA released into the cytoplasm by endogenous damage. cGAS can be activated to produce cyclic dinucleosides (CDNs), and then activate the STING-TBK1-IRF3 signaling pathway, promote the secretion of type I interferon pathway, thereby activating innate immune cells, including APC cells and NK cells, and promoting the formation of anti-tumor immune responses and adaptive immune responses.

Among them, the cGAS-STING-TBK1-IRF3 pathway plays a key role in anti-tumor immunity. Activation of this pathway stimulates the production and secretion of type I interferons, which in turn activates the anti-tumor immune response. Activated cells involved mainly include dendritic cells (DC), monocytes, macrophages, and natural killer cells (NK), which induce and activate killer T cells, thereby killing tumor cells. In addition, activation of this pathway can directly enhance the activity of killing tumor cells, including pyroptosis, apoptosis and endoplasmic reticulum stress death and other death methods, which play a part of the anti-tumor effect. In addition, defects in the cGAS-STING pathway have been found in some tumors, such as colon cancer and breast cancer, leading to immune escape.

In conclusion, targeting the cGAS-STING-TBK1-IRF3 pathway has become an important strategy for anti-tumor immunotherapy. Among them, the activation of STING molecule to induce the production of type I interferon has become a promising direction in anti-tumor immunotherapy. At present, there are several STING agonists in clinical and preclinical research, such as DXMAA, ADU-S100, etc. Among them, ADU-S100 is a cyclic dinucleotide (CDN) analog. The main problems are poor stability of compounds, not easy to synthesize, unstable metabolism, etc., so the method of administration can only be intratumoral injection, which limits its clinical application to deep tumors. Although DMXAA is a small molecule STING agonist, it only has an agonistic effect on murine STING, while it was ineffective in the human-derived STING, so the phase I clinical study was terminated.

Therefore, it is urgent to develop a new generation of small-molecule STING agonists with simple structure, easy synthesis and stable metabolism, which can activate anti-tumor immune responses and exert anti-tumor effects.

## SUMMARY OF THE INVENTION

The present invention provides a novel small molecule STING agonist with simple structure, easy synthesis and stable metabolism, which can activate anti-tumor immune response and exert anti-tumor effect.

The purpose of the present invention is to provide a five-membered heterocyclic oxocarboxylic acid compound, which has the function of inducing the secretion of STING-dependent type I interferon.

In the first aspect of the present invention, it provides a compound represented by formula (I), or a pharmaceutically acceptable salt, prodrug, hydrate, solvate or crystal form thereof:

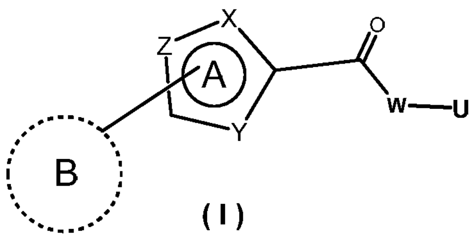

(I)

wherein,

W is selected from the group consisting of substituted or unsubstituted C3-C8 cycloalkyl, substituted or unsubstituted 3-8 membered heterocyclyl,

, 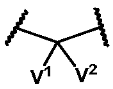 , 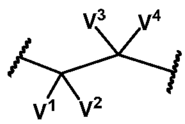 ,

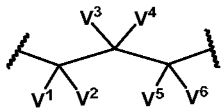 and 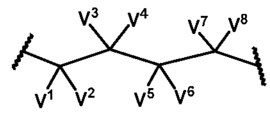 ,

, wherein, $V^1$, $V^2$, $V^3$, $V^4$, $V^5$, $V^6$, $V^7$ and $V^8$ are each independently selected from the group consisting of hydrogen atom, halogen, deuterium, cyano, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl and substituted or unsubstituted 3-8-membered heterocyclyl;

or $V^1$ and $V^2$ together with the C atom to which they are attached form a substituted or unsubstituted C3-C8 cycloalkyl or substituted or unsubstituted 3-8 membered heterocyclyl;

or $V^3$ and $V^4$ together with the C atom to which they are attached form a substituted or unsubstituted C3-C8 cycloalkyl or substituted or unsubstituted 3-8 membered heterocyclyl;

or $V^5$ and $V^6$ together with the C atom to which they are attached form a substituted or unsubstituted C3-C8 cycloalkyl or substituted or unsubstituted 3-8 membered heterocyclyl;

or $V^7$ and $V^8$ together with the C atom to which they are attached form a substituted or unsubstituted C3-C8 cycloalkyl or substituted or unsubstituted 3-8 membered heterocyclyl;

or $V^1$ and $V^3$ together with the atoms to which they are attached form a substituted or unsubstituted C3-C8 cycloalkyl or a substituted or unsubstituted 3-8 membered heterocyclyl;

or $V^1$ and $V^5$ together with the atoms to which they are attached form a substituted or unsubstituted C3-C8 cycloalkyl or a substituted or unsubstituted 3-8 membered heterocyclyl;

or $V^3$ and $V^7$ together with the atoms to which they are attached form a substituted or unsubstituted C3-C8 cycloalkyl or a substituted or unsubstituted 3-8 membered heterocyclyl;

or $V^5$ and $V^7$ together with the atoms to which they are attached form a substituted or unsubstituted C3-C8 cycloalkyl or a substituted or unsubstituted 3-8 membered heterocyclyl;

or $V^1$ and $V^7$ together with the atoms to which they are attached form a substituted or unsubstituted C4-C8 cycloalkyl

or a substituted or unsubstituted 4-8 membered heterocyclyl;
U is independently selected from the group consisting of $COOR^1$, COOH, CN, $CONH_2$ and

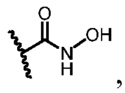

,

wherein, $R^1$ is selected from the group consisting of substituted or unsubstituted C1-C6 alkyl, substituted or unsubstituted C3-C8 cycloalkyl and substituted or unsubstituted 3-8 membered heterocyclyl;
ring A is a five-membered heteroaromatic ring, wherein X, Y, and Z are each independently selected from C, CH, C-$R^2$, NH, N, O or S, wherein, $R^2$ is selected from the group consisting of halogen, deuterium, hydroxyl, amino, cyano, carboxyl, formyl, substituted or unsubstituted formamido, substituted or unsubstituted C1-C6 alkyl, substituted or unsubstituted C1-C6 alkoxy, substituted or unsubstituted C3-C8 cycloalkyl, and substituted or unsubstituted 3-8 membered heterocyclyl;
ring B is independently selected from the group consisting of substituted or unsubstituted 5-14 membered heteroaromatic ring and substituted or unsubstituted C6-C14 aromatic ring;
wherein, unless otherwise specified, the "substituted" refers to being substituted by one or more groups selected from the group consisting of deuterium, halogen, hydroxyl, cyano, carboxyl, amino,

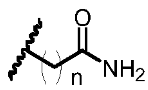

,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

In another preferred embodiment, the compound of formula (I), or a pharmaceutically acceptable salt, prodrug, hydrate, solvate or crystal form thereof has a structure shown in formula (I'):

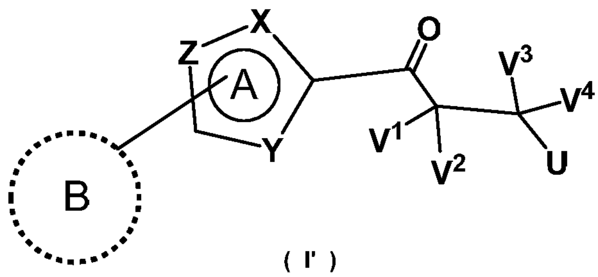

**( I' )**

wherein, $V^1$, $V^2$, $V^3$ and $V^4$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;
U is independently selected from the group consisting of $COOR^m$, COOH, CN, $CONH_2$ and

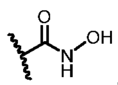

,

wherein, $R^m$ is selected from substituted or unsubstituted C1-C6 alkyl, substituted or unsubstituted C3-C8 cycloalkyl or substituted or unsubstituted 3-8 membered heterocyclyl;
ring A is a five-membered heteroaromatic ring, wherein X, Y and Z are each independently selected from CH, C-

$R^n$, NH, N, O or S, wherein, $R^n$ is selected from the group consisting of halogen, deuterium, hydroxyl, amino, cyano, carboxyl, formyl, substituted or unsubstituted formamido, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

ring B is independently selected from the group consisting of substituted or unsubstituted 5-14 membered heteroaromatic ring and substituted or unsubstituted C6-C14 aromatic ring;

wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

In another preferred embodiment, W is selected from the group consisting of

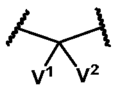, 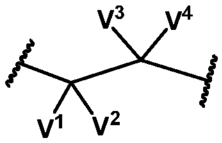, 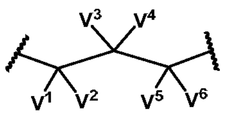 and 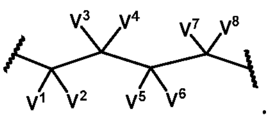,

, wherein, $V^1$, $V^2$, $V^3$, $V^4$, $V^5$, $V^6$, $V^7$ and $V^8$ are defined as above.

In another preferred embodiment, ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;

the "substituted" refers to being substituted by one or more groups selected from the group consisting of deuterium, halogen, hydroxyl, cyano, carboxyl, amino,

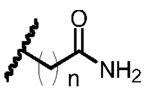,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;

n is an integer of 1-6.

In another preferred embodiment, the compound of formula (I), or a pharmaceutically acceptable salt, prodrug, hydrate, solvate or crystal form thereof has the structure shown in formula (I'):

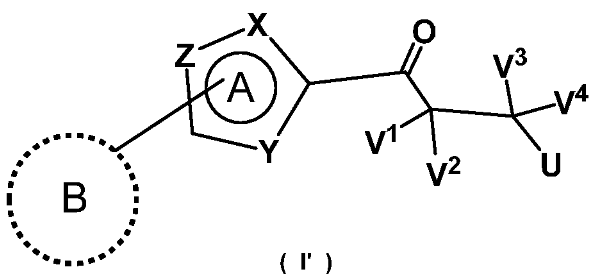

( I' )

wherein, $V^1$, $V^2$, $V^3$ and $V^4$ are each independently selected from the group consisting of hydrogen atom, halogen,

substituted or unsubstituted C1-C3 alkyl, substituted or unsubstituted C1-C3 alkoxy, substituted or unsubstituted C3-C6 cycloalkyl and substituted or unsubstituted 3-8 membered heterocyclyl;

U is independently slected from the group consisting of $COOR^m$, COOH, CN, $CONH_2$ and

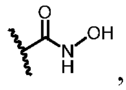
,

wherein, $R^m$ is selected from substituted or unsubstituted C1-C6 alkyl and substituted or unsubstituted C3-C6 cycloalkyl;
ring A is a five-membered heteroaromatic ring, wherein X, Y and Z are each independently selected from CH, C-$R^n$, NH, N, O or S, wherein $R^n$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
ring B and

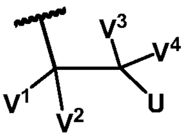

are located in the ortho or meta position of ring A; wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl;
wherein, the "substituted" in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

In another preferred embodiment, in formula I', $V^1$, $V^2$, $V^3$ and $V^4$ are each independently selected from hydrogen atom, halogen, C1-C3 alkyl, C1-C3 alkoxy and C3~C6 cycloalkyl.

In another preferred embodiment, the compound of formula (I), or a pharmaceutically acceptable salt, prodrug, hydrate, solvate or crystal form thereof:

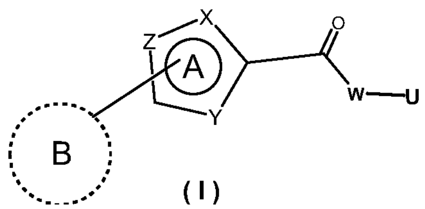
(I)

wherein W is selected from the group consisting of

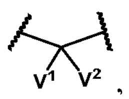
,
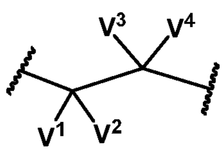
,

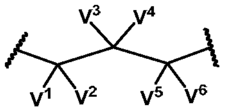 and 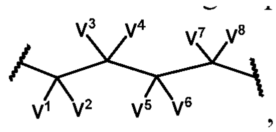,

wherein $V^1$, $V^2$, $V^3$, $V^4$, $V^5$, $V^6$, $V^7$ and $V^8$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is independently selected from the group consisting of $COOR^1$, COOH, CN, $CONH_2$ and

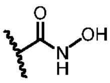,

wherein, $R^1$ is selected from substituted or unsubstituted C1-C6 alkyl, substituted or unsubstituted C3-C8 cycloalkyl and substituted or unsubstituted 3-8 membered heterocyclyl;
ring A is a five-membered heteroaromatic ring, wherein X, Y and Z are each independently selected from the group consisting of C, CH, C-$R^2$, NH, N, O or S, wherein $R^2$ is selected from halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
ring B and

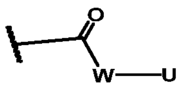

are located in the ortho or meta position of ring A;
wherein, the "substituted" refers to being substituted by one or more groups selected from the group consisting of deuterium halogen, hydroxyl, cyano, carboxyl, amino,

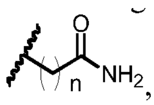,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

In another preferred embodiment, ring A is selected from substituted or unsubstituted thienyl, furyl, thiazolyl, or pyrrolyl; wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl; wherein, the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

In another preferred embodiment, X is O, S or NH.

In another preferred embodiment, Y is NH or $CR^2$, wherein $R^2$ is defined as above.

In another preferred embodiment, Z is NH or $CR^2$, wherein $R^2$ is defined as above.

In another preferred embodiment, X is O, S or NH; Y is NH or $CR^2$; Z is NH or $CR^2$, wherein $R^2$ is defined as above.

In another preferred embodiment, the compound of formula (I), or a pharmaceutically acceptable salt, prodrug, hydrate, solvate or crystal form thereof has the structure shown in formula (**II**), (**III**), (**IV**), (**V**) or (**VI**):

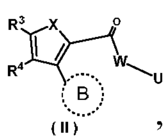
(II) ,
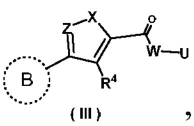
(III) ,
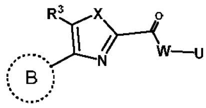
(IV) ,
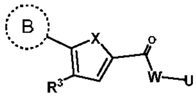
(V) or
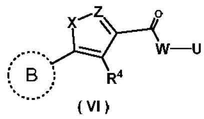
(VI)

wherein, X is selected form O, S or NH;

Z is selected from: CH, N or C-$R^2$; $R^2$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;

$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C8 cycloalkyl and substituted or unsubstituted 3-8-membered heterocyclyl; wherein, the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;

wherein, unless otherwise specified, the "substituted" refers to being substituted by one or more groups selected from the group consisting of deuterium, halogen, hydroxyl, cyano, carboxyl, amino,

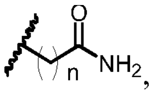
,

amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;

n is an integer of 1-6;

W, U and B are defined as above.

In another preferred embodiment, the compound of formula (I), or a pharmaceutically acceptable salt, prodrug, hydrate, solvate or crystal form thereof has the structure shown in formula **(II), (III), (IV), (V)** or **(VI):**

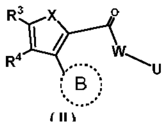
(II) ,
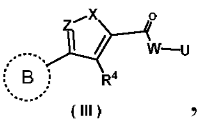
(III) ,
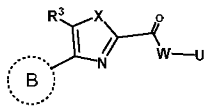
(IV) ,
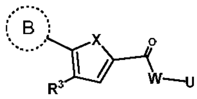
(V) or
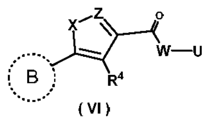
(VI)

wherein W is selected from the group consisting of

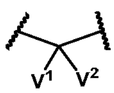
,
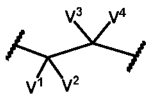
,

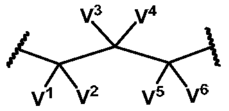 and 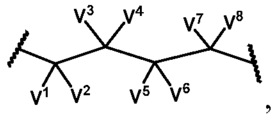 ,

wherein $V^1$, $V^2$, $V^3$, $V^4$, $V^5$, $V^6$, $V^7$ and $V^8$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is selected from the group consisting of $COOR^1$, COOH, CN, $CONH_2$ and

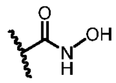 ,

wherein, $R^1$ is selected from the group consisting of substituted and unsubstituted C1-C6 alkyl and substituted or unsubstituted C3-C6 cycloalkyl;
wherein, X is selected form O, S or NH;
Z is selected from CH, N or C-$R^2$;
wherein, $R^2$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C8 cycloalkyl and substituted or unsubstituted 3-8-membered heterocyclyl; wherein, the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
wherein, unless otherwise specified, the "substituted" refers to being substituted by one or more groups selected from the group consisting of deuterium halogen, hydroxyl, cyano, carboxyl, amino,

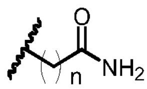 ,

amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

In another preferred embodiment, the compound of formula (I), or a pharmaceutically acceptable salt, prodrug, hydrate, solvate or crystal form thereof has the structure shown in formula (**II**-1), (**III**-1), **(IV-1),** (**V-1**) or (**VI**-1):

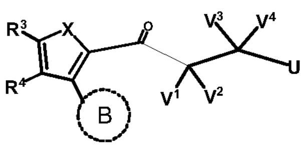
( II-1 ) ,

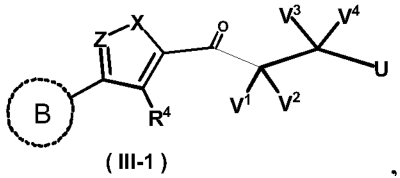
( III-1 ) ,

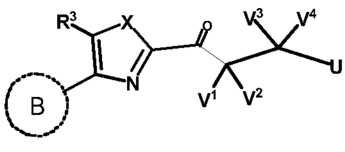
( IV-1 ) ,

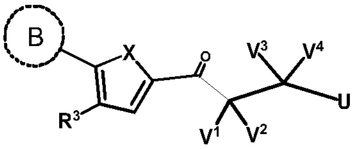

( V -1) or

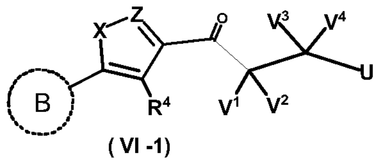

( VI -1) ,

wherein, X is selected form O, S or NH.

Z is selected from N or C-$R^3$;

$V^1$, $V^2$, $V^3$ and $V^4$ are each independently selected from hydrogen atom, halogen, C1-C3 alkyl, C1-C3 alkoxy or C3-C6 cycloalkyl;

U is selected from $COOR^m$, COOH, CN, $CONH_2$ or

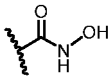

,

wherein, $R^m$ is selected from substituted or unsubstituted C1-C6 alkyl or substituted or unsubstituted C3-C6 cycloalkyl;

$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C6 cycloalkyl and substituted or unsubstituted 3-8-membered heterocyclyl;

ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;

wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

In another preferred embodiment, the compound of formula (I), or a pharmaceutically acceptable salt, prodrug, hydrate, solvate or crystal form thereof, wherein ring B is selected from

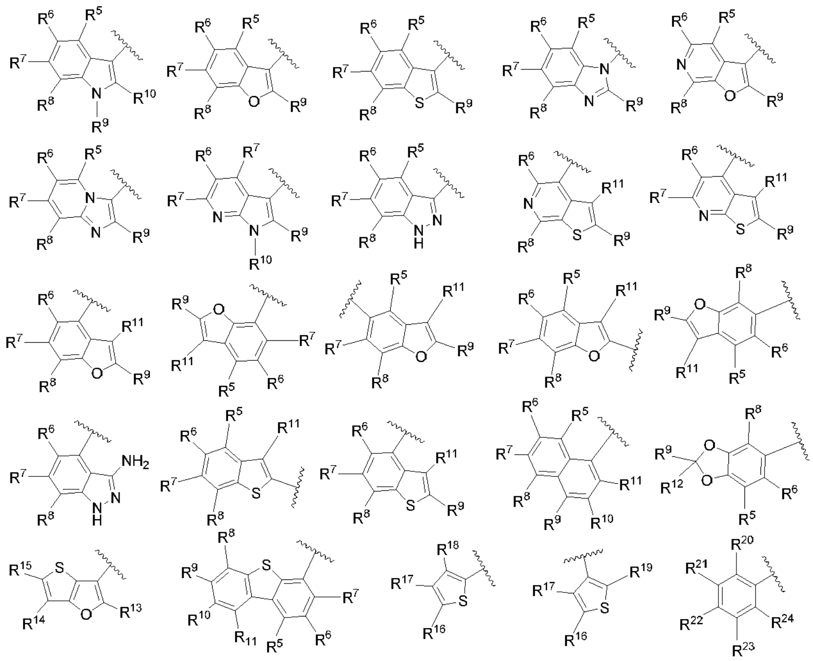

wherein, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$ and $R^{24}$ are each independently selected from the group consisting of hydrogen atom, halogen, deuterium, substituted or unsubstituted C1-C6 alkyl, C2-C6 alkenyl, substituted or unsubstituted C1-C6 alkoxy, substituted or unsubstituted C3-C6 cycloalkyl, substituted or unsubstituted 3-8 membered heterocyclyl, amino, amido, sulfonamido, hydroxyl,

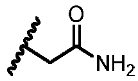,

cyano, carboxyl, formyl, formamido or substituted formamido and C1 -C6 alkylamino;

wherein, the "substituted" refers to being substituted by one or more groups selected from the group consisting of deuterium, halogen, hydroxyl, cyano, carboxyl, amino, amido, sulfonamido, C1-C6 alkylamino, C1-C3 alkyl, C1-C3 alkoxy, C3-C6 cycloalkyl and 3-8 membered heterocyclyl.

In another preferred embodiment, ring B is selected from the following structures:

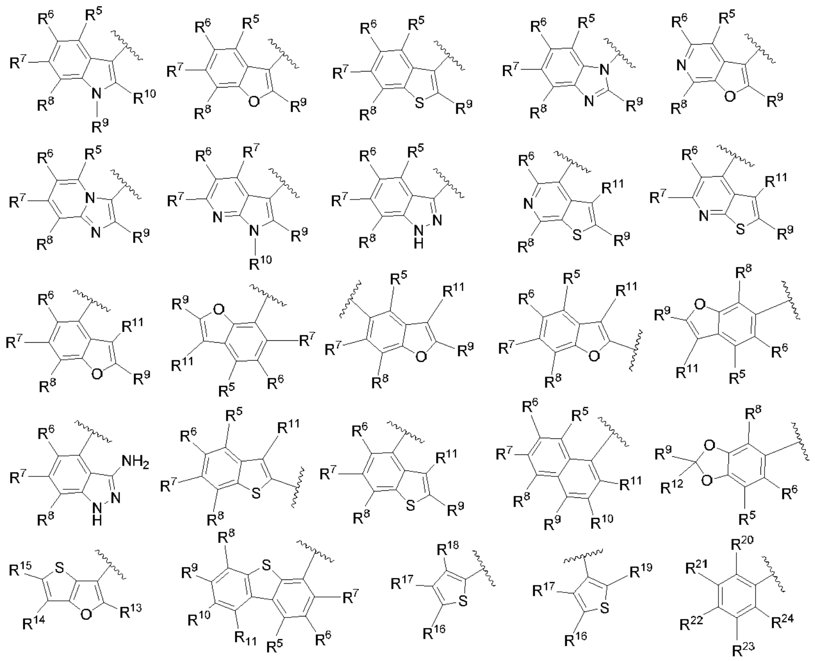

wherein, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$ and $R^{24}$ are each independently selected from the group consisting of hydrogen atom, halogen, deuterium, substituted or unsubstituted C1-C3 alkyl, C2-C3 alkenyl, substituted or unsubstituted C1-C3 alkoxy, substituted or unsubstituted C3-C6 cycloalkyl, amino, hydroxyl,

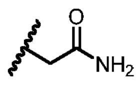,

cyano, carboxyl, formyl, formamido or substituted formamido and C1-C6 alkylamino;

wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

In another preferred embodiment, the prodrug of the compound of formula (I) has the structure shown in formula (**I**"):

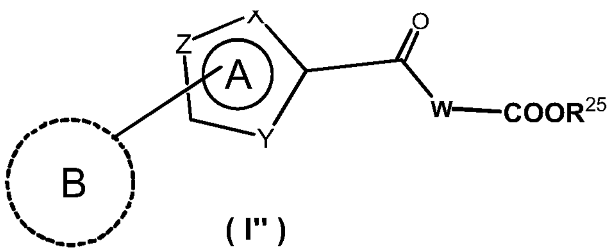

**( I" )**

wherein,

$R^{25}$ is selected from substituted or unsubstituted C1-C6 alkyl, substituted or unsubstituted C2-C6 alkenyl, substituted or unsubstituted C1-C6 alkoxy, substituted or unsubstituted C3-C6 cycloalkyl, substituted or unsubstituted 3-8 membered heterocyclyl, substituted or unsubstituted C6-C12 aryl or substituted or unsubstituted 5-14 membered heteroaryl;

wherein, the "substituted" refers to being substituted by one or more groups selected from the group consisting of deuterium, halogen, hydroxyl, cyano, carboxyl, amino, amido, ester group, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl, substituted or unsubstituted 3-8 membered heterocyclyl, substituted or unsubstituted C6-C12 aryl and substituted or unsubstituted 5-14-membered heteroaryl;

wherein, the substituted in the "substituted" refers to being substituted by one or more groups selected from the group consisting of deuterium, halogen, hydroxyl, cyano, carboxyl, amino, ester group, amido, sulfonamido, C1-C3 alkyl, C1-C3 alkoxy, C3-C6 cycloalkyl and 3-8 membered heterocyclyl;

W, U, ring A, X, Y, Z and ring B are defined as above.

In another preferred embodiment, $R^{25}$ is selected from the corresponding group of the specific compound in the Example.

In another preferred embodiment, the compound of formula (I), or a pharmaceutically acceptable salt, prodrug, hydrate, solvate or crystal form thereof, wherein the compound is selected from the group consisting of

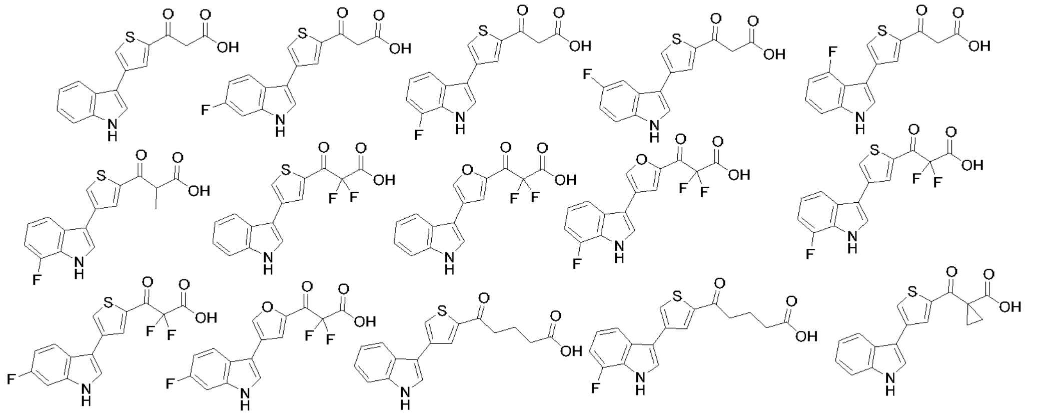

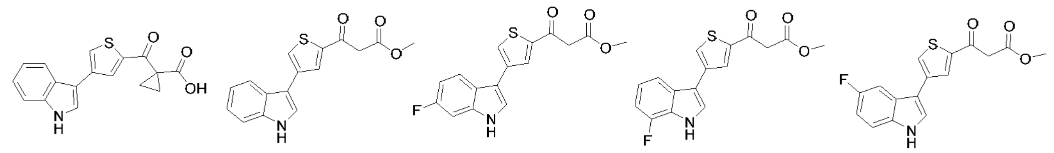

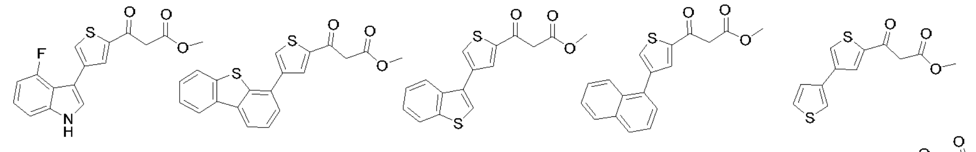

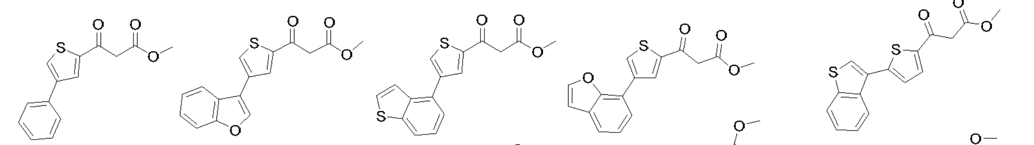

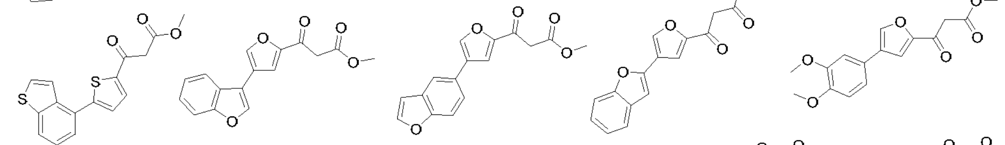

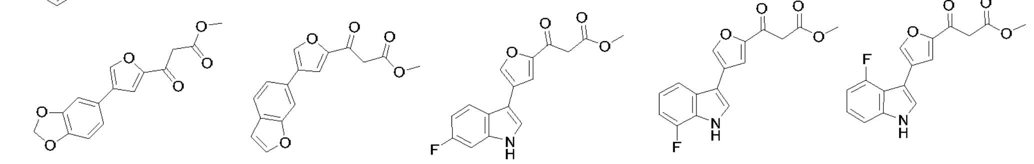

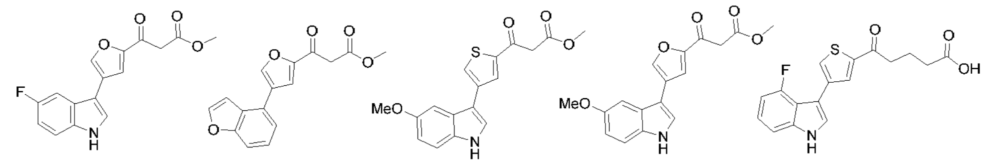

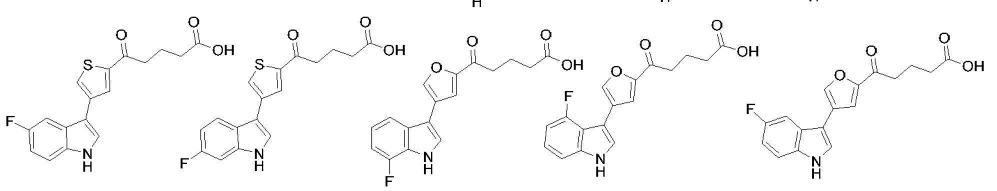

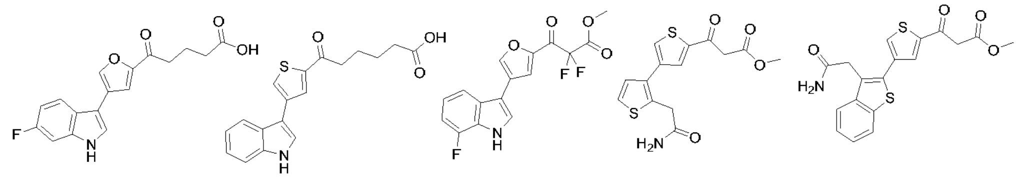

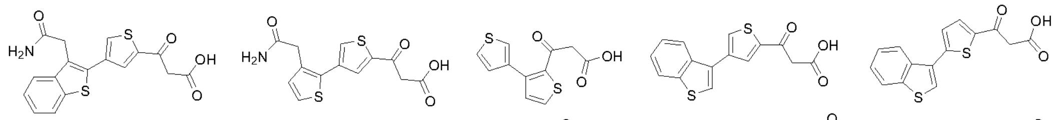

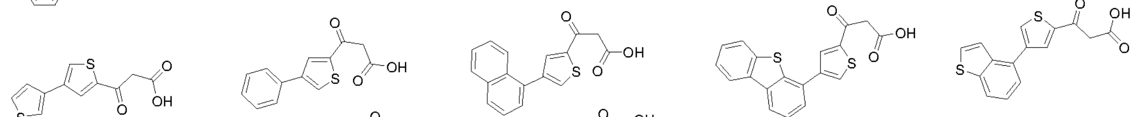

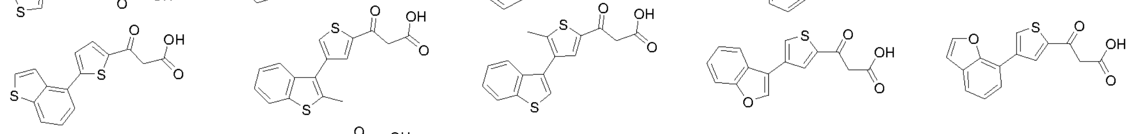

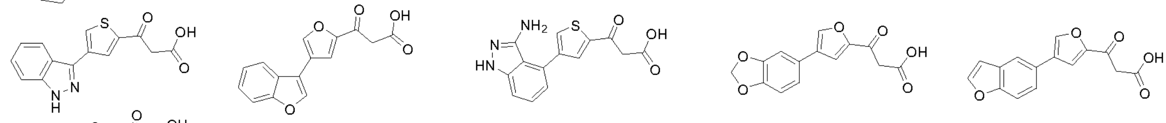

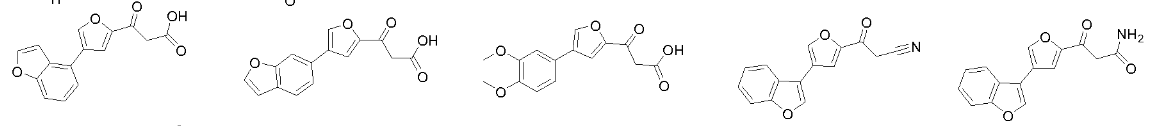

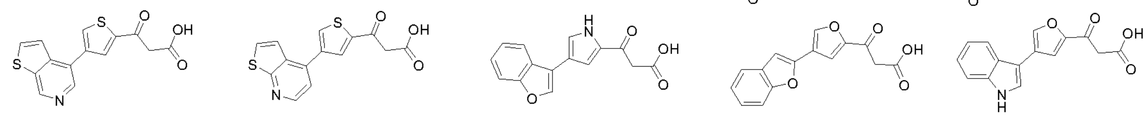

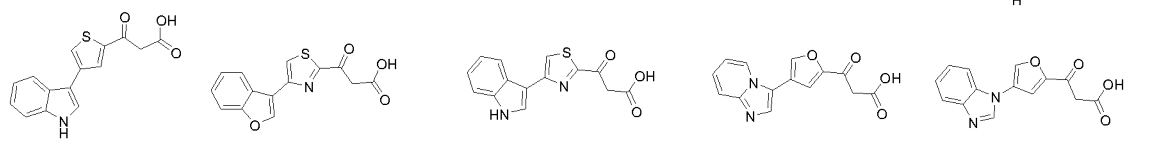

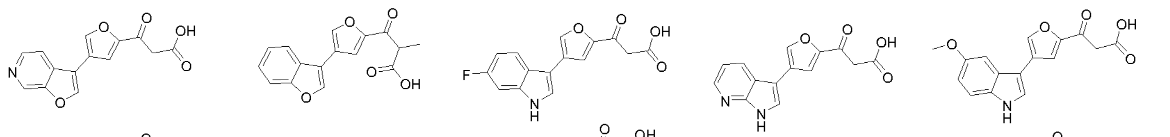

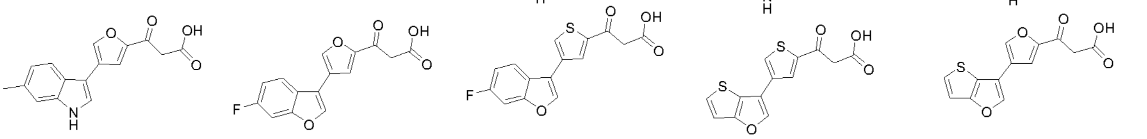

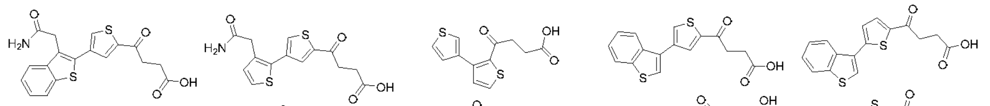

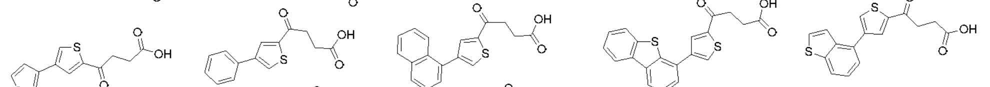

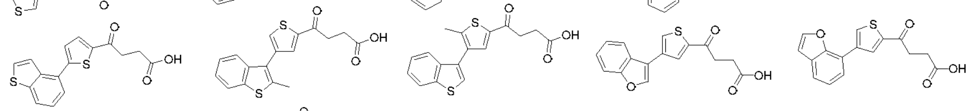

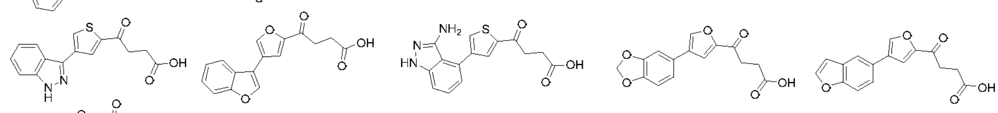

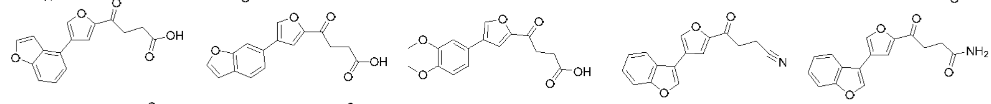

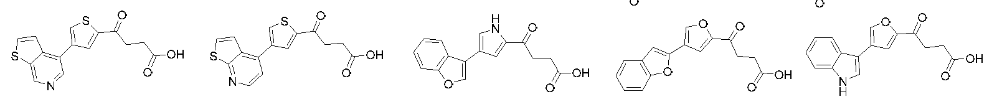

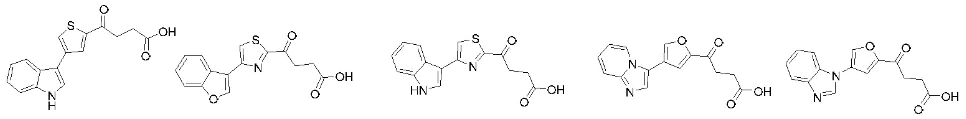

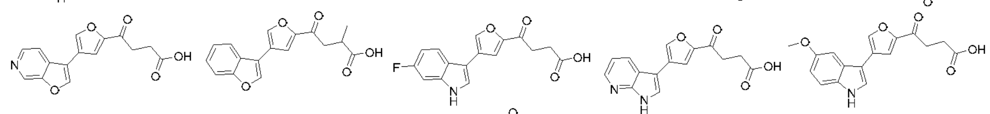

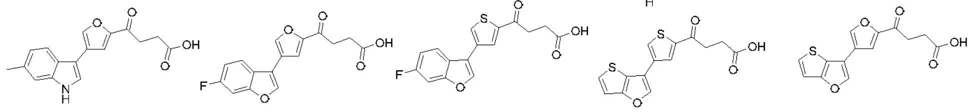

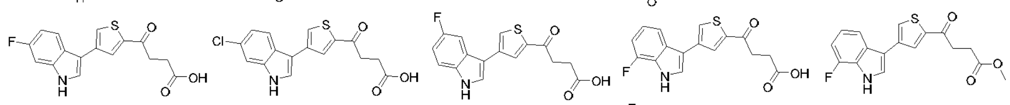

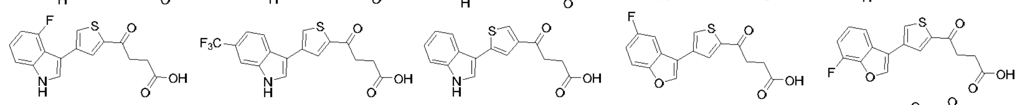

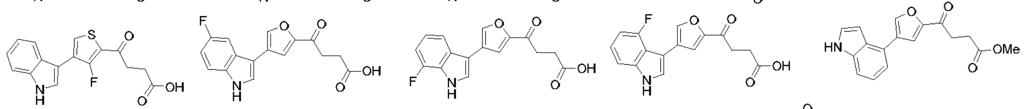

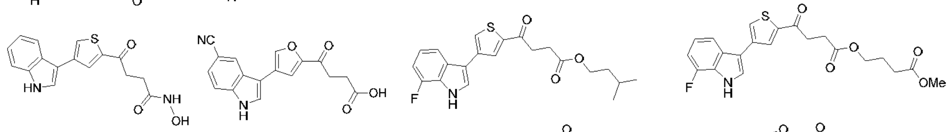

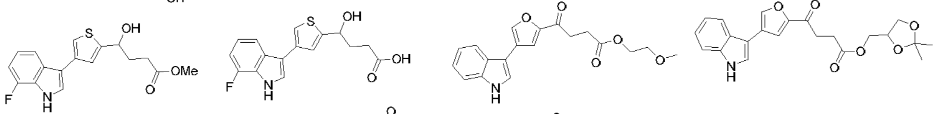

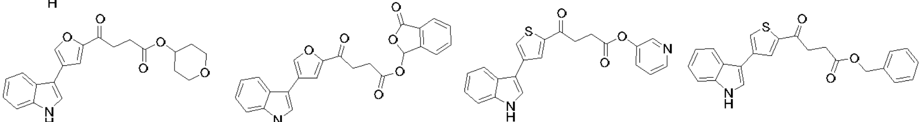

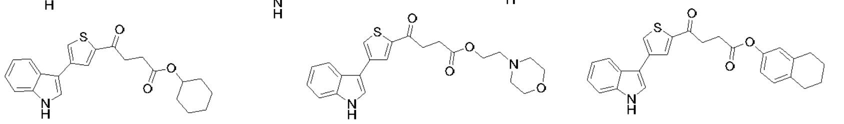

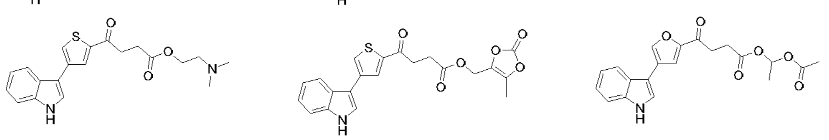

.

In another preferred embodiment, the compound is selected from the compounds shown in Table 1.

In the second aspect of the present invention, it provides a pharmaceutical composition comprising a therapeutically effective amount of one or more of the compound of formula (I), or a pharmaceutically acceptable salt, prodrug, hydrate, solvate or crystal form thereof of the first aspect; and optionally a pharmaceutically acceptable carrier.

In another preferred embodiment, the pharmaceutical composition further comprises:
one or more other active drugs: STING agonists, antiviral compounds, antigens, adjuvants, anticancer agents, CTLA-4, LAG-3 and PD-1 antagonists, lipids, liposomes, peptides, cytotoxic agent, chemotherapeutic agent, immunomodulators, immune checkpoint inhibitors, vascular growth factor (VEGF) receptor inhibitors, topoisomerase II inhibitors, smoothing inhibitors, alkylating agents, antitumor antibiotics, antimetabolites, retinoic acid and immunomodulators (including but not limited to anticancer vaccines).

In another preferred embodiment, a method for preparing a pharmaceutical composition is provided, comprising the steps of mixing a pharmaceutically acceptable carrier with the compound, or a pharmaceutically acceptable salt, prodrug, hydrate, solvate or the crystal form thereof of the first aspect of the present invention, thereby forming the pharmaceutical composition.

In the third aspect of the present invention, it provides a method for preparing the compound of formula (I), or a pharmaceutically acceptable salt, prodrug, solvate or the crystal form thereof of the first aspect,

Method 1:

it comprises the following steps:

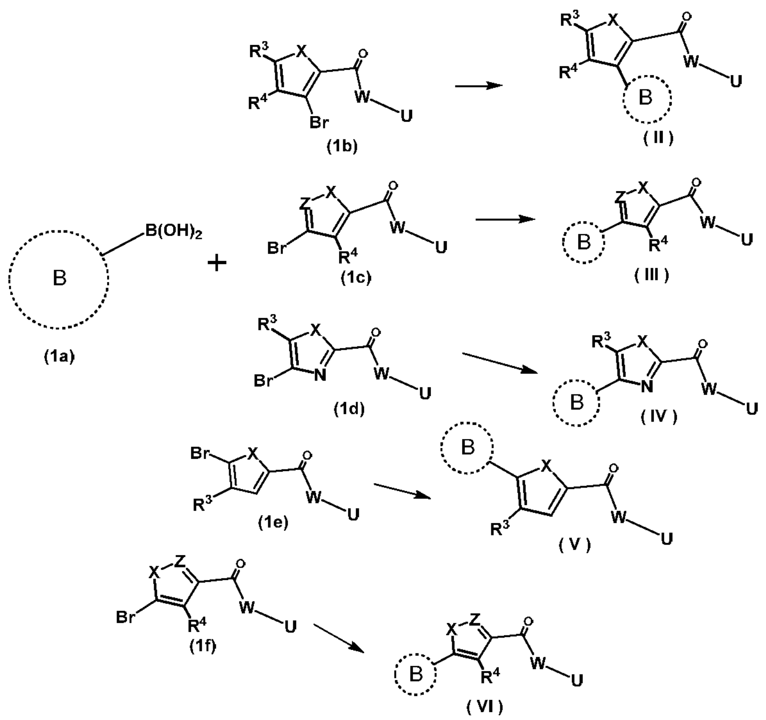

(1a) (1b) (II) (1c) (III) (1d) (IV) (1e) (V) (1f) (VI)

in the presence of a catalyst, a boronic acid intermediate (**1a**) is reacted with an intermediate (**1b**) to obtain a compound of formula (II);

in the presence of a catalyst, a boronic acid intermediate (**1a**) is reacted with an intermediate (**1c**) to obtain a compound of formula (III);
in the presence of a catalyst, a boronic acid intermediate (**1a**) is reacted with an intermediate (**1d**) to obtain a compound of formula (IV);
in the presence of a catalyst, a boronic acid intermediate (**1a**) is reacted with an intermediate (**1e**) to obtain a compound of formula (V);
in the presence of a catalyst, a boronic acid intermediate (**1a**) is reacted with an intermediate (**1f**) to obtain a compound of formula (VI);

**Method 2:**

it comprises the following steps:

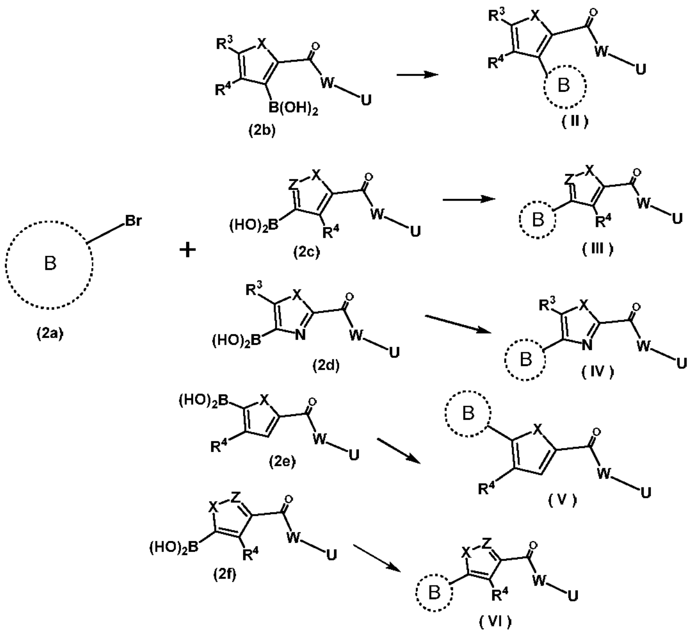

in the presence of a catalyst, the bromine-containing intermediate **(2a)** is reacted with a boronic acid intermediate **(2b)** to obtain a compound of formula (II);

in the presence of a catalyst, the bromine-containing intermediate **(2a)** is reacted with a boronic acid intermediate **(2c)** to obtain a compound of formula (III);
in the presence of a catalyst, the bromine-containing intermediate **(2a)** is reacted with a boronic acid intermediate **(2d)** to obtain a compound of formula (IV);
in the presence of a catalyst, the bromine-containing intermediate **(2a)** is reacted with a boronic acid intermediate **(2e)** to obtain a compound of formula (V);
in the presence of a catalyst, a bromine-containing intermediate **(2a)** is reacted with a boronic acid intermediate **(2f)** to obtain a compound of formula (VI);
wherein, $R^3$, $R^4$, W, X, Z, U, and ring B are defined as above.

In another preferred embodiment, the method for the compound of formula (I), or a pharmaceutically acceptable salt, prodrug, solvate or crystal form thereof,

Method A:

it comprises the following steps:

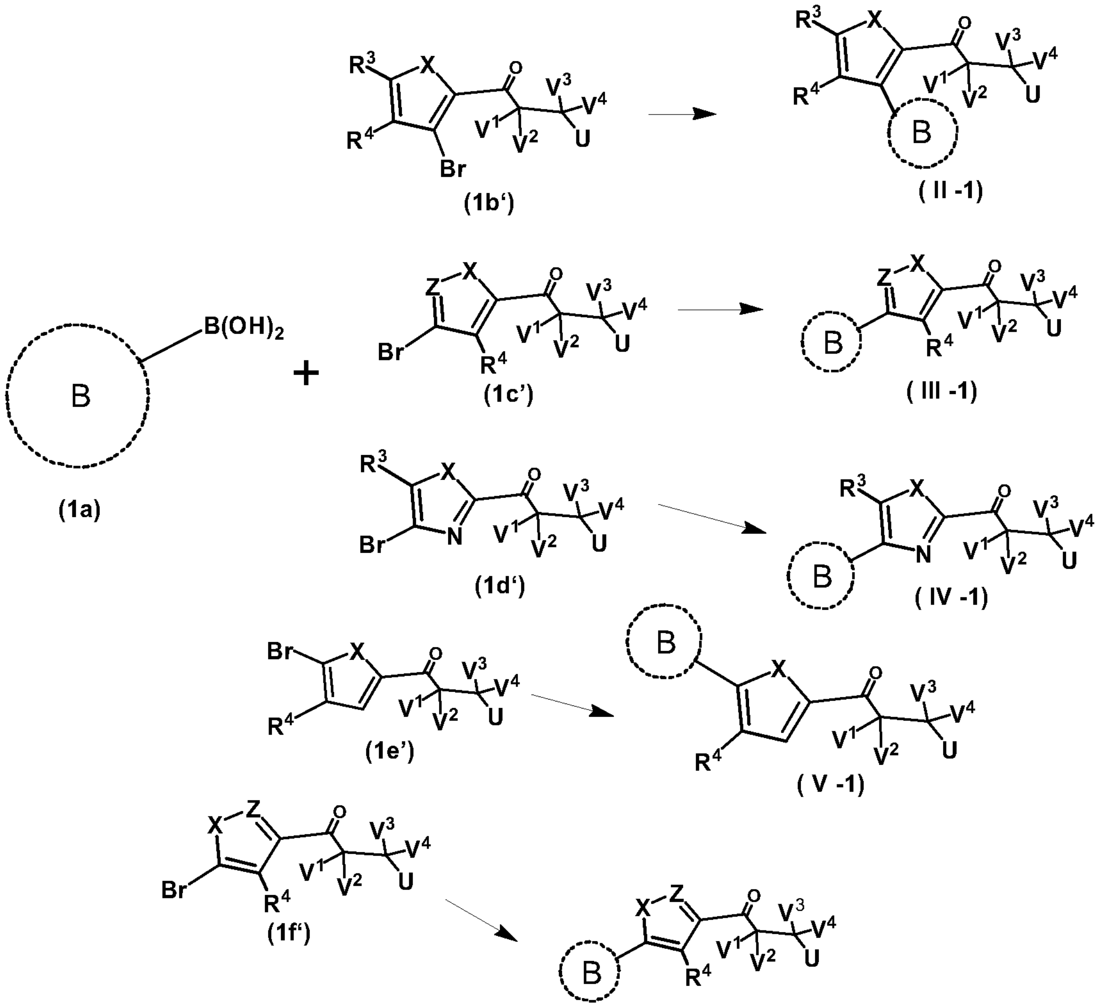

(1b‘) ( II -1)

(1a) (1c’) ( III -1)

(1d‘) ( IV -1)

(1e’) ( V -1)

(1f‘) ( VI-1 )

In the presence of a catalyst, a boronic acid intermediate (**1a**) is reacted with an intermediate (**1b’**) to obtain a compound of formula (II-1);

in the presence of a catalyst, a boronic acid intermediate (**1a**) is reacted with an intermediate (**1c’**) to obtain a compound of formula (III-1);
in the presence of a catalyst, a boronic acid intermediate (**1a**) is reacted with an intermediate (**1d’**) to obtain a compound of formula (IV-1);
in the presence of a catalyst, a boronic acid intermediate (**1a**) is reacted with an intermediate (**1e’**) to obtain a compound of formula (V-1);
in the presence of a catalyst, a boronic acid intermediate (**1a**) is reacted with an intermediate (**1f’**) to obtain a compound of formula (VI-1);

Method B:

it comprises the following steps:

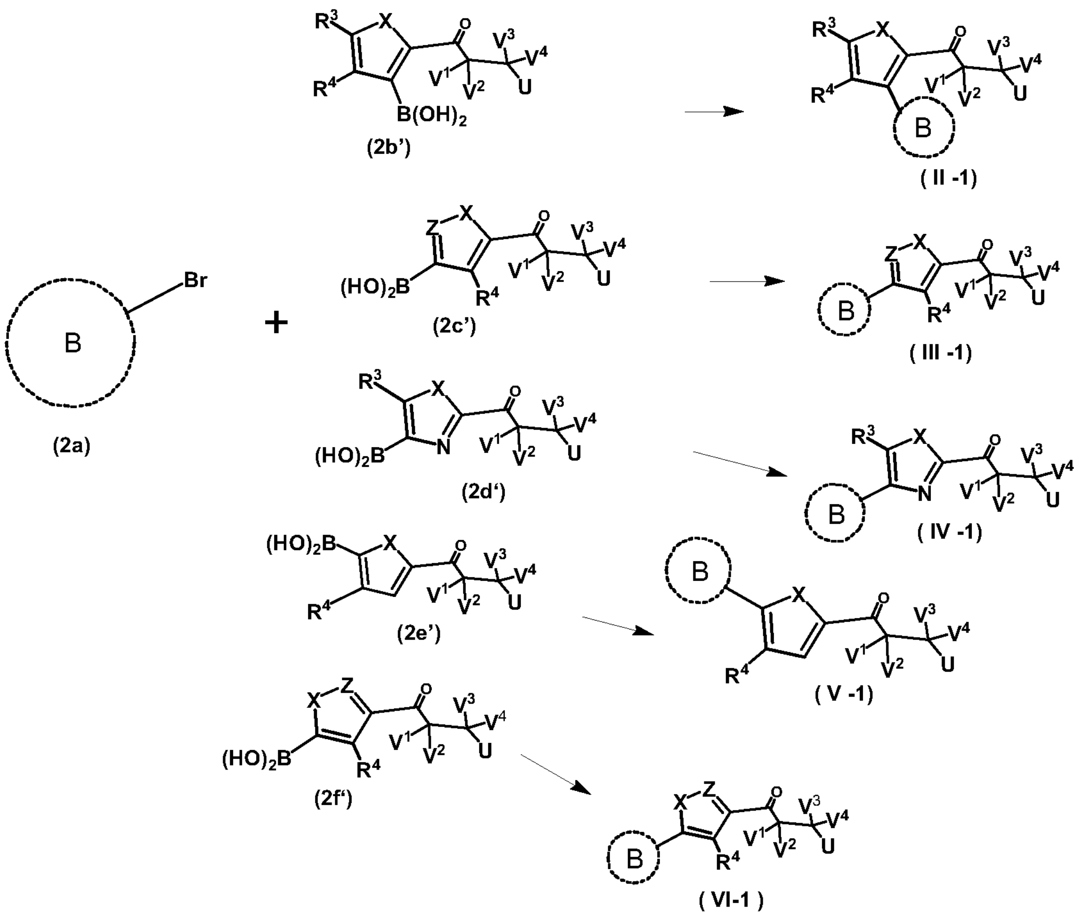

(2a) (2b') (II-1) (2c') (III-1) (2d') (IV-1) (2e') (V-1) (2f') (VI-1)

in the presence of a catalyst, a bromine-containing intermediate **(2a)** is reacted with a boronic acid intermediate **(2b')** to obtain a compound of formula (II-1);

in the presence of a catalyst, the bromine-containing intermediate **(2a)** is reacted with a boronic acid intermediate **(2c')** to obtain a compound of formula (III-1);
in the presence of a catalyst, a bromine-containing intermediate **(2a)** is reacted with a boronic acid intermediate **(2d')** to obtain a compound of formula (IV-1);
In the presence of a catalyst, a bromine-containing intermediate **(2a)** is reacted with a boronic acid intermediate **(2e')** to obtain a compound of formula (V-1);
in the presence of a catalyst, a bromine-containing intermediate **(2a)** is reacted with a boronic acid intermediate **(2f')** to obtain a compound of formula (VI-1);
wherein, $V^1$, $V^2$, $V^3$, $V^4$, $R^3$, $R^4$, X, Z, U and ring B are defined as above.

In another preferred embodiment, the catalyst is a palladium catalyst.
In another preferred embodiment, the catalyst is [1,1'-bis(diphenylphosphino)ferrocene]dichloride palladium, palladium(II)bis(triphenylphosphine) dichloride or tetrakis(triphenylphosphine)palladium.
In the fourth aspect of the present invention, it provides a use of the compound, or a pharmaceutically acceptable salt, prodrug, hydrate, solvate or crystal form thereof of the first aspect, for preparing a medicament, and the medicament is used for a use selected from the group consisting of

1) for the prevention and/or treatment of immune response-related disease;
2) for the prevention and/or treatment of STING activity-related disease;
3) for the prevention and/or treatment of immune response anti-infection-related disease.

In another preferred embodiment, the immune response-related disease and/or STING activity-related disease is tumor or cancer.
In another preferred embodiment, the cancer is selected from colon cancer, breast cancer, lung cancer, melanoma, liver cancer, stomach cancer, cervical cancer, ovarian cancer, fibrosarcoma and squamous cell carcinoma, brain cancer and spine cancer, head and neck cancer, leukemia and blood cancer, skin cancer, genital system cancer, gastrointestinal system cancer, liver and bile duct cancer, kidney cancer and bladder cancer, bone cancer, lung cancer, malignant mesothelioma, sarcoma, lymphoma, adenocarcinoma, thyroid cancer, cardiac tumor, germ cell tumor, malignant neuroendocrine (carcinoid) tumor, midline beam cancer, and unknown primary cancer (i.e., metastatic cancer found but the primary cancer origin is unknown)

In another preferred embodiment, the immune response anti-infection-related disease is bacterial or viral infection, especially hepatitis B virus (HBV), hepatitis C virus (HCV), human papilloma virus (HPV), nasopharyngeal cancer-related EB virus (EBV) and human immunodeficiency virus (HIV) infection-related disease.

In another preferred embodiment, the STING activity-related disease includes inflammatory disease. The inflammatory disease is selected from acne vulgaris, asthma, celiaca, chronic prostatitis, glomerulonephritis, inflammatory bowel disease, pelvic inflammatory disease, reperfusion injury, rheumatoid arthritis, sarcoidosis, vasculitis, airway inflammation caused by house dust mites and interstitial cystitis.

In the fifth aspect of the present invention, it provides a method for inducing an immune response comprising administering to a subject a therapeutically effective amount of the compound, or a pharmaceutically acceptable salt, prodrug, hydrate, solvate, or the crystal form thereof of the first aspect or the composition of the second aspect.

In another preferred embodiment, the inducing immune response is injecting the therapeutically effective amount of the compound or a pharmaceutically acceptable salt or the pharmaceutical composition of the present invention into the subject.

In the sixth aspect of the present invention, it provides a method for inducing a subject to produce STING-dependent type I interferon comprising administering to the subject a therapeutically effective amount of the compound, or the pharmaceutically acceptable salt, prodrug, hydrate, solvate, or the crystal form thereof of the first aspect or a pharmaceutical composition of the second aspect.

In the seventh aspect of the present invention, it provides a method for treating malignant cell proliferation comprising administering to a subject a therapeutically effective amount of the compound of the first aspect, the pharmaceutical composition, a pharmaceutically acceptable salt or the solvate thereof.

In another preferred embodiment, the malignant cell proliferation disease is cancer.

In another preferred embodiment, the compound of the present invention can be prepared into powder, tablet, granule, capsule, solution, emulsion, suspension and the like.

It should be understood that in the present invention, any of the technical features specifically described above and below (such as in the Example) can be combined with each other, thereby constituting new or preferred technical solutions. Limited by space, it will not be repeated here.

## DESCRIPTION OF FIGURES

Figure 1 shows the in vivo antitumor activity of compound I'-31 in the 4T1 breast cancer model.
Figure 2 shows the in vivo antitumor activity of compound I'-31 in the CT26 colon cancer model.

## DETAILED DESCRIPTION OF THE INVENTION

After long-term and in-depth research, the inventors have unexpectedly developed a five-membered heterocyclic oxocarboxylic acid compound with novel structure and easy synthesis, which has the activity of inducing the secretion of type I interferon, and excellent activating properties on STING, and can significantly promote the expression of interferon factor IFN-β and activate antigen-presenting cells (APC), thereby activating T cells to produce anti-tumor immune responses, which can be used for anti-tumor and anti-infective diseases. It is of great significance for the development of new antiviral drugs and antitumor drugs. On this basis, the inventor has completed the present invention.

## TERMS

In the present invention, unless otherwise specified, the terms used have the general meanings known to those skilled in the art.

When a substituent is described by a conventional chemical formula written from left to right, the substituent also includes a chemically equivalent substituent obtained by writing a structural formula from right to left. For example, $-CH_2O-$ is equivalent to $-OCH_2-$.

In the present invention, the term "halogen" refers to F, Cl, Br or I.

The term "alkyl" by itself or as part of another substituent refers to a straight or branched chain hydrocarbon

group having the specified number of carbon atoms, "C1-C6 alkyl" refers to straight or branched chain alkyl containing 1-6 (for example, containing 1, 2, 3, 4, 5 or 6) carbon atoms, preferably C1-C3 alkyl, such as methyl, ethyl, propyl, isopropyl, butyl, isobutyl , tert-butyl, neopentyl, tert-amyl, or similar groups. In the present invention, the alkyl is intended to include substituted alkyl.

In the present invention, the term "C3-C8 cycloalkyl" refers to a cyclic alkyl having 3 to 8 (for example, containing 3, 4, 5, 6, 7 or 8) carbon atoms in the ring, preferably C3 -C6 cycloalkyl, which includes but not limited to cyclopropyl, cyclobutyl, cyclopentyl, cyclohexyl, cycloheptyl, cyclooctyl, etc. In the present invention, cycloalkyl is intended to include substituted cycloalkyl.

The term "alkenyl" refers to a straight or branched chain hydrocarbon group containing one or more double bonds and usually 2 to 20 carbon atoms (or C2-C8) in length. For example, in the present invention, "C2-C6 alkenyl" contains two to six carbon atoms. The alkenyl includes but is not limited to for example, vinyl, propenyl, butenyl, 1-methyl-2-buten-1-yl, etc.

In the present invention, the term "C1-C6 alkoxy" refers to a straight or branched chain alkoxy or cyclic alkoxy (such as C3-C6 cycloalkoxy) having 1 to 6 carbon atoms with a structure of C1-C6 alkyl-O- or -C1-C5 alkyl-O-C1-C5 alkyl including, but not limited to, methoxy, ethoxy, propoxy, isopropoxy and butoxy, etc. Preferably C1-C3 alkoxy.

In the present invention, the term "C1-C6 alkylamino" may be mono- or di-substituted, has the following structure: -NH-C1-C6 alkyl or -N-(C1-C6 alkyl)$_2$, wherein, alkyl is defined as above, representative examples include but are not limited to methylamino, ethylamino, propylamino, isopropylamino, butylamino, isobutylamino, sec-butylamino, tert-butylamino, di- methylamino, diethylamino, dipropylamino, diisopropylamino, dibutylamino, diisobutylamino, di-sec-butylamino, di-tert-butylamino, etc.

In the present invention, the term "3-8 membered heterocyclyl" is a 3-8 membered saturated or unsaturated heterocyclyl containing 1, 2 or 3 heteroatoms selected from N, O, S or Se, wherein, the ring system of each heterocyclyl may be monocyclic or polycyclic, including (but not limited to) the following groups: tetrahydrofuranyl, tetrahydropyranyl, tetrahydropyrrolyl, tetrahydrothienyl, piperidyl, azetidinyl, azepanyl, morpholinyl and the like. The heterocyclyl can be fused to aryl, heteroaryl, heterocyclyl or cycloalkyl (e.g., forming [6+5], [6+6] fused ring systems, etc.), and the ring attached to the parent structure is heterocyclyl.

In the present invention, the term "C6-C14 aryl" refers to an aromatic ring group with 6 to 14 carbon atoms that does not contain heteroatoms on the ring, and the aryl can be fused to a heteroaryl, heterocyclyl or cycloalkyl, wherein the ring attached to the parent structure is aryl. Such as phenyl (i.e., six-membered aromatic ring), naphthyl (i.e., [6+6] aromatic ring), etc., wherein the six-membered aromatic ring is also intended to include six-membered aromatic ring fused 5-6 membered cycloalkyl and six-membered aromatic ring fused 5-6 membered heterocycloalkyl. 6 to 10 carbon atoms are preferred. Aryl can be optionally substituted or unsubstituted.

In the present invention, the term "5-14 membered heteroaryl" refers to a heteroaromatic group containing 1, 2 or 3 heteroatoms selected from N, O, S or Se, and the ring system of the heteroaryl may be monocyclic or polycyclic (including fused ring forms). Non-limiting examples include pyrrolyl, pyrazolyl, imidazolyl, oxazolyl, oxadiazolyl, isoxazolyl, thiazolyl, thiadiazolyl, isothiazolyl, furyl, pyridyl, pyrazinyl, pyrimidinyl, pyridazinyl, triazinyl, triazolyl, purinyl, carbazolyl, indolyl, indazolyl, benzothienyl, benzofuranyl, benzimidazolyl, benzotriazolyl, benzothiazolyl, benzothiadiazolyl, benzoxazolyl, isomerized quinolinyl, phthalazinyl, quinoxalinyl, quinazolinyl, cinnolinyl or naphthyridinyl and tetrazolyl, etc.. The heteroaryl ring may be fused to aryl, heterocyclyl or cycloalkyl, and the ring attached to the parent structure is heteroaryl. The term "[6+5+6]aromatic heterocyclic ring" refers to a fused 6, 5 and 6 tricyclic ring system, such as dibenzo[b,d]thiophene, and the term "[6+5]aromatic heterocyclic ring" refers to a fused 6 and 5 bicyclic ring system, such as benzothienyl, benzofuranyl, benzimidazolyl, benzotriazolyl, benzothiazolyl, benzothiadiazolyl, benzoxazolyl, "[6+6] aromatic heterocyclic ring", "[5+5] aromatic heterocyclic ring or fused ring" have similar meanings. Heteroaryl can be optionally substituted or unsubstituted. When being substituted, the substituents are preferably one or more of the following groups independently selected from alkyl, deuterated alkyl, haloalkyl, alkoxy, haloalkoxy, alkenyl, alkynyl, alkylthio , alkylamino, halogen, amino, nitro, hydroxyl, mercapto, cyano, cycloalkyl, heterocyclyl, aryl, heteroaryl, cycloalkylthio, oxo, amido, sulfonamido, formyl, formamido, carboxyl and carboxylate, etc. In the present invention, among the substituents, "heteroaromatic ring", "aromatic heterocyclic ring" and "heteroaryl" have the same meaning.

In the present invention, the term "ester group" refers to a group with the structure -COOR, wherein R can independently represent hydrogen, alkyl or substituted alkyl, cycloalkyl or substituted cycloalkyl, cycloalkenyl or substituted cycloalkenyl, aryl or substituted aryl, heterocycle or substituted heterocycle, as defined above. In the present invention, the term "amido" refers to a group with the structure -CONRR', wherein R and R' can independently represent hydrogen, alkyl or substituted alkyl, cycloalkyl or substituted cycloalkyl, cycloalkenyl or substituted cycloalkenyl, aryl or substituted aryl, heterocycle or substituted heterocycle, as defined above. R and R' may be the same or different in the dialkylamino segment.

In the present invention, the term "sulfonamido" refers to a group with the structure -$SO_2$NRR', where R and R' can independently represent hydrogen, alkyl or substituted alkyl, cycloalkyl or substituted cycloalkyl, cycloalkenyl or substituted cycloalkenyl, aryl or substituted aryl, heterocycle or substituted heterocycle, as defined above. R and R' may

be the same or different in the dialkylamino segment.

In the present invention, the term "formyl" refers to a group comprising -CHO.

In the present invention, the term "formamido" refers to a group comprising

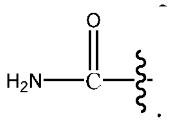

In the present invention, the term "substituted formamido" refers to a group comprising

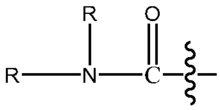,

wherein R each independently represents hydrogen, alkyl or substituted alkyl, cycloalkyl or substituted cycloalkyl, cycloalkenyl or substituted cycloalkenyl, aryl or substituted aryl, heteroaryl, heterocycle or substituted heterocycle, as defined above. R can be the same or different.

In the present invention, the term "substituted" refers to one or more hydrogen atoms on a specific group being substituted by specific substituent. The specific substituents are those described in the preceding paragraph or those present in each Example. Unless otherwise specified, a substituted group may have a substituent selected from a specific group at any substitutable position of the group, and the substituent may be the same or different in each position. Those skilled in the art should understand that the combinations of substituents contemplated by the present invention are those that are stable or chemically achievable.

"The substituted in the "substituted"" means that the substituent of a group is further substituted. In the present application, "Substituent of the substituent" refers to that C1-C6 alkyl, formamido, C1-C6 alkoxy, C3-C8 cycloalkyl or 3-8-membered heterocyclyl are further substituted.

In the present invention, the method is non-diagnostic and/or non-therapeutic.

"Immune response-related disease" refers to a disease in which an immune response is generated during the occurrence and development of the disease. The immune response plays an important role in the development, outcome or recovery of the disease. The immune response includes the innate immune response and the adaptive immune response, involving immune cells and antibodies and complement and other immune system, such as dendritic cells, mononuclear macrophages, T lymphocytes, B lymphocytes, natural killer cells, etc. The specific types of immune response-related diseases mainly include tumors, pathogenic microorganism infectious diseases, autoimmune diseases, neuropsychiatric diseases, and acute or chronic inflammatory diseases.

In the present invention, the term "multiple" independently refers to 2, 3, 4 or 5.

**ACTIVE INGREDIENT**

As used herein, the terms "compounds of the invention" or "active ingredient of the invention" are used interchangeably and refer to compounds of formula (I), or a pharmaceutically acceptable salt, hydrate, solvate, isotope compound (e.g. deuterated compound) or prodrug thereof. The term also includes racemate and optical isomer.

The compound of formula I in the present invention has the following structure:

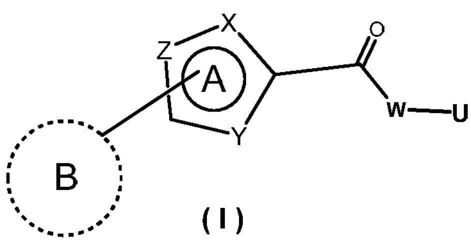

(I)

wherein,

W is selected from the group consisting of substituted or unsubstituted C3-C8 cycloalkyl, substituted or unsubstituted 3-8 membered heterocyclyl,

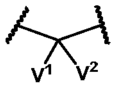, 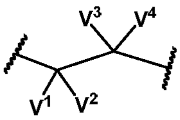,

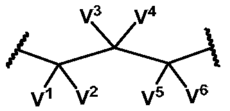 and 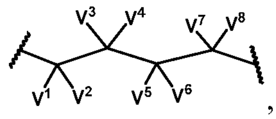,

wherein, $V^1$, $V^2$, $V^3$, $V^4$, $V^5$, $V^6$, $V^7$, $V^8$, U, ring A, ring B, X, Y, and Z are defined as above.

Preferably, the compound of formula I has a structure represented by formula (**I**'):

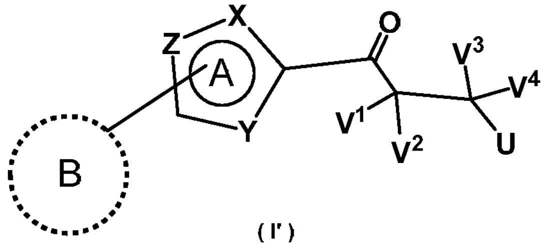

(I')

wherein, $V^1$, $V^2$, $V^3$ and $V^4$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is independently selected from the group consisting of $COOR^m$. COOH, CN, $CONH_2$ and

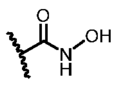,

wherein, $R^m$ is selected from substituted or unsubstituted C1-C6 alkyl, substituted or unsubstituted C3-C8 cycloalkyl and substituted or unsubstituted 3-8 membered heterocyclyl;

ring A is a five-membered heteroaromatic ring, wherein X, Y, Z are each independently selected from CH, C-$R^n$, NH, N, O or S, wherein, $R^n$ is selected from the group consisting of halogen, deuterium, hydroxyl, amino, cyano, carboxyl, formyl, substituted or unsubstituted formamido, substituted or unsubstituted C1-C6 alkyl, substituted or unsubstituted C1-C6 alkoxy, substituted or unsubstituted C3-C8 cycloalkyl, and substituted or unsubstituted 3-8 membered heterocyclyl;

ring B is independently selected from the group consisting of substituted or unsubstituted 5-14 membered heteroaromatic ring and substituted or unsubstituted C6-C14 aromatic ring;

wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

Preferably, in the above formulas, ring A is selected from substituted or unsubstituted thienyl, furanyl, thiazolyl or pyrrolyl; wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl; wherein, the substituted in the "substituted" refers to being substituted by one or more groups selected from the group

consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;

Preferably, in the above formulas, ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;

wherein, the "substituted" refers to being substituted by one or more groups selected from the group consisting of deuterium, halogen, hydroxyl, cyano, carboxyl, amino,

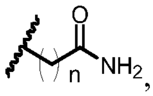,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl; wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

Preferably, the compound of formula I has the structure shown in formula (II), (III), (IV), (V) or (VI):

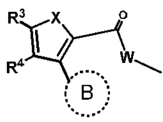
(II) ,
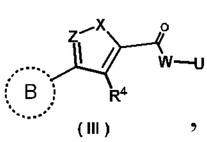
(III) ,
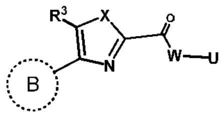
(IV) ,
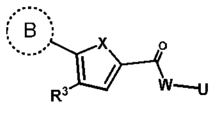
(V) or
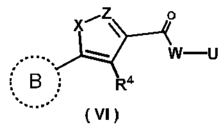
(VI)

wherein, $R^3$, $R^4$, U, ring B, W, X, and Z are defined as above.

Preferably, the compound of formula I has the structure shown in formula (**II-1**), (**III-1**), (**IV-1**), (**V-1**) or (**VI-1**):

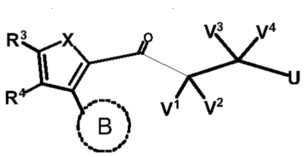
(II-1) ,
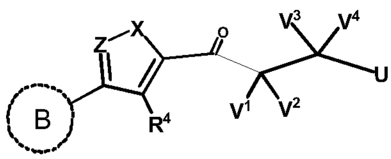
(III-1) ,
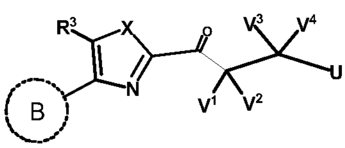
(IV-1) ,
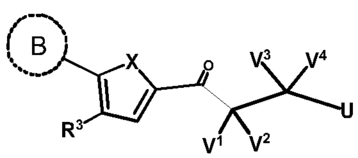
(V-1) or
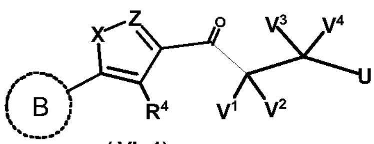
(VI-1) ,

wherein, X is selected form O, S or NH.

Z is selected from N or C-$R^3$;

$V^1$, $V^2$, $V^3$ and $V^4$ are each independently selected from hydrogen atom, halogen, C1-C3 alkyl, C1-C3 alkoxy or C3-C6 cycloalkyl;

U is selected from $COOR^m$, COOH, CN, $CONH_2$ or

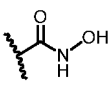,

wherein, $R^m$ is selected from substituted or unsubstituted C1-C6 alkyl or substituted or unsubstituted C3-C6 cycloalkyl;

$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C6 cycloalkyl and substituted or unsubstituted 3-8-membered heterocyclyl;

ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;

wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;

wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

Preferably, in the above formulas, ring B is selected from:

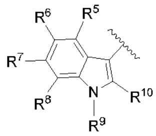
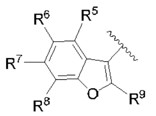
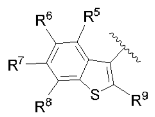
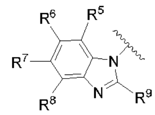
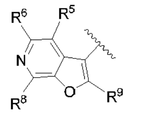
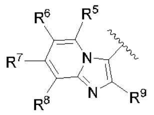
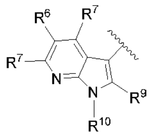
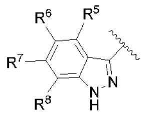
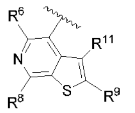
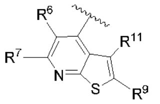
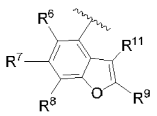
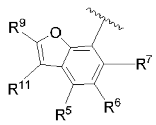
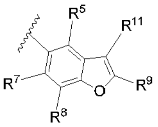
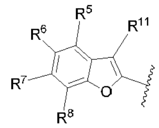
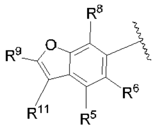
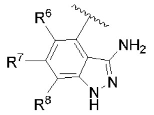
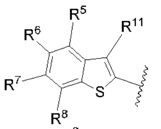
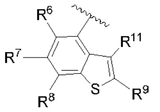
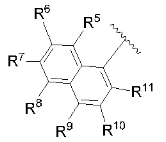
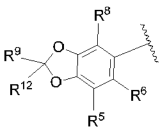
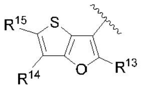
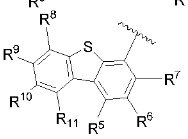
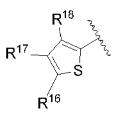
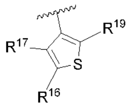
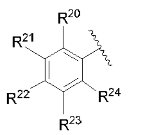

wherein, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$ and $R^{24}$ are each independently selected from the group consisting of hydrogen atom, halogen, deuterium, substituted and unsubstituted C1-C6 alkyl, C2-C6 alkenyl, substituted and unsubstituted C1-C6 alkoxy, substituted and unsubstituted C3-C6 cycloalkyl, substituted or unsubstituted 3-8 membered heterocyclyl, amino, amido, sulfonamido, hydroxyl,

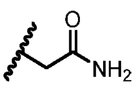,

cyano, carboxyl, formyl, formamido or substituted formamido and C1 -C6 alkylamino; preferably selected from hydrogen atom, halogen, deuterium, substituted or unsubstituted C1-C3 alkyl, C2-C3 alkenyl, substituted or unsubstituted C1-C3 alkoxy, substituted or unsubstituted C3-C6 cycloalkyl, amino, hydroxyl,

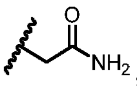

, cyano, carboxyl, formyl, formamido or substituted formamido and C1-C6 alkylamino;

wherein, the "substituted" refers to being substituted by one or more groups selected from the group consisting of deuterium, halogen, hydroxyl, cyano, carboxyl, amino, amido, sulfonamido, C1-C6 alkylamino, C1-C3 alkyl, C1-C3 alkoxy, C3-C6 cycloalkyl and 3-8 membered heterocyclyl; preferably, the compound of formula I or the prodrug of the compound of formula I is selected from the compounds listed in Table 1.

Table 1

| | | |
|---|---|---|
| **I-1** | 3-(4-(1H-indol-3-yl)thiophen-2-yl)- 3-oxopropionic acid | 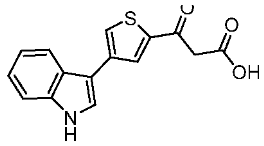 |
| **I-2** | 3-(4-(6-fluoro-1H-indol-3 -yl)thiophen-2-yl)-3 -oxopropi onic acid | 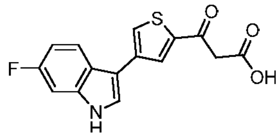 |
| **I-3** | 3-(4-(7-fluoro-1H-indol-3 -yl)thiophen-2-yl)-3 -oxopropi onic acid | 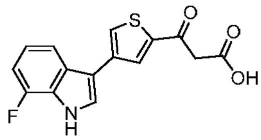 |
| **I-4** | 3-(4-(5-fluoro-1H-indol-3 -yl)thiophen-2-yl)-3 -oxopropi onic acid | 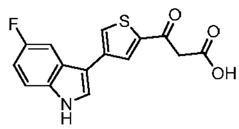 |
| **I-5** | 3-(4-(4-fluoro-1H-indol-3 -yl)thiophen-2-yl)-3 -oxopropi onic acid | 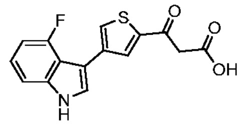 |
| **I-6** | 2-methyl-3-(4-(7-fluoro-1H-indol-3-yl)thiophen-2-yl)-3-oxopropionic acid | 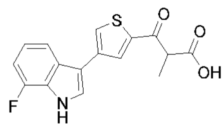 |
| **I-7** | 2,2-difluoro-3-(4-(1 H-indol-3 -yl)thiophen-2-yl)-3 -oxopr opionic acid | 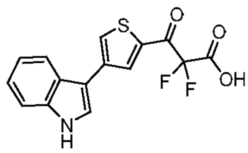 |
| **I-8** | 2,2-difluoro-3-(4-(1H-indol-3-yl)furan-2-yl)-3-oxopropi onic acid | 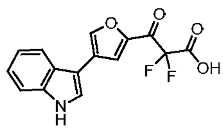 |
| **I-9** | 2,2-difluoro-3-(4-(7-fluoro-1H-indol-3-yl)furan-2-yl)-3-oxopropionic acid | 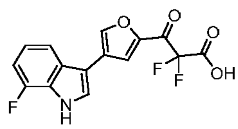 |
| **I-10** | 2,2-difluoro-3-(4-(7-fluoro-1H-indol-3-yl)thiophen-2-yl) -3-oxopropionic acid | 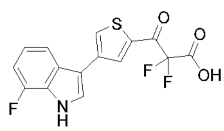 |

(continued)

| | | |
|---|---|---|
| **I-11** | 2,2-difluoro-3-(4-(6-fluoro-1H-indol-3-yl)thiophen-2-yl) -3-oxopropionic acid | 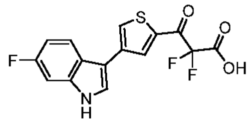 |
| **I-12** | 2,2-difluoro-3-(4-(6-fluoro-1H-indol-3-yl)furan-2-yl)-3-oxopropionic acid | 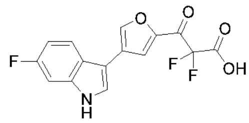 |
| **I-13** | 5-(4-(1H-indol-3-yl)thiophen-2-yl)-5-oxopentanoic acid | 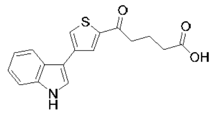 |
| **I-14** | 5-(4-(7-fluoro-1H-indol-3 -yl)thiophen-2-yl)-5 -oxopenta noic acid | 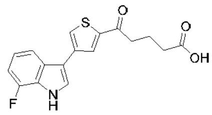 |
| **I-15** | 1-(4-(1H-indol-3-yl)thiophen-2-carbonyl)cyclopropyl-1-carboxylic acid | 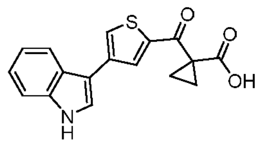 |
| **I-16** | Methyl 3-(4-(1H-indol-3 -yl)thiophen-2-yl)-3 -oxopropanoate | 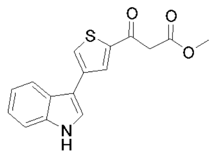 |
| **I-17** | Methyl 3-(4-(6-fluoro-1H-indol-3-yl)thiophen-2-yl)-3 -oxopropa noate | 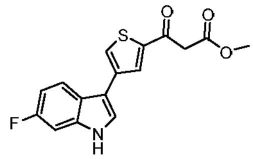 |
| **I-18** | Methyl 3-(4-(7-fluoro-1H-indol-3-yl)thiophen-2-yl)-3-oxopropa noate | 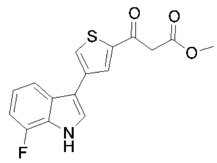 |
| **I-19** | Methyl 3-(4-(5-fluoro-1H-indol-3-yl)thiophen-2-yl)-3-oxopropa noate | 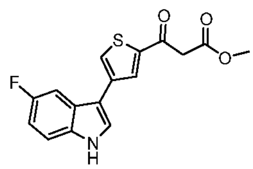 |
| **I-20** | Methyl 3-(4-(4-fluoro-1H-indol-3-yl)thiophen-2-yl)-3-oxopropa noate | 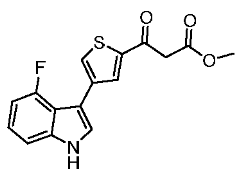 |

(continued)

| | | |
|---|---|---|
| **I-21** | Methyl 3-(4-(dibenzo[b,d]thiophen-4-yl)thiophen-2-yl)-3-oxopr opanoate | 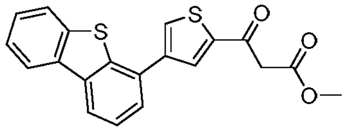 |
| **I-22** | Methyl 3-(4-(benzo [b]thiophen-3-yl)thiophen-2-yl)-3-oxopropa noate | 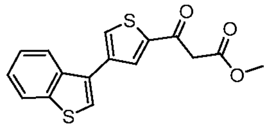 |
| **I-23** | Methyl 3-(4-(naphthalen-1-yl)thiophen-2-yl)-3-oxopropanoate | 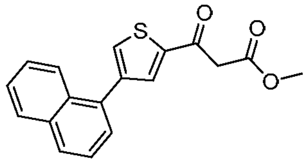 |
| **I-24** | Methyl 3-([3,3'-bithiophen]-5-yl)-3-oxopropanoate | 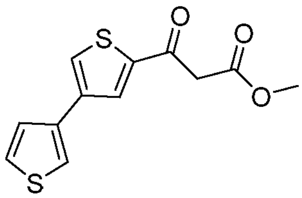 |
| **I-25** | Methyl (4-phenylthiophen-2-yl)-3-oxopropanoate | 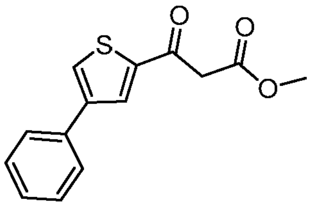 |
| **I-26** | Methyl 3-(4-(benzofuran-3-yl)thiophen-2-yl)-3-oxopropanoate | 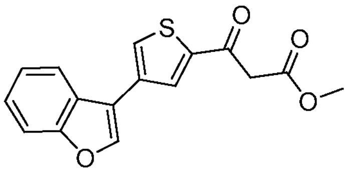 |
| **I-27** | 3-(4-(benzo[b]thiophen-4-yl)thiophen-2-yl)-3-oxopropio nic acid | 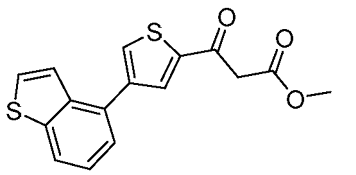 |
| **I-28** | Methyl 3-(4-(benzofuran-7-yl)thiophen-2-yl)-3-oxopropanoate | 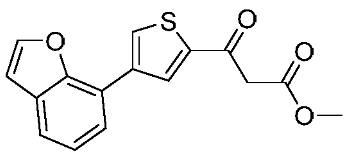 |

(continued)

| | | |
|---|---|---|
| **I-29** | Methyl 3 -(5 -(benzo [b]thiophen-3 -yl)thiophen-2-yl)-3 -oxopropa noate | 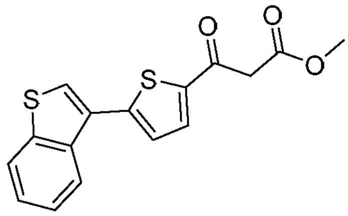 |
| **I-30** | Methyl 3 -(5 -(benzo [b]thiophen-4-yl)thiophen-2-yl)-3 -oxopropa noate | 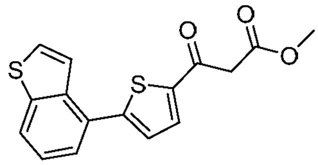 |
| **I-31** | Methyl 3-(4-(benzofuran-3-yl)furan-2-yl)-3-oxopropanoate | 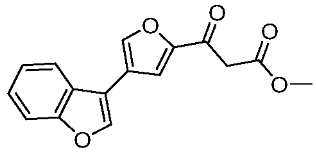 |
| **I-32** | Methyl 3-(4-(benzofuran-5-yl)furan-2-yl)-3-oxopropanoate | 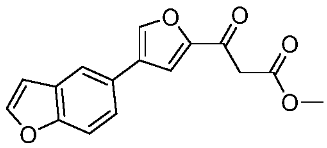 |
| **I-33** | Methyl 3-(4-(benzofuran-2-yl)furan-2-yl)-3-oxopropanoate | 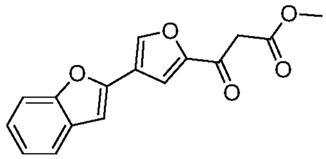 |
| **I-34** | Methyl 3-(4-(3,4-dimethoxyphenyl)furan-2-yl)-3-oxopropanoate | 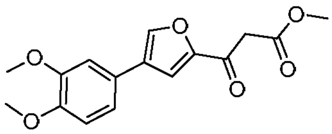 |
| **I-35** | Methyl 3-(4-(benzo[d][1,3]dioxolan-5-yl)furan-2-yl)-3-oxoprop anoate | 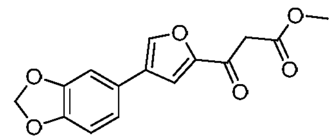 |
| **I-36** | Methyl 3-(4-(benzofuran-6-yl)furan-2-yl)-3-oxopropanoate | 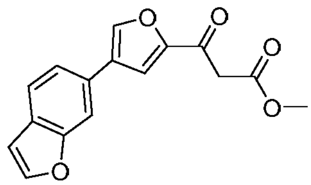 |
| **I-37** | Methyl 3-(4-(6-fluoro-1H-indol-3-yl)furan-2-yl)-3-oxopropanoa te | 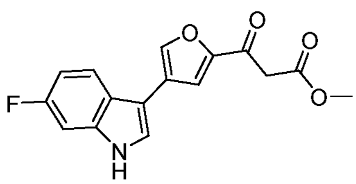 |

(continued)

| | | |
|---|---|---|
| **I-38** | Methyl 3-(4-(7-fluoro-1H-indol-3-yl)furan-2-yl)-3-oxopropanoa te | 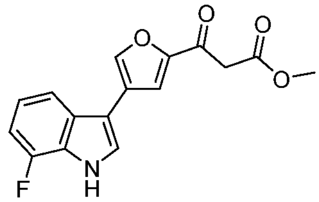 |
| **I-39** | Methyl 3-(4-(4-fluoro-1H-indol-3-yl)furan-2-yl)-3-oxopropanoa te | 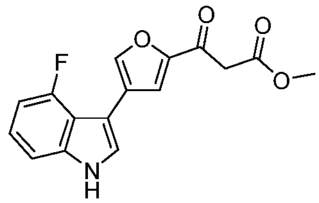 |
| **I-40** | Methyl 3-(4-(5-fluoro-1H-indol-3-yl)furan-2-yl)-3-oxopropanoa te | 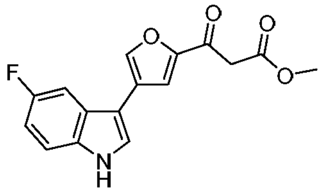 |
| **I-41** | Methyl 3-(4-(benzofuran-4-yl)furan-2-yl)-3-oxopropanoate | 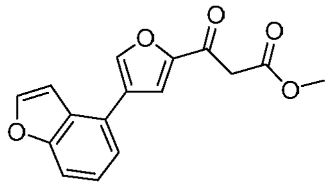 |
| **I-42** | Methyl 3 -(4-(5 -methoxy-1H-indol-3-yl)thiophen-2-yl)-3 -oxopro panoate | 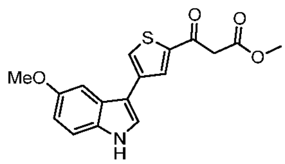 |
| **I-43** | Methyl 3-(4-(5-methoxy-1H-indol-3-yl)furan-2-yl)-3-oxopropan oate | 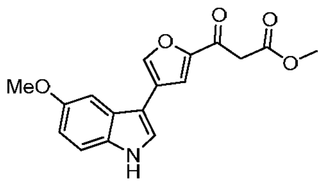 |
| **I-44** | 5-(4-(4-fluoro-1H-indol-3 -yl)thiophen-2-yl)-5 -oxopenta noic acid | 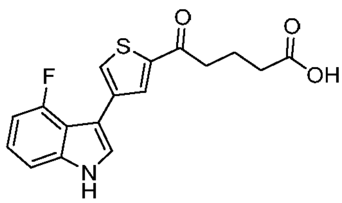 |
| **I-45** | 5 -(4-(5 -fluoro-1 H-indol-3 -yl)thiophen-2-yl)-5 -oxopenta noic acid | 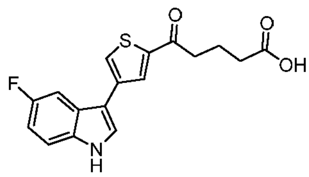 |

(continued)

| | | |
|---|---|---|
| **I-46** | 5-(4-(6-fluoro-1H-indol-3 -yl)thiophen-2-yl)-5 -oxopenta noic acid | 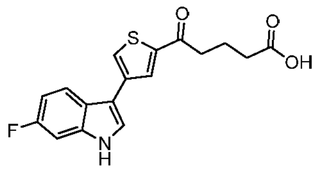 |
| **I-47** | 5-(4-(7-fluoro-1H-indol-3-yl)furan-2-yl)-5-oxopentanoic acid | 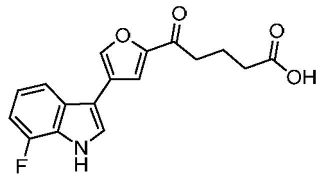 |
| **I-48** | 5-(4-(4-fluoro-1H-indol-3-yl)furan-2-yl)-5-oxopentanoic acid | 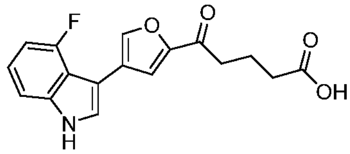 |
| **I-49** | 5-(4-(5-fluoro-1H-indol-3-yl)furan-2-yl)-5-oxopentanoic acid | 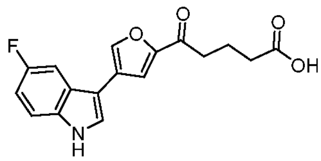 |
| **I-50** | 5-(4-(6-fluoro-1H-indol-3-yl)furan-2-yl)-5-oxopentanoic acid | 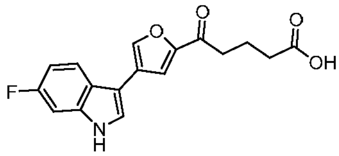 |
| **I-51** | 6-(4-(1H-indol-3-yl)thiophen-2-yl)-6-oxohexanoic acid | 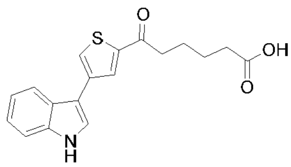 |
| **I-52** | Methyl 2,2-difluoro-3-(4-(7-fluoro-1H-indol-3-yl)thiophen-2-yl) -3 -oxopropanoate | 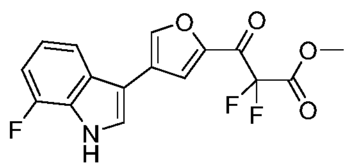 |
| **I-53** | Methyl 3 -(2'-(2-amino-2-oxoethyl)- [3,3 '-bithiophen]-5 -yl)-3-ox opropanoate | 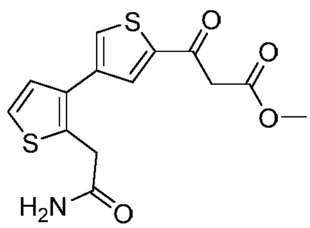 |

(continued)

| | | |
|---|---|---|
| **I-54** | Methyl 3-(4-(3-(2-amino-2-oxoethyl)benzo[b]thiophen-2-yl)thio phen-2-yl)-3 -oxopropanoate | 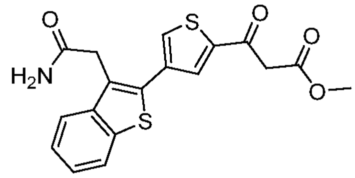 |
| **I'-1** | 4-(4-(3-(2-amino-2-oxoethyl)benzo[b]thiophen-2-yl)thio phen-2-yl)-4-oxobutyric acid | 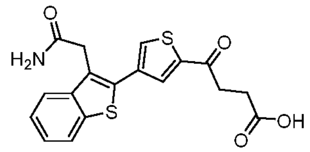 |
| **I'-2** | 4-(3-(2-amino-2-oxoethyl)-[2,3'-thiophen]-5'-yl)-4-oxob utyric acid | 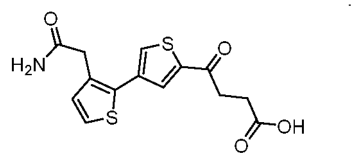 |
| **I'-3** | 4-([3,3'-dithiophen]-2-yl)-4-oxo butyric acid | 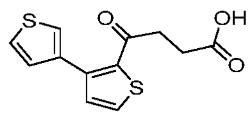 |
| **I'-4** | 4-(4-(benzo[b]thiophen-3-yl)thiophen-2-yl)-4-oxobutyri c acid | 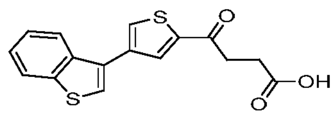 |
| **I'-5** | 4-(5-(benzo[b]thiophen-3-yl)thiophen-2-yl)-4-oxobutyri c acid | 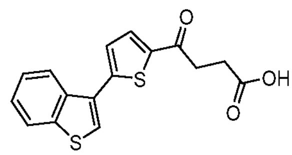 |
| **I'-6** | 4-([3,3'-dithiophen]-5-yl)-4-oxobutyric acid | 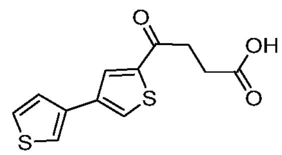 |
| **I'-7** | 4-(4-phenylthiophen-2-yl)-4-oxobutyric acid | 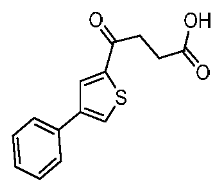 |
| **I'-8** | 4-(4-(naphthalen-1-yl)thiophen-2-yl)-4-oxobutyric acid | 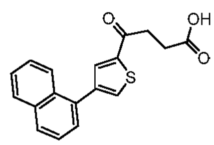 |
| **I'-9** | 4-(4-(dibenzo[b,d]thiophen-4-yl)thiophen-2-yl)-4-oxobu tyric acid | 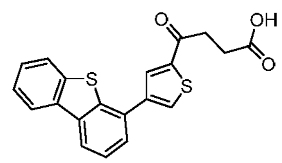 |

(continued)

| | | |
|---|---|---|
| **I'-10** | 4-(4-(benzo[b]thiophen-4-yl)thiophen-2-yl)-4-oxobutyri c acid | 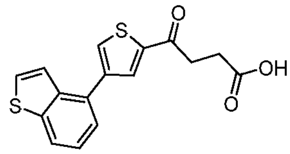 |
| **I'-11** | 4-(5-(benzo[b]thiophen-4-yl)thiophen-2-yl)-4-oxobutyri c acid | 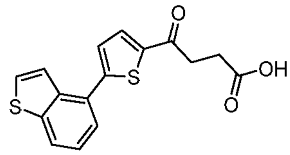 |
| **I'-12** | 4-(4-(2-methylbenzo[b]thiophen-3-yl)thiophen-2-yl)-4-o xobutyric acid | 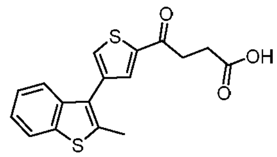 |
| **I'-13** | 4-(4-(benzo[b]thiophen-3-yl)-5-methylthiophen-2-yl)-4-oxobutyric acid | 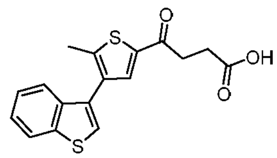 |
| **I'-14** | 4-(4-(benzofuran-3-yl)thiophen-2-yl)-4-oxobutyric acid | 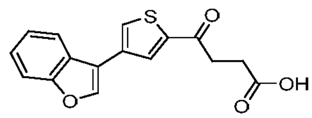 |
| **I'-15** | 4-(4-(benzofuran-7-yl)thiophen-2-yl)-4-oxobutyric acid | 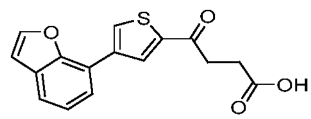 |
| **I'-16** | 4-(4-(1H-indazol-3-yl)thiophen-2-yl)-4-oxobutyric acid | 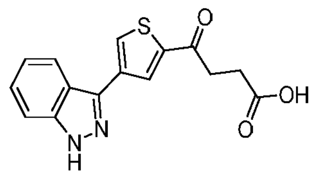 |
| **I'-17** | 4-(4-(benzofuran-3-yl)furan-2-yl)-4-oxo butyric acid | 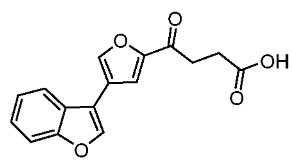 |
| **I'-18** | 4-(4-(3-amino-1H-indazol-4-yl)thiophen-2-yl)-4-oxobut yric acid | 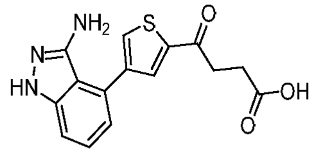 |
| **I'-19** | 4-(4-(benzo[d][1,3]dioxol-5-yl)furan-2-yl)-4-oxobutyric acid | 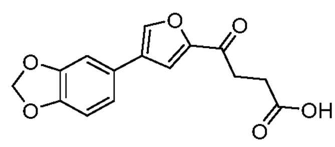 |

(continued)

| | | |
|---|---|---|
| **I'-20** | 4-(4-(benzofuran-5-yl)furan-2-yl)-4-oxobutyric acid | 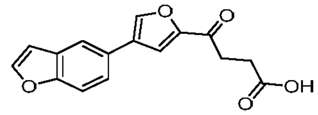 |
| **I'-21** | 4-(4-(benzofuran-4-yl)furan-2-yl)-4-oxobutyric acid | 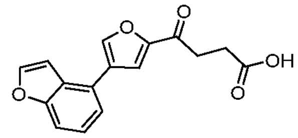 |
| **I'-22** | 4-(4-(benzofuran-6-yl)furan-2-yl)-4-oxobutyric acid | 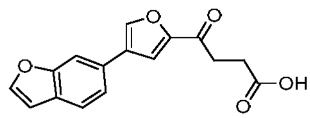 |
| **I'-23** | 4-(4-(3,4-dimethoxyphenyl)furan-2-yl)-4-oxobutyric acid | 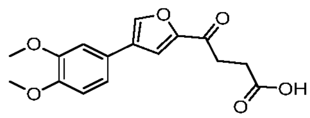 |
| **I'-24** | 4-(4-(benzofuran-3-yl)furan-2-yl)-4-oxobutyronitrile | 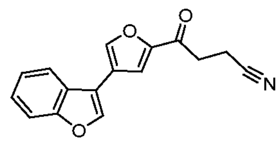 |
| **I'-25** | 4-(4-(benzofuran-3-yl)furan-2-yl)-4-oxobutanamide | 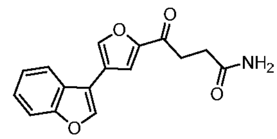 |
| **I'-26** | 4-(4-(thieno[2,3-c]pyridin-4-yl)thiophen-2-yl)-4-oxobut yric acid | 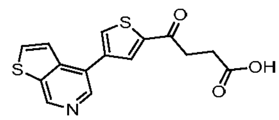 |
| **I'-27** | 4-(4-(thieno[2,3-b]pyridin-4-yl)thiophen-2-yl)-4-oxobut yric acid | 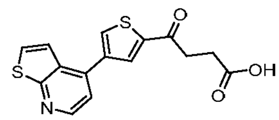 |
| **I'-28** | 4-(4-(benzofuran-3-yl)-1H-pyrrol-2-yl)-4-oxobutyric acid | 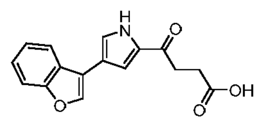 |
| **I'-29** | 4-(4-(benzofuran-2-yl)furan-2-yl)-4-oxobutyric acid | 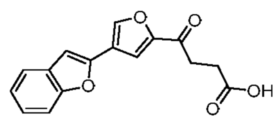 |
| **I'-30** | 4-(4-(1H-indol-3-yl)furan-2-yl)-4-oxobutyric acid | 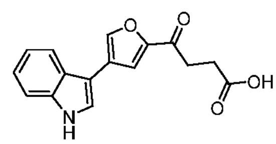 |

(continued)

| | | |
|---|---|---|
| **I'-31** | 4-(4-(1H-indol-3-yl)thiophen-2-yl)-4-oxobutyric acid | 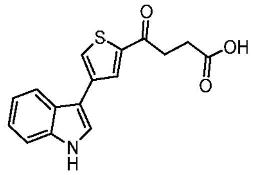 |
| **I'-32** | 4-(4-(benzofuran-3-yl)thiazol-2-yl)-4-oxobutyric acid | 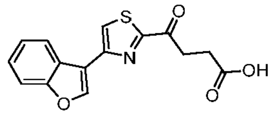 |
| **I'-33** | 4-(4-(1H-indol-3-yl)thiazol-2-yl)-4-oxobutyric acid | 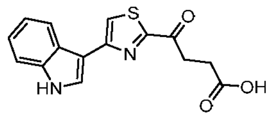 |
| **I'-34** | 4-(4-(imidazo[1,2-a]pyridin-3-yl)furan-2-yl)-4-oxobutyr ic acid | 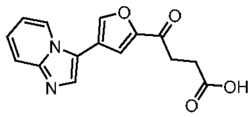 |
| **I'-35** | 4-(4-(1H-benzo[d]imidazol-1-yl)furan-2-yl)-4-oxobutyri c acid | 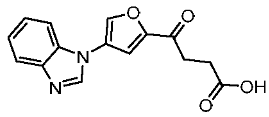 |
| **I'-36** | 4-(4-(furo[2,3-c]pyridin-3-yl)furan-2-yl)-4-oxobutyric acid | 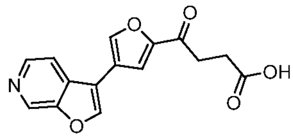 |
| **I'-37** | 4-(4-(benzofuran-3-yl)furan-2-yl)-2-methyl-4-oxobbutyr ic acid | 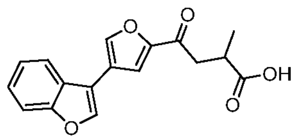 |
| **I'-38** | 4-(4-(6-fluoro-1H-indol-3-yl)furan-2-yl)-4-oxobutyric acid | 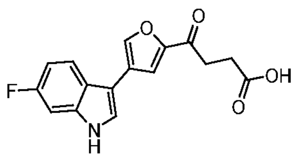 |
| **I'-39** | 4-(4-(1H-pyrro[2,3-b]pyridin-3-yl)furan-2-yl)-2-methyl-4-oxobutyric acid | 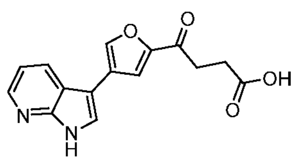 |
| **I'-40** | 4-(4-(5-methoxy-1H-indol-3-yl)furan-2-yl)-4-oxobutyric acid | 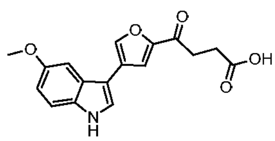 |

(continued)

| | | |
|---|---|---|
| **I'-41** | 4-(4-(6-methyl-1H-indol-3-yl)furan-2-yl)-4-oxobutyric acid | 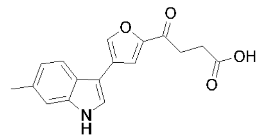 |
| **I'-42** | 4-(4-(6-fluorobenzofuran-3-yl)furan-2-yl)-4-oxobutyric acid | 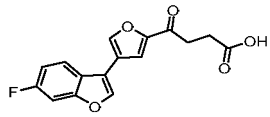 |
| **I'-43** | 4-(4-(6-fluorobenzofuran-3-yl)thiophen-2-yl)-4-oxobuty ric acid | 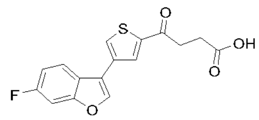 |
| **I'-44** | 4-(4-(thieno[3,2-b]furan-3-yl)thiophen-2-yl)-4-oxobutyri c acid | 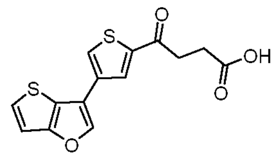 |
| **I'-45** | 4-(4-(thieno[3,2-b]furan-3-yl)furan-2-yl)-4-oxobutyric acid | 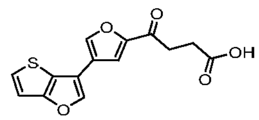 |
| **I'-46** | 4-(4-(6-fluoro-1H-indol-3-yl)thiophen-2-yl)-4-oxobutyri c acid | 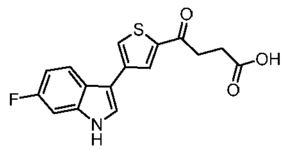 |
| **I'-47** | 4-(4-(6-chloro-1H-indol-3-yl)thiophen-2-yl)-4-oxobutyri c acid | 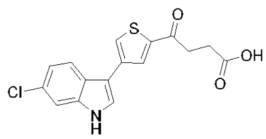 |
| **I'-48** | 4-(4-(5-fluoro-1H-indol-3-yl)thiophen-2-yl)-4-oxobutyri c acid | 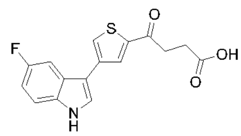 |
| **I'-49** | 4-(4-(7-fluoro-1H-indol-3-yl)thiophen-2-yl)-4-oxobutyri c acid | 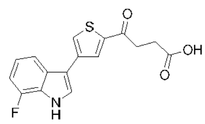 |
| **I'-50** | Methyl 4-(4-(7-fluoro-1H-indol-3-yl)thiophen-2-yl)-4-oxobutyr ate | 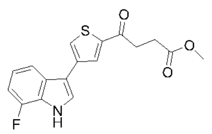 |
| **I'-51** | 4-(4-(4-fluoro-1H-indol-3-yl)thiophen-2-yl)-4-oxobutyri c acid | 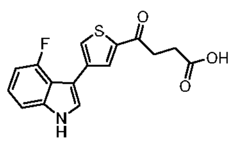 |

(continued)

| | | |
|---|---|---|
| **I'-52** | 4-(4-(6-trifluoromethyl-1H-indol-3-yl)thiophen-2-yl)-4-oxobutyric acid | 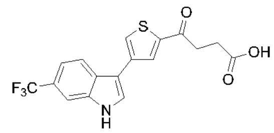 |
| **I'-53** | 4-(5-(1H-indol-3-yl)thiophen-3-yl)-4-oxobutyric acid | 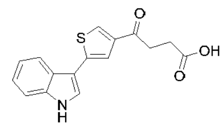 |
| **I'-54** | 4-(4-(5-fluorobenzofuran-3-yl)thiophen-2-yl)-4-oxobuty ric acid | 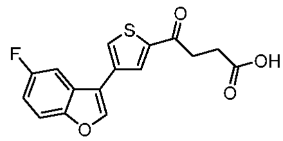 |
| **I'-55** | 4-(4-(7-fluorobenzofuran-3-yl)thiophen-2-yl)-4-oxobuty ric acid | 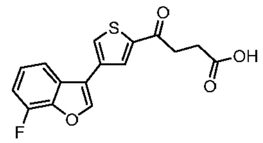 |
| **I'-56** | 4-(3-fluoro-4-(1H-indol-3 -yl)thiophen-2-yl)-4-oxobutyri c acid | 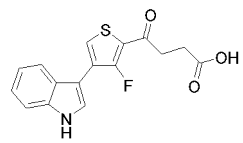 |
| **I'-57** | 4-(4-(5-fluoro-1H-indol-3-yl)furan-2-yl)-4-oxobutyric acid | 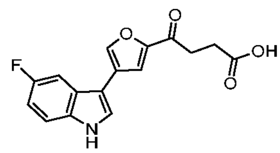 |
| **I'-58** | 4-(4-(7-fluoro-1H-indol-3-yl)furan-2-yl)-4-oxobutyric acid | 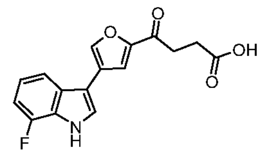 |
| **I'-59** | 4-(4-(4-fluoro-1H-indol-3-yl)furan-2-yl)-4-oxobutyric acid | 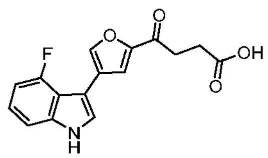 |
| **I'-60** | Methyl 4-(4-(1H-indol-4-yl)furan-2-yl)-4-oxobutyrate | 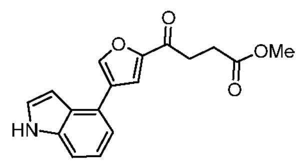 |
| **I'-61** | 4-(4-(1H-indol-3-yl)thiophen-2-yl)-N-hydroxy-4-oxobut anamide | 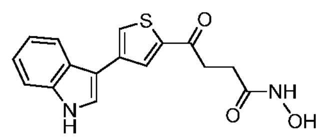 |

(continued)

| | | |
|---|---|---|
| **I'-62** | 4-(4-(5-cyano-1H-indol-3-yl)furan-2-yl)-4-oxobutyric acid | 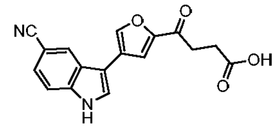 |
| **63-1** | Isoamyl 4-(4-(7-fluoro-1H-indol-3-yl)thiophen-2-yl)-4-oxo butyrate | 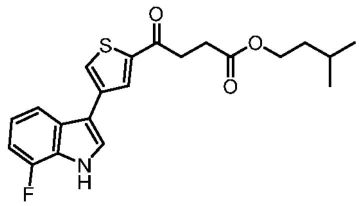 |
| **63-2** | Butyl 4-(4-(7-fluoro-1H-indol-3-yl)thiophen-2-yl)-4-oxobutyr ate (4-methoxy-4-oxo) | 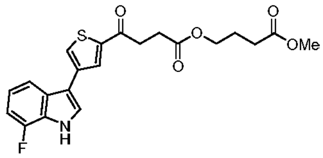 |
| **63-3** | 4-(4-(7-fluoro-1H-indol-3-yl)thiophen-2-yl)-4-hydroxyb utyric acid | 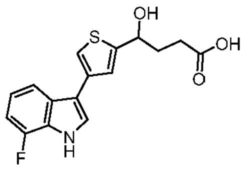 |
| **63-4** | Methyl 4-(4-(7-fluoro-1H-indol-3-yl)thiophen-2-yl)-4-hydroxyb utyrate | 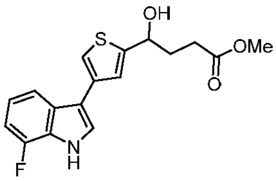 |
| **63-5** | Ethyl 4-(4-(1H-indol-3-yl)furan-2-yl)-4-oxobutyrate (2-methoxy) | 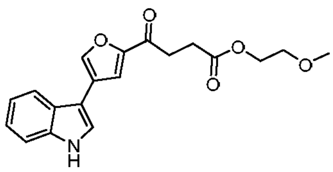 |
| **63-6** | (2,2-dimethyl-1,3-dioxolan-4-yl)methyl 4-(4-(1H-indol-3-yl)furan-2-yl)-4-oxobutyrate | 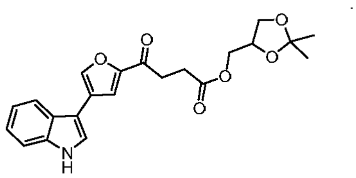 |
| **63-7** | (Tetrahydropyran-4-yl) 4-(4-(1H-indol-3-yl)furan-2-yl)-4-oxobutyrate | 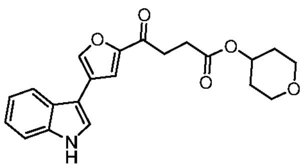 |
| **63-8** | (3-Oxo-1,3-dihydroisobenzofuran-1-yl) 4-(4-(1H-indol-3-yl)furan-2-yl)-4-oxobutyrate | 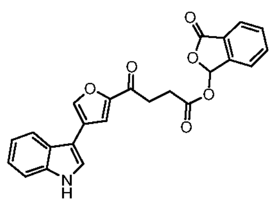 |

(continued)

| | | |
|---|---|---|
| **63-9** | (Pyridin-3-yl) 4-(4-(1H-indol-3-yl)thiophen-2-yl)-4-oxobutyrate | 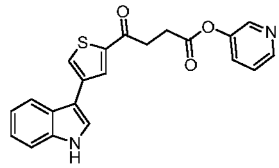 |
| **63-10** | Benzyl 4-(4-(1H-indol-3-yl)thiophen-2-yl)-4-oxobutyrate | 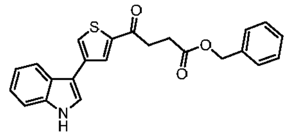 |
| **63-11** | Cyclohexyl 4-(4-(1H-indol-3-yl)thiophen-2-yl)-4-oxobutyrate | 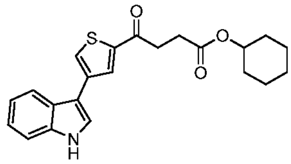 |
| **63-12** | (2-Morpholinyl) ethyl 4-(4-(1H-indol-3-yl)thiophen-2-yl)-4-oxobutyrate | 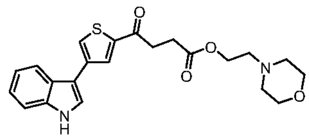 |
| **63-13** | (5,6,7,8-Tetrahydronaphthalen-2-yl) 4-(4-(1H-indol-3-yl)thiophen-2-yl)-4-oxobutyrate | 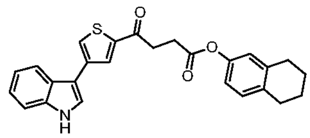 |
| **63-14** | (2-Dimethylamino) ethyl 4-(4-(1H-indol-3-yl)thiophen-2-yl)-4-oxobutyrate | 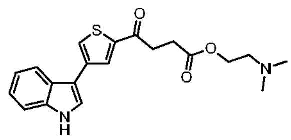 |
| **63-15** | (5-Methyl-[1,3]dioxol-2-one-4 -yl) methyl 4-(4-(1H-indol-3-yl) thiophen-2-yl)-4-oxobutyrate | 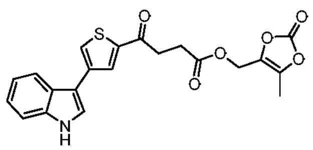 |
| **63-16** | (1-Acetoxy) ethyl 4-(4-(1H-indol-3-yl)furan-2-yl)-4-oxobutyrate | 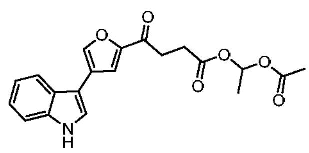 |

PHARMACEUTICALLY ACCEPTABLE SALT

The term "pharmaceutically acceptable salt" or "pharmaceutical salt" means a salt (including amphoteric ions, etc.) that has an effect similar to the parent compound and is acceptable biologically or otherwise (e.g., neither toxic nor harmful to the subject). Accordingly, the examples of the present invention provide a pharmaceutically acceptable salt of the compound of the present invention. The term "salt" as used herein refers to any of the following acid salts formed from inorganic and/or organic acids, as well as basic salts formed from inorganic and/or organic bases. Salt of the compound of the present invention can be formed by methods known to those skilled in the art, for example, by reacting a compound of the present invention with an amount of acid or base (e.g., an equivalent amount of acid or base) in a medium (e.g., the medium can allow the salt to precipitate in it; or using water as the medium and then lyophilizing).

Exemplary acid salts include acetate, ascorbate, benzoate, benzenesulfonate, bisulfate, borate, butyrate, citrate, camphorate, camphorsulfonate, fumarate, hydrochloride, hydrobromide, hydroiodide, lactate, maleate, methanesulfonate ("mesylate"), naphthalenesulfonate, nitrate, oxalate, phosphate, propionate, salicylate, succinate, sulfate, tartrate, thiocyanate, tosylate, etc. Suitable acid salts can be prepared by mixing a solution of the compound with a solution of a pharmaceutically acceptable acid such as hydrochloric acid, sulfuric acid, acetic acid, trifluoroacetic acid or benzoic acid. In addition, the acids suitable for forming pharmaceutical salts are also selected from the following references: 1. P. Stahl et al, Camille G (eds.) Handbook of Pharmaceutical Salts. Properties, Selection and Use. (2002); 2. Zurich, Wiley-VCH. S. Berge el al, Journal of Pharmaceutical Sciences (1977) 66(1), 1-19; 3. P. Gould, International J. of Pharmaceutics (1986) 33, 201-217; 4. Anderson el al, The Practice of Medicinal Chemistry (1996), Academic Press, New York; 5. *T*he Orange Book (Food & Drug Administration, Washing, D. C. on their website).

Exemplary base salts include ammonium salts, alkali metal salts (e.g., sodium, lithium, and potassium salts), alkaline earth metal salts (e.g., calcium and magnesium salts), salts containing organic bases (e.g., organic amines) (e.g., dicyclohexylamine) , tert-butylamine, choline and salts containing amino acids (such as arginine, lysine), etc. Basic nitrogen-containing groups can react with compounds such as lower alkyl halides (e.g., methyl, ethyl, and butyl chlorides, bromides, and iodides), dialkyl sulfates (e.g., dimethyl, diethyl and dibutyl sulfate), long-chain halides (e.g., decyl, lauryl, and stearyl chlorides, bromides, and iodides), aralkyl halides (e.g., benzyl and phenethyl bromide) and others to form quaternary ammonium salts. Compounds bearing acidic groups can be mixed with suitable pharmaceutically acceptable salts to prepare alkali metal salts (such as sodium or potassium salts), alkaline earth metal salts (such as calcium or magnesium salts) and salts prepared from suitable organic ligands (such as quaternary ammonium salts). In addition, in the presence of carboxyl or hydroxyl, pharmaceutically acceptable esters can be used to improve the solubility or hydrolysis properties of the compound.

All such acid salts and base salts are pharmaceutically acceptable salts within the scope of the present invention, and for the purposes of the present invention, all acid salts and base salts are considered equivalent to the corresponding parent compounds of the present invention.

In addition, when the compounds of the present invention contain both basic groups (such as but not limited to primary, secondary, tertiary aliphatic or cyclic amines, aromatic or heteroaryl amines, pyridine or imidazole) and acidic groups (such as but not limited to tetrazole or carboxylic acid), amphoteric ions ("inner salts") that can be formed are also encompassed by the term "salts" in the present patent. Certain compounds of the present invention may exist in the form of amphoteric ions, having both anionic and cationic centers in the same compound, and have a net neutral charge, and such amphoteric ions are also included in the present invention.

## PREPARATION METHOD OF COMPOUND

The preparations of formula **(I), (I')**, (I"), **(II), (III), (IV), (V), (VI)**, (**II**-1), (**III**-1), **(IV-1)**, (**V-1**) or **(VI-1)** are described in the following schemes and examples, preferably several methods for the preparations of formula **(II), (III), (IV), (V), (VI)**, (**II**-1), (**III-1**), **(IV-1)**, (V-1) **or** (VI-1) and the above pharmaceutically acceptable salts. Raw materials and intermediates are purchased from commercial sources, prepared by known steps, or otherwise described. In some cases, the sequence of steps to perform the reaction scheme may be changed to facilitate the reaction or avoid unwanted side reaction products.

In the following methods and schemes, the variables $R^3$, $R^4$, W, X, Z, U and ring B have the same meanings as above.

Preferably, **Method 1**

A boronic acid intermediate (**1a**) is reacted with an intermediate (**1b**), (**1c**), (**1d**), (**1e**) or **(If)** through palladium-catalyzed Suzuki coupling reaction to obtain the compounds of formula **(II), (III), (IV), (V)** or **(VI)** of the present invention.

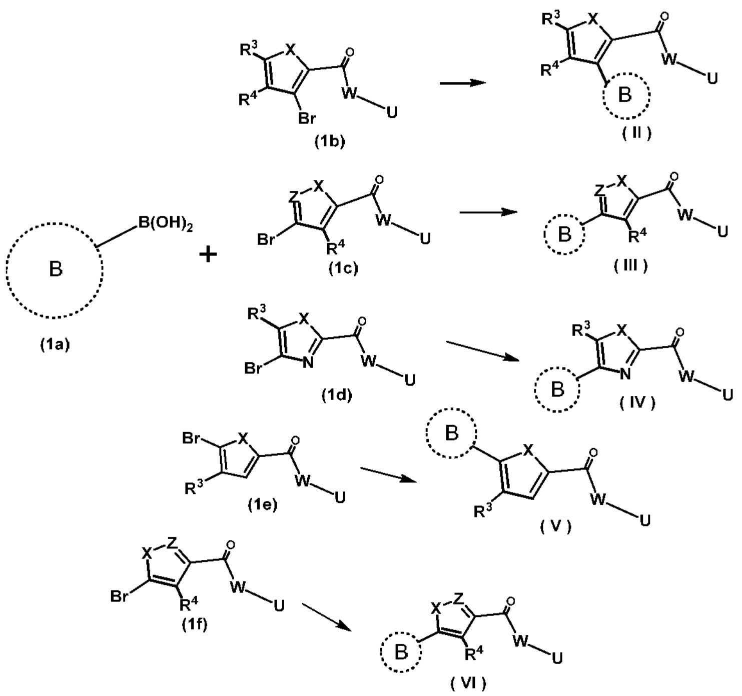

in the presence of a catalyst, a boronic acid intermediate (**1a**) is reacted with an intermediate (**1b**) to obtain a compound of formula (II);

in the presence of a catalyst, a boronic acid intermediate (**1a**) is reacted with an intermediate (**1c**) to obtain a compound of formula (III);
in the presence of a catalyst, a boronic acid intermediate (**1a**) is reacted with an intermediate (**1d**) to obtain a compound of formula (IV);
in the presence of a catalyst, a boronic acid intermediate (**1a**) is reacted with an intermediate (**1e**) to obtain a compound of formula (V);
in the presence of a catalyst, a boronic acid intermediate (**1a**) is reacted with an intermediate **(If)** to obtain a compound of formula (VI);

**Method 2**

Compounds of formula **(II), (III), (IV), (V)** or **(VI)** of the present invention can also be prepared by the following synthetic routes, namely an intermediate **(2a)** is reacted with a boronic acid intermediate **(2b), (2c), (2d), (2e)** or **(2f)** through palladium-catalyzed Suzuki coupling reaction.

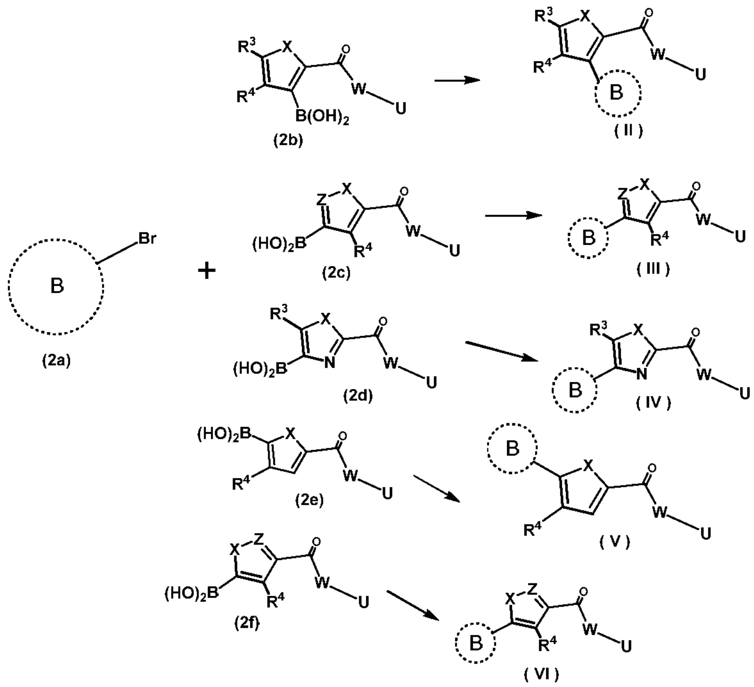

(2a) (2b) (II) (2c) (III) (2d) (IV) (2e) (V) (2f) (VI)

in the presence of a catalyst, the bromine-containing intermediate **(2a)** is reacted with a boronic acid intermediate **(2b)** to obtain a compound of formula (II);

in the presence of a catalyst, the bromine-containing intermediate **(2a)** is reacted with a boronic acid intermediate **(2c)** to obtain a compound of formula (III);
in the presence of a catalyst, the bromine-containing intermediate **(2a)** is reacted with a boronic acid intermediate **(2d)** to obtain a compound of formula (IV);
in the presence of a catalyst, the bromine-containing intermediate **(2a)** is reacted with a boronic acid intermediate **(2e)** to obtain a compound of formula (V);
in the presence of a catalyst, a bromine-containing intermediate **(2a)** is reacted with a boronic acid intermediate **(2f)** to obtain a compound of formula (VI);
preferably, (**II**-1), (**III-1**), **(IV-1), (V-1)** or **(VI-1)** is prepared by the following methods,

Method A:

it comprises the following steps:

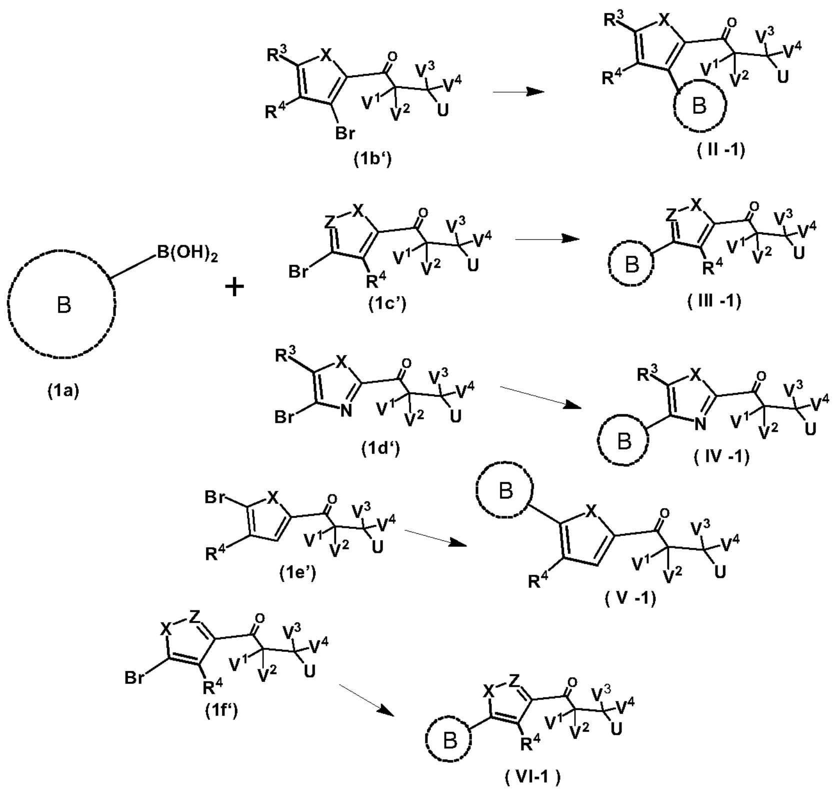

(1b') (II-1) (1a) (1c') (III-1) (1d') (IV-1) (1e') (V-1) (1f') (VI-1)

in the presence of a catalyst, a boronic acid intermediate (**1a**) is reacted with an intermediate (**1b'**) to obtain a compound of formula (II-1);

in the presence of a catalyst, a boronic acid intermediate (**1a**) is reacted with an intermediate (**1c'**) to obtain a compound of formula (III-1);
in the presence of a catalyst, a boronic acid intermediate (**1a**) is reacted with an intermediate (**1d'**) to obtain a compound of formula (IV-1);
in the presence of a catalyst, a boronic acid intermediate (**1a**) is reacted with an intermediate (**1e'**) to obtain a compound of formula (V-1);
in the presence of a catalyst, a boronic acid intermediate (**1a**) is reacted with an intermediate (**1f'**) to obtain a compound of formula (VI-1);

Method B:

it comprises the following steps:

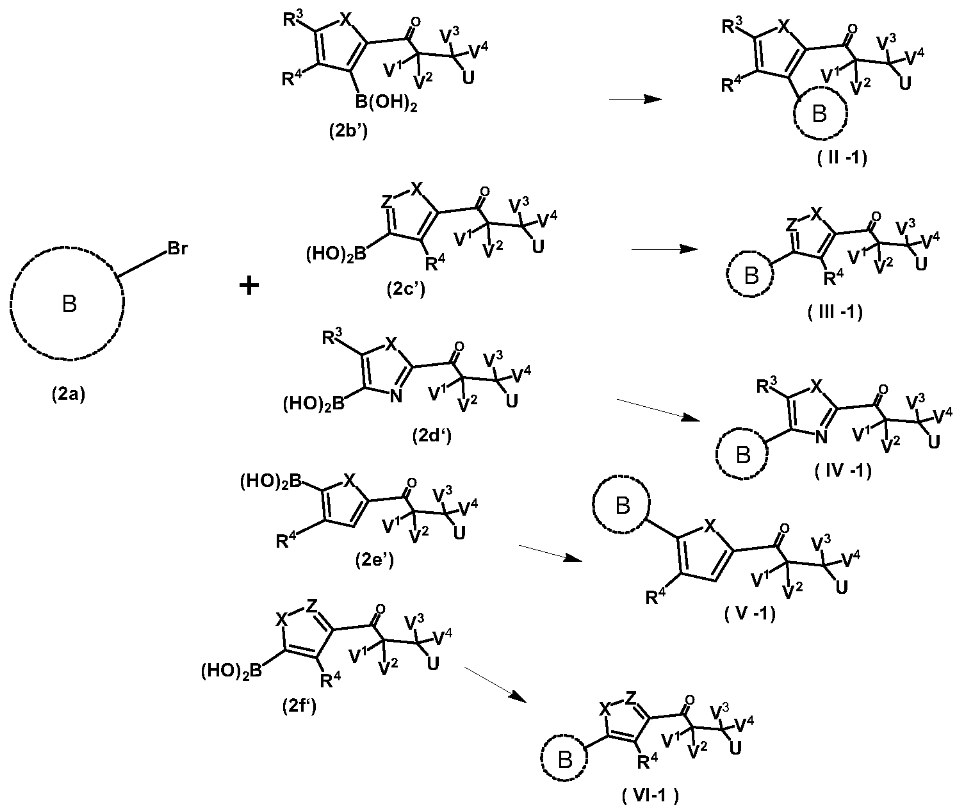

(2b')

(II -1)

(2a)

+

(2c')

(III -1)

(2d')

(IV -1)

(2e')

(V -1)

(2f')

(VI-1)

in the presence of a catalyst, a bromine-containing intermediate **(2a)** is reacted with a boronic acid intermediate **(2b')** to obtain a compound of formula (II-1);

in the presence of a catalyst, the bromine-containing intermediate **(2a)** is reacted with a boronic acid intermediate **(2c')** to obtain a compound of formula (III-1);
in the presence of a catalyst, a bromine-containing intermediate **(2a)** is reacted with a boronic acid intermediate **(2d')** to obtain a compound of formula (IV-1);
in the presence of a catalyst, a bromine-containing intermediate **(2a)** is reacted with a boronic acid intermediate **(2e')** to obtain a compound of formula (V-1);
in the presence of a catalyst, a bromine-containing intermediate **(2a)** is reacted with a boronic acid intermediate **(2f')** to obtain a compound of formula (VI-1);
wherein, $V^1$, $V^2$, $V^3$, $V^4$, $R^3$, $R^4$, X, Z, U and ring B are defined as above.

## USE

The therapeutically useful compound of the present invention includes, for example, the compound of formulae **(I), (I')**, (I"), **(II), (III), (IV),** (V), **(VI),** (**II**-1), (**III-1**), (**IV-1**), **(V-1)** or **(VI-1).** The compounds and pharmaceutically acceptable salts of the above compounds described in the Examples are administered to patients for the purpose of inducing an immune response, inducing STING-dependent cytokine production and/or inducing anti-tumor activity. The term "administration" refers to providing a compound to an individual in need of treatment. When the compound of the present invention is administered in combination with one or more other drugs (e.g., antiviral for the treatment of HCV or antineoplastic drugs), "administration" means providing the compound of the present invention, the pharmaceutically acceptable salt thereof and in combination with other drugs to a patient.

The compound disclosed herein can be STING agonists. Disease or disorder that these compounds can be used to treat includes but is not limited to cell proliferation disorder. Cell proliferation disorder includes but is not limited to cancer, benign papillomatosis, gestational trophoblastic disease, and benign neoplastic diseases such as papillomas (warts) and genital papilloma.

In certain embodiments, the cell proliferation disorder is cancer. In specific embodiment, the cancer is selected from brain cancer and spine cancer, head and neck cancer, leukemia and blood cancer, skin cancer, genital system cancer, gastrointestinal system cancer, liver and bile duct cancer, kidney cancer and bladder cancer, bone cancer, lung cancer, malignant mesothelioma, sarcoma, lymphoma, adenocarcinoma, thyroid cancer, cardiac tumor, germ cell tumor,

malignant neuroendocrine (carcinoid) tumor, midline beam cancer, and unknown primary cancer(i.e., metastatic cancer found but the primary cancer origin is unknown). In particular embodiments, the cancer is present in an adult patient; in other embodiments, the cancer is present in a pediatric patient. In particular embodiments, the cancer is associated with AIDS.

In particular embodiments, the cancer is selected from brain cancer and spine cancer. In particular embodiments, the cancer is selected from anaplastic astrocytoma, glioblastoma, astrocytoma or estuarine neuroblastoma (also known as olfactory blastoma). In particular embodiments, the brain cancer is selected from astrocytomas (e.g., pilocytic astrocytoma, subependymal giant cell astrocytoma, diffuse astrocytoma, pleomorphic yellow astrocytoma tumor, anaplastic astrocytoma, astrocytoma, giant cell glioblastoma, glioblastoma, secondary glioblastoma, primary adult glioblastoma), primary childhood glioblastoma, oligodendroglioma (e.g., oligodendroglioma, anaplastic oligodendroglioma), oligoastrocytoma (e.g., oligoastrocytoma, anaplastic oligoastrocytoma), ependymoma (e.g., mucus nipples ependymoma and anaplastic ependymoma), medulloblastoma, primitive neuroectodermal tumor, schwannoma, meningioma, atypical meningioma, anaplastic meningioma, pituitary adenomas, brain stem gliomas, cerebellar astrocytomas, cerebral astrocytomas/malignant gliomas, optic nerve pathway and hypothalamic gliomas and primary CNS lymphomas. In certain embodiments of these specific embodiments, the brain cancer is selected from glioma, glioblastoma multiforme, paraganglioma or supratentorial primary neuroectodermal tumor (SPNET).

In particular embodiments, the cancer is selected from head and neck cancer, including nasopharyngeal cancer, nasal cavity and paranasal sinus cancer, hypopharyngeal cancer, oral cancer (eg, squamous cell carcinoma, lymphoma and sarcoma), lip cancer, oropharyngeal cancer, salivary gland tumors, laryngeal cancer (eg, laryngeal squamous cell carcinoma, rhabdomyosarcoma), or eye cancer. In particular embodiments, the eye cancer is selected from intraocular melanoma or retinoblastoma.

In particular embodiments, the cancer is selected from leukemia or hematological cancer. In certain embodiments, the cancer is selected from myeloproliferative neoplasms, myelodysplastic syndromes, myelodysplastic/myeloproliferative neoplasms, acute myeloid leukemia (AML), myelodysplastic syndromes (MDS) or chronic myeloid leukemia (CML). Myeloproliferative neoplasm (MPN), post-MIPN acute myeloid leukemia, post-MDS acute myeloid leukemia, del(5q)-related high-risk MDS or AML, advanced chronic myeloid leukemia, angioimmunoblastic lymphoma, acute lymphoblastic leukemia, Langerans cell histiocytosis, hairy cell leukemia or plasmacytoma including plasmacytoma and multiple myeloma. The leukemia mentioned here may be acute or chronic.

In particular embodiments, the cancer is selected from skin cancer. In particular embodiments, the skin cancer is selected from melanoma, squamous cell carcinoma or basal cell carcinoma.

In specific embodiments, the cancer is selected from cancers of the reproductive system. In particular embodiments, the cancer is selected from breast cancer, cervical cancer, vaginal cancer, ovarian cancer, prostate cancer, penile cancer or testicular cancer. In particular examples of these embodiments, the cancer is breast cancer selected from ductal carcinoma or phyllodes tumor. In particular examples of these embodiments, the breast cancer can be male breast cancer or female breast cancer. In particular examples of these embodiments, the cancer is cervical cancer selected from squamous cell carcinoma or adenocarcinoma. In particular examples of these embodiments, the cancer is ovarian cancer selected from epithelial cancers.

In particular embodiments, the cancer is selected from cancers of the gastrointestinal system. In specific embodiments, the cancer is selected from esophageal cancer, gastric cancer, gastrointestinal carcinoid, pancreatic cancer, gallbladder cancer, colorectal cancer or anal cancer. In the examples of these embodiments, the cancer is selected from esophageal squamous cell carcinoma, esophageal adenocarcinoma, gastric adenocarcinoma, gastrointestinal carcinoid, gastrointestinal stromal tumor, gastric lymphoholema, gastrointestinal lymphoma, solid pseudopapilloma tumor of the pancreas, pancreatic blastoma, islet cell tumor, pancreatic cancer including acinar cell carcinoma and ductal adenocarcinoma, gallbladder adenocarcinoma, colorectal adenocarcinoma or anal squamous cell carcinoma.

In particular embodiments, the cancer is selected from liver and cholangiocarcinoma. In particular embodiments, the cancer is liver cancer (also known as hepatocellular carcinoma). In particular embodiments, the cancer is cholangiocarcinoma; in the examples of these embodiments, the cholangiocarcinoma is selected from intrahepatic cholangiocarcinoma or extrahepatic cholangiocarcinoma.

In particular embodiments, the cancer is selected from kidney cancer or bladder cancer. In particular embodiments, the renal cancer is selected from renal cell carcinoma, Wilms tumor or transitional cell carcinoma. In particular embodiments, the bladder cancer is selected from urothelial carcinoma (transitional cell carcinoma), squamous cell carcinoma or adenocarcinoma.

In particular embodiments, the cancer is selected from bone cancer. In particular embodiments, the bone cancer is selected from osteosarcoma, malignant fibrous histiocytoma of bone, Ewing's sarcoma or chordoma (bone cancer along the spine).

In particular embodiments, the cancer is selected from lung cancer. In particular embodiments, the lung cancer is selected from non-small cell lung cancer, small cell lung cancer, bronchial tumors or pleuropulmonary blastoma.

In particular embodiments, the cancer is selected from malignant mesothelioma. In particular embodiments,

the cancer is selected from epithelial mesothelioma or sarcoma.

In particular embodiments, the cancer is selected from sarcoma. In particular embodiments, the sarcoma is selected from central chondrosarcoma, central and periosteal chondroma, fibrosarcoma, tenosynovial clear cell sarcoma or Kaposi's sarcoma.

In particular embodiments, the cancer is selected from lymphoma. In particular embodiments, the cancer is selected from Hodgkin lymphoma (eg, Reed Sternberg cells), non-Hodgkin lymphoma (eg, diffuse large B-cell lymphoma, follicular lymphoma, mycosis fungoides, SZAREY syndrome, primary central nervous system lymphoma), cutaneous T-cell lymphoma or primary central nervous system lymphoma.

In particular embodiments, the cancer is selected from adenocarcinoma. In particular embodiments, the cancer is selected from adrenocortical carcinoma, pheochromocytoma, paraganglioma, pituitary tumor, thymoma or thymoma.

In particular embodiments, the cancer is selected from thyroid cancer. In particular embodiments, the thyroid cancer is selected from medullary thyroid cancer, papillary thyroid cancer or follicular thyroid cancer.

In particular embodiments, the cancer is selected from germ cell tumor. In particular embodiments, the cancer is selected from malignant extracranial germ cell tumor and malignant extragonadal germ cell tumor. In particular examples of these embodiments, the malignant extragonadal germ cell tumor is selected from non-seminomas or seminomas.

In particular embodiments, the cancer is selected from cardiac tumor. In particular embodiments, the cardiac tumor is selected from malignant teratoma, lymphoma, rhabdomyosarcoma, angiosarcoma, chondrosarcoma, infantile fibrosarcoma or synovial sarcoma.

In particular embodiments, the cell proliferation disorder is selected from benign papillomatosis, benign neoplastic disease, or gestational trophoblastic disease. In particular embodiments, the benign neoplastic disease is selected from cutaneous papilloma (wart) or genital papilloma. In particular embodiments, the gestational trophoblastic disease is selected from hydatid nevus or gestational trophoblastic tumor (e.g., invasive nevus, choriocarcinoma, placental site trophoblastic tumor, and epithelioid trophoblastic tumor).

In particular embodiments, the disease associated with STING activity comprises an inflammatory disease. The inflammatory disease is selected from acne vulgaris, asthma, celiaca, chronic prostatitis, glomerulonephritis, inflammatory bowel disease, pelvic inflammatory disease, reperfusion injury, rheumatoid arthritis, sarcoidosis, vasculitis, airway inflammation caused by house dust mites and interstitial cystitis.

The term "treatment" as used herein refers to all processes in which there may be slowing, interrupting, arresting, controlling or halting the development of the disease or disorder described herein. These terms do not necessarily imply the complete elimination of all symptoms of the disease or disorder.

The term "administeration" is understood to include providing to a subject a compound described herein, or a pharmaceutically acceptable salt thereof, or a pharmaceutical composition as described above.

The amount of the compound administered to the subject is an amount sufficient to induce an immune response and/or to induce STING-dependent Type I interferon production in the subject. In one embodiment, the amount of compound may be an "effective amount" or "therapeutically effective amount" that will cause the biological or medical (i.e., treatment-related) reactions of the tissue, system, animal or human being sought by the researcher, veterinarian, physician or other clinician. In one embodiment, the amount of the compound may be an "effective amount" or "therapeutically effective amount" such that the administered amount of the test compound will induce response in a tissue, system, animal or human, being sought by the researcher, veterinarian, physician or other clinician. The effective amount is not necessarily based on toxicity and safety considerations associated with the administered compound.

The effective amount of the compound will vary with the particular compound selected (eg, taking into account the activity, efficacy and/or half-life of the compound); mode of administration; treatment status and severity of the condition being treated; age, size, weight and physical condition of the subject being treated; subject's medical history; duration of treatment; nature of synergistic treatment; desired therapeutic effect; and similar factors that can be determined by those skilled in the art.

The compounds disclosed herein can be administered by any suitable route, including oral and parenteral administration. Parenteral administration is usually by injection or infusion, including intravenous, intramuscular, and subcutaneous injection or infusion.

The compounds disclosed in this patent may be administered once or in a dosing regimen wherein multiple doses are administered at different time intervals over a given period of time. For example, it may be administered once, twice, three times or four times per day. This dose is administered until the desired therapeutic effect is achieved or maintained indefinitely. A reasonable dosing regimen for a compound disclosed herein depends on the pharmacokinetic properties of the compound, such as absorption, distribution, and half-life, which can be determined by those skilled in the art. Furthermore, for the compounds disclosed herein, a reasonable dosage regimen, including the duration of administration of such regimen, depends on the disease or condition being treated, the severity of the disease or condition, the age and physical condition of the subject being treated, the medical history of the subject being treated, the nature of the co-combination, the desired therapeutic effect, and similar factors within the knowledge and expertise of those

skilled in the art. Those skilled in the art make corresponding adjustments to a given dosing regimen based on the individual subject's response to the dosing regimen, or according to the individual subject's needs. The typical daily dose may vary with the particular route of administration chosen.

One embodiment of the present invention provides a method of treating a cell proliferation disorder comprising administering to a subject a therapeutically effective amount of the compound of formula **(I),** (**I'**), (**I"**), (**II** ), **(III), (IV), (V), (VI),** (**II**-1), (**III-1**), **(IV-1), (V-1)** or **(VI-1),** or a pharmaceutically acceptable salt of the above compound. In particular embodiments, the disease or disorder to be treated is a cell proliferation disorder. In specific embodiments of these embodiments, the cell proliferation disorder is cancer, wherein the cancer is selected from brain cancer and spine cancer, head and neck cancer, leukemia and blood cancer, skin cancer, reproductive system cancer, gastrointestinal system cancer, liver and bile duct cancer, kidney cancer and bladder cancer, bone cancer, lung cancer, malignant mesothelioma, sarcomas, lymphoma, adenocarcinomas, thyroid cancer, cardiac tumor, germ cell tumor, malignant neuroendocrine (carcinoid) tumor, midline beam cancer or primary tumors of unknown origin.

In one embodiment, disclosed herein is a use of the compound of formula (**I**), (**I'**), (**I"**), (**II**), (**III**), (**IV**), (**V**), (**VI**), (**II-1**), (**III-1**), (**IV-1**), (**V-1**) or (**VI-1**), or the pharmaceutically acceptable salt for medicine. The primary therapeutic effect of the compound is to induce an immune response and/or to induce STING-dependent type I interferon in a subject (e.g., a mammal) by administering to the subject an effective amount of the compound.

One embodiment disclosed herein is a pharmaceutical composition comprising at least one of the compounds of formula (**I**), (**I'**), (**I"**), (**II**), (**III**), ( **IV**), (**V**), (**VI**), (**II-1**), (**III-1**), (**IV-1**), (**V-1**) or (**VI-1**), or at least one of the pharmaceutically acceptable salt of the above compounds for use in inducing an immune response and/or inducing STING-dependent type I interferon production.

One embodiment disclosed herein is the compound of formula (**I**), (**I'**), (**I"**), (**II**), (**III**), (**IV**), (**V**) , (**VI**), (**II-1**), (**III-1**), (**IV-1**), (**V-1**) or (**VI-1**), or the pharmaceutically acceptable salts of the above compounds for used in the manufacture of a medicament for inducing an immune response and/or inducing the production of STING-dependent type I interferon. In embodiments, the disease or disorder to be treated is a cell proliferation disorder. In specific embodiments of these embodiments, the cell proliferation disorder is cancer, wherein the cancer is selected from brain cancer and spine cancer, head and neck cancer, leukemia and blood cancer, skin cancer, reproductive system cancer, gastrointestinal system cancer, liver and bile duct cancer, kidney cancer, bladder cancer, bone cancer, lung cancer, malignant mesothelioma, sarcomas, lymphoma, adenocarcinomas, thyroid cancer, cardiac tumor, germ cell tumor, malignant neuroendocrine (carcinoid) tumor, midline beam cancer or primary tumors of unknown origin.

## PHARMACEUTICAL COMPOSITION

The term "pharmaceutical composition" refers to a dosage form comprising a specified amount of the specified compound, as well as any dosage form prepared directly or indirectly from the specified amount of the specified compound. This term includes the dosage form of the compound of formula (**I**), (**I'**), (**I"**), (**II**), (**III**), (**IV**), (**V**), (**VI**), (**II-1**), (**III-1**), (**IV-1**), (**V-1**) or (**VI-1**) or the pharmaceutically acceptable salt of the above compound, and one or more pharmaceutically acceptable carriers or excipients. Therefore, the pharmaceutical compositions disclosed in the present invention comprise any pharmaceutical dosage form prepared by mixing a compound of the present invention with one or more pharmaceutically acceptable carriers or excipients. The term "pharmaceutically acceptable" means that a carrier or excipient is compatible with the compound disclosed herein and the other ingredients of the pharmaceutical composition.

To induce an immune response and/or to induce STING-dependent type I interferon production, a preparation formed by the compound of formula (**I**), (**I'**), (**I' '**), (**II**), (**III**), ( **IV**), (**V**), (**VI**), (**II-1**), (**III-1**), (**IV-1**), (**V-1**) or (**VI-1**) or the pharmaceutically acceptable salt of the above compound and corresponding carrier or excipient is administrated. These compounds can be administered in conventional manner with other drugs, either as therapeutic drug alone or in combination with other drugs. These compounds may be administered alone, but will generally be administered with the choice of pharmaceutical carrier according to the chosen route of administration and standard pharmaceutical practice.

In one embodiment, the pharmaceutical composition disclosed herein comprises the compound of formula (**I**), (**I'**), (**I"**), (**II**), (**III**), (**IV**), (**V**), (**VI**), (**II-1**), (**III-1**), (**IV-1**), (**V-1**) or (**VI-1**), or the pharmaceutically acceptable salt of the above compound, and one or more pharmaceutically acceptable carriers or adjuvants. The pharmaceutical composition can be prepared in the form of a bulk pack in which a therapeutically effective dose of a compound of the present invention can be provided, and then administered to a subject, such as a powder or syrup. Alternatively, the pharmaceutical composition may be prepared in unit-package form, wherein each individual package contains a therapeutically effective amount of the compound of formula (**I**), (**I'**), (**I"**), (**II**), (**III**), (**IV**), (**V**), (**VI**), (**II-1**), (**III-1**), (**IV-1**), (**V-1**) or (**VI-1**), or the pharmaceutically acceptable salt of the above compound.

The compounds and the pharmaceutically acceptable carriers or excipients disclosed herein are formulated into a suitable dosage form for administration to a subject in a reasonable manner of administration. Dosage form includes (1) oral dosage form such as tablet, capsule, tablet, pill, tablet, powder, syrup, elixir, suspension, solution, emulsion, sachet and ointment; (2) parenteral administration, such as sterile solution, suspension, and reconstituted powder.

Suitable pharmaceutically acceptable carriers or excipients are determined by the particular dosage form chosen. In addition, the choice of a pharmaceutically acceptable carrier or excipient also depends on its specific function in the pharmaceutical composition. For example, certain pharmaceutically acceptable carriers or excipients are selected to facilitate the manufacture of uniform dosage forms; certain pharmaceutically acceptable carriers or excipients are selected to help stabilize the manufacture of dosage forms; certain pharmaceutically acceptable carriers or excipients are selected to facilitate delivery or transport of a compound disclosed herein from one organ or part of the body to another organ or part of the body; certain pharmaceutically acceptable carriers or excipients are selected to enhance patient compliance.

Suitable pharmaceutically acceptable excipients include the following types: diluents, lubricants, binders, disintegrants, fillers, glidants, granulating agents, coating agents, wetting agents, solvents, co-solvents, suspending agents, emulsifiers, sweeteners, flavoring agents, flavor masking agents, colorants, anti-caking agents, hemostatic agents, chelating agents, plasticizers, tackifiers, antioxidants, preservatives, stabilizers, surfactants agents and buffers.

Those skilled in the art have the knowledge and skills in the art, and can select suitable pharmaceutically acceptable carriers and excipients to prepare the pharmaceutical composition of the compound of the present invention. The following documents describe pharmaceutically acceptable carriers and excipients for reference by those skilled in the art: 1.REMINGTON'S PHARMACEUTICAL SCIENCES (Mack Publishing Company); 2.THE HANDBOOK OF PHARMACEUTICAL ADDITIVES (Gower Publishing Limited); 3.THE HANDBOOK OF PHARMACEUTICAL EXCIPIENTS (the American Pharmaceutical Association and the Pharmaceutical Press).

The pharmaceutical compositions of the present invention are prepared using techniques and methods known to those skilled in the art. Methods commonly used in the art are generally described in Remington's Pharmaceutical Sciences (Mack Publishing Company).

In one embodiment, the present invention relates to a solid oral dosage form, such as a tablet or capsule, comprising a therapeutically effective amount of the compound of formula (**I**), (**I'**), (**I"**), (**II**), (**III**), (**IV**), (**V**), (**VI**), (**II-1**), (**III-1**), (**IV-1**), (**V-1**) or (**VI-1**), or the pharmaceutically acceptable salt of the above and a diluent or filler. Suitable diluents and fillers include lactose, sucrose, dextrose, mannitol, sorbitol, starches (e.g. corn starch, potato starch and pregelatinized starch), cellulose and derivatives thereof (e.g. microcrystalline cellulose), calcium sulfate and calcium dihydrogen phosphate. Solid oral dosage forms may also include binders. Suitable binders include starches (e.g. corn starch, potato starch and pregelatinized starch), gelatin, gum arabic, sodium alginate, alginic acid, astragalus, guar gum, povidone, cellulose and derivatives thereof (e.g. microcrystalline cellulose). Solid oral dosage forms may further include disintegrants, including polyvinylpyrrolidone, sodium starch glycolate, croscarmellose, alginic acid, and sodium carboxymethyl cellulose. Solid oral dosage forms may also include lubricants. Suitable lubricants include stearic acid, magnesium stearate, calcium stearate and talc.

Formulations for oral administration may be microencapsulated, where appropriate. The compositions can also be prepared to prolong or sustain release, for example, by coating or embedding particulate materials in polymers, waxes, and the like.

The compounds disclosed herein can also be coupled with soluble polymers as targeted drug carriers. Such polymers include polyvinylpyrrolidone, pyrrole polymers, polyhydroxypropylmethacrylamidophenol, polyhydroxyethylasparticphenol, or polyvinyloxypolylysine substituted with palmitoyl residues. In addition, the compound of the present invention can be coupled with a class of biodegradable polymers useful for controlled release of drugs, such as polylactic acid, poly $\varepsilon$-caprolactone, polyhydroxybutyric acid, polyorthoesters, polyacetal, polydihydropyran, polycyanoacrylate, and hydrogel cross-linked or amphiphilic block copolymers.

In one embodiment, the present invention relates to a liquid oral dosage form. Oral solutions, such as solutions, syrups and elixirs, can be prepared in unit dosage form to provide a subject with a predetermined amount of a compound or a pharmaceutically acceptable salt thereof. Syrups can be prepared by dissolving the compound of this patent in a suitably flavored aqueous solution, and by using a non-toxic alcoholic vehicle to prepare the dosage form. Suspensions can be formulated by dispersing the compound disclosed in the present patent in nontoxic transporters. Solubilizers and emulsifiers such as ethoxylated isostearol and polyoxyethylenesorbitol, preservatives, flavor additives such as peppermint oil, or other natural sweeteners or saccharin or other artificial sweeteners may also be added.

In one embodiment, the present invention also relates to pharmaceutical composition for parenteral administration. Ingredients suitable for parenteral administration include aqueous and non-aqueous sterile injectable solutions comprising antioxidants, buffers, bacteriostatic agents and solutes to render the preparation isotonic with the blood of the intended recipient; and sterile suspensions may include suspending and thickening agents. The ingredients can be presented in unit-dose or multi-dose containers, such as sealed ampoules and vials, and can be stored under lyophilized condition requiring only the addition of a sterile liquid carrier (such as water for injection) before use. Extemporaneous injection solution and suspension can be prepared from sterile powder, granule and tablet.

## COMBINATION ADMINISTRATION

The compound of formula (**I**), (**I'**), (**I"**), (**II**), (**III**), (**IV**), (**V**), (**VI**), (**II-1**), (**III-1**), (**IV-1**), (**V-1**) or (**VI-1**) and/or pharmaceutically acceptable salts thereof may be used in combination with one or more other active drugs. In specific embodiments, one or more of the compounds of formula (**I**), (**I'**), (**I"**), (**II**), (**III**), (**IV**), (**V**), (**VI**), ( **II-1**), (**III-1**), (**IV-1**), (**V-1**) or (**VI-1**), or one or more of the pharmaceutically acceptable salts of the above compounds, and one or more other active drugs may be co-administered. Other active drugs may be administrated with the compound of formula (**I**), (**I'**), (**I"**), (**II**), (**III**), (**IV**), (**V**), ( **VI**), **(II-1**), (**III-1**), (**IV-1**), (**V-1**) or (**VI-1**) or a pharmaceutically acceptable salt of the above compound as a single dosage form, or other active drug formulations and formulations containing the compound of formula (**I**), (**I'**), (**I"**), (**II**), (**III**), (**IV**), (**V**), (**VI**), **(II-1**), (**III-1**), (**IV-1**), (**V-1**) or ( **VI-1**) or a pharmaceutically acceptable salt of the above drug may be administered alone.

Where appropriate, other active drugs may be administered as pharmaceutically acceptable salts.

The other active drug may be one or more other active drugs selected from STING agonists, antiviral compounds, antigens, adjuvants, anticancer agents, CTLA-4, LAG-3 and PD-1 antagonists, lipids, liposomes, peptides, cytotoxic agent, chemotherapeutic agent, immunomodulators, immune checkpoint inhibitors, vascular growth factor (VEGF) receptor inhibitors, topoisomerase II inhibitors, smoothing inhibitors, alkylating agents, antitumor antibiotics, antimetabolites, retinoic acid and immunomodulators (including but not limited to anticancer vaccines). It is understood that the other active drugs can be provided as a pharmaceutically acceptable salt. It is understood that the description of the other active drugs above may be repeated. It is also understood that these combination regimens need to be optimized, i.e. the combination regimen of the compound of formula (**I**), (**I'**), (**I"**), (**II**), (**III**), ( **IV**), (**V**), (**VI**), **(II-1**), (**III-1**), (**IV-1**), (**V-1**) or ( **VI-1**) or the above pharmaceutically acceptable salt with one or more other active drugs will be determined according to the needs of the individual patient.

The compounds disclosed in the present patent may be used in combination with one or more other active drugs, including, but not limited to, other anticancer drugs for the prevention, treatment, control, amelioration, or reduction of the risk of a particular disease or disorder (e.g., cell proliferation disorder). In one embodiment, the compounds disclosed in this patent are used in combination with one or more other anticancer drugs for the prevention, treatment, control amelioration or reduction of the risk of a particular disease or disease, wherein the compounds disclosed in this patent are effective, so that other active drugs can be administered simultaneously or sequentially with the compounds disclosed in this patent by a specific mode of administration and dosage.

When the compounds disclosed in this patent are administered simultaneously with one or more other active drugs, such pharmaceutical compositions that contain other active drugs in addition to the compounds disclosed herein are also within the scope of protection. Therefore, the pharmaceutical compositions disclosed in this patent contain, in addition to the active compounds of this patent, one or more other active drugs. Also disclosed are the compounds disclosed herein. The compounds disclosed in this patent may be administered simultaneously with or before or after one or more other active drugs. The compounds disclosed in the present invention can be administered alone by the same or different modes of administration as other active drugs, and can also be administered simultaneously with pharmaceutical compositions composed of other active drugs.

The pharmaceutical composition disclosed in this patent can either be one comprising the compound of formula (I), (I'), (I"), (II), (III), (IV), (V), ( VI), (II-1), (III-1), (IV-1), (V-1) or (VI-1) or a pharmaceutically acceptable salt of the above compound, and one or more other active drugs; or a pharmaceutical composition comprising the compound of formula (I), (I'), (I"), (II), (III), (IV), (V), (VI), (II-1), (III-1), (IV-1), (V-1) or (VI-1) or a pharmaceutically acceptable salt of the above compound, and a separate pharmaceutical composition of one or more other active drugs which can be in kit form or any other forms are administered simultaneously or on a separate schedule for independent administrations.

The weight ratio of the compound of formula (I), (I'), (I"), (II), (III), (IV), (V), (VI), (II-1), (III-1), (IV-1), (V-1)or (VI-1) or a pharmaceutically acceptable salt of the above compound and the other active drugs is variable and will depend on the therapeutically effective dose of the individual drug. Typically, a therapeutically effective dose of each drug will be used. Combinations of the compounds disclosed in this patent with other active agents should generally be within the above weight ranges, but in each case a therapeutically effective dose of each active agent should be used. In such combinations, the compounds disclosed herein and the other active drugs may be administered separately or simultaneously. In addition, the administration of a single drug can be administrated before, during, or after the administration of the other drugs.

In one embodiment, the present invention provides a composition comprising the compound of formula (I), (I'), (I"), (II), (III), (IV), (V), ( VI), (II-1), (III-1), (IV-1), (V-1)or (VI-1) or a pharmaceutically acceptable salt of the above compound, and at least one other active drug, as a combined preparation for simultaneous, separate or sequential use. In one embodiment, the therapy is for the treatment of cell proliferation disorders, such as cancer.

In one embodiment, the present invention provides a kit comprising two or more separate pharmaceutical compositions, at least one of which contains the compound of formula (I), (I'), (I"), (II), (III), (IV), (V), ( VI), (II-1), (III-1), (IV-1), (V-1)or (VI-1) or a pharmaceutically acceptable salt of the above compound. In particular embodiments, the kit includes a device for retaining the composition separately, such as a container, bottle, or oil bag. An example of such a kit is a blister pack, typically used for the packaging of tablets, capsules, etc.

The kits of the present invention can be used for administration of different dosage forms, such as oral and

parenteral administration, for administration of separate pharmaceutical compositions at different dosage intervals, or for titration of separate pharmaceutical composition between each other. For ease of compliance, such kits usually contain instructions for administration.

The present invention discloses the use of the compound of formula (**I**), (**I'**), (**I"**), (**II**), (**III**), (**IV**), (**V**), (**VI**), **(II-1**), (**III-1**), (**IV-1**), (**V-1**) or (**VI-1**) or a pharmaceutically acceptable salt of the above compound for the treatment of cell proliferation disorders, and the method of use of which is administered in combination with another active drug. The present invention also provides other active drugs for the treatment of cell proliferation disorders, and the method of use of which is co-administered with the compound of formula (**I**), (**I'**), (**I"**), (**II**), (**III**), (**IV**), (**V**), (**VI**), (**II-1**), (**III-1**), (**IV-1**), (**V-1**) or (**VI-1**) or a pharmaceutically acceptable salt of the above compound.

The present invention also provides the use of the compound of formula (**I**), (**I'**), (**I"**), (**II**), (**III**), (**IV**), (**V**), (**VI**), (**II-1**), (**III-1**), (**IV-1**), (**V-1**) or (**VI-1**) or a pharmaceutically acceptable salt of the above compound for the treatment of cell proliferation disorders, wherein the patient has been previously (e.g., within 24 hours) treated with another active drug. The present invention also provides other active drugs for the treatment of cell proliferation disorders, wherein the patient has previously (e.g., within 24 hours) been treated with the compound of formula (**I**), (**I'**), (**I"**), (**II**), (**III**), (**IV**), (**V**), (**VI**), (**II-1**), (**III-1**), (**IV-1**), (**V-1**) or (**VI-1**) or a pharmaceutically acceptable salt of the above compound. The second drug can be administered one week, several weeks, one month, or several months after administration.

STING agonists can be combined with the compound of formula (**I**), (**I'**), (**I"**), (**II**), (**III**), (**IV**), (**V**), (**VI**), (**II-1**), (**III-1**), (**IV-1**), (**V-1**) or (**VI-1**) or a pharmaceutically acceptable salt of the above compound, and the STING agonists referred to in the present invention include but are not limited to cyclic dinucleotide compounds.

Antiviral compounds can be combined with the compound of formula (**I**), (**I'**), (**I"**), (**II**), (**III**), (**IV**), (**V**), (**VI**), (**II-1**), (**III-1**), (**IV-1**), (**V-1**) or ( **VI-1)** or a pharmaceutically acceptable salt of the above compound, and the antiviral compounds referred to in the present invention include hepatitis B virus (HBV) inhibitors, hepatitis C virus (HCV) protease inhibitors, HCV polymerase inhibitors, HCV NS4A inhibitors, HCV NS5A inhibitors, HCVNS5B inhibitors, human papillomavirus (HPV) inhibitors, nasopharyngeal carcinoma-associated EB virus (EBV) inhibitors and human immunodeficiency virus (HIV) inhibitors. These antiviral compounds may be pharmaceutically acceptable salts if necessary.

The compound of formula (**I**), (**I'**), (**I"**), (**II**), (**III**), (**IV**), (**V**), (**VI**), (**II-1**), (**III-1**), (**IV-1**), (**V-1**) or (**VI-1**) or a pharmaceutically acceptable salt of the above compound may be co-administrated with antigens and adjuvants including B7 co-stimulatory molecule interleukin-2, interferon y, GM-CSF, CTLA-4 antagonist, OX-40/OX-40 ligand, CD40/CD40 ligand , salnacedin, levamisole, vaccinia virus, Bacille Calmette Guerin (BCG), liposomes, Alum, Freund complete or incomplete adjuvant, detoxification endotoxin, mineral oil, surfactants such as lecithin, pluronic polyols, polyanionic, peptide and oil or hydrocarbon emulsions. Adjuvants, such as aluminum hydroxide or aluminum phosphate, are added to enhance the vaccine's ability to trigger, enhance or prolong an immune response. Other substances, such as cytokines, chemokines, and bacterial nucleic acid sequences (such as CpG, TLR9 agonists), and other TLR 2, TLR 4, TLR 5, TLR 7, TLR 8, TLR 9 agonists (including lipoproteins, LPS, monophosphoryl lipid A, lipoteichoic acid, imiquimod, resimod), retinoic acid-inducible gene I (RIG-I) agonists such as poly I:C, these adjuvants are used alone or in combination which are all potential adjuvants. Where appropriate, such antigens and adjuvants may be pharmaceutically acceptable salts.

The CLTA4 and PD-1 pathways are important negative regulators of immune responses. Activated T cells upregulate CTLA-4, which binds antigen-presenting cells and inhibits T cell activation, IL-2 gene expression, and T cell proliferation, antitumor effects are observed in mouse models of colon cancer, metastatic prostate cancer, and metastatic melanoma. PD-1 binds to active T cells and inhibits T cell activation, and Pd-1 antagonists have also been shown to have antitumor effects. CTLA-4 and PD-1 antagonists, including ipilimumab, tremelimumab, nivolumab, pembrolizumab, CT-011, AMP-224, and MDX-1106, may be co-administrated with the compound of formula (**I**), (**I'**), (**I"**), (**II**), (**III**), (**IV**), (**V**), (**VI**), (**II-1**), (**III-1**), (**IV-1**), (**V-1**) or ( **VI-1**) or a pharmaceutically acceptable salt of the above compound.

"PD-1 antagonist" or "PD-1 pathway antagonist" refers to any compound or biological macromolecule that prevents the binding of PD-L1 expressed on cancer cells to PD-1 expressed on immune cells (T cells, B cells or NKT cells), and also tend to prevent the binding of PD-L2 expressed on cancer cells to PD-1 expressed on immune cells. Names with the same meaning as PD-1 also include PDCD1, PD1, CD279 and SLEB2. Names with the same meaning as PD-L1 also include PDCD1L1, PDL1, B7H1, B7-4, CD274 and B7-H. Names with the same meaning as PD-L2 also include PDCD1L2, PDL2 B7-DC, Btdc and CD273. In any of the therapeutic methods, medicaments and uses of the present invention for treating the human body, PD-1 antagonist blocks the binding of human PD-L1 to human PD-1, in particular, blocks the binding of human PD-L1 and PD-L2 to human PD-1 combination. Human PD-1 amino acid sequences can be found in NCBI Locus No.: NP_005009. Human PD-L1 and PD-L2 amino acid sequences can be found in NCBI Locus No.: NP_054862 and NP_079515, respectively.

PD-1 antagonists include a monoclonal antibody, or antigen binding fragment thereof, which specifically binds to PD-1 or PD-L1, and preferably specifically binds to human PD-1 or human PD-L1 and can be used in combination with any of the methods, medicaments and uses of the present invention. The monoclonal antibody may be a human antibody, a humanized antibody or a chimeric antibody, and may include a human constant region. In some embodiments, the human constant region is selected from IgG1, IgG2, IgG3 or IgG4 constant regions, and in preferred embodiments,

the human constant region is an IgG1 or IgG4 constant region. In some embodiments, the antigen-binding fragment is selected from Fab, Fab'-SH, $F(ab')_2$, scFv, or Fv fragments.

Examples of monoclonal antibodies that bind to human PD-1, and are administered in combination with the methods of treatment, medicaments and uses disclosed herein are described in US7488802, US7521051, US8008449, US8354509, US8168757, WO2004/004771, WO2004 /072286 and WO2004/056875.

Examples of monoclonal antibodies that bind to human PD-L1 and can be administered in combination with the methods of treatment, medicaments and uses of the present invention are described in patents WO2013/019906, WO2010/077634 A1 and US8383796, which are specific against human PD-L1 monoclonal antibodies include MPDL3280A, BMS-936559, MEDI4736 and MSB0010718C. WO2013/019906 also discloses an anti-PD-Ll antibody comprising a heavy chain region (SEQ ID NO: 24) and a light chain variable region (SEQ ID NO: 21).

Other PD-1 antagonists that may be administered in combination with any of the methods of treatment, medicaments and uses disclosed in this patent include immune adhesives that specifically bind to PD-1 or PD-L1, and preferably specifically bind to human PD-1 or human PD-L1, for example, fusion proteins containing the extracellular segment, or PD-1 binding portions of PD-L1 or PD-L2, are fused to a constant region of an immunoglobulin molecule, such as the Fc region. Examples of immunoadhesion molecules that specifically bind PD-1 are described in patents WO2010/027827 and WO2011/066342. Specific fusion proteins of PD-1 antagonists that can be used in combination with the therapeutic methods, medicaments and uses of the present invention include AMP-224 (also known as B7-DCIg), which is a PD-L2-FC fusion protein, and binds to human PD-1.

The cytotoxic agents can be combined with the compound of formula (I), (I'), (I"), (II), (III), (IV), (V), (VI), (II-1), (III-1), (IV-1), (V-1) or (VI-1) or a pharmaceutically acceptable salt of the above compound including but not limited to arsenic trioxide (trade name: TRISENOX®), asparaginase (also known as L-asparaginase, and Erwinia L-asparaginase, trade name ELSARAR® and KIDROLASE®).

The chemotherapy drugs that can be combined with the compound of formula (I), (I'), (I"), (II), (III), (IV), (V), ( VI), (II-1), (III-1), (IV-1), (V-1)or (VI-1) or a pharmaceutically acceptable salt of the above compound includes abiraterone acetate, altretamine, dehydrovinblastine, oxaliplatin, bexarotene, bicalutamide, BMS 184476, 2,3,4,5,6 -pentafluoro-N-(3-fluoro-4-methoxyphenyl)benzenesulfonamide, bleomycin, N,N-dimethyl-L-valine-L-valine-N- methyl-L-valine-L-prolyl-1-proline-tert-butylamine, tumor necrosis factor, gibberellin, chloropyrilene, cyclophosphamide, docetaxel, carboplatin, carmustine, cisplatin, nostoxanthin, cytarabine, dacarbazine, dactinomycin, daunorubicin, decitabine, dorastatin, doxorubicin, etoposide, 5-fluorouracil, finasteride, flutamide, hydroxyurea, ifosfamide, temozolomide, lomustine, rhodamine, MDV3100, allyl cyanamide (nitrogen mustard), melphalan, mivolbrin isothiocyanate, rhizomycin, streptomycin, mitomycin, methotrexate, taxanes, nilutamide, nivacizumab, onapristone, pembrolizumab, prednisone, procarbazine, RPR10981, estramustine phosphate, tamoxifen, tasonermin, paclitaxel, retinoic acid, vinblastine, vincristine, vindesine sulfate, and vinorelbine. Where appropriate, these chemotherapeutic agents may be administered in the form of pharmaceutically acceptable salts.

Examples of vascular endothelial growth factor (VEGF) receptor inhibitors include but are not limited to bevacizumab (trade name: AVASTIN®), axitinib (patent number: WO01/002369), Brivanib, motesanib (patent number: WO02/068470), pasireotide (also known as SO 230, patent number: WO02/010192) and sorafenib (trade name: NEXAVAR®). Where appropriate, these inhibitors can be administered in the form of pharmaceutically acceptable salts.

Examples of topoisomerase II inhibitors include but are not limited to etoposide (also known as VP-16 and etoposide phosphate, trade name: TOPOSAR®, VEPESID® and ETOPOPHOS®), teniposide (also known as VM-26, trade name: VUMON®). Where appropriate, these inhibitors can be administered in the form of pharmaceutically acceptable salts.

Examples of alkylating agents include but are not limited to 5-azacytidine (trade name: VIDAZA®), decitabine (trade name: DECOGEN®), temozolomide (trade name: TEMODAR® and TEMODAL®), dactinomycin (also known as actinomycin-D, trade name: COSMEGEN®), melphalan (trade name: ALKERAN®), altretamine (also known as hexamethylmelamine, trade name: HEXALEN®), carmustine (trade name: BCNU®), bendamustine (trade name: TREANDA®), busulfan (trade name: BUSULFEX® and MYLERAN®), carboplatin (trade name: PARAPLATIN®) , lomustine (also known as CCNU, trade name: CEENU®), cisplatin (also known as CDDP, trade name: PLATINOL® and PLATINOL®-AQ), chloramphenicol (trade name: LEUKERAN®), cyclophosphamide (trade names: CYTOXAN® and NEOSAR®), dacarbazine (also known as DTIC, DIC, trade name: DTIC-DOME®), ifosfamide (trade name: IFEX®), methylbenzyl hydrazine (trade name: MATULANE®), nitrogen mustard (trade name: MUSTARGEN®), streptomycin (trade name: ZANOSAR®) and cetepine (also known as thiophosphoramide, trade name: THIOPLEX ®). Where appropriate, these alkylating agents can be administered in the form of pharmaceutically acceptable salts.

Examples of antitumor antibiotics include but are not limited to doxorubicin (trade name: ADRIMYCIN® and RUBEX®), bleomycin (trade name: ENOXANE®), daunorubicin (trade name: CERUBIDINE®), daunorubicin liposome (trade name: DAUNOXOME®), mitoxantrone (also known as DHAD, trade name: NOVANTRON®), epirubicin (trade name: ELLENCE" TM), idamycin (also known as demethoxydaunorubicin, trade name: DAMYCIN® and IDAMYCIN PFS®) and mitomycin C (trade name: MUTAMYCIN®). Where appropriate, these antitumor antibiotics can be administered in the form of pharmaceutically acceptable salts.

Examples of antimetabolites include, but are not limited to, 2-chlorodeoxyadenosine (trade name: LEUSTATIN®), 5-fluorouracil (trade name: ADRUCIL®), 6-thioguanine (trade name: PURINETHOL ®), pemetrexed (trade name: ALIMTA®), cytarabine (also known as Ara-C, trade name: CYTOSAR-U ®), cytarabine liposome (also known as Liposome Ara-c, trade name: DEPOCYT™), decitabine (trade name: DACEGEN®), hydroxyurea (trade name: HYDREA®, DROXIA™ and MYLOCEL™ ), fludarabine (trade name: FLUDARA®), floxuridine (trade name: FUDR®), cladribine (also known as 2-chlorodeoxyadenosine, trade name: LEUSTATIN™), methotrexate (trade name: RHEUMATREX® and TREXALL™) and pentostatin (trade name: NIPENT®). Where appropriate, these antimetabolites can be administered in the form of pharmaceutically acceptable salts.

Examples of retinoic acids include but are not limited to alitretinoin (trade name: PANRETIN®), retinoic acid (also known as all-trans retinoic acid, trade name: VESANOID®), isotretinoin (also known as 13- c/s-retinoic acid, trade name: ACCUTANE®, AMNESTEEM®, CLARAVIS®, CLARUS®, DECUTAN®, ISOTANE®, IZOTECH®, ORATANE®, ISOTRET® and SOTRET ®) and bexarotene (trade name: TARGRITIN®). Where appropriate, these compounds may be administered in the form of pharmaceutically acceptable salts.

**The main advantages of the present invention:**

1. The five-membered heterocyclic oxocarboxylic acid compound of the present invention has a novel structure, and has the activity of inducing the secretion of type I interferon which can be used for anti-tumor and anti-infective diseases.
2. The five-membered heterocyclic oxocarboxylic acid compound of the present invention can activate the immune system of the human body, thereby having a therapeutic effect on diseases related to immune response.
3. Compared with the cyclic dinucleotide-based STING agonists, the five-membered heterocyclic oxocarboxylic acid compound of the present invention has the advantages of simple structure, simple synthesis, good metabolic stability and the like.

The detailed experimental procedures in the following examples will further illustrate the present invention. These example compounds are drawn in neutral form in the Examples below. In some cases, compounds are isolated as salts depending on the method used for final purification and/or the intrinsic molecular properties. These examples are used only to illustrate the invention and are not intended to limit the scope of this patent in any way. Unless otherwise defined or indicated, all professional and scientific terms used herein have the same meaning as is familiar to those skilled in the art.

**Example 1** 3-(4-(1H-indol-3-yl)thiophen-2-yl)-3-oxopropionic acid (**I-1**)

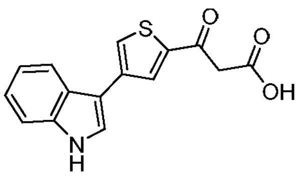

Step 1: 2-acetyl-4,5-dibromothiophene (**1-1**)

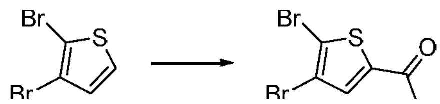

A mixture of 1 g of 2,3-dibromothiophene and 0.36 g of acetyl chloride was dissolved in 10 mL of dichloromethane, and 0.72 g of aluminum trichloride was added in five batches under ice bath conditions. The mixture was reacted for 2 hours under the ice bath, and TLC showed that the reaction was completed. The reaction solution was poured into dilute

hydrochloric acid, extracted with dichloromethane, and the organic phase was dried over anhydrous sodium sulfate, concentrated to dryness, and the residue was subjected to column chromatography with ethyl acetate:petroleum ether=1:100 to obtain 0.8 g of an orange-yellow solid ( **1-1**) with a yield of 68%. $^1$H NMR (400 MHz, $CDCl_3$) $\delta$ 7.47 (s, 1H), 2.51 (s, 3H).

Step 2: methyl 3-(4,5-dibromothiophen-2-yl)-3-oxopropionate(**1-2**)

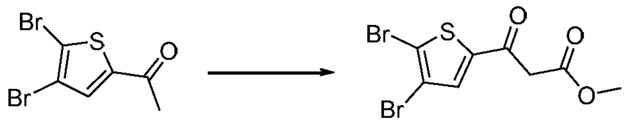

282 mg of sodium hydrogen was dissolved in 15 mL of redistilled tetrahydrofuran, and 400 mg of compound **1-1** was added under ice bath under the protection of argon. After stirring for 30 minutes under an ice bath, dimethyl carbonate in tetrahydrofuran (380 mg/5 mL) was slowly added dropwise. After addition, the mixture was refluxed at 70°C for 2 hours under an oil bath, and TLC showed that the reaction was completed. The reaction solution was poured into dilute hydrochloric acid, extracted with ethyl acetate, dried over anhydrous sodium sulfate, concentrated to dryness, and the residue was subjected to column chromatography with ethyl acetate: petroleum ether = 1:100 to obtain 295 mg of yellow oily liquid (**1-2**) with a yield of 68%. $^1$H NMR (400 MHz, $CDCl_3$) $\delta$ 7.52 (s, 1H), 3.86(s, 2H), 3.77 (s, 3H).

Step 3: methyl 3-(4-bromothiophen-2-yl)-3-oxopropanoate (**1-3**)

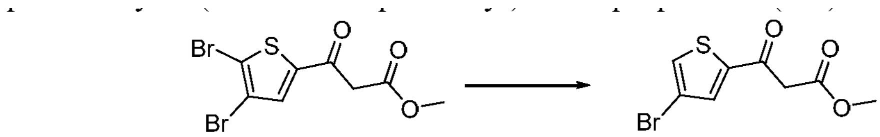

A mixture of 250 mg of compound **1-2,** 50 mg of zinc powder, 1.5 mL of water and 0.5 mL of glacial acetic acid was heated to reflux for 2 hours. After the reaction solution was cooled to room temperature, diluted with water, extracted with dichloromethane, and the organic phase was concentrated to dryness, and purified by preparative thin layer chromatography (ethyl acetate : petroleum ether=5:95) to obtain 127 mg of yellow oil (**1-3**) with a yield of 66%. $^1$H NMR (400 MHz, $CDCl_3$) $\delta$ 7.63 (s, 1H), 7.60 (s, 1H), 3.91(s, 2H), 3.77 (s, 3H).

Step 4: methyl 3-(4-(1-Boc-1H-indol-3-yl)thiophen-2-yl)-3-oxopropanoate (**1-4**)

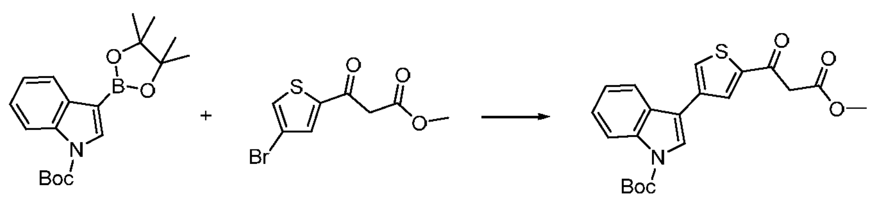

A mixture of 144 mg of 3-(4,4,5,5-tetramethyl-1,3,2-dioxaborolan-2-yl)-1-Boc-1H-indole, 100 mg of compound **1-3,** 14 mg [1,1-bis(diphenylphosphino)ferrocene]dichloride palladium, 310 mg of cesium carbonate, 1 mL tetrahydrofuran and 1 mL deionized water was stirred overnight under an oil bath at 70°C under the protection of argon. The reaction solution was concentrated to dryness, and purified by preparative thin layer chromatography (ethyl acetate: petroleum ether = 1:10) to obtain 54 mg of a yellow oily liquid (**1-4**) with a yield of 35%. H NMR (400 MHz, CDCl $_3$) $\delta$ 8.22 (d, *J* = 7.7 Hz, 1H), 8.00 (d, *J* = 1.3 Hz, 1H), 7.85 (d, " J = 1.3 Hz, 1H), 7.73-8.80 (m, 2H), 7.40 (t, *J* = 7.2 Hz, 1H), 7.33 (t, *J* = 7.6 Hz, 1H), 4.00 (s, 2H), 3.39 (s, 3H), 1.72 (s, 9H).

Step 5: 3-(4-(1H-indol-3-yl)thiophen-2-yl)-3-oxopropionic acid (1-1)

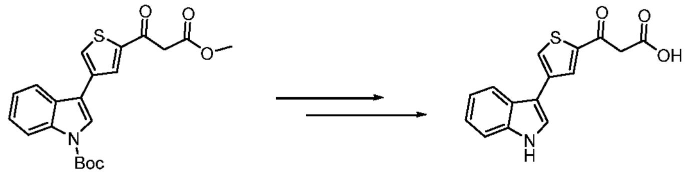

A mixture of 40 mg of compound **1-4,** 0.35 mL of dichloromethane and 0.08 mL of trifluoroacetic acid was stirred at room temperature for 6 hours. The reaction solution was adjusted to pH=7 with saturated sodium bicarbonate solution, extracted with dichloromethane, and the organic phase was concentrated to dryness, 0.5 mL of tetrahydrofuran, 0.5 mL of water and 0.11 mL of 1M lithium hydroxide solution were added, stirred at room temperature for 2 hours. The reaction solution was adjusted pH to 1-3 with 2M hydrochloric acid, and then concentrated to dryness, purified by preparative thin layer chromatography (dichloromethane: methanol = 30 : 1 (1% acetic acid)) to obtain 16 mg of a yellow solid (**I-1**) with a two-step yield of 56%. $^1$H NMR (400 MHz, DMSO-$d_6$) $\delta$ 11.44 (s, 1H), 8.43 (d, $J$ = 1.4 Hz, 1H), 8.21 (d, $J$ = 1.4 Hz, 1H), 7.97 (d, $J$ = 7.5 Hz, 1H ), 7.85 (d, $J$ = 2.6 Hz, 1H), 7.46 (d, $J$ = 7.8 Hz, 1H),7.10-7.21 (m, 2H), 4.26 (s, 2H).

**Example 2** 3-(4-(6-fluoro-1H-indol-3-yl)thiophen-2-yl)-3-oxopropionic acid (**I-2**)

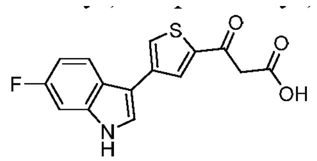

Step 1: methyl 3-(4-(4,4,5,5-tetramethyl-1,3,2-dioxaboran-2-yl)thiophen-2-yl)-3-oxopropanoate (**2-1**)

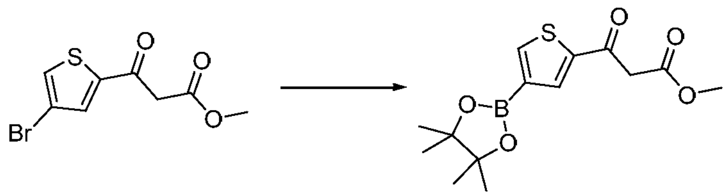

A mixture of 100 mg of compound **1-3,** 106 mg of pinacol biboronate, 94 mg of potassium acetate, 14 mg of [1,1-bis(diphenylphosphino)ferrocene]palladium dichloride and 1 mL of 1,4-dioxane was stirred overnight at 80°C under argon protection. The reaction solution was concentrated to dryness, and subjected to column chromatography with ethyl acetate: petroleum ether=5:95 to obtain 94 mg of a yellow solid (2-1) with a yield of 79%. $^1$H NMR (400 MHz, $CDCl_3$) $\delta$ 8.20 (d, $J$= 0.9 Hz, 1H), 7.97 (d, $J$= 0.9 Hz, 1H), 3.95 (s, 2H), 3.76 (s, 3H), 1.34 (s, 12H).

Step 2: methyl 3-(4-(1-Boc-6-fluoro-1H-indol-3-yl)thiophen-2-yl)-3-oxopropanoate (**2-2**)

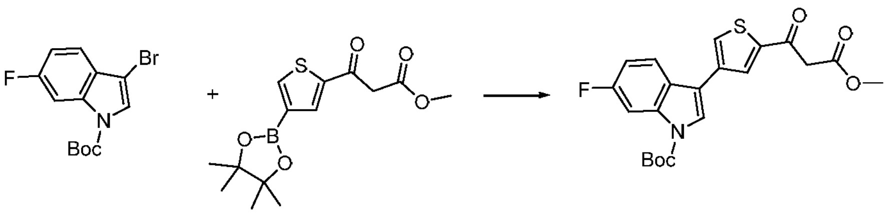

The preparation method of methyl 3-(4-(1-Boc-6-fluoro-1H-indol-3-yl)thiophen-2-yl)-3-oxopropanoate (**2-2**) was the same as that of compound **1-4,** and a pale yellow oily liquid was obtained with a yield of 46%. $^{1}$H NMR (400 MHz, $CDCl_3$) $\delta$7.88-8.02 (m, 2H), 7.82 (s, 1H), 7.73 (s, 1H), 7.63-7.70 (m, 1H), 7.08 (t, *J*= 8.9 Hz, 1H), 3.99 (s, 2H), 3.78 (s, 3H), 1.70 (s, 9H).

Step 3: 3-4-6-fluoro-1H-indol-3-yl)thiophen-2-yl)-3-oxopropionic acid (**I-2** )

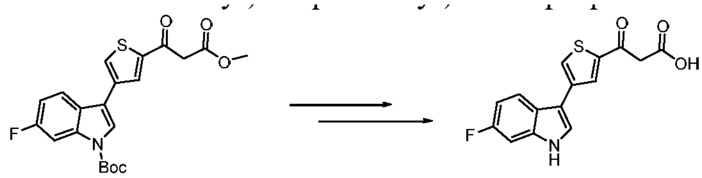

The preparation of 3-(4-(6-fluoro-1H-indol-3-yl)thiophen-2-yl)-3-oxopropionic acid (1-2) was the same as that of compound **I-1**, and a yellow solid was obtained with a yield of 84%. $^{1}$H NMR (400 MHz, DMSO-$d_6$) $\delta$ 11.50 (s, 1H), 8.42 (d, *J* = 1.2 Hz, 1H), 8.22 (d, *J* = 1.2 Hz, 1H), 7.96 (dd, *J* = 5.3, 8.7 Hz, 1H ), 7.85 (d, *J* = 2.5 Hz, 1H ), 7.24 (dd, *J* = 2.3, 10.0 Hz, 1H ), 6.99 (td, *J* = 2.4, 9.5 Hz, 1H), 4.26 (s, 2H).

**Example 3** 3-(4-(7-fluoro-1H-indol-3-yl)thiophen-2-yl)-3-oxopropionic acid (**I-3**)

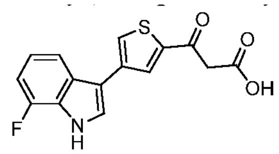

Step 1: methyl 3-(4-(7-fluoro-1-Boc-1H-indol-3-yl)thiophen-2-yl)-3-oxopropanoate ( **3-1**)

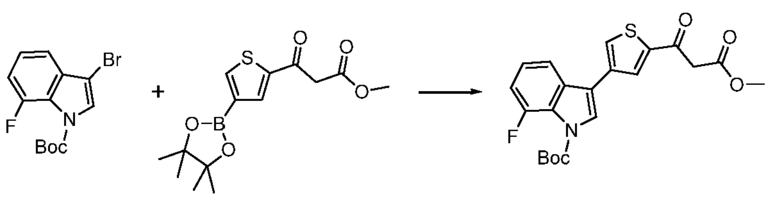

The preparation of methyl 3-(4-(7-fluoro-1-Boc-1H-indol-3-yl)thiophen-2-yl)-3-oxopropanoate (**3-1**) was the same as that of compound **1**-4, and a yellow solid was obtained with a yield of 47%. $^{1}$H NMR (400 MHz, $CDCl_3$) $\delta$7.98 (s, 1H), 7.80-7.87 (m, 2H), 7.54 (d, *J*= 7.9 Hz, 1H), 7.23-7.34 (m, 1H), 7.13 (dd, *J* = 7.9, 12.4 Hz, 1H), 4.01 (s, 2H), 3.81 (s, 3H), 1.70 (s, 9H).

Step 2: 3-(4-(7-fluoro-1H-indol-3-yl)thiophen-2-yl)-3-oxopropionic acid (**I-3**)

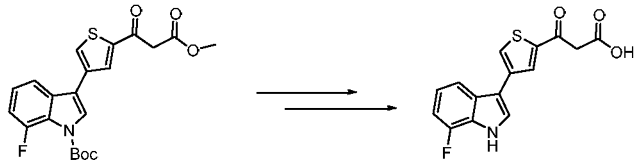

The preparation of 3-(4-(7-fluoro-1H-indol-3-yl)thiophen-2-yl)-3-oxopropanoic acid (**I-3**) was the same as that of compound **I-1**, and a yellow solid was obtained with a yield of 37%. $^1$H NMR (400 MHz, DMSO-$d_6$) $\delta$ 11.95(s, 1H), 8.45 (s, 1H), 8.25 (s, 1H), 7.93 (d, *J* = 2.5 Hz, 1H), 7.79 (d, *J* = 7.9 Hz, 1H), 6.96-7.16 (m, 2H), 4.26 (s, 2H).

**Example 4** 3-(4-(5-fluoro-1H-indol-3-yl)thiophen-2-yl)-3-oxopropionic acid (**I-4**)

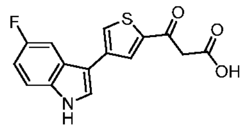

Step 1: methyl 3-(4-(5-fluoro-1-Boc-1H-indol-3-yl)thiophen-2-yl)-3-oxopropanoate ( **4-1** )

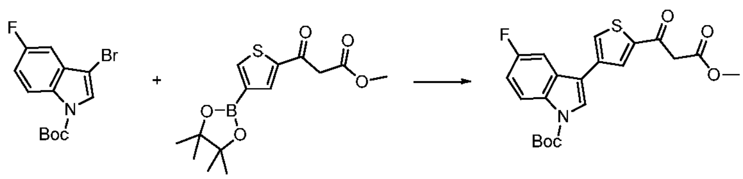

The preparation of methyl 3-(4-(5-fluoro-1-Boc-1H-indol-3-yl)thiophen-2-yl)-3-oxopropanoate (**4-1**) was the same as that of compound **1-4,** and a yellow oily liquid was obtained with a yield of 42%. $^1$H NMR (400 MHz, $CDCl_3$) $\delta$ 8.17 (s, 1H), 7.96 (d, *J*= 1.3 Hz, 1H), 7.76-7.83 (m, 2H), 7.40 (dd, *J* = 2.5, 8.9 Hz, 1H), 7.12 (td, *J*= 2.6, 9.1 Hz, 1H), 4.00 (s, 2H), 3.79 (s, 3H), 1.70 (s, 9H).

Step 2: 3-(4-(5-fluoro-1H-indol-3-yl)thiophen-2-yl)-3-oxopropionic acid (**I-4**)

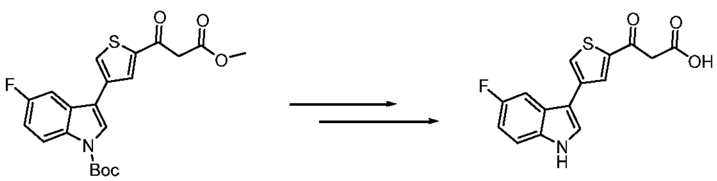

The preparation of 3-(4-(5-fluoro-1H-indol-3-yl)thiophen-2-yl)-3-oxopropanoic acid (**I-4**) was the same as that of compound **I-1**, and a yellow solid was obtained with a yield of 78%. $^1$H NMR (400 MHz, DMSO-$d_6$) $\delta$ 11.55 (s, 1H), 8.42 (s, 1H ), 8.23 (s, 1H), 7.94 (d, *J* = 2.3 Hz, 1H), 7.75 (d, *J* = 8.6 Hz, 1H), 7.46 (dd, *J* = 4.7, 8.8 Hz, 1H), 7.46 (td, *J* = 2.1, 9.3 Hz, 1H), 4.27 (s, 2H).

**Example 5** 3-(4-(4-fluoro-1H-indol-3-yl)thiophen-2-yl)-3-oxopropionic acid (**I-5**)

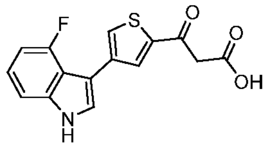

Step 1: methyl 3-(4-(4-fluoro-1-Boc-1H-indol-3-yl)thiophen-2-yl)-3-oxopropanoate (**5-1**)

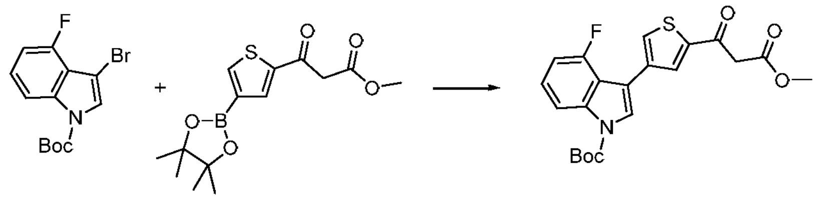

The preparation of methyl 3-(4-(4-fluoro-1-Boc-1H-indol-3-yl)thiophen-2-yl)-3-oxopropanoate (**5-1**) was the same as that of compound **1-4,** and a pale yellow oily liquid was obtained with a yield of 32%. $^1$H NMR (400 MHz, $CDCl_3$) $\delta$ 8.04 (d, *J*= 8.4 Hz, 1H), 7.97 (s, 1H), 7.68 (s, 1H), 7.31 (td, *J* = 5.3, 8.2 Hz, 1H), 6.99 (dd, *J* = 8.0, 11.3 Hz, 1H), 3.99 (s, 2H), 3.78 (s, 3H), 1.70 (s, 9H).

Step 2: 3-(4-(4-fluoro-1H-indol-3-yl)thiophen-2-yl)-3-oxopropionic acid (**I-5**)

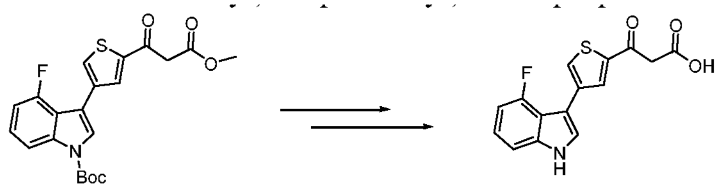

The preparation of 3-(4-(4-fluoro-1H-indol-3-yl)thiophen-2-yl)-3-oxopropanoic acid (**I-5**) was the same as that of compound **I-1**, and a yellow solid was obtained with a yield of 42%. $^1$H NMR (400 MHz, DMSO-$d_6$) $\delta$ 11.78 (s, 1H), 8.25 (s, 1H ), 7.95 (s, 1H), 7.81 (t, *J* = 4.8 Hz, 1H), 7.29 (d, *J* = 8.1 Hz, 1H), 7.07-7.19 (m, 1H), 6.86 (dd, *J* = 7.8, 12.2 Hz, 1H), 4.28 (s, 2H).

**Example 6** 2-methyl-3-(4-(7-fluoro-1H-indol-3-yl)thiophen-2-yl)-3-oxopropionic acid (**I-6**)

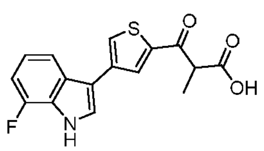

Step 1: 1-(4,5-dibromothiophen-2-yl)propan-1-one (**6-1**)

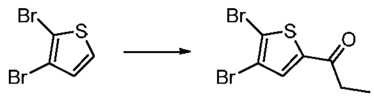

The preparation of 1-(4,5-dibromothiophen-2-yl)propan-1-one (**6-1**) was the same as that of compound **1-1,** and a yellow oily liquid was obtained with a yield of 81%. $^1$H NMR (400 MHz, $CDCl_3$) $\delta$7.50 (s, 1H), 2.88 (q, *J* = 7.3 Hz, 2H), 1.24 (t, *J* = 7.2 Hz, 3H).

Step 2: methyl 2-methyl-3-(4,5-dibromothiophen-2-yl)-3-oxopropionate (**6-2**)

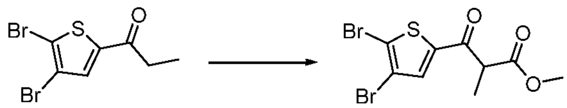

The preparation of methyl 3-(4,5-dibromothiophen-2-yl)-2-methyl-3-oxopropionate (**6-2**) was the same as that of compound **1-2, and** an orange-red oily liquid was obtained with a yield of 56%. $^1$H NMR (400 MHz, $CDCl_3$) $\delta$7.55 (s, 1H), 4.13 (q, *J*= 7.1 Hz, 2H), 3.72 (s, H), 1.49 (d, *J* = 7.0 Hz, 3H).

Step 2: methyl 2-methyl-3-(4-bromothiophen-2-yl)-3-oxopropionate (**6-3**)

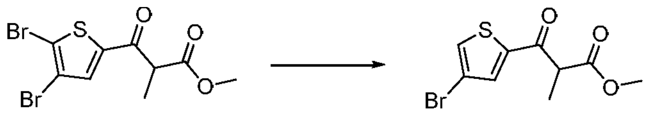

The preparation of methyl 3-(4-bromothiophen-2-yl)-2-methyl-3-oxopropionate (**6-3**) was the same as that of compound **1-3,** and a yellow oily liquid was obtained with a yield of 74 %. $^1$H NMR (400 MHz, $CDCl_3$) $\delta$7.60 (s, 1H), 7.52 (s, 1H), 4.13 (q, *J* = 7.0 Hz, 1H), 3.66 (s, 3H), 1.44 (t, *J* = 7.0 Hz, 3H).

Step 4: methyl 2-methyl-3-(4-(7-fluoro-1-Boc-1H-indol-3-yl)thiophen-2-yl)-3-oxopropanoate (**6-4**)

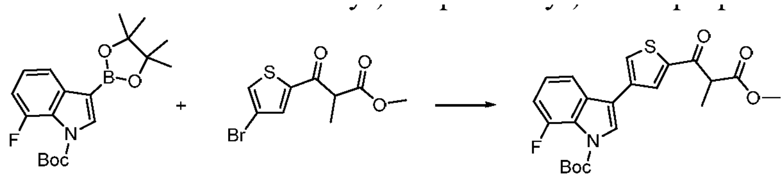

The preparation of methyl 2-methyl-3-(4-(7-fluoro-1-Boc-1H-indol-3-yl)thiophen-2-yl)-3-oxopropanoate (**6-4**) was the same as that of compound **1-4** with a yield of 36%. $^1$H NMR (400 MHz, $CDCl_3$) $\delta$8.17 (s, 1H), 7.98 (s, 1H), 7.70-7.86 (m, 2H), 7.42 (d, *J* = 8.9 Hz, 1H), 7.11 (s, 1H), 3.73 (s, 3H), 3.33 (q, *J* = 7.0 Hz, 1H), 1.70 (s, 9H), 1.56 (t, *J* = 7.0Hz, 3H).

Step ⑤: 2-methyl-3-(4-(7-fluoro-1H-indol-3-yl)thiophen-2-yl)-3-oxopropionic acid (**I-6**)

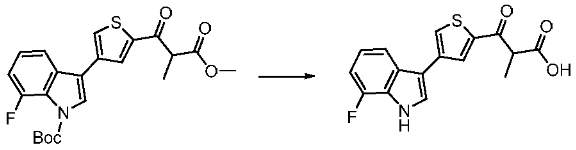

The preparation of 3-(4-(7-fluoro-1H-indol-3-yl)thiophen-2-yl)-2-methyl-3-oxopropionic acid (**I-6**) was the same as that of compound I-1, and a yellow solid was obtained with a yield of 41%. $^1$H NMR (400 MHz, DMSO-$d_6$) $\delta$ 11.92(s, 1H), 8.37 (s, 1H), 8.15 (s, 1H), 7.96 (d, $J$ = 2.4 Hz, 1H), 7.78 (d, $J$ = 7.8 Hz, 1H ), 6.96-7.14 (m, 2H), 3.09 (q, $J$ = 7.1 Hz, 1H), 1.14 (d, $J$ = 7.3 Hz, 3H).

**Example 7** 2,2-difluoro-3-(4-(1H-indol-3-yl)thiophen-2-yl)-3-oxopropionic acid (**I-7**)

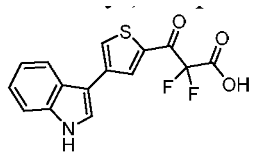

Step 1: 3-(5-formylthiophen-3-yl)-1-Boc-1H-indole (**7-1**)

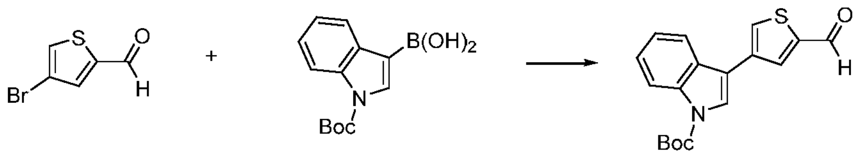

A mixture of 146 mg of 4-bromo-2-thiophenecarboxaldehyde, 240 mg of (1-Boc-1H-indol-3-yl)boronic acid, 31 mg of 1,1-bis(diphenylphosphino)ferrocene palladium dichloride and 680 mg of cesium carbonate was dissolved in a mixed solution of 1.5 mL of tetrahydrofuran and 1.5 mL of water. It was heated and stirred at 80°C overnight under the protection of argon. The reaction solution was diluted with water, extracted with ethyl acetate, and the organic phase was concentrated to dryness. The residue was subjected to column chromatography with petroleum ether : ethyl acetate= 9:1 to obtain 214 mg of yellow solid (**7-1**) with a yield of 71%. $^1$H NMR (400 MHz, $CDCl_3$) $\delta$ 10.01 (d, $J$ = 1.3 Hz, 1H), 8.23 (d, $J$ = 8.3 Hz, 1H), 8.06 (d, $J$ = 1.5 Hz, 1H), 7.96-7.89 (m, 1H), 7.83-7.74 (m, 2H), 7.45-7.37 (m, 1H), 7.33 (td, $J$ = 1.2, 7.6 Hz, 1H), 1.70 (s, 9H).

Step 2: ethyl 2,2-difluoro-3-(4-(1-Boc-1H-indol-3-yl)thiophen-2-yl)-3-hydroxypropanoate (**7 -2**)

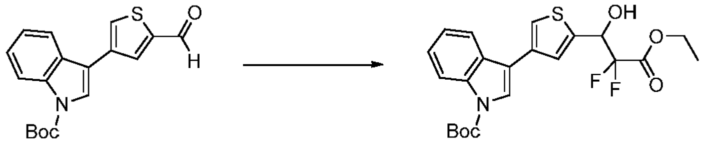

A mixture of 106 mg of compound **7-1**, 52 μL of ethyl difluorobromoacetate and 27 mg of zinc powder was dissolved in 5 mL of tetrahydrofuran, and heated and stirred at 55° C for 3 hours under the protection of argon. The reaction solution was adjusted to pH=5 with 1N hydrochloric acid, extracted with ethyl acetate, and the organic layer was concentrated to dryness, and subjected to column chromatography with petroleum ether : ethyl acetate=20:1 to obtain 112 mg of a yellow solid (**7-2**) with a yield of 76.1%. $^1$H NMR (400 MHz, $CDCl_3$) $\delta$ 8.21 (d, $J$ = 8.3 Hz, 1H), 7.79 (d, $J$ = 7.8 Hz, 1H), 7.72 (s, 1H), 7.55 (s, 1H), 7.46 (s, 1H), 7.37 (t, $J$ = 7.7 Hz, 1H), 7.34-7.27 (m, 1H), 5.48 (dt, $J$ = 6.7, 14.2 Hz, 1H), 4.36 (q, $J$ = 7.2 Hz, 2H), 2.95 (d, $J$ = 6.2 Hz, 1H), 1.69 (s, 9H), 1.33 (d, $J$= 0.7, 7.1 Hz, 3H).

Step 3: ethyl 2,2-difluoro-3-(4-(1-Boc-1H-indol-3-yl)thiophen-2-yl)-3-oxopropanoate (7-3)

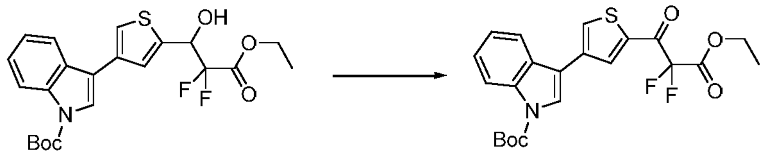

105 mg of compound **7-2** was dissolved in 13 mL of dichloromethane, 138 mg of Dess Martin oxidant was slowly added, and the mixture was stirred for 3 hours. The reaction solution was diluted with saturated aqueous sodium bicarbonate solution, extracted with ethyl acetate, and the organic layer was concentrated to dryness, and subjected to column chromatography with petroleum ether : ethyl acetate=98:2 to obtain 88 mg of yellow solid (7-3) with a yield of 84.3%. $^1$H NMR (400 MHz, $CDCl_3$) $\delta$ 8.28 (d, $J$ = 1.5 Hz, 1H), 8.23 (d, $J$= 8.3 Hz, 1H), 8.00 (d, $J$ = 1.3 Hz, 1H), 7.80 (s, 1H), 7.75 (d, $J$ = 7.8 Hz, 1H), 7.44 - 7.37 (m, 1H), 7.37-7.30 (m, 1H), 4.42 (q, $J$ = 7.1 Hz, 2H), 1.71 (s, 9H), 1.36 (t, $J$ = 7.1 Hz, 3H).

Step 4: 2,2-difluoro-3-(4-(1H-indol-3-yl)thiophen-2-yl)-3-oxoroionic acid (**I-7**)

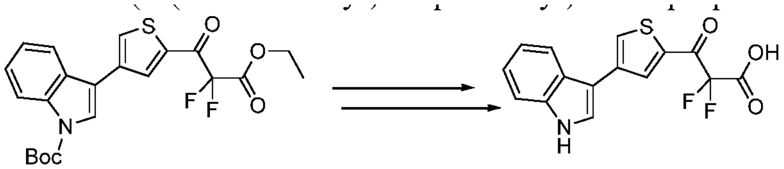

50 mg of compound **7-3** was dissolved in 18 mL of dichloromethane, 7.5 mL of trifluoroacetic acid was added dropwise, and the mixture was stirred at room temperature for 12 hours. The reaction mixture was directly concentrated to dryness. The residue was dissolved in a mixed solution of 6 mL of tetrahydrofuran and 6 mL of water, a 1M aqueous lithium hydroxide solution was added dropwise, and the mixture was stirred at room temperature for 1 hour. The reaction solution was neutralized with 1N hydrochloric acid, extracted with ethyl acetate, and the organic layer was concentrated to dryness, and purified by preparative thin layer chromatography (dichloromethane : methanol : acetic acid = 100:10:1) to obtain 21 mg of a yellow solid (**I-7**) with a yield of 75%. $^1$H NMR (400 MHz, DMSO-$d_6$) $\delta$ 11.43 (s, 1H), 8.35 (s, 1H), 8.19 (d, $J$ = 1.4 Hz, 1H), 7.85 (d, $J$ = 7.8 Hz, 1H), 7.72 (d, $J$ = 2.6 Hz, 1H), 7.45 (d, $J$= 7.9 Hz, 1H), 7.24-7.08 (m, 2H). LRMS (ESI) $[M + H]^+$ 322.2.

**Example 8** 2,2-difluoro-3-(4-(1H-indol-3-yl)furan-2-yl)-3-oxopropionic acid (**I-8**)

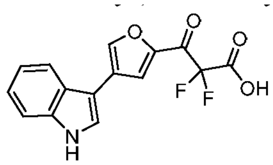

Step 1: 3-(5-formylfuran-3-yl)-1-Boc-1 H-indole (**8-1**)

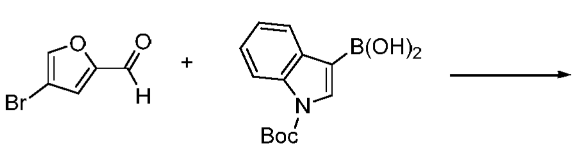

The preparation of 3-(5-formylfuran-3-yl)-1-Boc-1 H-indole (**8-1**) was the same as that of compound **7-1,** and a yellow solid was obtained with a yield of 71.4% . LRMS (ESI) [M + H]$^+$ 312.1.

Step 2: ethyl 2,2-difluoro-3-(4-(1-Boc-1H-indol-3-yl)furan-2-yl)-3-hydroxypropanoate (**8-2**)

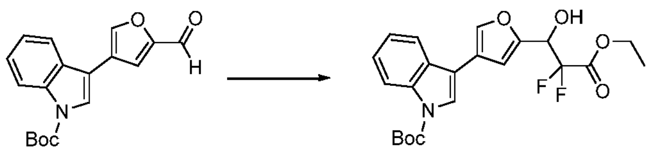

The preparation of ethyl 2,2-difluoro-3-(4-(1-Boc-1H-indol-3-yl)furan-2-yl)-3-hydroxypropanoate(**8-2**) was the same as that of compound **7-2,** and a yellow solid was obtained with a yield of 73.2%. $^1$H NMR (400 MHz, $CDCl_3$) $\delta$ 8.20 (d, *J*= 7.9 Hz, 1H), 7.84 (d, *J*= 0.9 Hz, 1H), 7.67 (d, *J*= 8.0 Hz, 2H), 7.37 (ddd, *J* = 1.3, 7.2, 8.4 Hz, 1H), 7.30 (td, *J*= 1.1, 7.5 Hz, 1H), 6.83 (s, 1H), 5.25 (dd, *J* = 7.7, 15.0 Hz, 1H), 4.38 (q, *J* = 7.2 Hz, 2H), 2.85 (d, *J* = 8.2 Hz, 1H), 1.69 (s, 9H), 1.35 (t, *J* = 7.1 Hz, 3H).

Step 3: ethyl 2,2-difluoro-3-(4-(1-Boc-1H-indol-3-yl)furan-2-yl)-3-oxopropanoate (**8-3**)

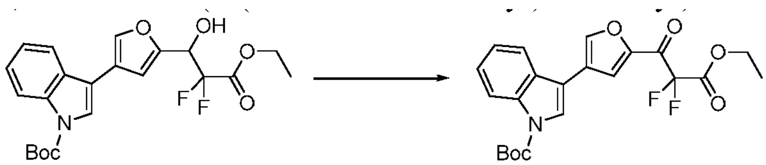

The preparation of ethyl 2,2-difluoro-3-(4-(1-Boc-1H-indol-3-yl)furan-2-yl)-3-oxopropanoate (**8-3**) was the same as that of compound **7-3,** and a white solid was obtained with a yield of 82%. LRMS (ESI) [M + H]$^+$ 434.3.

Step 4: 2,2-difluoro-3-(4-(1H-indol-3-yl)furan-2-yl)-3-oxopropionic acid (**I-8**)

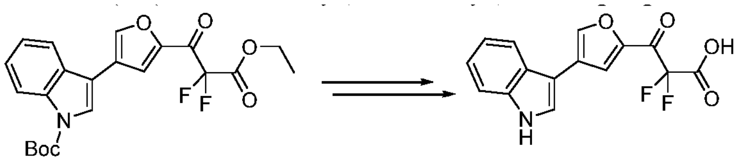

The preparation of 3-(4-(1H-indol-3-yl)furan-2-yl)-2,2-difluoro-3-oxopropionic acid (**I-8**) was the same as that of compound **I** -7, and a white solid was obtained with a yield of 76.4%. $^1$H NMR (400 MHz, DMSO-$d_6$) $\delta$ 11.42 (s, 1H), 8.52 (s, 1H), 7.84-7.75 (m, 3H), 7.43 (d, *J*= 8.0 Hz, 1H), 7.13 (dt, *J*= 7.2, 22.9 Hz, 2H). LRMS (ESI) [M + H]$^+$ 306.1.

**Example 9** 2,2-difluoro-3-(4-(7-fluoro-1H-indol-3-yl)furan-2-yl)-3-oxopropionic acid (**I-9**)

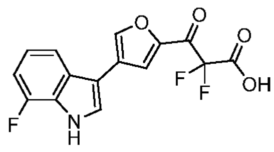

Step 1: 2-formylfuran-4-boronic acid pinacol ester 4-(4,4,5,5-tetramethyl-1,3,2-dioxaboran-2-yl)furan-2-formaldehyde (**9-1**)

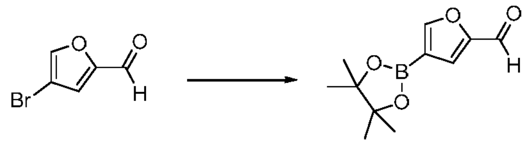

The preparation of 2-formylfuran-4-boronic acid pinacol ester 4-(4,4,5,5-tetramethyl-1,3,2-dioxaboran-2-yl)furan-2-carbaldehyde (**9-1**) was the same as that of compound **2-1** with a yield of 79.5%. LRMS (ESI) $[M + H]^+$ 223.2.

Step 2: 7-fluoro-3-(5-formylfuran-3-yl)-1-Boc-1H-indole (**9-2**)

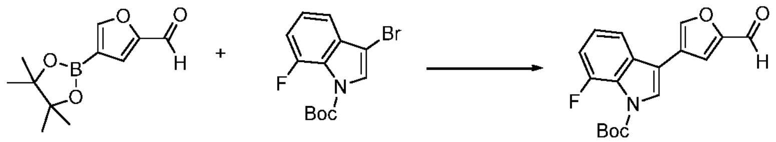

The preparation of 7-fluoro-3-(5-formylfuran-3-yl)-1-Boc-1H-indole (**9-2**) was the same as that of compound **7-1** with a yield of 33.8%. $^1$H NMR (400 MHz, $CDCl_3$) $\delta$ 9.76 (s, 1H), 8.03 (s, 1H), 7.81 (s, 1H), 7.54 (d, *J* = 0.9 Hz, 1H), 7.46 (dd, *J*= 1.0, 7.9 Hz, 1H), 7.34-7.24 (m, 1H), 7.14 (ddd, *J* = 1.1, 8.0, 12.5 Hz, 1H), 1.70 (s, 9H).

Step 3: ethyl 2,2-difluoro-3-(4-(1-Boc-7-fluoro-1H-indol-3-yl)furan-2-yl)-3-hydroxypropanoate (**9-3**)

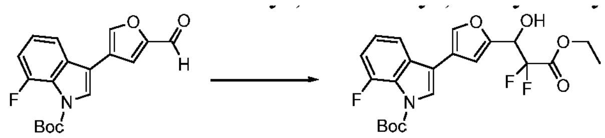

The preparation of ethyl 2,2-difluoro-3-(4-(1-Boc-7-fluoro-1H-indol-3-yl)furan-2-yl)-3-hydroxypropanoate (**9-3** ) was the same as that of compound **7-2** with a yield of 25.7%. $^1$H NMR (400 MHz, $CDCl_3$) $\delta$ 7.79 (s, 1H), 7.70 (s, 1H), 7.42 (d, *J* = 7.8 Hz, 1H), 7.23 (td, *J* = 4.1, 8.0 Hz, 1H), 7.08 (dd, *J* = 7.9, 12.5 Hz, 1H), 6.80 (s, 1H), 5.26 (dt, *J* = 7.1, 14.8 Hz, 1H), 4.38 (q, *J* = 7.1 Hz, 2H), 3.04 (d, *J*= 8.0 Hz, 1H), 1.66 (s, 9H), 1.35 (t, *J* = 7.1 Hz, 3H).

Step 4: ethyl 2,2-difluoro-3-(4-(1-Boc-7-fluoro-1H-indol-3-yl)furan-2-yl)-3-oxopropanoate(**9-4**)

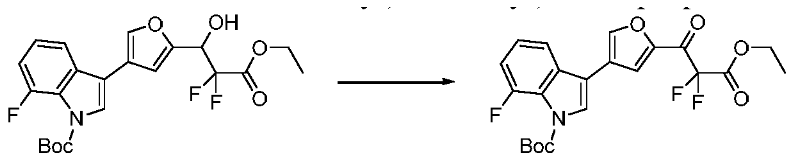

The preparation of ethyl 2,2-difluoro-3-(4-(1-Boc-7-fluoro-1H-indol-3-yl)furan-2-yl)-3-oxopropanoate(**9-4** ) was the same as that of compound **7-3** with a yield of 100%. LRMS (ESI) [M + H]$^+$ 452.3.

Step 5: 2,2-difluoro-3-(4-(7-fluoro-1H-indol-3-yl)furan-2-yl)-3-oxopropionic acid (**I-9**)

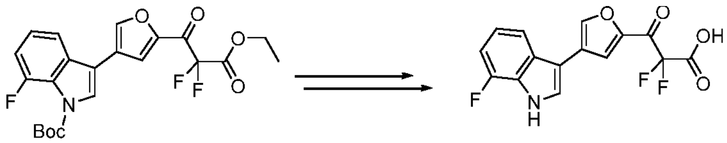

The preparation of 2,2-difluoro-3-(4-(7-fluoro-1H-indol-3-yl)furan-2-yl)-3-oxopropionic acid (**I-9**) was the same as that of compound **1-7** with a yield of 27.3%. $^1$H NMR (400 MHz, $CD_3OD$) $\delta$ 8.28 (d, *J* = 0.8 Hz, 1H), 7.92 (d, *J* = 1.1 Hz, 1H), 7.63 (s, 1H), 7.57 (d, *J* = 8.0 Hz, 1H), 7.08 (td, *J* = 4.8, 7.9 Hz, 1H), 6.91 (dd, *J* = 7.8, 11.4 Hz, 1H). LRMS (ESI) [M + H]$^+$ 324.1.

**Example 10** 2,2-difluoro-3-(4-(7-fluoro-1H-indol-3-yl)thiophen-2-yl)-3-oxopropionic acid (**I-10**)

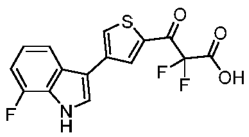

Step 1: 4-(4,4,5,5-tetramethyl-1,3,2-dioxaboran-2-yl)thiophen-2-carbaldehyde (**10-1**)

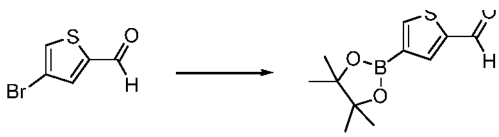

The preparation of 4-(4,4,5,5-tetramethyl-1,3,2-dioxaboran-2-yl)thiophene-2-carbaldehyde (**10-1**) was the same as that of compound **2-1** with a yield of 85%. $^1$H NMR (400 MHz, $CDCl_3$) $\delta$ 9.94 (d, *J* = 1.5 Hz, 1H), 8.28-8.22 (m, 1H), 8.06 (s, 1H), 1.34 (s, 12H).

Step 2: 7-fluoro-3-(5-formylthiophen-3-yl)-1-Boc-1H-indole (**10-2**)

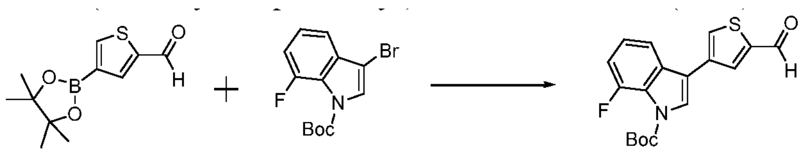

The preparation of 7-fluoro-3-(5-formylthiophen-3-yl)-1-Boc-1H-indole (**10-2**) was the same as that of compound **7-1** with a yield of 53.2%. LRMS (ESI) $[M + H]^+$ 346.3.

Step 3: ethyl 2,2-difluoro-3-(4-(1-Boc-7-fluoro-1H-indol-3-yl)thiophen-2-yl)-3-hydroxypropanoate (**10-3**)

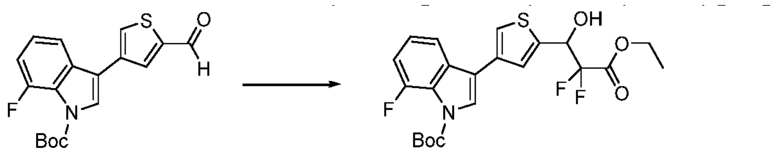

The preparation of ethyl 2,2-difluoro-3-(4-(1-Boc-7-fluoro-1H-indol-3-yl)thiophen-2-yl)-3-hydroxypropanoate (**10-3**) was the same as that of compound **7-2** with a yield of 47.8%. $^1$H NMR (400 MHz, $CDCl_3$) $\delta$ 7.77 (s, 1H), 7.60-7.52 (m, 2H), 7.45 (d, *J* = 0.7 Hz, 1H), 7.25 (dt, *J* = 4.0, 7.9 Hz, 1H), 7.11 (ddd, *J* = 1.0, 8.0, 12.6 Hz, 1H), 5.51 (dt, *J* = 6.4, 13.9 Hz, 1H), 4.39 (q, *J* = 7.1 Hz, 2H), 2.97 (d, *J* = 6.1 Hz, 1H), 1.69 (s, 9H), 1.36 (t, *J*= 7.1 Hz, 3H).

Step 4: ethyl 2,2-difluoro-3-(4-(1-Boc-7-fluoro-1H-indol-3-yl)thiophen-2-yl)-3-oxopropanoate (**10-4**)

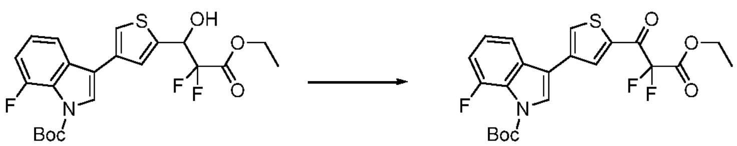

The preparation of ethyl 2,2-difluoro-3-(4-(1-Boc-7-fluoro-1H-indol-3-yl)thiophen-2-yl)-3-oxopropanoate (**10-4**) was the same as that of compound **7-3** with a yield of 88%. $^1$H NMR (400 MHz, $CDCl_3$) $\delta$ 8.25 (d, *J* = 1.5 Hz, 1H), 7.97 (d, *J* = 1.3 Hz, 1H), 7.82 (s, 1H), 7.50 (dd, *J* = 1.0, 7.9 Hz, 1H), 7.34-7.22 (m, 1H), 7.12 (ddd, *J*= 1.0, 8.0, 12.5 Hz, 1H), 4.41 (q, *J*= 7.1 Hz, 2H), 1.68 (s, 9H), 1.36 (t, *J* = 7.1 Hz, 3H).

Step 5: 2,2-difluoro-3-(4-(7-fluoro-1H-indol-3-yl)thiophen-2-yl)-3-oxopropionic acid (**I-10**)

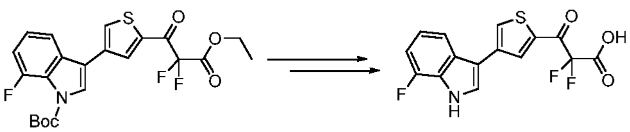

The preparation of 2,2-difluoro-3-(4-(7-fluoro-1H-indol-3-yl)thiophen-2-yl)-3-oxopropionic acid (**I-10**) was the same as that of compound **1-7** with a yield of 95%. $^1$H NMR (400 MHz, $CD_3OD$) $\delta$ 8.40 (d, *J* = 1.4 Hz, 1H), 8.06 (d, *J* = 1.4 Hz, 1H), 7.67 (d, *J* = 8.3 Hz, 2H), 7.08 (td, *J* = 4.8, 8.0 Hz, 1H), 6.95-6.87 (m, 1H). LRMS (ESI) $[M + H]^+$ 340.1.

**Example 11** 2,2-difluoro-3-(4-(6-fluoro-1H-indol-3-yl)thiophen-2-yl)-3-oxopropionic acid (**I-11**)

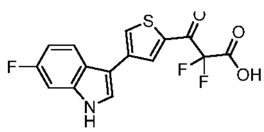

Step 1: 6-fluoro-3-(5-formylthiophen-3-yl)-1-Boc-1H-indole (**11-1**)

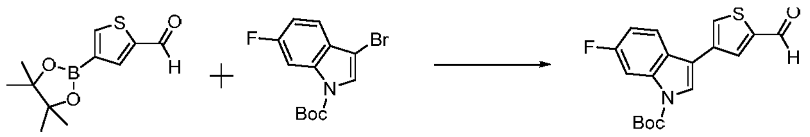

The preparation of 6-fluoro-3-(5-formylthiophen-3-yl)-1-Boc-1H-indole (**11-1**) was the same as that of compound **7-1** with a yield of 25%. $^1H$ NMR (400 MHz, $CDCl_3$) $\delta$ 9.99 (s, 1H), 8.01 (d, *J* = 1.6 Hz, 1H), 7.99-7.90 (m, 1H), 7.87 (s, 1H), 7.74 (s, 1H), 7.67 (dd, *J* = 5.2, 8.7 Hz, 1H), 7.07 (td, *J* = 2.4, 8.9 Hz, 1H), 1.70 (s, 9H).

Step 2: ethyl 2,2-difluoro-3-(4-(1-Boc-6-fluoro-1H-indol-3-yl)thiophen-2-yl)-3-hydroxypropanoate(**11-2**)

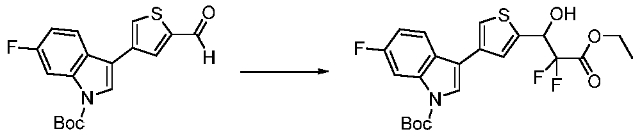

The preparation of ethyl 2,2-difluoro-3-(4-(1-Boc-6-fluoro-1H-indol-3-yl)thiophen-2-yl)-3-hydroxypropanoate (**11-2**) was the same as that of compound **7-2** with a yield of 69%. $^1H$ NMR (400 MHz, $CDCl_3$) $\delta$ 7.92 (d, *J* = 10.0 Hz, 1H), 7.74-7.63 (m, 2H), 7.52 (s, 1H), 7.43 (s, 1H), 7.09-7.02 (m, 1H), 5.49 (dd, *J* = 7.0, 14.5 Hz, 1H), 4.36 (q, *J* = 7.2 Hz, 2H), 3.00 (d, *J* = 5.9 Hz, 1H), 1.69 (s, 9H), 1.33 (t, *J* = 7.1 Hz, 3H).

Step 3: ethyl 2,2-difluoro-3-(4-(1-Boc-6-fluoro-1H-indol-3-yl)thiophen-2-yl)-3-oxopropanoate (**11-3**)

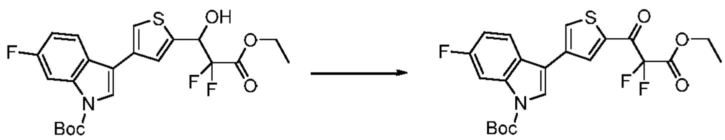

The preparation of ethyl 2,2-difluoro-3-(4-(1-Boc-6-fluoro-1H-indol-3-yl)thiophen-2-yl)-3-oxopropanoate(**11-3**) was the same as that of compound **7-3** with a yield of 81%. $^1H$ NMR (400 MHz, $CDCl_3$) $\delta$ 8.29-8.22 (m, 1H), 8.02-7.91 (m, 2H), 7.76 (s, 1H), 7.65 (dd, *J* = 5.2, 8.8 Hz, 1H), 7.09 (td, *J* = 2.4, 8.9 Hz, 1H), 4.42 (q, *J*= 7.2 Hz, 2H), 1.71 (s, 9H), 1.36 (t, *J* = 7.1 Hz, 3H).

Step 4: 2,2-difluoro-3-(4-(6-fluoro-1H-indol-3-yl)thiophen-2-yl)-3-oxopropionic acid (**I-11**)

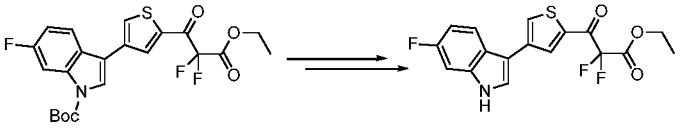

The preparation of 2,2-difluoro-3-(4-(6-fluoro-1H-indol-3-yl)thiophen-2-yl)-3-oxopropionic acid (**I-11**) was the same as that of compound **1-7** with a yield of 55%. $^1H$ NMR (400 MHz, $CD_3OD$) $\delta$ 8.38 (d, *J* = 1.3 Hz, 1H), 8.03 (d, *J* = 1.4 Hz, 1H), 7.83 (dd, *J*= 5.2, 8.8 Hz, 1H), 7.60 (s, 1H), 7.12 (dd, *J*= 2.3, 9.8 Hz, 1H), 6.92 (td, *J*= 2.3, 9.3 Hz, 1H). LRMS (ESI) $[M + H]^+$ 340.4.

**Example 12** 2,2-difluoro-3-(4-(6-fluoro-1H-indol-3-yl)furan-2-yl)-3-oxopropionic acid (**I-12**)

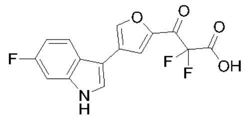

Step 1: 6-fluoro-3-(5-formylfuran-3-yl)-1-Boc-1H-indole (**12-1**)

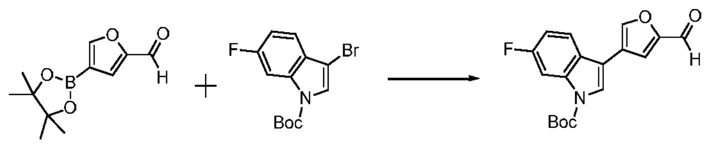

The preparation of 6-fluoro-3-(5-formylfuran-3-yl)-1-Boc-1H-indole (**12-1**) was the same as that of compound **7-1** with a yield of 17%. $^{1}$H NMR (400 MHz, $CDCl_3$) $\delta$ 9.73 (s, 1H), 8.01 (s, 1H), 7.98-7.89 (m, 1H), 7.72 (s, 1H), 7.57 (dd, *J*= 5.3, 8.7 Hz, 1H), 7.51 (s, 1H), 7.07 (td, *J* = 2.4, 8.9 Hz, 1H), 1.70 (s, 9H).

Step 2: ethyl 2,2-difluoro-3-(4-(1-Boc-6-fluoro-1H-indol-3-yl)furan-2-yl)-3-hydroxypropanoate (**12-2**)

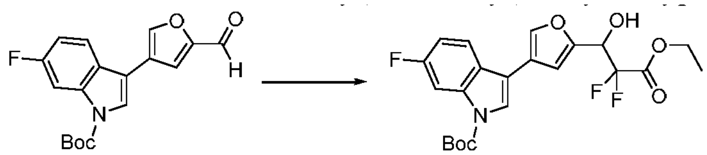

The preparation of ethyl 2,2-difluoro-3-(4-(1-Boc-6-fluoro-1H-indol-3-yl)furan-2-yl)-3-hydroxypropanoate (**12-**2) was the same as that of compound **7-2** with a yield of 49%. $^{1}$H NMR (400 MHz, $CDCl_3$) $\delta$ 7.89 (dd, *J* = 6.6, 16.8 Hz, 1H), 7.80 (s, 1H), 7.64 (s, 1H), 7.56 (dd, *J* = 5.3, 8.7 Hz, 1H), 7.04 (td, *J* = 2.5, 9.0 Hz, 1H), 6.80 (s, 1H), 5.26 (dt, *J* = 7.7, 15.1 Hz, 1H), 4.38 (q, *J* = 7.1 Hz, 2H), 3.02 (d, *J*= 7.9 Hz, 1H), 1.69 (s, 9H), 1.35 (t, *J* = 7.1 Hz, 3H).

Step 3: ethyl 2,2-difluoro-3-4-1-**B**oc-6-fluoro-1H-indol-3-lfuran-2-l-3-oxoroanoate (**12 -3**)

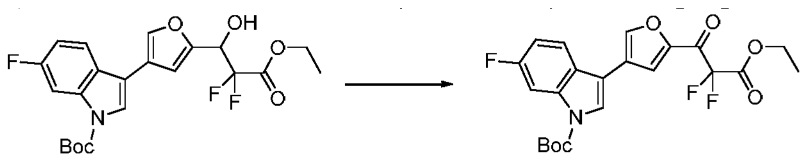

The preparation of ethyl 2,2-difluoro-3-(4-(1-Boc-6-fluoro-1H-indol-3-yl)furan-2-yl)-3-oxopropanoate(**12-3** ) was the same as that of compound **7-3** with a yield of 65%. $^{1}$H NMR (400 MHz, $CDCl_3$) $\delta$ 8.08 (s, 1H), 7.98-7.90 (m, 1H), 7.78 (s, 1H), 7.75 (s, 1H), 7.63-7.51 (m, 1H), 7.09 (td, *J* = 2.4, 8.9 Hz, 1H), 4.41 (q, *J* = 7.1 Hz, 2H), 1.70 (s, 9H), 1.36 (t, *J*= 7.2 Hz, 3H).

Step 4: 2,2-difluoro-3-(4-(6-fluoro-1H-indol-3-yl)furan-2-yl)-3-oxopropionic acid (**I-12**)

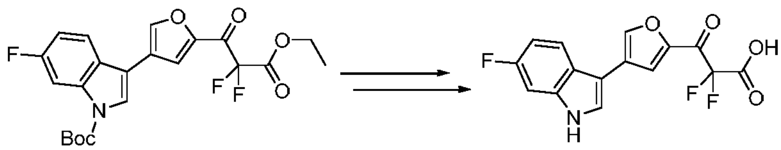

The preparation of 2,2-difluoro-3-(4-(6-fluoro-1H-indol-3-yl)furan-2-yl)-3-oxopropionic acid (**I-12**) was the same as that of compound **1-7** with a yield of 46%. $^1$H NMR (400 MHz, $CD_3OD$) $\delta$ 8.26 (s, 1H), 7.90 (s, 1H), 7.72 (dd, $J$ = 5.2, 8.8 Hz, 1H), 7.56 (s, 1H), 7.12 (dd, $J$ = 2.3, 9.9 Hz, 1H), 6.91 (td, $J$ = 2.3, 9.2 Hz, 1H). LRMS (ESI) $[M + H]^+$ 324.1.

**Example 13** 5-(4-(1H-indol-3-yl)thiophen-2-yl)-5-oxopentanoic acid (**I-13**)

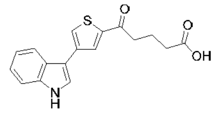

Step 1:methyl 5-(4,5-dibromothiophen-2-yl)-5-oxopentanoate (**13-1**)

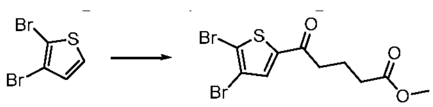

The preparation of methyl 5-(4,5-dibromothiophen-2-yl)-5-oxopentanoate (**13-1**) was the same as that of compound **1-1** with a yield of 34%. $^1$H NMR (400MHz, $CDCl_3$) $\delta$ 7.51(s, 1H), 3.68 (s, 3H), 2.91(t, $J$ =7.2 Hz, 2H), 2.43 (t, $J$ = 7.2 Hz, 2H), 2.05 (m, 2H).

Step 2: methyl 5-(4-bromothiophen-2-yl)-5-oxopentanoate (**13-2**)

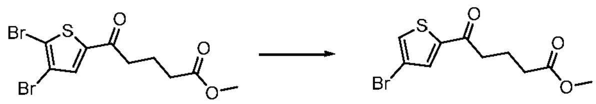

The preparation of methyl 5-(4-bromothiophen-2-yl)-5-oxopentanoate (**13-2**) was the same as that of compound **1-3** with a yield of 79%. $^1$H NMR (400 MHz, $CDCl_3$) $\delta$ 7.62 (d, $J$ = 1.3 Hz, 1H), 7.53 (d, $J$ = 1.3 Hz, 1H), 3.69 (s, 3H), 2.97 (t, $J$ = 7.2 Hz, 2H), 2.44 (t, $J$ = 7.1 Hz, 2H), 2.07 (m, 2H).

Step 3: methyl 5-(4-(1H-indol-1-Boc-3-yl)thiophen-2-yl)-5-oxopentanoate (**13-3**)

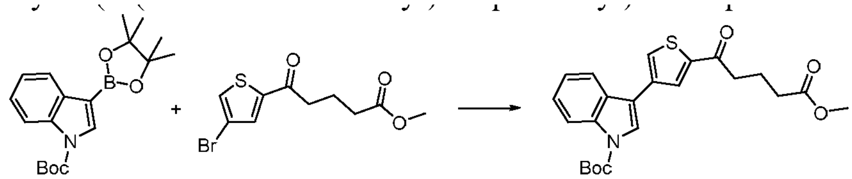

The preparation of methyl 5-(4-(1H-indol-1-Boc-3-yl)thiophen-2-yl)-5-oxopentanoate (**13-3**) was the same as that of compound **1-4** with a yield of 55%. $^1$H NMR (400 MHz, $CDCl_3$) $\delta$ 8.24 (d, $J$ = 8.0 Hz, 1H), 7.99 (d, $J$= 1.2 Hz, 1H),

7.73-7.80 (m, 3H), 7.31-7.41 (m, 2H), 3.70 (s, 3H), 3.08 (t, *J* = 7.2 Hz, 2H), 2.50 (t, *J* =7.2 Hz, 2H), 2.09-2.16 (m, 2H), 1.71 (s, 9H).

Step 4: 5-(4-(1H-indol-3-yl)thiophen-2-yl)-5-oxopentanoic acid (**I-13**)

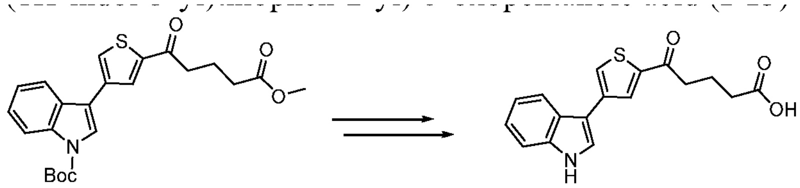

The preparation of 5-(4-(1H-indol-3-yl)thiophen-2-yl)-5-oxopentanoic acid (**I-13**) was the same as that of compound **1-1** with a yield of 75%. $^1$H NMR (400 MHz, DMSO-*d*$_6$) $\delta$ 12.15 (s, 1H), 11.42 (s, 1H), 8.35 (d, *J* = 1.2 Hz, 1H), 8.12 (d, *J* = 1.2 Hz, 1H), 7.97 (d, *J* = 7.6 Hz, 1H), 7.89 (d, *J* = 2.8 Hz, 1H), 7.45 (d, *J* = 8.0 Hz, 1H), 7.11-7.20 (m, 2H), 3.12 (t, *J* = 7.2 Hz, 2H), 2.36 (t, *J*= 7.2 Hz, 2H), 1.84-1.91 (m, 2H).

**Example 14** 5-(4-(7-fluoro-1H-indol-3-yl)thiophen-2-yl)-5-oxopentanoic acid (**I-14**)

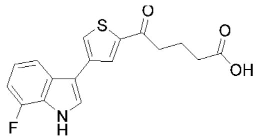

Step 1: methyl 5-(4-(7-fluoro-1H-1-Boc-indol-3-yl)thiophen-2-yl)-5-oxopentanoate (**14-1**)

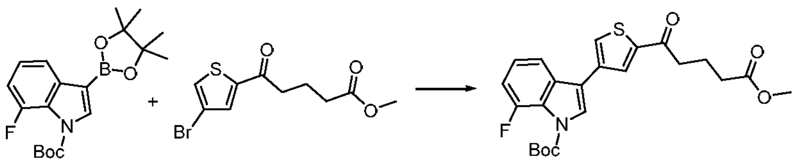

The preparation of methyl 5-(4-(7-fluoro-1H-1-Boc-indol-3-yl)thiophen-2-yl)-5-oxopentanoate (**14-1**) was same as that of compound **1**-4 with a yield of 25%. $^1$H NMR (400 MHz, $CDCl_3$) $\delta$ 7.95 (d, *J* = 1.20 Hz, 1H), 7.79 (s, 1H), 7.76 (d, *J*= 1.20 Hz, 1H), 7.55 (d, J = 7.6 Hz, 1H), 7.24-7.29 (m, 1H), 7.08-7.13 (m, 1H), 3.69 (s, 3H), 3.07 (t, *J* = 7.2 Hz, 2H), 2.50 (t, *J* =7.2 Hz, 2H), 2.08-2.15 (m, 2H), 1.68 (s, 9H).

Step 2: 5-(4-(7-fluoro-1H-indol-3-yl)thiophen-2-yl)-5-oxopentanoic acid (**I-14**)

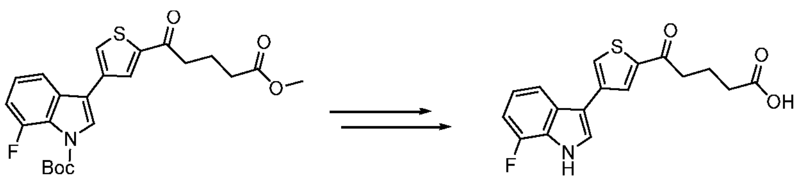

The preparation of 5-(4-(7-fluoro-1H-indol-3-yl)thiophen-2-yl)-5-oxopentanoic acid (**I-14**) was the same as that

of compound **1-1** with a yield of 80%. $^1$H NMR (400 MHz, DMSO-$d_6$) $\delta$ 12.16 (s, 1H), 11.94 (s, 1H), 8.38 (s, 1H), 8.16 (s, 1H), 7.97 (d, $J$ = 2.8 Hz, 1H), 7.80 (d, $J$ = 8.0 Hz, 1H), 7.00-7.10 (m, 2H), 3.12 (t, $J$ = 7.6 Hz, 2H), 2.36 (t, $J$ = 7.6 Hz, 2H), 1.84-1.93 (m, 2H).

**Example 15**

**4-(4-(3-(2-amino-2-oxoethyl)benzo[b]thiophen-2-yl)thiophen-2-yl)-4-oxobutyric acid (I'-1)**

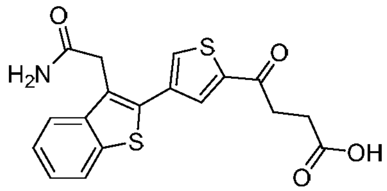

Step 1: ethyl 2-(2-bromobenzo[b]thiophen-3-yl)acetate (**1'-1**)

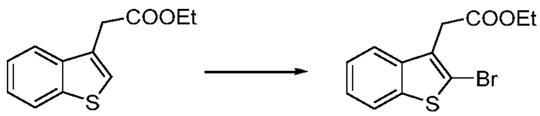

Ethyl 2-(benzo[b]thiophen-3-yl)acetate (254 mg) was dissolved in dichloromethane (4 mL), and a 1M solution of bromine in dichloromethane (11.5 mL) was added dropwise under ice bath. After addition, the mixture was stirred under an ice bath for 1 hour, saturated sodium thiosulfate solution (2 mL) was added, and stirred at room temperature for 20 minutes. The organic phase was separated, concentrated to dryness, and subjected to column chromatography with ethyl acetate : petroleum ether=5:95 to obtain 331 mg of ethyl 2-(2-bromobenzo[b]thiophen-3-yl)acetate (**1'-1**) as a yellow oil with a yield of 96%. $^1$H NMR (400 MHz, $CDCl_3$) $\delta$ 7.69-7.76 (m, 2H), 7.28-7.41 (m, 2H), 4.16-4.22 (m, 2H), 3.90 (s, 2H), 1.28 (t, $J$ = 7.2 Hz, 3H).

**Step 2:** 2-(2-bromobenzo[b]thiophen-3-yl)acetamide (**1'-2**)

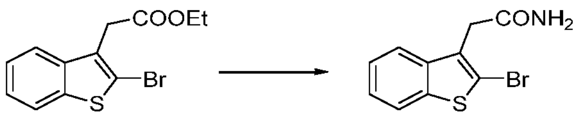

The mixture of compound **1'-1** (300 mg) and a solution of 7 M ammonia in methanol (5 mL) was stirred at room temperature for 3 days. The reaction solution was concentrated to dryness, and subjected to column chromatography with dichloromethane: methanol = 95:5 to obtain 100mg of 2-(2-bromobenzo[b]thiophen-3-yl)acetamide (**1'-2**) as a light brown solid with a yield of 33%. $^1$H NMR (400MHz, $CDCl_3$) $\delta$ 7.93-7.95 (m, 1H), 7.77-7.79 (m, 1H), 7.63 (br s, 1H), 7.33-7.43 (m, 2H), 7.09 (br s, 1H), 3.70 (s, 2H).

Step 3: methyl 4-(4-(4,4,5,5-tetramethyl-1,3,2-dioxaboran-2-yl)thiophen-2-yl)-4-oxobutyrate (**1'-3**)

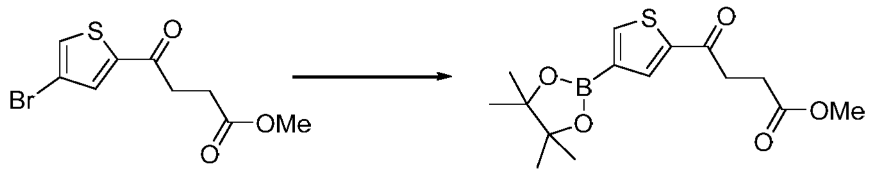

A mixture of compound 31'-**2** (1.18g), pinacol biboronate (1.3g), potassium acetate (1.26g), [1,1-bis(diphenylphosphino)ferrocene]palladium dichloride (94 mg) and 1,4-dioxane (20 mL) was stirred at 80 °C overnight under the protection of argon. The reaction solution was concentrated to dryness, and subjected to column chromatography with ethyl acetate: petroleum ether = 5:95 to obtain 1.08 g of methyl 4-(4-(4,4,5,5-tetramethyl-1,3,2-dioxaboran-2-yl)thiophen-2-yl)4-oxybutyrate (**1'-3**) as a yellow oily liquid with a yield of 78%. $^1$H NMR (400 MHz, $CDCl_3$) $\delta$ 8.15 (s, 1H), 8.03 (s, 1H), 3.69 (s, 3H), 3.28 (t, J = 6.8 Hz, 2H), 2.75 (t, J = 6.7 Hz, 2H), 1.34 (s, 12H).

Step 4: methyl 4-(4-(3-(2-amino-2-oxoethyl)benzo[b]thiophen-2-yl)thiophen-2-yl)-4-oxobutyrate (**1'-4**)

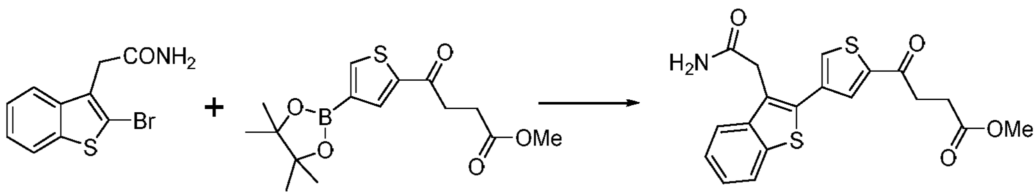

A mixture of compound **1'-2 (** 44mg **)**, compound **1'-3** (125mg), [1,1-bis(diphenylphosphino)ferrocene]palladium dichloride (6 mg), cesium carbonate (120 mg), tetrahydrofuran (0.75 mL) and water (0.75 mL) was stirred at 70 °C for 7 hours under the protection of argon. The reaction solution was concentrated to dryness and purified by preparative thin layer chromatography (dichloromethane: methanol = 20:1) to obtain 24 mg of methyl 4-(4-(3-(2-amino-2-oxoethyl)benzo[b]thiophen-2-yl)thiophen-2-yl)-4-oxybutyrate (**1'-4**) as a white solid with a yield of 41%. $^1$H NMR (400MHz, $CDCl_3$+$CD_3OD$) $\delta$7.68-7.91 (m, 4H), 7.32-7.38 (m, 3H), 3.75-3.78 (m, 2H), 3.65 (s, 3H), 3.24-3.32 (m, 3H), 2.74 (s, 2H).

Step 5: 4-(4-(3-(2-amino-2-oxoethyl)benzo[b]thiophen-2-yl)thiophen-2-yl)-4-oxobutyric acid (**I'-1**)

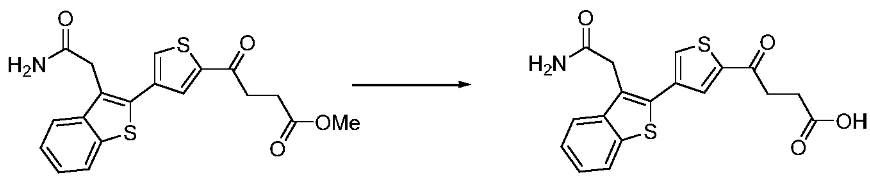

Compound **1'-4** (10mg) was dissolved in tetrahydrofuran (0.3mL) and water (0.3mL), 1M lithium hydroxide aqueous solution (60$\mu$L) was added, and the mixture was stirred at room temperature for 0.5 h. The reaction solution was concentrated to dryness, and purified by preparative thin layer chromatography (dichloromethane: methanol = 20:1 (1% acetic acid)) to obtain 9.7 mg of pale yellow solid (**I'-1**) with a yield of 100%. $^1$H NMR (400MHz, DMSO-$d_6$) $\delta$8.34 (s ,1H), 8.27 (s,1H), 8.00 (d, *J*= 6.8 Hz, 1H), 7.90-7.92 (m ,2H), 7.39-7.47 (m, 2H), 7.21 (s, 1H), 3.76 (s, 2H), 3.23 (t, *J* = 6.4 Hz, 2H) , 2.55 (t, *J* = 6.4 Hz, 2H).

**Example 16 4-(3-(2-amino-2-oxoethyl)-[2,3'-thiophen]-5'-yl)-4-oxobutyric acid (1-2)**

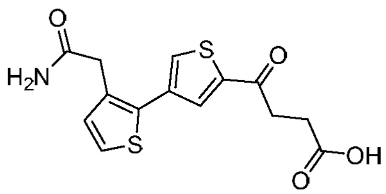

Step 1: ethyl 2-(2-bromothiophen-3-yl)acetate (**2'-1**)

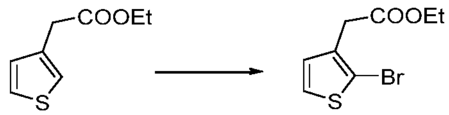

Ethyl 2-(thiophen-3-yl)acetate (1 g) was dissolved in chloroform (3 mL) and glacial acetic acid (3 mL), and N-bromosuccinimide (1.0 g) was added in batches with stirring in an ice bath. After addition, the mixture was stirred at room temperature for 2 days. The reaction solution was concentrated to dryness, and subjected to column chromatography with petroleum ether to obtain 1.42g of ethyl 2-(2-bromothiophen-3-yl) acetate (2'-1) **as a colorless oil with a** yield of 97%. $^1$H NMR (400 MHz, $CDCl_3$) $\delta$7.25 (d, *J*= 5.6 Hz, 1H), 6.95 (d, *J* = 5.6 Hz, 1H), 4.15-4.20 (m, 2H), 3.62 (s, 2H), 1.29 (t, *J*= 9.2 Hz, 3H).

Step 2: 2-(2-bromothiophen-3-yl)acetamide (**2'-2**)

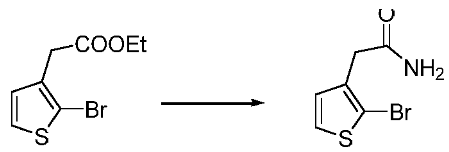

The preparation method of 2-(2-bromothiophen-3-yl)acetamide (**2'-2**) was the same as that of compound **1'-2**, and a white solid was obtained with a yield of 69%. $^1$H NMR (400 MHz, DMSO-$d_6$) $\delta$7.54 (d, *J*= 8.0 Hz, 1H), 7.48 (br s, 1H), 6.99 (d, *J* = 8.0 Hz, 2H), 3.39 (s, 2H).

Step 3: methyl 4-(3-(2-amino-2-oxoethyl)-[2,3'-dithiophene]-5'-yl)-4-oxobutyrate (**2'-3**)

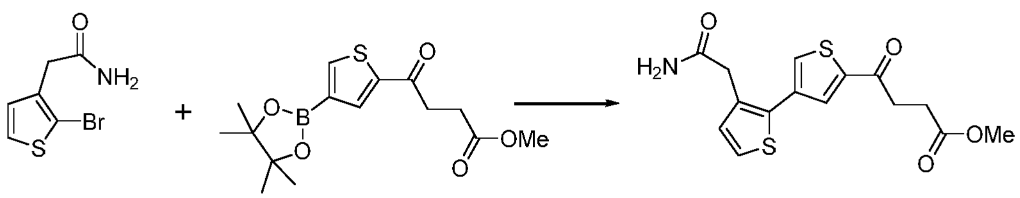

The preparation method of methyl 4-(3-(2-amino-2-oxoethyl)-[2',3-dithiophen]-5'-yl)-4-oxobutyrate (**2'-3**) was the same as that of compound **1'-4**, and a yellow oil was obtained with a yield of 52%. $^1$H NMR (400MHz, $CDCl_3$+$CD_3OD$) $\delta$7.82 (d, *J* = 1.2 Hz, 1H), 7.69 (d, *J* = 0.8 Hz, 1H), 7.25 (d, *J* = 0.8 Hz, 1H), 6.99 (d, *J* = 1.2 Hz, 1H), 3.64 (s, 3H), 3.54 (s, 2H), 3.24 (t, *J* = 6.4 Hz, 2H), 2.72 (t, *J* = 6.4 Hz, 2H).

Step 4: 4-(3-(2-amino-2-oxoethyl)-[2,3'-thiophen]-5'-yl)-4-oxobutyric acid (**I'-2**)

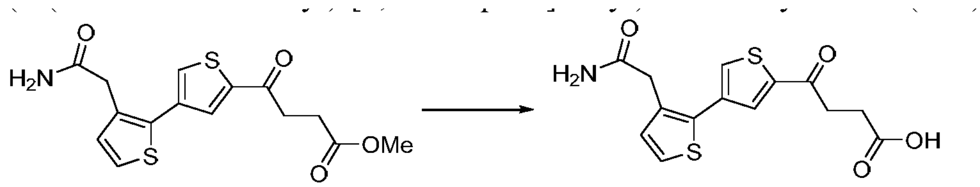

The preparation method of 4-(3-(2-amino-2-oxoethyl)-[2,3'-thiophen]-5'-yl)-4-oxobutyric acid (**I'-2**) was the same as that of compound **I'-1**, and an off-white solid was obtained with a yield of 42%. $^1$H NMR (400MHz, $CD_3OD$) $\delta$ 8.06 (d, $J$ = 0.4 Hz, 1H), 7.94 (d, $J$ = 1.2 Hz, 1H), 7.38 (d, $J$ = 5.6 Hz, 1H), 7.08 (d, $J$ = 5.2 Hz, 1H), 3.60 (s, 2H), 3.27 (t, $J$ = 6.8 Hz, 2H) , 2.71 (t, $J$ = 6.8 Hz, 2H).

**Example 17 4-([3,3'-dithiophen]-2-yl)-4-oxobutyric acid (I'-3)**

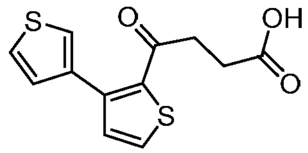

Step 1: methyl 4-(3-chlorothiophen-2-yl)-4-oxobutyrate (**3'-1**)

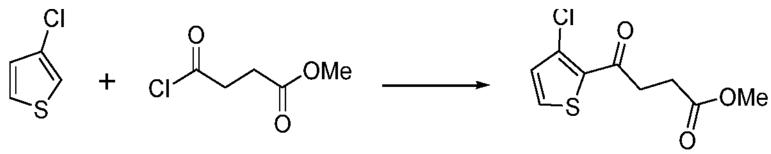

3-chlorothiophene (2.82g) was dissolved in dichloromethane (30mL), monomethyl succinate chloride (6.44g) was slowly added dropwise with stirring under an ice bath. After addition, the mixture was stirred at room temperature overnight, 2M hydrochloric acid was slowly added dropwise to the reaction solution under the ice bath. The liquid was separated after stirring, and the organic phase was concentrated to dryness, and subjected to column chromatography with ethyl acetate: petroleum ether=4:96 to obtain 2.93 g of methyl 4-(3-chlorothien-2-yl)-4-oxobutyrate (**3'-1**) as a brown oil with a yield of 53%. $^1$H NMR (400MHz, $CDCl_3$) $\delta$ 7.58 (d, $J$= 5.2 Hz, 1H), 7.05 (d, $J$= 5.2 Hz, 1H), 3.72 (s, 3H), 3.98 (t, $J$ = 6.4 Hz, 2H), 2.78 (t, $J$ = 6.4 Hz, 2H).

Step 2: methyl 4-([3,3'-dithiophen]-2-yl)-4-oxobutyrate (**3'-2**)

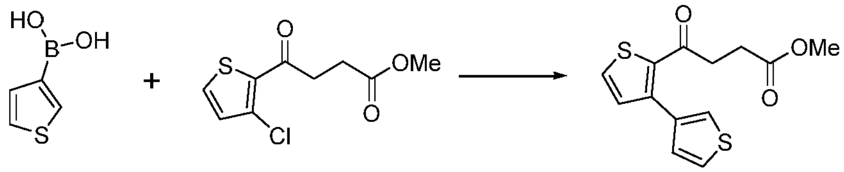

The preparation method of methyl 4-([3,3'-dithiophen]-2-yl)-4-oxobutyrate (**3'-2**) was the same as that of compound **1'-4**, and a brownish yellow oil was obtained with a yield of 56%. $^1$H NMR (400 MHz, $CD_3OD$) $\delta$ 7.74 (d, $J$ = 5.0 Hz ,1H), 7.59 (dd, $J$ = 1.2, 3.0 Hz ,1H), 7.50 (dd, $J$ = 3.0, 5.0 Hz ,1H), 7.24 (dd, $J$ = 1.2, 5.0 Hz, 1H), 7.17 (d, $J$ = 5.0 Hz ,1H), 3.64 (s, 3H), 2.94 (t, $J$ = 6.4 Hz, 2H), 2.58 (t, $J$ = 6.4 Hz, 2H).

Step 3: 4-([3,3'-dithiophenl-2-yl)-4-oxobutyric acid (**I'-3**)

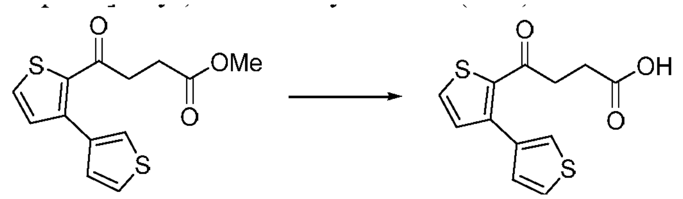

The preparation method of 4-([3,3'-dithiophen]-2-yl)-4-oxobutyric acid (**I'-3**) was the same as that of compound **I'-1**, and an off-white solid was obtained with a yield of 95%. $^1$H NMR (400MHz, $CD_3OD$) $\delta$ 7.73 (d, *J*= 5.0 Hz ,1H), 7.57-7.60 (m, 1H), 7.45-7.50 (m, 1H), 7.22-7.26 (m, 1H), 7.16 (d, *J* = 5.0 Hz, 1H), 3.64 (s, 3H), 2.92 (t, *J* = 6.4 Hz, 2H) , 2.56 (t, *J* = 6.4 Hz, 2H).

**Example 18 4-(4-(benzo[b]thiophen-3-yl)thiophen-2-yl)-4-oxobutyric acid (I'-4)**

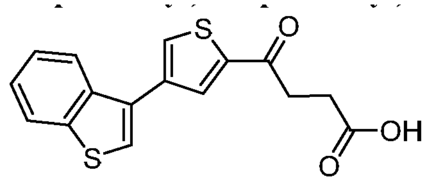

Step 1: methyl 4-(4-(benzo[b]thiophen-3-yl)thiophen-2-yl)-4-oxobutyrate (**4'-1**)

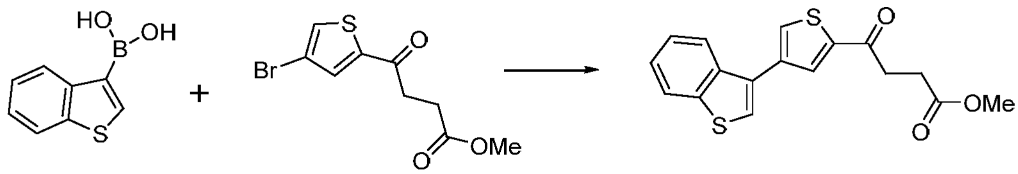

A mixture of benzothiopen-3-boronic acid (50mg), methyl 4-(4-bromothiophen-2-yl)-4-oxobutyrate (87mg), [1,1'-bis(diphenylphosphino)ferrocene] palladium dichloride (10 mg), cesium carbonate (229 mg), tetrahydrofuran (1 mL) and deionized water (1 mL) was stirred at 70 °C under an oil bath overnight under argon protection, and the reaction solution was concentrated to dryness, and purified by preparative thin layer chromatography (ethyl acetate: petroleum ether = 1:10) to obtain 59 mg of methyl 4-(4-(benzo[b]thiophen-3-yl)thiophen-2-yl)-4-oxobutanoate as a yellow oily liquid (**4-1**) with a yield of 64%. $^1$H NMR (400 MHz, $CD_3OD$) $\delta$ 8.20 (d, *J*= 1.4 Hz ,1H), 8.04 (d, *J*= 1.4 Hz ,1H), 7.98-8.03 (m, 1H), 7.93-7.97 (m, 1H), 7.74 (s,1H), 7.38-7.49 (m, 2H), 3.69 (s,3H), 3.38 (t, *J*= 6 .4Hz, 2H) , 2.75 (t, *J* = 6.4 Hz, 2H).

Step 2: 4-(4-(benzo[b]thiophen-3-yl)thiophen-2-yl)-4-oxobutyric acid (**I'-4**)

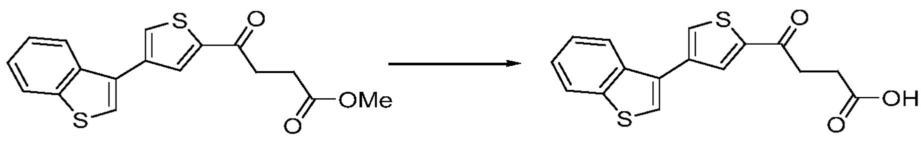

Compound **4'-1** (59mg) was dissolved in tetrahydrofuran (1.5mL) and deionized water (0.5mL), 1M lithium

hydroxide aqueous solution (410μL) was added, and the mixture was stirred at room temperature for 2 hours. The pH was adjusted to 1-3 with 2M hydrochloric acid, and the reaction solution was concentrated to dryness, and purified by preparative thin layer chromatography( dichloromethane: methanol = 30:1 (1% acetic acid)) to obtain 46 mg of 4-(4-(benzo[b]thiophen-3-yl)thiophen-2-yl)-4-oxobutyric acid (**I'-4**) as a pale yellow solid with a yield of 81%. $^1$H NMR (400 MHz, $CD_3OD$) $\delta$8.20 (d, *J*= 1.4 Hz ,1H), 8.04 (d, *J*= 1.4 Hz ,1H), 7.99-8.03 (m, 1H), 7.93-7.97 (m, 1H), 7.74(s,1H), 7.38-7.49 (m, 2H), 3.69(s,3H), 3.35 (t, *J* = 6 .4Hz, 2H) , 2.73 (t, *J* = 6.4 Hz, 2H).

**Example 19 4-(5-(benzo[b]thiophen-3-yl)thiophen-2-yl)-4-oxobutyric acid (I'-5)**

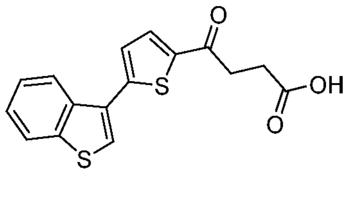

Step 1: methyl 4-(5-chlorothiophen-2-yl)-4-oxobutyrate (**5'-1**)

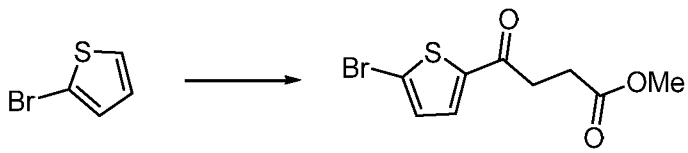

2-bromothiophene (200 mg) and monomethyl succinate chloride (203 mg) were dissolved in 1,2-dichloromethane (3 mL), aluminum trichloride (213 mg) was added, and the mixture was stirred at room temperature overnight. The reaction solution was poured into dilute hydrochloric acid, extracted with dichloromethane, and the organic layer was concentrated to dryness, and subjected to column chromatography with ethyl acetate : petroleum ether=1:50 to obtain 261 mg of methyl 4-(5-chlorothiophen- 2-yl)-4-oxobutyrate (5'-1) as a yellow solid with a yield of 77%. $^1$H NMR (400 MHz, $CDCl_3$) $\delta$7.50 (d, *J*= 4.0 Hz, 1H), 7.11 (d, *J* = 4.0 Hz, 1H), 3.70 (s, 3H), 3.18 (t, *J*= 6.7 Hz, 2H), 2.75 (t, *J* = 6.7 Hz, 2H).

Step 2: methyl 4-(5-(4,4,5,5-tetramethyl-1,3,2-dioxaboran-2-yl)thiophen-2-yl)-4-oxobutyrate (**5'-2**)

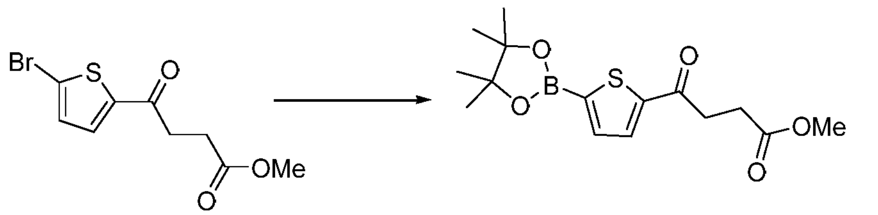

A mixture of compound **5'-1** (220mg), pinacol diboronate (202mg), [1,1'-bis(diphenylphosphino)ferrocene]palladium dichloride (12mg), potassium acetate (125 mg) and ethylene glycol dimethyl ether (2 mL) was stirred at 80 °C overnight under the protection of argon. The reaction solution was concentrated to dryness, and subjected to column chromatography with ethyl acetate: petroleum ether = 1:7 to obtain 76 mg of methyl 4-(5-(4,4,5,5-tetramethyl-1,3,2-dioxaboran-2-yl)thiophen-2-yl)-4-oxobutyrate (**5'-2**) as a yellow solid with a yield of 30%. $^1$H NMR (400MHz, DMSO-$d_6$) $\delta$8.02 (d, *J* = 4.0 Hz,1H), 7.65 (d, *J* = 4.0 Hz ,1H), 3.59 (s, 3H), 3.27 (t, *J* = 6.4 Hz, 2H) , 2.67 (t, *J* = 6.4 Hz, 2H).

Step 3: methyl 4-(5-(benzo[b]thiophen-3-yl)thiophen-2-yl)-4-oxobutyrate (**5'-3**)

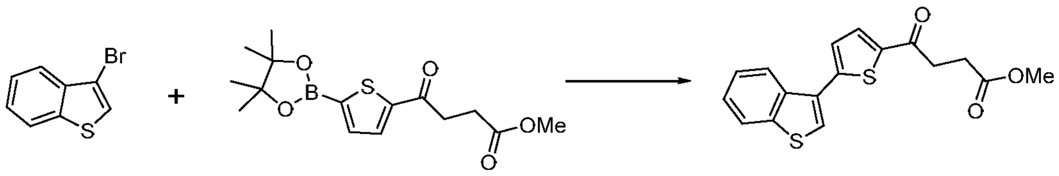

The preparation method of methyl 4-(5-(benzo[b]thiophen-3-yl)thiophen-2-yl)-4-oxobutyrate (**5'-3**) was the same as that of compound **4'-1**, and a yellow solid was obtained with a yield of 64%. $^1H$ NMR (400 MHz, $CDCl_3$) $\delta$ 8.15 (d, *J* = 8.1 Hz ,1H), 7.92 (d, *J* = 7.6 Hz ,1H), 7.81(d, *J* = 3.9 Hz ,1H), 7.66 (s, 1H), 7.40-7.51(m, 2H), 7.38 (d, *J* = 3.9 Hz ,1H) 3.73 (s, 3H), 3.30 (t, *J* = 6.8 Hz, 2H) , 2.81 (t, *J*= 6.8 Hz, 2H).

Step 4: 4-(5-(benzo[b]thiophen-3-yl)thiophen-2-yl)-4-oxobutyric acid **(I'-5)**

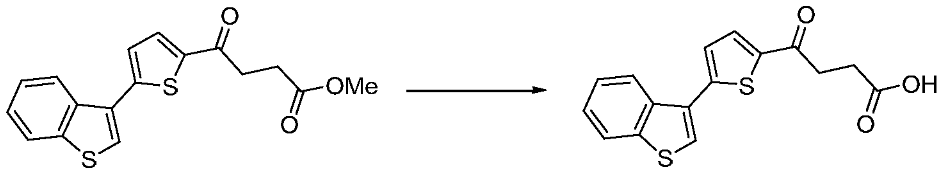

The preparation method of 4-(5-(benzo[b]thiophen-3-yl)thiophen-2-yl)-4-oxobutyric acid (**I'-5**) was the same as that of compound **I'-4**, and a yellow solid was obtained with a yield of 86%. $^1H$ NMR (400 MHz, $CD_3OD$) $\delta$ 8.11 (d, *J* = 7.6 Hz, 1H), 7.89 (d, *J* = 7.6 Hz ,1H), 7.79 (d, *J* = 3.2 Hz ,1H), 7.62 (s, 1H), 7.36-7.49 (m, 2H), 7.33 (d, *J* = 3.2 Hz, 1H), 3.27 (t, *J* = 6.4 Hz, 2H), 2.78 (t, *J*= 6.4 Hz, 2H).

**Example 20 4-([3,3'-dithiophen]-5-yl)-4-oxobutyric acid (I'-6)**

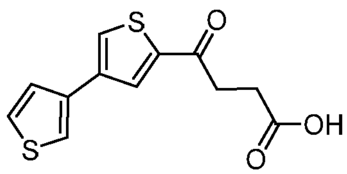

Step 1: methyl 4-([3,3'-dithiophen]-5-yl)-4-oxobutyrate **(6'-1)**

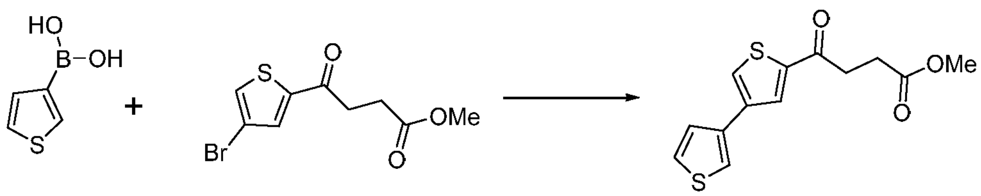

The preparation method of methyl 4-([3,3'-dithiophen]-5-yl)-4-oxobutyrate **(6'-1)** was the same as that of compound **4'-1,** and a yellow solid was obtained with a yield of 69%. $^1H$ NMR (400 MHz, $CDCl_3$) $\delta$ 7.95 (d, *J* = 1.4 Hz ,1H), 7.66 (d, *J* = 1.4 Hz, 1H), 7.37-7.44 (m, 2H), 7.30-7.44 (m, 1H), 3.72 (s, 3H), 3.30 (t, *J*= 6.7 Hz, 2H), 2.79 (t, *J* = 6.7 Hz, 2H).

Step 2: 4-([3,3'-dithiophen]-5-yl)-4-oxobutyric acid **(I'-6)**

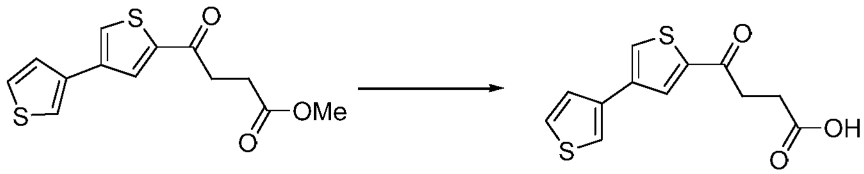

The preparation method of 4-([3,3'-dithiophen]-5-yl)-4-oxobutyric acid **(I'-6)** was the same as that of compound **I'-4,** and an off-white solid was obtained with a yield of 100%. $^1$H NMR (400 MHz, DMSO-$d_6$) $\delta$ 8.39 (s, 1H), 8.18 (s, 1H), 7.94 (d, 1H), 7.55-7.65 (m, 2H), 3.20 (t, $J$= 6.3 Hz, 2H), 2.51 (t, $J$ = 6.3 Hz, 2H).

**Example 21 4-(4-phenylthiophen-2-yl)-4-oxobutyric acid (I'-7)**

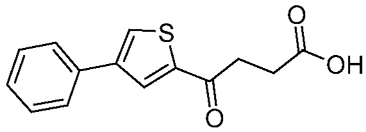

Step 1: methyl 4-(4-phenylthiophen-2-yl)-4-oxobutyrate **(7'-1)**

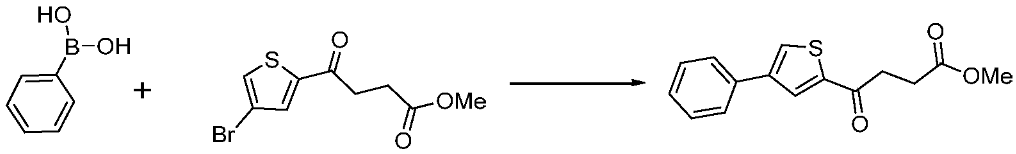

The preparation method of methyl 4-(4-phenylthiophen-2-yl)-4-oxobutyrate **(7'-1)** was the same as that of compound **4'-1,** and a yellow oily liquid was obtained with a yield of 75%. $^1$H NMR (400MHz, $CDCl_3$) $\delta$ 8.02 (d, $J$ = 1.4 Hz ,1H), 7.74 (d, $J$= 1.4 Hz ,1H), 7.58 (d, $J$ = 7.1 Hz ,2H), 7.43 (d, $J$ = 7.6 Hz ,1H) , 7.35 (d, $J$ = 7.3 Hz ,2H), 3.72 (s, 3H), 3.31(t, $J$ = 6.8 Hz, 2H) , 2.80 (t, $J$= 6.8 Hz, 2H).

Step 2: 4-(4-phenylthiophen-2-yl)-4-oxobutyric acid **(I'-7)**

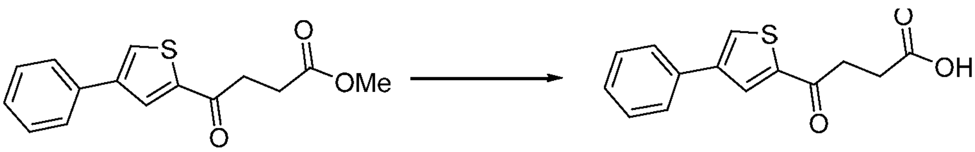

The preparation method of 4-(4-phenylthiophen-2-yl)-4-oxobutyric acid **(I'-7)** was the same as that of compound **I'-4,** and a pale yellow solid was obtained with a yield of 87%. $^1$H NMR (400MHz, DMSO-$d_6$) $\delta$ 12.1(br s, 1H), 8.43 (s, 1H), 8.29 (s, 1H), 7.80 (d, $J$ = 7.5 Hz ,1H), 7.28-7.52 (m, 3H), 3.25 (t, $J$= 6.2 Hz, 2H), 2.54 (t, $J$ = 6.2 Hz, 2H).

**Example 22 4-(4-(naphthalen-1-yl)thiophen-2-yl)-4-oxobutyric acid (I'-8)**

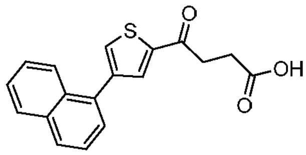

Step 1: methyl 4-(4-(naphthalen-1-yl)thiophen-2-yl)-4-oxobutyrate **(8'-1)**

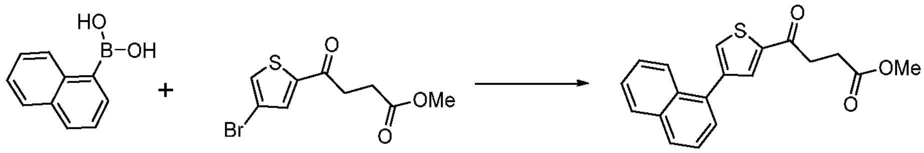

The preparation method of methyl 4-(4-(naphthalen-1-yl)thiophen-2-yl)-4-oxobutyrate **(8'-1)** was the same as that of compound **4'-1,** and a yellow oil was obtained with a yield of 79%. $^1$H NMR (400 MHz, $CDCl_3$) $\delta$ 7.36-7.98 (m, 4H), 7.69 (d, *J*= 0.9 Hz, 1H), 7.45-7.56 (m, 4H), 3.72 (s, 3H), 3.31 (t, *J*= 6.7 Hz, 2H), 2.80 (t, *J* = 6.7 Hz, 2H).

Step 2: 4-(4-(naphthalen-1-yl)thiophen-2-yl)-4-oxobutyric acid **(I'-8)**

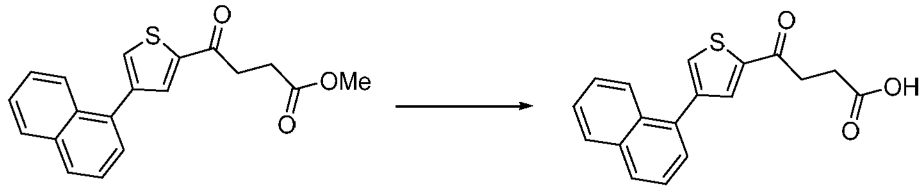

The preparation method of 4-(4-(naphthalen-1-yl)thiophen-2-yl)-4-oxobutyric acid **(I'-8)** was the same as that of compound **I'-4, and** an off-white solid was obtained with a yield of 100 %. $^1$H NMR (400 MHz, DMSO-$d_6$) $\delta$ 8.21 (d, *J*= 1.2 Hz, 1H), 8.14 (d, *J*= 1.2 Hz, 1H), 7.95-8.06 (m, 3H), 7.53-7.64 (m, 4H), 3.28 (t, *J*= 6.2 Hz, 2H), 2.59 (t, *J* = 6.2 Hz, 2H).

**Example 23 4-(4-(dibenzo[b,d]thiophen-4-yl)thiophen-2-yl)-4-oxobutyric acid (I'-9)**

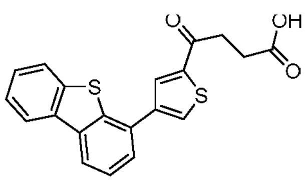

Step 1: methyl 4-(4-(dibenzo[b,d]thiophen-4-yl)thiophen-2-yl)-4-oxobutyrate **(9'-1)**

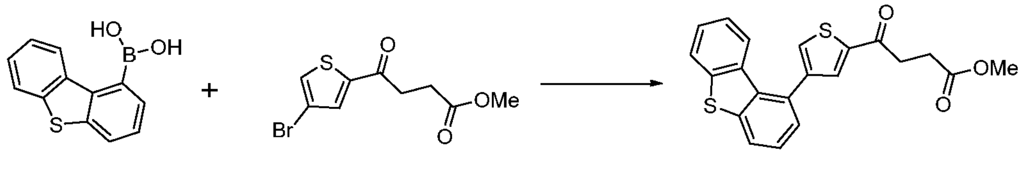

The preparation method of methyl 4-(4-(dibenzo[b,d]thiophen-4-yl)thiophen-2-yl)-4-oxobutyrate **(9'-1)** was the same as that of compound **4'-1,** and an orange-yellow solid was obtained with a yield of 54%. $^1$H NMR (400 MHz, $CDCl_3$) $\delta$8.34 (d, *J* = 1.4 Hz, 1H), 8.20-8.27 (m, 1H), 8.14 (d, *J* = 1.4 Hz , 1H), 7.81-7.93 (m, 3H), 7.68 (dd, *J* = 1.7, 8.3 Hz ,1H), 7.47-7.53 (m, 2H), 3.73 (s, 3H), 3.36 (t, *J* = 6.7 Hz, 2H), 2.83 (t, *J*= 6.7 Hz, 2H).

Step 2: 4-(4-(dibenzo[b,d]thiophen-4-yl)thiophen-2-yl)-4-oxobutyric acid **(I'-9)**

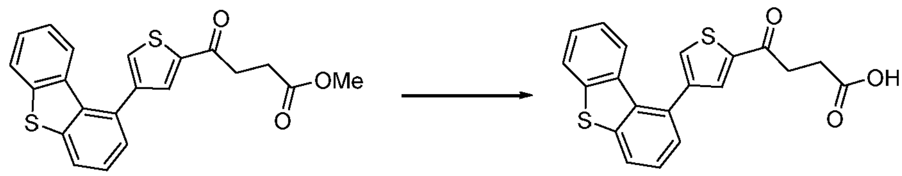

The preparation method of 4-(4-(dibenzo[b,d]thiophen-4-yl)thiophen-2-yl)-4-oxobutyric acid **(I'-9)** was the same as that of compound **I'-4,** and a yellow solid was obtained with a yield of 95%. $^1$H NMR (400 MHz, DMSO-$d_6$) $\delta$8.88 (s, 1H), 8.69 (s, 1H), 8.46-8.58 (m, 2H), 7.96-8.14 (m, 3H), 7.52-7.61 (m, 2H), 3.28 (t, *J* = 6.2 Hz, 2H), 2.61 (t, *J* = 6.2 Hz, 2H).

Example 24 4-(4-(benzo[b]thiophen-4-yl)thiophen-2-yl)-4-oxobutyric acid **(I'-10)**

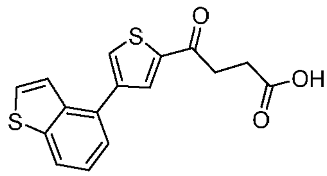

Step 1: 2-(benzo[b]thiophen-4-yl)-4,4,5,5-tetramethyl-1,3,2-dioxaborane **(10'-1)**

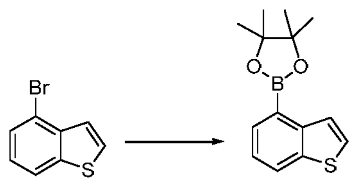

A mixture of 4-bromobenzo[b]thiophene (220 mg), pinacol diboronate (202 mg), [1,1'-bis(diphenylphosphino)ferrocene]palladium dichloride (12 mg), glycol dimethyl ether (2 mL) and potassium acetate (125 mg) was placed in a sealed tube, and stirred at 80 °C overnight under argon protection. The reaction solution was concentrated to dryness, and subjected to column chromatography with ethyl acetate: petroleum ether = 1:7 to obtain 156mg of 2-(benzo[b]thiophen-4-yl)-4,4,5,5-tetramethyl-1,3,2-dioxaborane (10'-1) as a yellow solid with a yield of 60%. $^1$H NMR (400 MHz, DMSO-$d_6$) $\delta$8.02 (d, *J* = 4.0 Hz ,1H), 7.65 (d, *J* = 4.0 Hz ,1H), 3.59 (s, 3H), 3.27 (t, *J* = 6.4 Hz, 2H), 2.67 (t, *J* = 6.4 Hz, 2H).

Step 2: methyl 4-(4-(benzo[b]thiophen-4-yl)thiophen-2-yl)-4-oxobutyrate(**10'-2)**

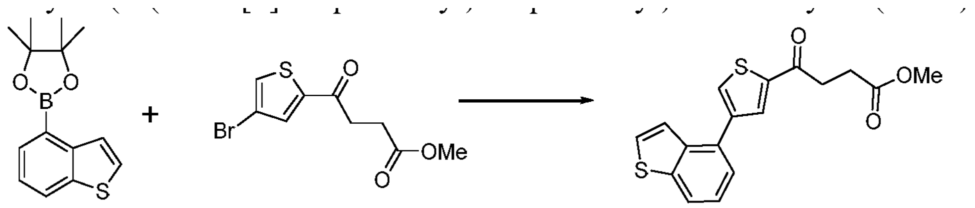

The preparation method of methyl 4-(4-(benzo[b]thiophen-4-yl)thiophen-2-yl)-4-oxobutyrate (**10'-2**) was the same as that of compound **4'-1,** and a yellow oily liquid was obtained with a yield of 70%. $^1$H NMR (400 MHz, $CDCl_3$) $\delta$8.01 (d, *J* = 1.2 Hz, 1H), 7.90 (dd, *J* = 2.6, 6.2 Hz, 1H), 7.76(d, *J* = 1.2 Hz, 1H), 7.48-7.54 (m , 2H), 7.39-7.42 (m, 2H), 3.72 (s, 3H), 3.32 (t, *J*= 6.7 Hz, 2H), 2.81 (t, *J* = 6.7 Hz, 2H).

Step 3: 4-(4-(benzo[b]thiophen-4-yl)thiophen-2-yl)-4-oxobutyric acid (**I'-10**)

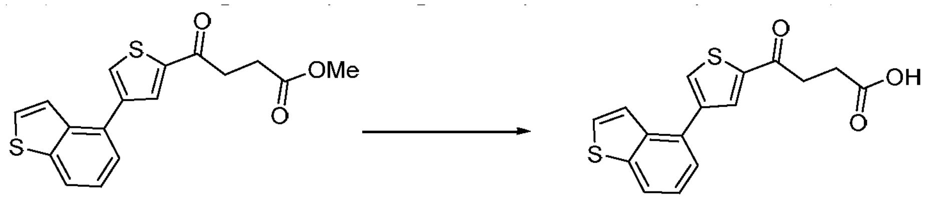

The preparation method of 4-(4-(benzo[b]thiophen-4-yl)thiophen-2-yl)-4-oxobutyric acid **(I'-10)** was the same as that of compound **I'-4,** and an off-white solid was obtained with a yield of 94%. $^1$H NMR (400 MHz, DMSO-$d_6$) $\delta$12.2 (br s, 1H), 8.28 (d, *J*= 1.4 Hz , 1H), 7.22 (d, *J* = 1.4 Hz , 1H), 8.04 (d, *J* = 7.8 Hz ,1H), 7.85 (d, *J* = 5.5 Hz , 1H), 7.61 (d, *J* = 5.6 Hz , 1H), 7.53 (d, *J* = 7.4 Hz , 1H), 7.44 (t, *J* = 7.6 Hz , 1H), 3.30 (t, *J* = 6 .4 Hz, 2H), 2.58 (t, *J* = 6.7 Hz, 2H).

**Example 25 4-(5-(benzo[b]thiophen-4-yl)thiophen-2-yl)-4-oxobutyric acid (I'-11)**

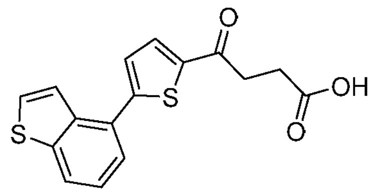

Step 1: methyl 4-(5-benzo[b]thiophen-4-yl)thiophen-2-yl)-4-oxobutyrate **(11'-1)**

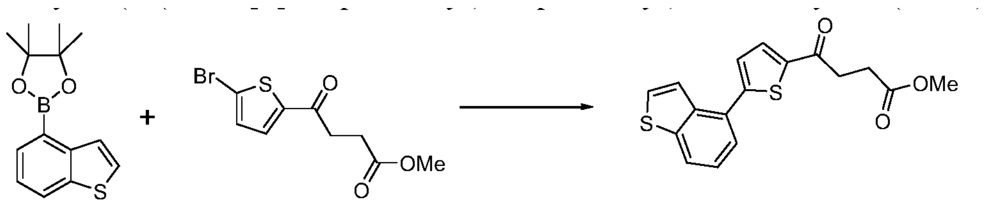

The preparation method of methyl 4-(5-(benzo[b]thiophen-4-yl)thiophen-2-yl)-4-oxobutyrate **(11'-1)** was the same as that of compound **4'-1,** and a yellow oil was obtained with a yield of 59%. $^1$H NMR (400 MHz, $CDCl_3$) $\delta$8.01(d, *J* = 1.2 Hz, 1H), 7.90 (dd, *J*= 2.6, 6.2 Hz, 1H), 7.76 (d, *J*= 1.2 Hz, 1H), 7.48-7.54 (m, 2H), 7.39-7.42 (m, 2H), 3.72 (s,

3H), 3.32 (t, J= 6.7 Hz, 2H), 2.81 (t, J = 6.7 Hz, 2H).

Step 2: 4-(5-(benzo[b]thiophen-4-yl)thiophen-2-yl)-4-oxobutyric acid **(I'-11)**

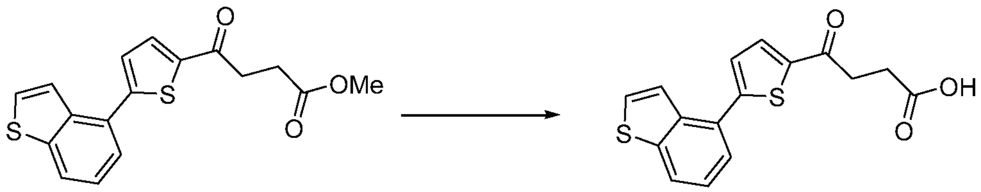

The preparation method of 4-(5-(benzo[b]thiophen-4-yl)thiophen-2-yl)-4-oxobutyric acid **(I'-11)** was the same as that of compound **I'-4,** and a pale yellow solid was obtained with a yield of 85%. $^1$H NMR (400 MHz, DMSO-$d_6$) δ 12.2 (br s, 1H), 8.11 (d, J = 8.0 Hz, 1H), 8.07 (d, J = 4.0 Hz, 1H), 7.94 (d, J= 5.5 Hz, 1H), 7.76 (d, J= 5.7 Hz, 1H), 7.57-7.64 (m, 2H), 7.46 (t , J = 7.7 Hz, 1H), 3.24 (t , J = 6.3 Hz, 2H), 2.60 (t, J = 6.3 Hz, 2H).

**Example 26 4-(4-(2-methylbenzo[b]thiophen-3-yl)thiophen-2-yl)-4-oxobutyric acid (I'-12**)

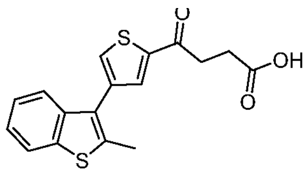

Step 1: 4,4,5,5-tetramethyl-2-(2-methylbenzo[b]thiophen-3-yl)-1,3,2-dioxaborane **(12'-1)**

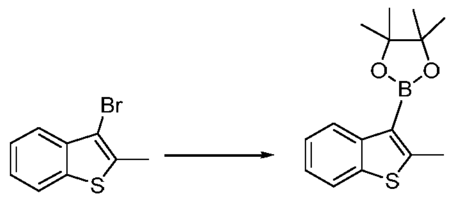

The preparation method of 4,4,5,5-tetramethyl-2-(2-methylbenzo[b]thiophen-3-yl)-1,3,2-dioxaborane **(12'-1)** was the same as that of compound **10'-1**, and a yellow oil was obtained with a yield of 42%. $^1$H NMR (400 MHz, $CDCl_3$) δ 8.28 (d, J= 8.0 Hz, 1H), 7.73 (d, J= 8.0 Hz, 1H), 7.32 (t, J= 7.6 Hz, 1H), 7.21-7.26 (m, 1H), 2.81 (s, 3H), 1.38 (s, 12H).

Step 2: methyl 4-(4-(2-methylbenzo[b]thiophen-3-yl)thiophen-2-yl)-4-oxobutyrate **(12'-2)**

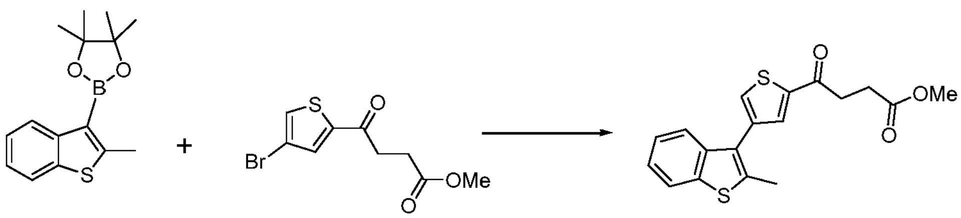

The preparation method of methyl 4-(4-(2-methylbenzo[b]thiophen-3-yl)thiophen-2-yl)-4-oxobutyrate(**12'-2**) was the same as that of compound **4'-1,** and a yellow oil was obtained with a yield of 57%. $^1$H NMR (400 MHz, $CDCl_3$) $\delta$ 7.85 (d, *J* = 1.1 Hz, 1H), 7.77-7.82 (m, 1H), 7.62 (d, *J* = 1.1 Hz, 1H), 7.51-7.56 (m, 1H), 7.28-7.36 (m, 2H), 3.72 (s, 3H), 3.31 (t, *J*= 6.7 Hz, 2H), 2.80 (t, *J*= 6.7 Hz, 2H), 2.54 (s, 3H).

Step 3: 4-(4-(2-methylbenzo[b]thiophen-3-yl)thiophen-2-yl)-4-oxobutyric acid **(I'-12)**

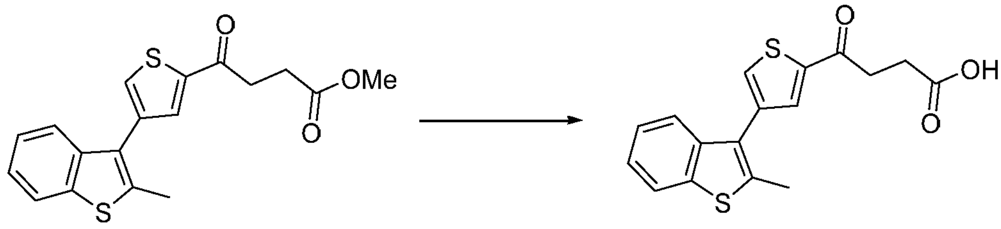

The preparation method of 4-(4-(2-methylbenzo[b]thiophen-3-yl)thiophen-2-yl)-4-oxobutyric acid **(I'-12)** was the same as that of compound **I'-4,** and an off-white solid was obtained with a yield of 100%. $^1$H NMR (400 MHz, DMSO-*d*$_6$) $\delta$ 12.1 (br s, 1H), 8.08-8.15 (m, 2H), 7.91-7.96 (m, 1H), 7.54-7.60 (m, 1H), 7.30-7.40 (m, 2H), 3.27 (t, *J*= 6.4 Hz, 2H), 2.58 (t, *J* = 6.3 Hz, 2H), 2.53 (s, 3H).

**Example 27 4-(4-(benzo[b]thiophen-3-yl)-5-methylthiophen-2-yl)-4-oxobutyric acid (I'-13)**

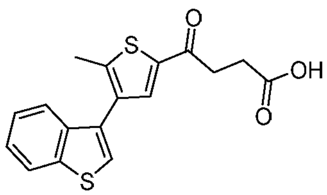

Step 1: methyl 4-(5-methylthiophen-2-yl)-4-oxobutyrate **(13'-1)**

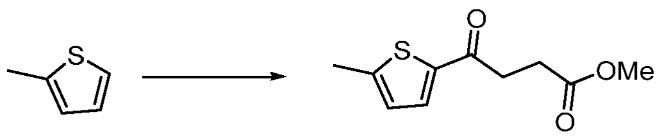

The preparation method of methyl 4-(5-methylthiophen-2-yl)-4-oxobutyrate **(13'-1)** was the same as that of compound **5'-1**, and a yellow solid was obtained with a yield of 74%. $^1$H NMR (400 MHz, $CDCl_3$) $\delta$ 7.58 (d, *J*= 3.8 Hz, 1H), 6.80 (d, *J*= 8.0 Hz, 1H), 3.70 (s, 3H), 3.20 (t, *J*= 6.8 Hz, 2H), 2.74 (t, *J*= 6.8 Hz, 2H), 2.53 (s, 3H).

Step 2: methyl 4-(4-bromo-5-methylthiophen-2-yl)-4-oxobutyrate **(13'-2)**

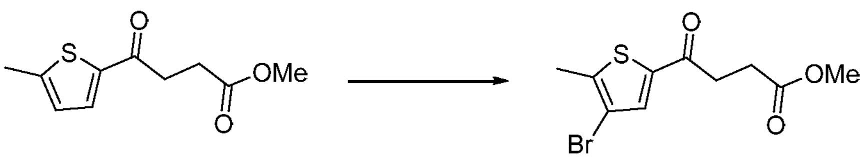

Aluminum trichloride (301 mg) was dissolved in dichloromethane (5 mL), compound **13'-1** (200 mg ) was added under ice bath under the protection of argon, stirred under an ice bath for 30 minutes, a solution of 1M bromine in dichloromethane (690 μL) was slowly added dropwise, and reacted under an ice bath for 3 hours. The reaction solution was poured into water, extracted with dichloromethane. The organic layer was concentrated to dryness, and purified by column chromatography (ethyl acetate: petroleum ether = 1: 100) to obtain 80 mg of methyl 4-(4-bromo-5-methylthiophen-2-yl)-4-oxobutyrate **(13'-2)** as a yellow solid with a yield of 29%. $^1$H NMR (400 MHz, $CDCl_3$) $\delta$ 7.57 (s, 1H), 3.70 (s, 3H), 3.17 (t, *J*= 6.7 Hz, 2H), 2.75 (t, *J*= 6.7 Hz, 2H), 2.45 (s, 3H).

Step 3: methyl 4-(4-(benzo[b]thiophen-3-yl)-5-methylthiophen-2-yl)-4-oxobutyrate **(13'-3)**

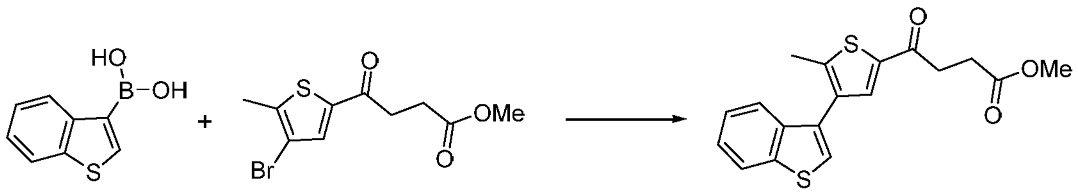

The preparation method of methyl 4-(4-(benzo[b]thiophen-3-yl)-5-methylthiophen-2-yl)-4-oxobutyrate **(13'-3)** was the same as that of compound **4'**-1, and a yellow oil was obtained with a yield of 85%. $^1$H NMR (400 MHz, $CDCl_3$) $\delta$ 7.89-7.97 (m, 1H), 7.74 (s, 1H), 7.56-7.64 (m , 1H), 7.37-7.45 (m, 2H), 7.35 (s, 1H), 3.71 (s, 3H), 3.24 (t, *J*= 6.7 Hz, 2H), 2.77 (t, *J*= 6.7 Hz, 2H), 2.45 (s, 3H).

Step 4: 4-(4-(benzo[b]thiophen-3-yl)-5-methylthiophen-2-yl)-4-oxobutyric acid **(I'-13)**

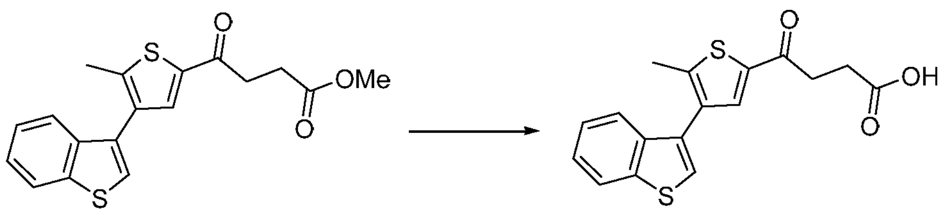

The preparation method of 4-(4-(benzo[b]thiophen-3-yl)-5-methylthiophen-2-yl)-4-oxobutyric acid **(I'-13)** was the same as that of compound **I'-4** , an off-white solid was obtained with a yield of 100%. $^1$H NMR (400 MHz, DMSO-*d*$_6$) $\delta$ 12.1 (br s, 1H), 8.07 (dd, *J* = 3.0, 5.9 Hz, 1H), 8.00 (s, 1H), 7.84 (s, 1H), 7.63 (dd, *J*= 3.2, 6.0 Hz, 1H), 7.40-7.60 (m, 2H), 3.20 (t, *J* = 6.3 Hz, 2H), 2.55 (t , *J* = 6.3 Hz, 2H), 2.43 (s, 3H).

**Example 28 4-(4-(benzofuran-3-yl)thiophen-2-yl)-4-oxobutyric acid (I'-14)**

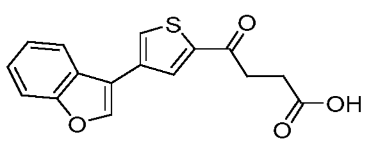

Step 1: methyl 4-(4-(benzofuran-3-yl)thiophen-2-yl)-4-oxobutyrate **(14'-1)**

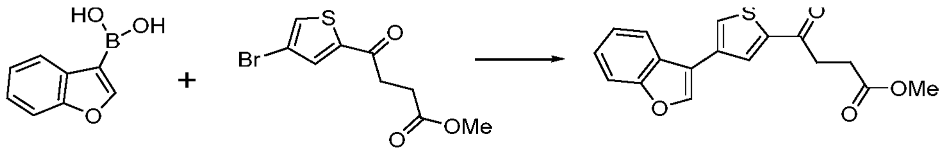

The preparation method of methyl 4-(4-(benzofuran-3-yl)thiophen-2-yl)-4-oxobutyrate **(14'-1**) was the same as that of compound **4'-1,** and a yellow solid was obtained with a yield of 72%. $^1$H NMR (400 MHz, $CDCl_3$) $\delta$ 8.03 (s, 1H), 7.88 (s, 1H), 7.80-7.86 (m, 2H), 7.59 (d, *J*= 7.7 Hz, 1H), 7.34-7.44 (m , 2H), 7.07 (s, 3H), 3.75 (s, 3H), 3.35 (t, *J*= 6.7 Hz, 2H), 2.84 (t, *J*= 6.7 Hz, 2H).

Step 2: 4-(4-(benzofuran-3-yl)thiophen-2-yl)-4-oxobutyric acid **(I'-14**)

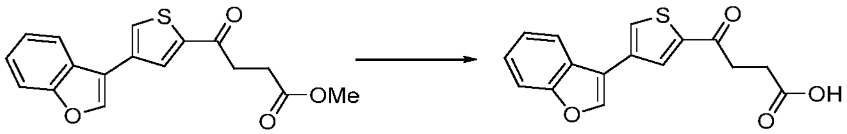

The preparation method of 4-(4-(benzofuran-3-yl)thiophen-2-yl)-4-oxobutyric acid **(I'-14**) was the same as that of compound **I'-4** with a yield of 100%. $^1$H NMR (400 MHz, DMSO-$d_6$) $\delta$ 12.25(s, 1H), 8.59 (s, 1H), 8.47 (s, 1H), 8.42 (s, 1H), 8.10 (d, *J* = 7.6 Hz, 1H), 7.69 (d, *J* = 7.8 Hz, 1H), 7.36-7.49 (m, 2H), 3.31 (t , *J* = 6.3 Hz , 2H), 2.63 (t , *J* = 6.3 Hz , 2H).

**Example 29 4-(4-(benzofuran-7-yl)thiophen-2-yl)-4-oxobutyric acid (I'-15)**

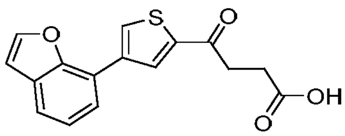

Step 1: 1-bromo-2-(2,2-diethoxyethoxy)benzene (**15'-1**)

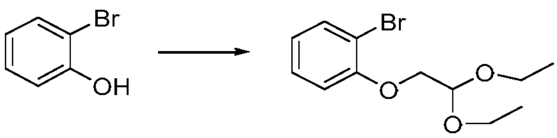

A mixture of 2-bromophenol (1 g), bromoacetaldehyde diethyl acetal (1.25 g), potassium carbonate (959 mg) and N,N-dimethylformamide (10 mL) was stirred at 160 °C for 3 hours under the protection of argon. The reaction solution was poured into water, extracted with ethyl acetate. The organic layer was concentrated to dryness, and subjected to column chromatography with ethyl acetate: petroleum ether=1:100 to obtain 1.56 g of 1-bromo-2-(2,2-diethoxyethoxy)benzene (15'-1) as a colorless oily liquid with a yield of 93%. $^1$H NMR (400 MHz, $CDCl_3$) $\delta$ 8.30 (s, 1H), 7.37-7.96 (m, 2H), 7.22 (t, *J*= 7.9 Hz, 1H), 3.71 (s, 3H), 3.27 (t, *J* = 6.6 Hz, 2H), 2.77 (t, *J*= 6.6 Hz, 2H).

Step 2: 7-bromobenzofuran **(15'-2)**

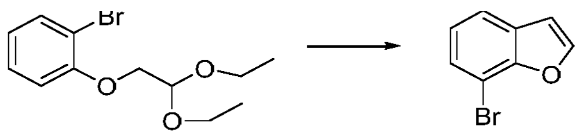

A mixture of polyphosphoric acid (467 mg), phosphorus pentoxide (45 mg) and chlorobenzene (10 mL) was heated to 130 ° C, a solution of compound **15'-1** (1 g) in chlorobenzene was added dropwise ( 5mL), and the temperature was raised to 160 ° C and stirred for 4.5 hours after addition. Chlorobenzene was spin-dried, and the residue was extracted with ethyl acetate. The organic layer was concentrated to dryness, and subjected to column chromatography with ethyl acetate: petroleum ether=1:100 to obtain 316 mg of 7-bromobenzofuran **(15'-2)** as a yellow oil with a yield of 46%. $^1$H NMR (400 MHz, $CDCl_3$) $\delta$ 7.69 (d, *J* = 2.1 Hz, 1H), 7.54 (dd, *J* = 0.7, 7.8 Hz 1H), 7.46 (dd, *J* = 0.7, 7.8 Hz, 1H), 7.21 (t, *J* = 7.8 Hz, 1H), 6.85 (t, *J*= 2.1 Hz, 1H).

Step 3: 2-(benzofuran-7-yl)-4,4,5,5-tetramethyl-1,3,2-dioxaborane (**15'-3**)

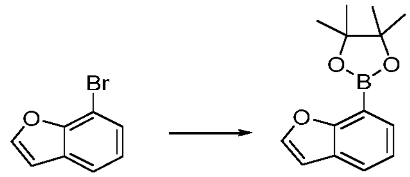

The preparation method of 2-(benzofuran-7-yl)-4,4,5,5-tetramethyl-1,3,2-dioxaborane **(15'-3)** was the same as that of compound **10'-1**, an off-white solid was obtained with a yield of 100%. $^1$H NMR (400MHz, $CDCl_3$) $\delta$ 7.68-7.77 (m, 3H), 7.23 (d, *J* = 7.4 Hz, 1H), 6.75 (d, *J*= 2.2 Hz, 1H), 1.41 (s, 12H).

Step 4: methyl 4-(4-(benzofuran-7-yl)thiophen-2-yl)-4-oxobutyrate **(15'-4)**

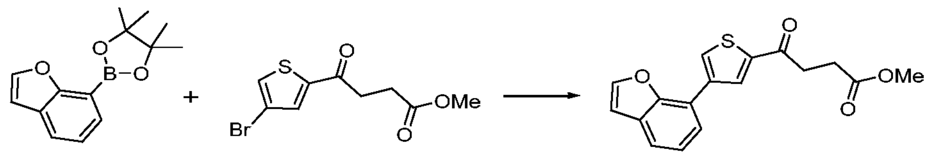

The preparation method of methyl 4-(4-(benzofuran-7-yl)thiophen-2-yl)-4-oxobutyrate **(15'-4)** was the same as that of compound **4'-1,** a yellow oil was obtained with a yield of 51%. $^1$H NMR (400 MHz, $CDCl_3$) $\delta$ 8.35 (d, *J*= 1.3 Hz, 1H), 8.25 (d, *J* = 1.3 Hz, 1H), 7.74 (d, *J* = 2.2 Hz, 1H), 7.53-7.61 (m, 2H), 7.32 (d, *J* = 7.7 Hz, 1H), 6.86 (d, *J* = 2.2 Hz, 1H), 3.72 (s, 3H), 3.37 (t, *J* = 6.7 Hz, 2H), 2.81 (t, *J* = 6.7 Hz, 2H).

Step 5: 4-(4-(benzofuran-7-yl)thiophen-2-yl)-4-oxobutyric acid **(I'-15)**

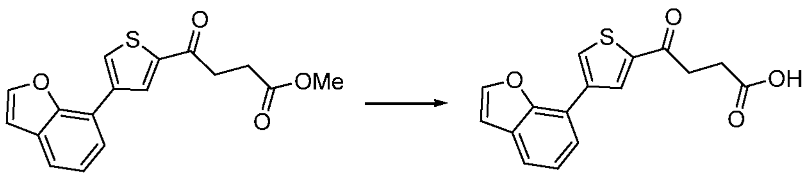

The preparation method of 4-(4-(benzofuran-7-yl)thiophen-2-yl)-4-oxobutyric acid **(I'-15)** was the same as that of compound **I'-4,** and an off-white solid was obtained with a yield of 68%. $^1$H NMR (400 MHz, $CDCl_3$) $\delta$ 7.97 (s, 1H), 7.72 (d, *J* = 7.7 Hz, 1H), 7.58 (d, *J* = 8.1 Hz, 1H), 7.51 (s, 1H), 7.32-7.46 (m, 2H), 3.27 (t, *J*= 6.6 Hz, 2H), 2.82 (t , *J* =

6.3 Hz, 2H).

**Example 30 4-(4-(lH-indazol-3-yl)thiophen-2-yl)-4-oxobutyric acid (I'-16)**

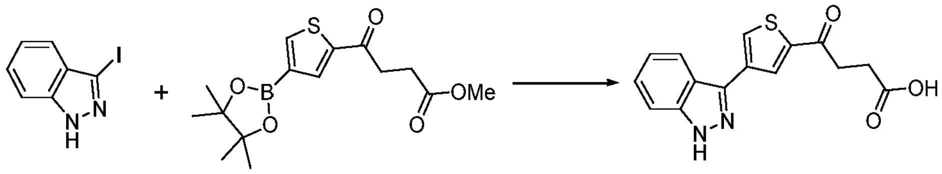

A mixture of 3-iodo-1H-indazole (100 mg), compound **1'-3** (146 mg), bistriphenylphosphonium palladium dichloride (14 mg), sodium carbonate (108 mg), dioxane (1 mL) and deionized water (1 mL) was stirred at 70 °C overnight under the protection of argon. The reaction solution was concentrated to dryness, and purified by preparative thin layer chromatography (dichloromethane: methanol = 10:1) to obtain 15 mg of 4-(4-(1H-indazol-3-yl)thiophen-2-yl)-4-oxobutyric acid **(I'-16)** as a pale yellow solid with a yield of 12%. $^1$H NMR (400 MHz, DMSO-$d_6$) $\delta$ 11.87 (s, 1H), 8.09 (s, 1H), 8.01 (s, 1H), 7.24-7.34 (m, 2H), 6.93 (d, *J* = 5.8 Hz, 2H), 4.52 (s, 1H), 3.08 (t, *J* = 6.6 Hz, 2H), 2.25 (t, *J* = 6.6 Hz, 2H).

**Example 31 4-(4-(benzofuran-3-yl)furan-2-yl)-4-oxobutyric acid (I'-17)**

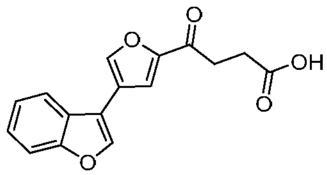

Step 1: 4-(benzofuran-3-yl)furan-2-carbaldehyde **(17'-1)**

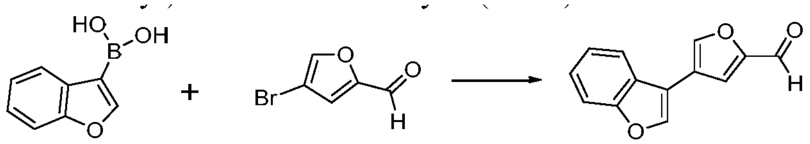

The preparation method of 4-(benzofuran-3-yl)furan-2-carbaldehyde **(17'-1)** was the same as that of compound **4'-1,** a yellow solid was obtained with a yield of 79%. $^1$H NMR (400 MHz, $CDCl_3$) $\delta$ 9.75(s, 1H), 8.04 (s, 1H), 7.84 (s, 1H), 7.70 (d, *J* = 7.3 Hz, 1H), 7.50-7.60 (m, 2H), 7.31-7.43 (m, 2H).

Step 2: methyl 4-(4-(benzofuran-3-yl)furan-2-yl)-4-oxobutyrate **(17'-2)**

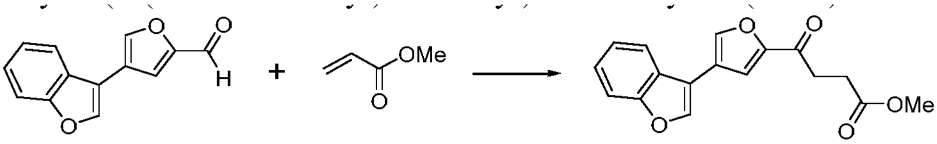

3-benzylhydroxyethylmethylthiazole lithium chloride (51 mg), 1-butyl-3-methylimidazol hexafluorophosphate (112 μL), 4-(benzofuran-3-yl) furan-2-carbaldehyde (200mg), methyl acrylate (163mg) and triethylamine (57mg) were placed in a sealed tube and reacted at 80 ° C overnight under the protection of argon. The reaction solution was concentrated to

dryness, and subjected by column chromatography with ethyl acetate: petroleum ether = 1:15 to obtain **57mg** of methyl 4-(4-(benzofuran-3-yl)furan-2-yl)-4-oxobutyrate (17'-2) with a yield of 20%. $^1$H NMR (400 MHz, $CDCl_3$) $\delta$ 7.97 (s, 1H), 7.84 (s, 1H), 7.72 (d, *J* = 7.7 Hz, 1H), 7.58 (d, *J* = 8.1 Hz, 1H), 7.51 (s, 1H), 7.32-7.46 (m, 2H), 3.74 (s,3H), 3.27 (t, *J* = 6.6 Hz, 2H), 2.82 (t, *J* = 6.6 Hz, 2H).

Step 3: 4-(4-(benzofuran-3-yl)furan-2-yl)-4-oxobutyric acid **(I'-17)**

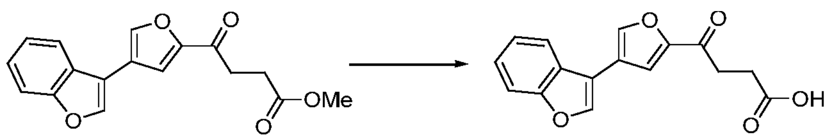

Methyl 4-(4-(benzo[b]thiophen-3-yl)thiophen-2-yl)-4-oxobutyrate was replaced with methyl 4-(4-(benzofuran-3-yl)furan- 2-yl)-4-oxobutyrate, other required raw materials, reagents and preparation methods were the same as the preparation of compound **I'-1**, **and** 4-(4-(benzofuran-3-yl)furan -2-yl)-4-oxobutyric acid **(I'-17)** was obtained as a yellow solid with a yield of 100%. $^1$H NMR (400 MHz, DMSO-$d_6$) $\delta$ 12.25 (br s, 1H), 8.66 (s, 1H), 8.49 (s, 1H), 7.06-7.97 (m, 3H), 7.67 (d , *J* = 7.8 Hz , 1H), 7.29-7.48 (m, 2H), 3.14 (t , *J* = 6.7 Hz , 2H), 2.61 (t , *J* = 6.7 Hz , 2H).

**Example 32 4-(4-(3-amino-1H-indazol-4-yl)thiophen-2-yl)-4-oxobutyric acid (I'-18)**

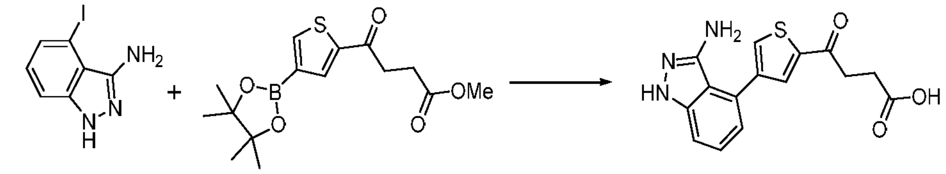

The preparation method of 4-(4-(3-amino-1H-indazol-4-yl)thiophen-2-yl)-4-oxobutyric acid **(I'-18)** was the same as that of compound **I'-16,** and a yellow solid was obtained with a yield of 9%. $^1$H NMR (400MHz, DMSO-$d_6$) $\delta$ 13.30 (s,1H), 12.23 (s, 1H), 8.52 (s, 1H), 8.47 (s, 1H), 8.18 (d, *J* = 8.3 Hz, 1H), 7.60 (d, *J* = 8.3 Hz, 1H), 7.43 (t, *J* = 7.6 Hz, 1H), 7.24 (d, *J* = 7.6 Hz, 1H), 4.52 (s,2H), 3.34 (t, *J*= 6.2 Hz, 2H), 2.61 (t, *J* = 6.2 Hz, 2H).

**Example 33 4-(4-(benzo[d][1,3]dioxol-5-yl)furan-2-yl)-4-oxobutyric acid (I'-19)**

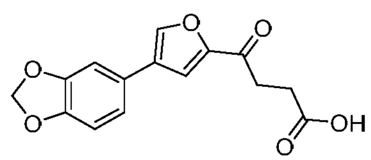

Step 1: methyl 4-(4-(benzo[d][1,3]dioxol-5-yl)furan-2-yl)-4-oxobutyrate **(19'-1** )

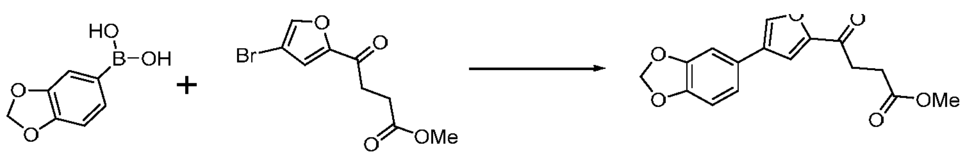

The preparation method of methyl 4-(4-(benzo[d][1,3]dioxol-5-yl)furan-2-yl)-4-oxobutyrate **(19'-1)** was the same as that of compound **4'-1,** and a white solid was obtained with a yield of 76%. $^1$H NMR (400MHz, $CDCl_3$) $\delta$ 7.75 (s, 1H), 7.41 (s, 1H), 6.95-6.98 (m, 2H), 6.86 (d, *J*= 7.6 Hz, 1H), 6.00 (s, 2H), 3.71 (s, 3H), 3.22 (t, *J* = 6.8 Hz, 2H) , 2.78 (t, *J* = 6.8 Hz, 2H).

Step 2: 4-(4-(benzo[d][1,3]dioxol-5-yl)furan-2-yl)-4-oxobutyric acid **(I'-19)**

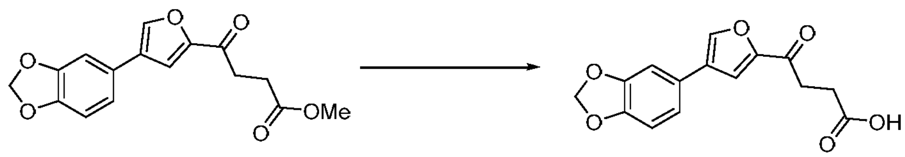

The preparation method of 4-(4-(benzo[d][1,3]dioxol-5-yl)furan-2-yl)-4-oxobutyric acid **(I'-19)** was the same as that of compound **I'-4,** and a pale yellow solid was obtained with a yield of 73%. $^1$H NMR (400 MHz, DMSO-$d_6$) $\delta$ 8.42 (d, *J* = 4.0 Hz, 1H), 7.95 (s, 1H), 7.35 (d, *J* = 4.0 Hz, 1H), 7.24 (dd, *J*= 4.0, 8.0 Hz, 1H), 6.98 (d, *J*= 8.0 Hz, 1H), 6.05 (s, 2H), 3.10 (t, *J* = 8.0 Hz, 2H), 2.59 (t, *J* = 8.0 Hz, 2H).

**Example 34 4-(4-(benzofuran-5-yl)furan-2-yl)-4-oxobutyric acid (I'-20)**

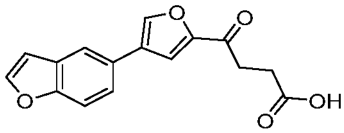

Step 1: methyl 4-(4-(benzofuran-5-yl)furan-2-yl)-4-oxobutyrate **(20'-1)**

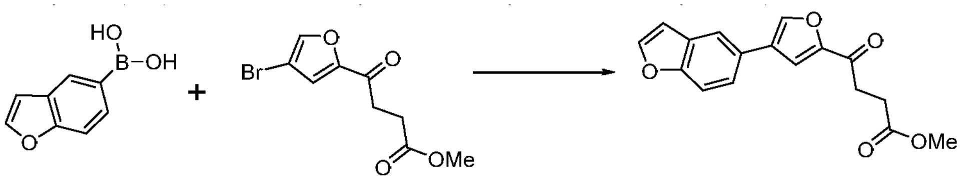

The preparation method of methyl 4-(4-(benzofuran-5-yl)furan-2-yl)-4-oxobutyrate **(20'-1)** was the same as that of compound **4'-1,** and a white solid was obtained with a yield of 79%. $^1$H NMR (400MHz, $CDCl_3$) $\delta$ 7.85 (s,1H), 7.72 (d, *J* = 2.0 Hz, 1H), 7.66 (d, *J*= 2.0 Hz, 1H), 7.52-7.55 (m, 2H), 7.44 (dd, *J*= 2.0, 8.4 Hz, 1H), 6.80 (dd, *J* = 0.8, 2.4 Hz, 1H), 3.72 (s, 3H), 3.25 (t, *J* = 6.8 Hz, 2H) , 2.80 (t, *J* = 6.8 Hz, 2H).

Step 2: 4-(4-(benzofuran-5-yl)furan-2-yl)-4-oxobutyric acid **(I'-20)**

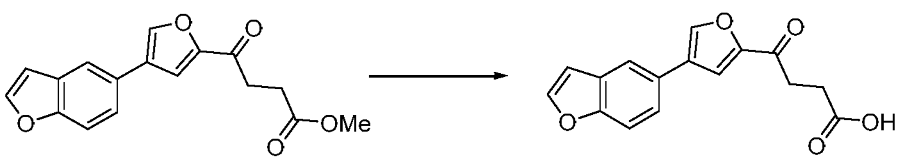

The preparation method of 4-(4-(benzofuran-5-yl)furan-2-yl)-4-oxobutyric acid (**I'-20**) was the same as that of

compound **I'-4,** and a white solid was obtained with a yield of 42%. $^1$H NMR (400 MHz, DMSO-$d_6$) $\delta$ 12.22 (s, 1H), 8.51 (s, 1H), 8.02-8.04 (m, 3H), 7.64-7.69 (m, 2H), 6.99 (d, $J$= 4.0 Hz, 1H), 3.14 (t, $J$= 8.0 Hz, 2H), 2.61 (t, $J$ = 8.0 Hz, 2H).

**Example 35 4-(4-(benzofuran-4-yl)furan-2-yl)-4-oxobutyric acid (I'-21)**

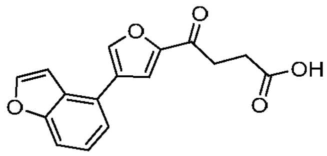

Step 1: 2-(2,6-dibromophenyl)acetaldehyde **(21'-1)**

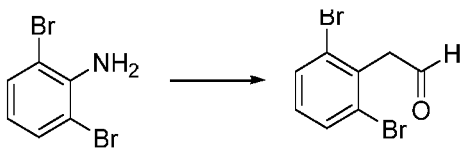

2,6-dibromoaniline (300mg) was dissolved in acetone (1mL), concentrated hydrochloric acid (0.3mL) was added and the mixture was cooled under an ice bath. Sodium nitrite (85mg sodium nitrite was dissolved in 3.8mL mixed solvent of water/acetone = 1 : 1) was slowly added, stirred for 5 minutes, and a mixture of vinyl ether (862mg) and acetone (2.6mL) and a mixture of ferrocene (38mg) dissolved in acetone (3.8mL) were added, respectively. The mixture was stirred for 10 minutes under an ice bath. The reaction solution was poured into saturated sodium bicarbonate solution, extracted with ethyl acetate, and the organic phase was concentrated to dryness, and subjected to column chromatography with petroleum ether to obtain 229 mg of 2-(2,6-dibromophenyl)acetaldehyde **(21'-1)** as an off-white solid with a yield of 69%. $^1$H NMR (400 MHz, $CDCl_3$) $\delta$ 9.60 (d, $J$ = 7.5 Hz, 1H), 8.17 (s, 1H), 7.02 (d, $J$= 8.1 Hz, 1H), 7.05(d, $J$ = 8.1 Hz, 2H).

Step 2: 4-bromobenzofuran **(21'-2)**

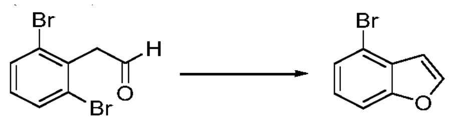

2-(2,6-dibromophenyl)acetaldehyde (200 mg) was dissolved in anhydrous N,N-dimethylformamide (3 mL), and cuprous iodide (7 mg) and potassium phosphate (204 mg) were added to react at 70 ° C for 2 hours under the protection of argon. The reaction solution was poured into water, extracted with ethyl acetate, and the organic phase was concentrated to dryness, and subjected to column chromatography with petroleum ether to obtain 50mg of 4-bromobenzofuran **(21'-2)** as a pale yellow solid with a yield of 35%. $^1$H NMR (400 MHz, $CDCl_3$) $\delta$ 7.67 (d, $J$ = 2.2 Hz, 1H), 7.46 (d, $J$ = 8.2 Hz, 1H), 7.40 (d, $J$ = 7.8 Hz, 1H), 7.17 (t, $J$ = 8.0 Hz, 1H), 6.86-6.79 (m, 1H).

Step 3: 2-(benzofuran-4-yl)-4,4,5,5-tetramethyl-1,3,2-dioxaborane **(21'-3)**

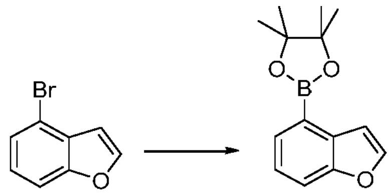

The preparation method of 2-(benzofuran-4-yl)-4,4,5,5-tetramethyl-1,3,2-dioxaborane **(21'-3)** was the same as that of compound **10'-1**, and a yellow solid was obtained, which is directly used in the next step.

Step 4: methyl 4-(4-bromofuran-2-yl)-4-oxobutyrate **(21'-4)**

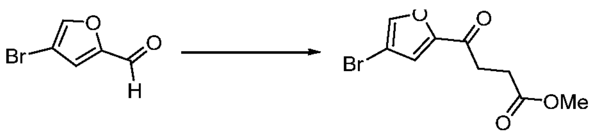

The preparation method of methyl 4-(4-bromofuran-2-yl)-4-oxobutyrate (**21'-4)** was the same as that of compound **17'-2,** and a yellow solid was obtained with a yield of 49%. $^1$H NMR (400 MHz, $CDCl_3$) $\delta$ 7.58 (s, 1H), 7.22 (s, 1H), 3.69 (s, 1H), 3.14 (t, *J*= 6.7 Hz, 1H), 2.74 (t, *J* = 6.7 Hz, 1H).

Step 5: methyl 4-(4-benzofuran-4-yl)furan-2-yl)-4-oxobutyrate(**21'-5**)

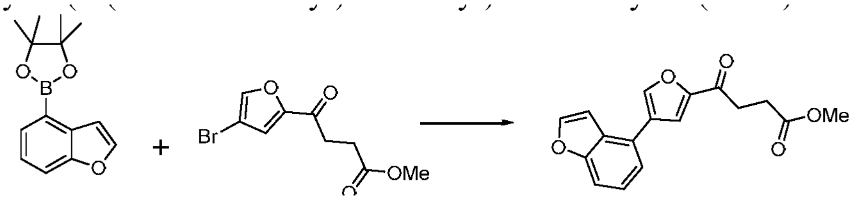

The preparation method of methyl 4-(4-(benzofuran-4-yl)furan-2-yl)-4-oxobutyrate **(21'-5)** was the same as that of compound **4'-1,** and a yellow solid was obtained with a yield of 46%. $^1$H NMR (400 MHz, $CDCl_3$) $\delta$ 7.92(s, 1H), 7.72 (s, 1H), 7.58 (s, 1H), 7.50 (d, *J* = 6.4 Hz, 1H), 7.31-7.36 (m, 2H), 6.93 (s, 1H), 3.72 (s, 3H), 3.26 (t, *J* = 6.8 Hz, 1H), 2.80 (t, *J* = 6.8 Hz, 1H).

Step 6: 4-(4-(benzofuran-4-yl)furan-2-yl)-4-oxobutyric acid **(I'-21)**

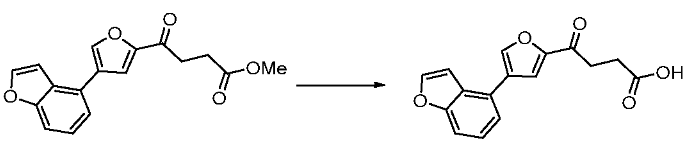

The preparation method of 4-(4-(benzofuran-4-yl)furan-2-yl)-4-oxobutyric acid **(I'-21)** was the same as that of compound **I'-4,** and a pale yellow solid was obtained with a yield of 93%. $^1$H NMR (400 MHz, DMSO-$d_6$) $\delta$ 12.2 (br s, 1H), 8.62 (s, 1H), 8.14 (s, 1H), 8.06 (s, 1H), 7.56-7.64 (m, 2H), 7.35-7.42 (m, 2H), 3.17 (t, *J* = 6.2 Hz, 2H), 2.60 (t, *J* = 6.2 Hz, 2H).

**Example 36** 4-(4-(benzofuran-6-yl)furan-2-yl)-4-oxobutyric acid **(I'-22)**

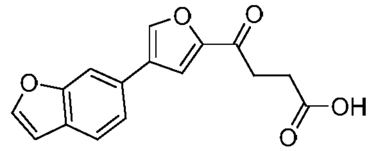

Step 1: 1-bromo-3-(2,2-diethoxyethoxy)benzene **(22'-1)**

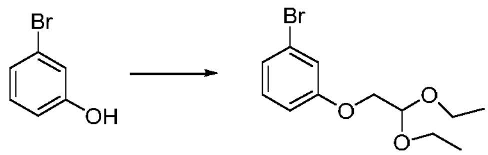

The preparation method of 1-bromo-3-(2,2-diethoxyethoxy)benzene **(22'-1)** was the same as that of compound **15'-1, and** a pale yellow solid was obtained with a yield of 93%. $^1$H NMR (400 MHz, $CDCl_3$) δ 7.05-7.16 (m, 3H), 6.83-6.88 (m, 1H), 4.81 (t, *J*= 5.2 Hz, 1H), 3.98 (d, *J* = 5.2 Hz, 2H), 3.71-3.81 (m, 2H), 3.57-3.68 (m, 2H), 1.25 (t, *J*= 7.1 Hz, 6H).

Step 2: 6-bromobenzofuran **(22'-2)**

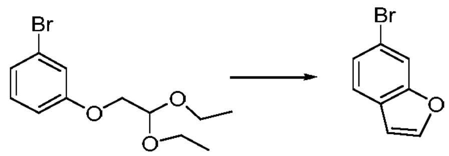

The preparation method of 6-bromobenzofuran **(22'-2)** was the same as that of compound **15'-2,** and a pale yellow solid was obtained with a yield of 32%. $^1$H NMR (400MHz, $CDCl_3$) δ 7.69 (s, 1H), 7.60(d, *J* = 2.2Hz, 1H), 7.46(d, *J* = 8.3Hz, 1H), 7.34-7.38(m, 1H), 6.75(dd, *J* = 2.2, 0.9Hz, 1H)

Step 3: 2-(benzofuran-6-yl)-4,4,5,5-tetramethyl-1,3,2-dioxaborane **(22'-3)**

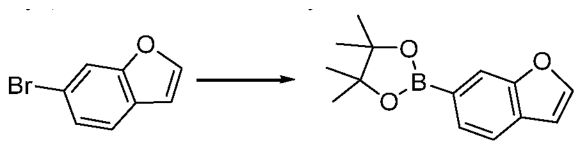

The preparation method of 2-(benzofuran-6-yl)-4,4,5,5-tetramethyl-1,3,2-dioxaborane **(22'-3)** was the same as that of compound **10'-1**, and a pale yellow solid was obtained, which was directly used in the next step.

Step 4: methyl 4-(4-(benzofuran-6-yl)furan-2-yl)-4-oxobutyrate **(22'-4)**

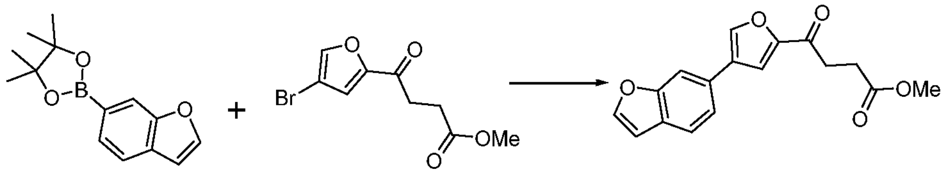

The preparation method of methyl 4-(4-(benzofuran-6-yl)furan-2-yl)-4-oxobutyrate **(22'-4)** was the same as that of compound **4'-1,** and a yellow solid was obtained with a yield of 46%. $^1$H NMR (400 MHz, $CDCl_3$) $\delta$ 7.92 (s, 1H), 7.72 (d, *J*= 2.0 Hz, 1H), 7.64(t, *J* = 8.6 Hz, 1H), 7.58 (s, 1H), 7.51 (d, *J* = 7.3 Hz, 1H), 7.34 (d, *J* = 1.3 Hz, 1H), 6.93 (s, 1H), 3.72 (s, 3H), 3.26 (t, *J* = 6.8 Hz, 2H), 2.80 (t, *J* = 6.8 Hz, 2H).

Step 5: 4-(4-(benzofuran-6-yl)furan-2-yl)-4-oxobutyric acid **(I'-22)**

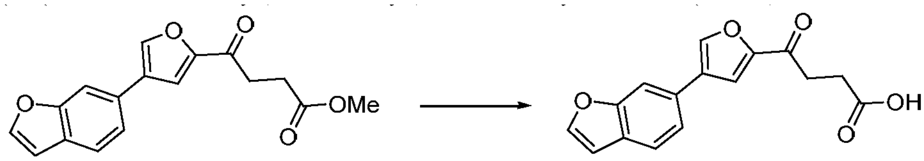

The preparation method of 4-(4-(benzofuran-6-yl)furan-2-yl)-4-oxobutyric acid (**I' -22)** was the same as that of compound **I'-4,** and a yellow solid was obtained with a yield of 48%. $^1$H NMR (400 MHz, DMSO-$d_6$) $\delta$ 12.1 (br s, 1H), 8.57 (s, 1H), 8.10 (s, 1H), 8.02-8.08 (m, 2H), 7.99-8.05 (m, 2H), 6.95-7.00 (m, 1H), 3.12 (t, *J* = 6.4 Hz, 2H), 2.60 (t, *J* = 6.4 Hz, 2H).

**Example 37 4-(4-(3,4-dimethoxyphenyl)furan-2-yl)-4-oxobutyric acid (I'-23)**

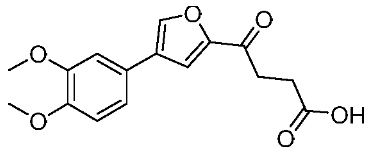

Step 1: methyl 4-(4-(3,4-dimethoxyphenyl)furan-2-yl)-4-oxobutyrate **(23'-1)**

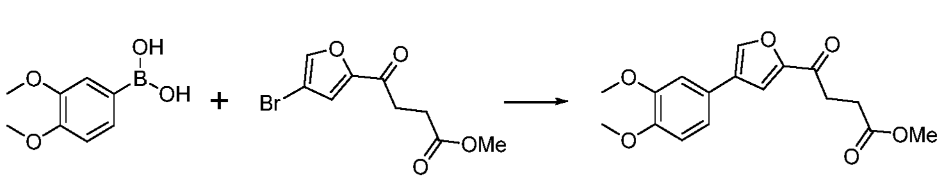

The preparation method of methyl 4-(4-(3,4-dimethoxyphenyl)furan-2-yl)-4-oxobutyrate **(23'-1)** was the same as that of compound **4'-1,** and a yellow solid was obtained with a yield of 39%. $^1$H NMR (400 MHz, $CDCl_3$) $\delta$ 7.78 (s, 1H), 7.06 (d, *J*= 8.2 Hz, 1H), 6.98 (s, 1H), 6.91 (d, *J*= 8.2 Hz, 1H), 3.94 (s, 3H), 3.91 (s, 3H), 3.71 (s, 3H), 3.22 (t, *J*= 6.8 Hz, 2H), 2.78 (t, *J* = 6.8 Hz, 2H).

Step 2: 4-(4-(3,4-dimethoxyphenyl)furan-2-yl)-4-oxobutyric acid **(I'-23)**

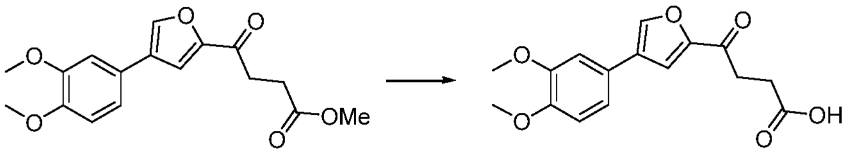

The preparation method of 4-(4-(3,4-dimethoxyphenyl)furan-2-yl)-4-oxobutyric acid **(I'-23)** was the same as that of compound **I'-4,** and a yellow solid was obtained with a yield of 48%. $^1$H NMR (400 MHz, DMSO-$d_6$) δ 12.2 (br s, 1H), 8.44 (s, 1H), 8.01 (s, 1H), 7.30 (s, 1H), 7.26 (d, *J* = 8.4 Hz, 1H), 6.99 (d, *J* = 8.4 Hz, 1H), 3.84 (s, 3H), 3.77 (s, 3H), 3.10 (t, *J* = 6.4 Hz, 2H), 2.58 (t, *J* = 6.4 Hz, 2H).

**Example 38 and Example 39**

4-(4-(benzofuran-3-yl)furan-2-yl)-4-oxobutyronitrile **(I'-24)** and 4-(4-(benzofuran-3-yl)furan- 2-yl)-4-oxobutanamide **(I'-25)**

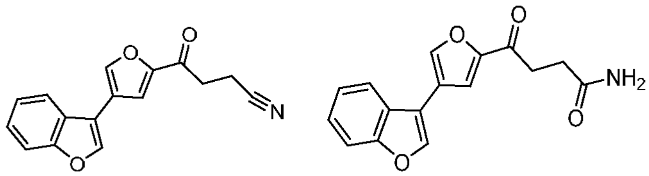

Step 1: 4-(4-bromofuran-2-yl)-4-oxobutyronitrile **(25'-1)**

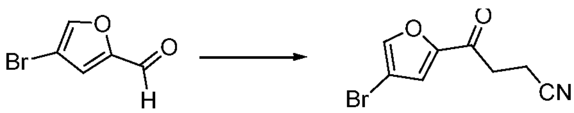

The preparation method of 4-(4-bromofuran-2-yl)-4-oxobutyronitrile **(25'-1)** was the same as that of compound **17'-2,** and a yellow solid was obtained with a yield of 47%. $^1$H NMR (400 MHz, $CDCl_3$) *δ*7.61 (s, 1H), 7.27 (s, 1H), 3.23 (t, *J* = 7.2 Hz, 1H), 2.74 (t, *J* = 7.2 Hz, 1H).

Step 2: 4-(4-(benzofuran-3-yl)furan-2-yl)-4-oxobutyronitrile **(I'-24)**

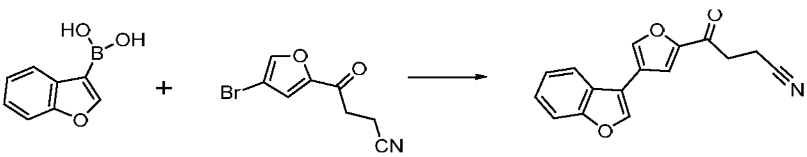

The preparation method of 4-(4-(benzofuran-3-yl)furan-2-yl)-4-oxobutyronitrile **(I'-24)** was the same as that of compound **4'-1,** and a yellow solid was obtained with a yield of 83%. $^1$H NMR (400 MHz, $CDCl_3$) δ 7.97 (s, 1H), 7.83 (s, 1H), 7.69 (d, *J*= 7.2 Hz, 1H), 7.52-7.61 (m, 2H), 7.32-7.43 (m, 2H), 7.51 (d, *J* = 7.3 Hz, 1H), 7.34 (d, *J* = 1.3 Hz, 1H), 6.93 (s, 1H), 3.72 (s, 3H), 3.26 (t, *J*= 6.8 Hz, 2H), 2.80 (t, *J* = 6.8 Hz, 2H).

Step 3: 4-(4-(benzofuran-3-yl)furan-2-yl)-4-oxobutanamide **(I'-25)**

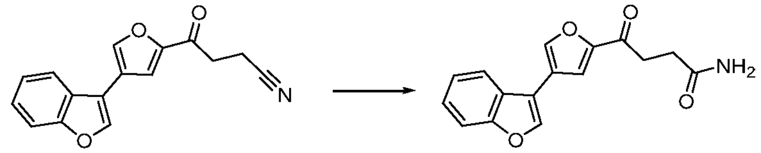

Compound **I'-24** (30mg) was dissolved in DMSO (0.2mL), potassium carbonate (47mg) and hydrogen peroxide (20μL) were added, and the mixture was stirred at room temperature overnight. The reaction solution was neutralized with dilute hydrochloric acid, extracted with ethyl acetate. The organic phase was concentrated to dryness, and purified by preparative thin layer chromatography (dichloromethane: methanol = 50:1 (1% acetic acid)) to obtain 40 mg of 4-(4-(benzofuran-3-yl)furan-2-yl)-4-oxobutanamide **(I'-25)** as a pale yellow solid with a yield of 64%. $^1H$ NMR (400 MHz, $CDCl_3$) $\delta$ 8.66 (s, 1H), 8.50 (s, 1H), 8.03 (d, $J$ = 6.7 Hz, 1H), 7.89 (s, 1H), 7.67 (d, $J$= 7.6 Hz, 1H), 7.35-7.45 (m, 3H), 6.85 (s, 1H), 3.10 (t, $J$ = 6.7 Hz, 2H), 2.47 (t, $J$ = 6.7 Hz, 2H).

**Example 40 4-(4-(thieno[2,3-c]pyridin-4-yl)thiophen-2-yl)-4-oxobutyric acid (I'-26)**

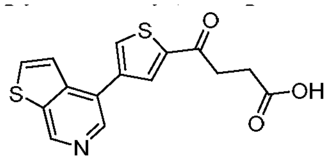

Step 1: Methyl 4-bromothieno[2,3-c]pyridin-2-carboxylate **(26'-1)**

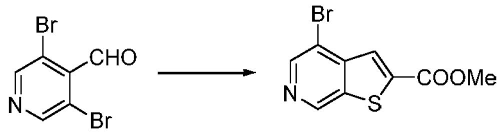

A mixture of 3,5-dibromo-4-pyridylaldehyde (1 g), tetrahydrofuran (5 mL) and methyl thioglycolate (400 mg) was cooled under an ice bath, cesium carbonate (1.23 g) was added, and the mixture was stirred overnight at room temperature under the protection of argon. The reaction solution was filtered through celite, washed with dichloromethane, and the organic layer was concentrated to dryness, and subjected to column chromatography with ethyl acetate : petroleum ether=1:100 to obtain 665 mg of methyl 4-bromothieno[2,3-c] pyridin-2-carboxylate **(26'-1)** as an off-white solid with a yield of 65%. $^1H$ NMR (400 MHz, $CDCl_3$) $\delta$ 9.11(s, 1H), 8.68 (s, 1H), 8.18 (s, 1H), 4.03 (s, 3H).

Step 2: 4-bromothieno[2,3-c]pyridin-2-carboxylic acid **(26'-2)**

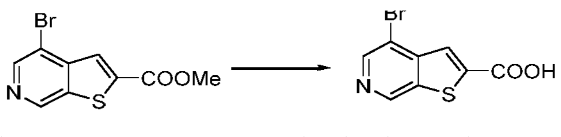

A mixture of compound **26'-1** (300 mg), tetrahydrofuran (1 mL), water (1 mL) and lithium hydroxide monohydrate (92 mg) was stirred at room temperature for 3 hours, and pH was adjusted to 7 with 2M hydrochloric acid, extracted with ethyl acetate. The organic phase was concentrated to dryness to obtain 261 mg of 4-bromothieno[2,3-c]pyridin-2-carboxylic acid **(26'-2)** as an off-white solid with a yield of 92%. $^1H$ NMR (400 MHz, DMSO-$d_6$) $\delta$ 9.35 (s, 1H), 8.71 (s, 1H), 7.96 (s, 1H)

Step 3: 4-bromothieno[2,3-c]pyridine **(26'-3)**

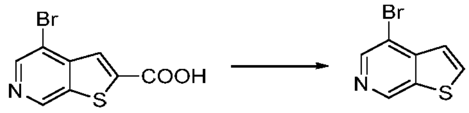

Compound **26'-2** (125 mg) was dissolved in diphenyl ether (1.5 mL), and stirred at 230 ° C for 2 hours. The reaction solution was subjected to column chromatography with ethyl acetate: petroleum ether=1:30 to obtain 72 mg of 4-bromothieno[2,3-c]pyridine **(26'-3)** as a yellow solid with a yield of 70%. 1H NMR (400MHz, $CDCl_3$) δ 9.06 (s, 1H), 8.60 (s, 1H), 7.81 (d, *J* = 5.5 Hz, 1H), 7.51 (d, *J* = 5.5 Hz, 1H).

Step 4: 4-(4-(thieno[2,3-c]pyridin-4-yl)thiophen-2-yl)-4-oxobutyric acid (**I'-26**)

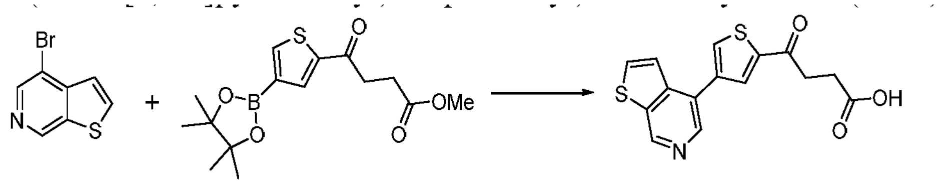

The preparation method of 4-(4-(thieno[2,3-c]pyridin-4-yl)thiophen-2-yl)-4-oxobutyric acid **(I'-26)** was the same as that of compound **I'-16,** and a pale yellow solid was obtained with a yield of 29%. 1H NMR (400 MHz, DMSO-d6) δ 12.3 (s, 1H), 9.29 (s, 1H), 8.66 (s, 1H), 8.35-8.41 (m, 2H), 8.25 (d, J = 5.4 Hz, 1H), 7.76 (d, J = 5.4 Hz, 1H), 3.30 (t, J = 6.4 Hz, 2H), 2.55 (t, J = 6.4 Hz, 2H).

**Example 41 4-(4-(thieno[2,3-b]pyridin-4-yl)thiophen-2-yl)-4-oxobutyric acid (I'-27)**

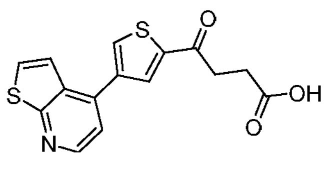

Step 1: Thieno[2,3-b]pyridine **(27'-1)**

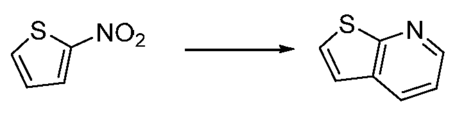

A mixture of 2-nitrothiophene (500 mg) and concentrated hydrochloric acid (7.5 mL) was added with tin (962 mg) under an ice bath, and after stirring for 10 minutes, a solution of zinc chloride (231 mg) in ethanol (2.7 mL) was added. The temperature was gradually raised to 85 ° C, and a solution of 1,1,3,3-tetramethoxypropane (543 mg) in ethanol (1.2 mL) was added, and stirred at 85 ° C for 1 hour. The reaction solution was poured into ice water, and pH was adjusted to be neutral with concentrated ammonia water, extracted with dichloromethane. The organic phase was concentrated to dryness, and subjected to column chromatography with ethyl acetate : petroleum ether=1:30 to obtain 94 mg of thieno[2,3-b]pyridine **(27'-1)** as a yellow oil with a yield of 18%. 1H NMR (400 MHz, $CDCl_3$) δ 8.55-8.62 (m, 1H), 8.08 (dd, J = 1.5, 8.0 Hz, 1H), 7.53 (d, J = 6.0 Hz, 1H), 7.24-7.35 (m, 2H).

Step 2: thieno[2,3-b]pyridine-7-oxide **(27'-2)**

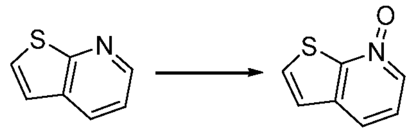

Compound **27'-1** (300 mg) was dissolved in a mixed solvent of ethyl acetate/dichloromethane = 1:1 (6 mL), a solution of m-chloroperoxybenzoic acid (541 mg) in ethyl acetate/dichloromethane (6 mL) was slowly added dropwise under ice bath, and stirred at room temperature overnight after addition. The reaction solution was concentrated, 2M NaOH solution was added, extracted with dichloromethane, and the organic phase was concentrated to dryness to obtain 439 mg of thieno[2,3-b]pyridine-7-oxide **(27'-2)** as a yellow solid with a yield of 100%. LRMS (ESI) *m/z* $[M+H]^+$: 152.10.

Step 3: 4-bromothieno[2,3-b]pyridine **(27'-3)**

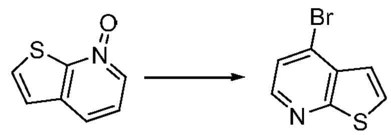

A mixture of compound **27'-2** (439 mg ), dichloromethane (12 mL), and tetrabutylammonium bromide (1 mg) was added to trifluoromethanesulfonic anhydride (1.25 g) under an ice bath. The mixture was stirred overnight at room temperature. The reaction solution was poured into water, the pH was adjusted to alkaline with lithium hydroxide, extracted with dichloromethane, and the organic phase was concentrated to dryness, and subjected to column chromatography with ethyl acetate: petroleum ether=1:100 to obtain 348 mg of 4- bromothieno[2,3-b]pyridine **(27'-3)** as a yellow solid with a yield of 73%. $^1H$ NMR (400 MHz, $CDCl_3$) $\delta$ 8.36 (d, *J*= 5.0 Hz, 1H), 7.61 (d, *J*= 6.1 Hz, 1H), 7.51 (d, *J*= 5.0 Hz, 1H), 7.40 (d, *J* = 6.1 Hz, 1H).

Step 4: 4-(4-(thieno[2,3-b]pyridin-4-yl)thiophen-2-yl)-4-oxobutyric acid **(I'-27)**

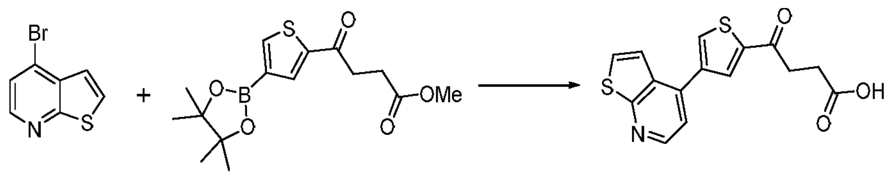

The preparation method of 4-(4-(thieno[2,3-b]pyridin-4-yl)thiophen-2-yl)-4-oxobutyric acid **(I'-27)** was the same as that of compound **I'-16,** and a pale yellow solid was obtained with a yield of 29%. $^1H$ NMR (400 MHz, DMSO-$d_6$) $\delta$ 12.2 (br s, 1H), 8.64 (d, *J*= 4.8 Hz, 1H), 8.50 (s, 1H), 8.44 (s, 1H), 8.00 (d, *J*= 6.1 Hz, 1H), 7.72 (d, *J*= 6.1 Hz, 1H), 7.65 (d, *J* = 4.8 Hz, 1H), 3.94 (t, *J* = 6.4 Hz, 2H), 2.59 (t, *J*= 6.4 Hz, 2H).

**Example 42 4-(4-(benzofuran-3-yl)-1H-pyrrol-2-yl)-4-oxobutyric acid (I'-28)**

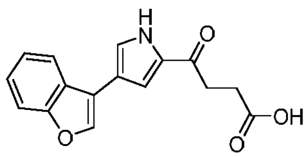

Step 1: methyl 4-(4-bromo-1H-pyrrol-2-yl)-4-oxobutyrate **(28'-1)**

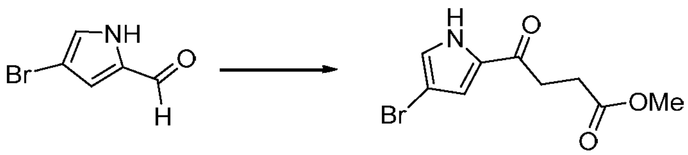

The preparation method of methyl 4-(4-bromo-1H-pyrrol-2-yl)-4-oxobutyrate **(28'-1)** was the same as that of compound **17'-2,** and a yellow solid was obtained with a yield of 48%. $^1$H NMR (400 MHz, $CDCl_3$) $\delta$ 9.46 (s, 1H), 7.04 (s, 1H), 6.92 (d, $J$= 1.8 Hz, 1H), 4.54 (t, $J$= 6.4 Hz, 1H), 3.68 (s, 3H), 2.80 (t, $J$ = 7.2 Hz, 1H).

Step 2: methyl 4-(4-(benzofuran-3-yl)-1H-pyrrol-2-yl)-4-oxobutyrate(**28'-2**)

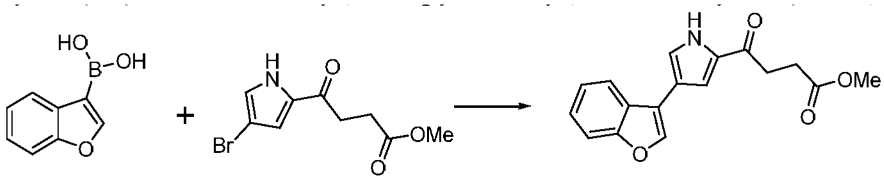

The preparation method of methyl 4-(4-(benzofuran-3-yl)-1H-pyrrol-2-yl)-4-oxobutyrate(**28'-2**) was the same as that of compound **4'-1,** and a yellow oily liquid was obtained with a yield of 72%. $^1$H NMR (400 MHz, $CDCl_3$) $\delta$ 9.61(s, 1H), 7.73-7.78 (m, 2H), 7.53 (d, $J$= 7.6 Hz, 1H), 7.39 (s, 1H), 7.29-7.38 (m, 2H), 7.22 (d, $J$= 1.9 Hz, 1H), 4.54 (t, $J$= 6.4 Hz, 1H), 3.68(s, 3H), 2.89 (t, $J$ = 7.2 Hz, 1H).

Step 3: 4-(4-(benzofuran-3-yl)-1H-pyrrol-2-yl)-4-oxobutyric acid **(I'-28)**

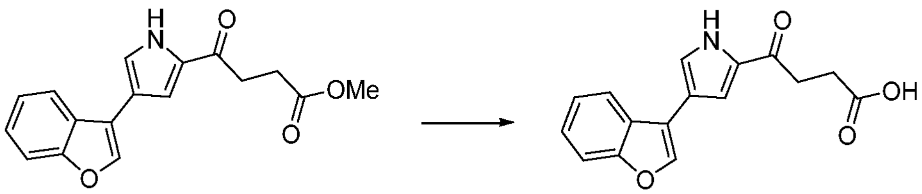

The preparation method of 4-(4-(benzofuran-3-yl)-1H-pyrrol-2-yl)-4-oxobutyric acid **(I'-28)** was the same as that of compound **I'-4,** and a yellow solid was obtained with a yield of 84%. $^1$H NMR (400 MHz, DMSO-$d_6$) $\delta$ 9.59 (s, 1H), 7.00-7.60 (m, 2H), 7.53 (d, $J$ = 7.9 Hz, 1H), 7.28-7.41 (m, 3H), 7.19 (s, 1H), 4.63 (t, $J$= 6.2 Hz, 2H), 2.94 (t, $J$ = 6.2 Hz, 2H).

**Example 43 4-(4-(benzofuran-2-yl)furan-2-yl)-4-oxobutyric acid (I'-29)**

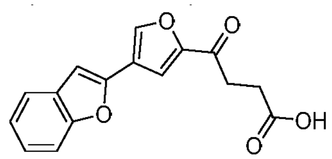

Step 1: methyl 4-4-benzofuran-2-lfuran-2-l-4-oxobutrate **(29'-1)**

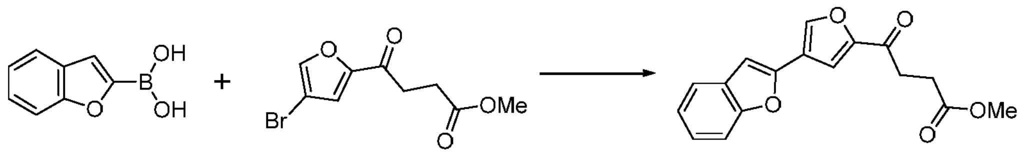

The preparation method of methyl 4-(4-(benzofuran-2-yl)furan-2-yl)-4-oxobutyrate **(29'-1)** was the same as that of compound **4'-1,** and an off-white solid was obtained with a yield of 71%. $^{1}$H NMR (400 MHz, $CDCl_3$) $\delta$ 8.00(s, 1H), 7.55 (d,*J*= 7.4 Hz, 1H), 7.44-7.51 (m, 2H), 7.19-7.31 (m, 2H), 6.82 (s, 1H), 3.69 (s, 3H), 3.21 (t, *J* = 6.7 Hz, 2H), 2.76 (t, *J* = 6.7 Hz, 2H).

Step 2: 4-(4-(benzofuran-2-yl)furan-2-yl)-4-oxobutyric acid **(I'-29)**

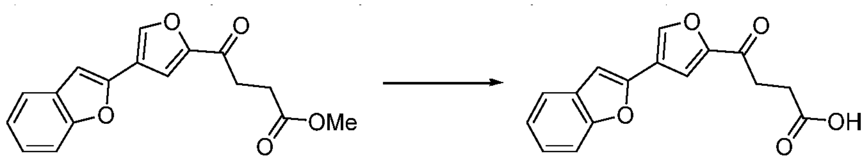

The preparation method of 4-(4-(benzofuran-2-yl)furan-2-yl)-4-oxobutyric acid **(I'-29)** was the same as that of compound **I'-4,** and a yellow solid was obtained with a yield of 76%. $^{1}$H NMR (400 MHz, DMSO-$d_6$) $\delta$ 12.23 (s, 1H), 8.55 (s, 1H), 8.02 (s, 1H), 7.67 (d, *J*= 7.3 Hz, 1H), 7.67 (d, *J* = 7.8 Hz, 1H), 7.24-7.38 (m, 3H), 3.13 (t, *J* = 6.3 Hz, 2H), 2.60 (t, *J* = 6.2 Hz, 2H).

**Example 44 4-(4-(1H-indol-3-yl)furan-2-yl)-4-oxobutyric acid (I'-30)**

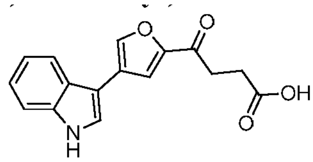

Step 1: methyl 4-(4-(1H-indol-1-Boc-3-yl)furan-2-yl)-4-oxobutyrate **(30'-1)**

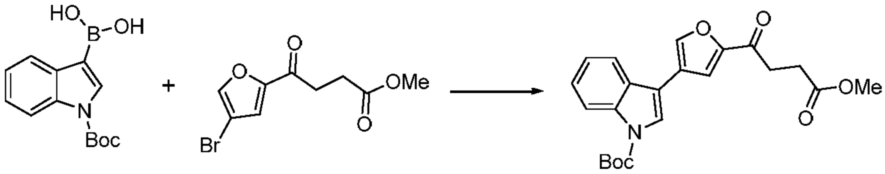

The preparation method of methyl 4-(4-(1H-indol-1-Boc-3-yl)furan-2-yl)-4-oxobutyrate(**30'-1**) was the same as that of compound **4'-1,** and an off-white solid was obtained with a yield of 71%. $^1$H NMR (400 MHz, $CDCl_3$) $\delta$ 8.02 (d, *J* = 6.4 Hz, 1H), 7.73 (s, 1H), 7.68 (d, *J*= 7.5 Hz, 1H), 7.52 (s, 1H), 7.29-7.42 (m, 2H),3.72 (s, 3H), 3.25 (t, *J* = 6.8 Hz, 2H), 2.79 (t, *J* = 6.8 Hz, 2H), 1.70 (s, 9H).

Step 2: methyl 4-(4-(1H-indol-3-yl)furan-2-yl)-4-oxobutyrate(**30'-2**)

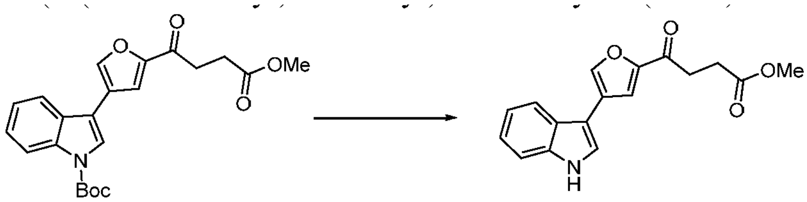

Methyl 4-(4-(1H-indol-1-Boc-3-yl)furan-2-yl)-4-oxobutyrate (50 mg) was dissolved in dichloromethane (0.85 mL), and trifluoroacetic acid (0.17 mL) was added and stirred at room temperature for 6 hours. The reaction solution was adjusted to pH=7 with saturated sodium bicarbonate solution, extracted with dichloromethane, and the organic phase was concentrated to dryness to obtain 28 mg of methyl 4-(4-(1H-indol-3-yl)furan-2-yl)-4-oxobutyrate(**30'-2**) as a pale yellow solid with a yield of 76%. $^1$H NMR (400 MHz, $CDCl_3$) $\delta$ 8.40 (br s, 1H), 7.91(s, 1H), 7.75(d, *J* = 7.8 Hz, 1H), 7.51 (s, 1H), 7.44 (d, *J* = 8.0 Hz, 1H), 7.19-7.30 (m, 2H), 3.72(s, 1H), 3.25(t, *J* = 6.7 Hz, 2H), 2.79 (t, *J* = 6.7 Hz, 2H).

Step 3: 4-(4-(1H-indol-3-yl)furan-2-yl)-4-oxobutyric acid **(I'-30)**

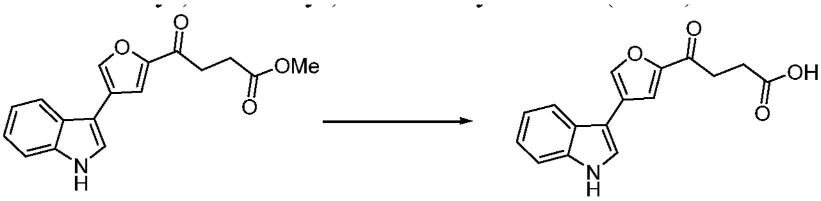

The preparation method of 4-(4-(1H-indol-3-yl)furan-2-yl)-4-oxobutyric acid **(I'-30)** was the same as that of compound **I'-4,** and a yellow solid was obtained with a yield of 76%. $^1$H NMR (400 MHz, DMSO-$d_6$) $\delta$ 12.23 (s, 1H), 11.38 (s, 1H), 8.46 (s, 1H), 7.93 (s, 1H), 7.86 (d, *J* = 8.0 Hz, 1H), 7.83 (d, *J* = 2.5 Hz, 1H), 7.44 (d, *J* = 8.0 Hz, 1H), 7.17 (t, *J* = 7.5 Hz, 1H), 7.11 (t, *J* = 7.4 Hz, 1H), 3.13 (t, *J* = 6.4 Hz, 2H), 2.60 (t, *J* = 6.4 Hz, 2H).

**Example 45 4-(4-(1H-indol-3-yl)thiophen-2-yl)-4-oxobutyric acid (I'-31)**

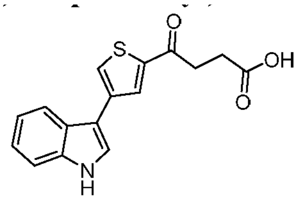

Step 1: methyl 4-(4,5-dibromothiophen-2-yl)-4-oxobutyrate (**31'-1**)

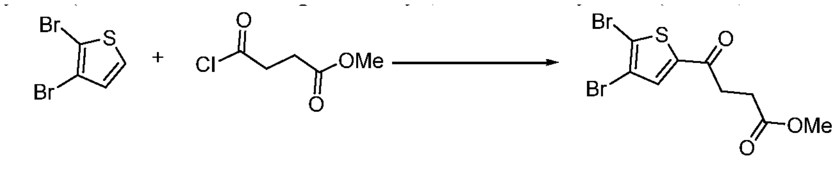

A mixture of 2,3-dibromothiophene (10 g) and succinic acid monomethyl chloride (6.8 g) was dissolved in dichloromethane (10 mL), and slowly added dropwise to aluminum trichloride (7.2 g ) in dichloromethane (90 mL) under ice bath. The reaction solution was transferred to a 50°C oil bath after addition, heated and stirred overnight. The reaction solution was cooled to room temperature, 1M hydrochloric acid (100 mL) was slowly added dropwise with stirring in an ice bath, and liquid seperation was carried out under stirring. The organic phase was concentrated to dryness, and subjected to column chromatography with ethyl acetate : petroleum ether=10:90 to obtain 6.4 g of methyl 4-(4,5-dibromothiophen-2-yl)-4-oxobutyrate **(31'-1)** as a brown oil with a yield of 43%. $^1$H NMR (400 MHz, $CDCl_3$) $\delta$ 7.55 (s, 1H), 3.70 (s, 3H), 3.18 (t, *J*= 8.0 Hz, 2H), 2.80 (t, *J* = 8.0 Hz, 2H).

Step 2: methyl 4-(4-bromothiophen-2-yl)-4-oxobutyrate (**31'-2**)

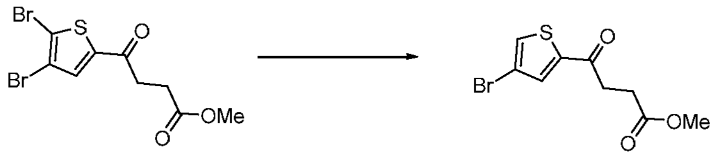

A mixture of methyl 4-(4,5-dibromothiophen-2-yl)-4-oxobutyrate (6.4 g), zinc powder (1.23 g), water (35 mL) and glacial acetic acid (9 mL) was heated and refluxed for 2 hours. After the reaction solution was cooled to room temperature, it was diluted with water, extracted with dichloromethane. The organic phase was concentrated to dryness, and subjected to column chromatography with ethyl acetate : petroleum ether=5:95 to obtain 3.75g of methyl 4-(4-bromothiophen-2-yl)-4-oxobutyrate **(31'-2)** as a yellow oil with a yield of 75%. $^1$H NMR (400 MHz, $CDCl_3$) $\delta$ 7.66 (s, 1H), 7.55 (s, 1H), 3.71 (s, 3H), 3.24 (t, *J* = 8.0 Hz, 2H), 2.78 (t, *J* = 8.0 Hz, 2H).

Step 3: methyl 4-(4-(1H-indol-1-Boc-3-yl)thiophen-2-yl)-4-oxobutyrate **(31'-3)**

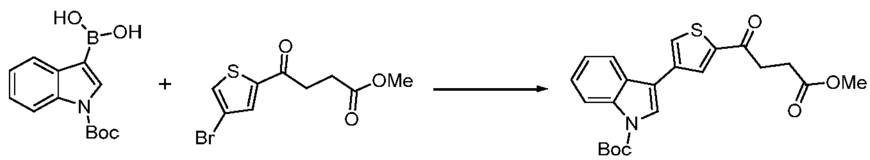

A mixture of methyl 4-(4-bromothiophen-2-yl)-4-oxobutyrate (500mg), 1-Boc-1H-indol-3-boronic acid (471mg), [1,1'-bis(diphenylphosphino)ferrocene]palladium dichloride(66 mg), cesium carbonate (1.47 g), tetrahydrofuran (4 mL) and water (4 mL) was heated to 80°C and stirred overnight under the protection of argon. The reaction solution was

concentrated to dryness, and the residue was subjected to column chromatography with ethyl acetate : petroleum ether=5:95 to obtain 401 mg of methyl 4-(4-(1H-indol-1-Boc-3-yl)thiophen-2-yl)-4-oxobutyrate **(31'-3**) as a pale yellow oil with a yield of 54%. $^1$H NMR (400 MHz, $CDCl_3$) $\delta$ 8.24 (d, *J*= 8.0 Hz, 1H), 8.03 *(d, J* = 1.6 Hz, 1H), 7.77-7.80 (m, 3H), 7.31-7.42 (m, 2H), 3.73 (s, 3H), 3.35 (t, *J* = 8.0 Hz, 2H), 2.83 (t, *J* = 8.0 Hz, 2H), 1.71 (s, 9H).

Step 4: methyl 4-(4-(1H-indol-3-yl)thiophen-2-yl)-4-oxobutyrate **(31'-4)**

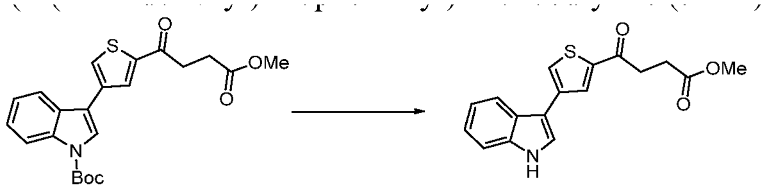

The reaction mixture of methyl 4-(4-(1H-indol-1-Boc-3-yl)thiophen-2-yl)-4-oxobutyrate (400 mg), trifluoroacetic acid (720 $\mu$L) and dichloromethane (5 mL) was stirred at room temperature overnight. The reaction solution was concentrated to dryness, and subjected to column chromatography with dichloromethane : methanol=1:99 to obtain 243 mg of methyl 4-(4-(1H-indol-3-yl)thiophen-2-yl)-4-oxobutyrate **(31'-4)** as a white solid with a yield of 80%. $^1$H NMR (400 MHz, $CDCl_3$) $\delta$ 8.31 (br s, 1H), 8.04 (d, *J* = 1.2 Hz, 1H),7.88 (d, *J* = 8.0 Hz, 1H), 7.74 (d, *J* = 1.2 Hz, 1H), 7.46 (d, *J* = 8.0 Hz, 1H), 7.42 (d, *J* = 2.4 Hz, 1H), 7.21-7.30 (m, 2H), 3.72 (s, 3H), 3.35 (t, *J*= 8.0 Hz, 2H), 2.82 (t, *J* = 8.0 Hz, 2H).

Step 5: 4-(4-(1H-indol-3-yl)thiophen-2-yl)-4-oxobutyric acid **(I'-31)**

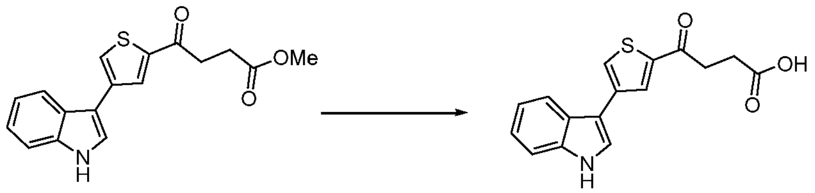

The reaction mixture of methyl 4-(4-(1H-indol-3-yl)thiophen-2-yl)-4-oxobutyrate (200 mg), lithium hydroxide monohydrate (62 mg), water (4 mL) and tetrahydrofuran (8 mL) was stirred at room temperature for 2 hours. The reaction solution was adjusted to pH=2-3 with 2M hydrochloric acid, extracted with ethyl acetate, and the organic phase was washed with saturated brine, and concentrated to dryness to obtain 178 mg of

4-(4-(1H-indol-3-yl)thiophen-2-yl)-4-oxobutyric acid **(I'-31)** as a yellow solid with a yield of 93%. $^1$H NMR (400 MHz, DMSO-$d_6$) $\delta$ 12.29 (br s, 1H), 11.42 (s, 1H), 8.39 (s, 1H), 8.12 (s, 1H), 7.97 (d, *J* = 8.0 Hz, 1H),7.91 (d, *J* = 2.4 Hz, 1H), 7.46 (d, *J* = 8.0 Hz, 1H), 7.11-7.19 (m, 2H), 3.32 (*t, J* = 8.0 Hz, 2H), 2.62 (t, *J* = 8.0 Hz, 2H). LRMS (ESI) *m*/*z* $[M+H]^+$: 300.10.

**Example 46 4-(4-(benzofuran-3-yl)thiazol-2-yl)-4-oxobutyric acid (I'-32)**

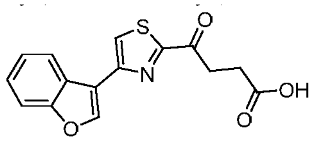

Step 1: methyl 4-(4-bromothiazol-2-yl)-4-oxobutyrate **(32'-1)**

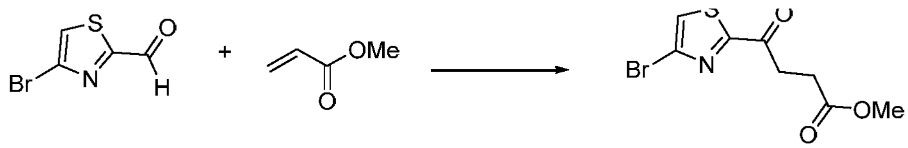

The preparation method of methyl 4-(4-bromothiazol-2-yl)-4-oxobutyrate **(32'-1)** was the same as that of compound **17'-2**, and a pale yellow oil was obtained with a yield of 39%. $^1$H NMR (400 MHz, $CDCl_3$) δ 7.59 (s, 1H), 3.70 (s, 3H), 3.50 (t, *J*= 8.0 Hz, 2H), 2.80 (t, *J* = 8.0 Hz, 2H).

Step 2: methyl 4-(4-(benzofuran-3-yl)thiazol-2-yl)-4-oxobutyrate **(32'-2)**

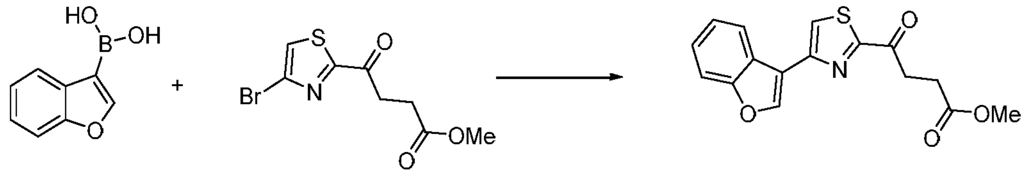

The preparation method of methyl 4-(4-(benzofuran-3-yl)thiazol-2-yl)-4-oxobutyrate **(32'-2)** was the same as that of compound **4'-1,** and a pale yellow solid was obtained with a yield of 67%. $^1$H NMR (400 MHz, $CDCl_3$) δ 8.22 (d, *J* = 1.2 Hz, 1H), 8.08 (d, *J* = 8.4 Hz, 1H), 7.85 (s, 1H), 7.59-7.62 (m, 1H), 7.38-7.44 (m, 2H), 3.75 (s, 3H), 3.66 (t, *J*= 6.4 Hz, 2H), 2.88 (t, *J* = 6.4 Hz, 2H).

Step 3: 4-(4-(benzofuran-3-yl)thiazol-2-yl)-4-oxobutyric acid **(I'-32)**

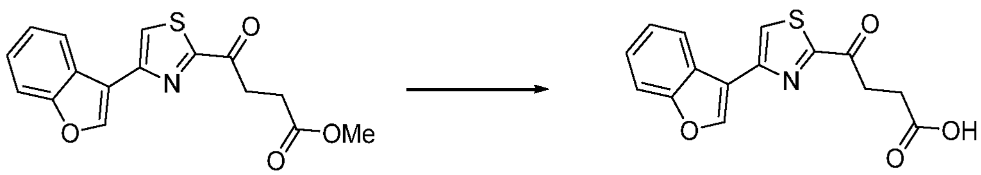

The preparation method of 4-(4-(benzofuran-3-yl)thiazol-2-yl)-4-oxobutyric acid **(I'-32)** was the same as that of compound **I'-4,** and a yellow solid was obtained with a yield of 92%. $^1$H NMR (400 MHz, DMSO-$d_6$) δ 12.30 (br s, 1H), 8.67 (s, 1H), 8.57 (s, 1H), 8.24-8.27 (m, 1H), 7.69-7.72 (m, 1H), 7.40-7.47 (m, 2H), 3.50 (t, *J* = 8.0 Hz, 2H), 2.71 (t, *J* = 8.0 Hz, 2H).

**Example 47 4-(4-(1H-indol-3-yl)thiazol-2-yl)-4-oxobutyric acid (I'-33)**

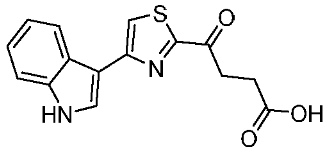

Step 1: methyl 4-(4-(1H-1-Boc-indol-3-yl)thiazol-2-yl)-4-oxobutyrate **(33'-1)**

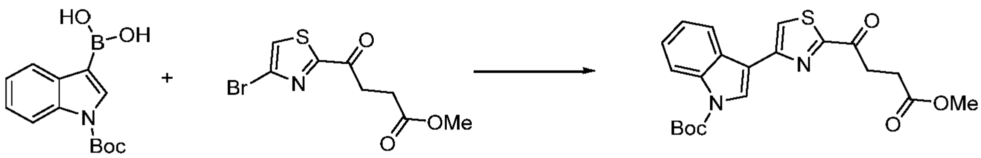

The preparation method of methyl 4-(4-(1H-1-Boc-indol-3-yl)thiazol-2-yl)-4-oxobutyrate **(33'-1)** was the same as that of compound **31'-3,** and a yellow solid was obtained with a yield of 72%. $^1$H NMR (400 MHz, $CDCl_3$) $\delta$ 8.27 (d, $J$ = 8.0 Hz, 1H), 8.13-8.16 (m, 2H), 7.85 (s, 1H), 7.29-7.45 (m, 2H), 3.75 (s, 3H), 3.68 (t, $J$ = 8.0 Hz, 2H), 2.88 (t, $J$ = 8.0 Hz, 2H), 1.74 (s, 9H).

Step 2: methyl 4-(4-(1H-indol-3-yl)thiazol-2-yl)-4-oxobutyrate **(33'-2)**

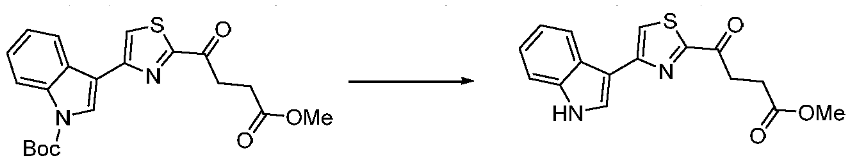

The preparation method of methyl 4-(4-(1H-indol-3-yl)thiazol-2-yl)-4-oxobutyrate **(33'-2)** was the same as that of compound **31'-4,** a yellow oil was obtained with a yield of 75%. $^1$H NMR (400 MHz, $CDCl_3$) $\delta$ 8.43 (br s, 1H), 8.10-8.13 (m, 1H), 7.80 (d, $J$= 4.0 Hz, 1H), 7.70 (s, 1H), 7.43-7.47 (m, 1H), 7.25-7.32 (m, 2H), 3.73 (s, 3H), 3.66 (t, $J$ = 8.0 Hz, 2H), 2.86 (t, $J$ = 8.0 Hz, 2H).

Step 3: **4-(4-(1H-indol-3-yl)thiazol-2-yl)-4-oxobutyric** acid **(I'-33)**

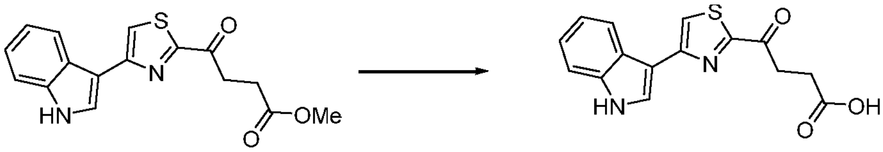

The preparation method of **4-(4-(1H-indol-3-yl)thiazol-2-yl)-4-oxobutyric** acid **(I'-33)** was the same as that of compound **I'-31**, and a yellow solid was obtained with a yield of 94%. $^1$H NMR (400 MHz, DMSO-$d_6$) $\delta$ 12.30 (br s, 1H), 11.54 (br s, 1H), 8.20-8.26 (m, 2H), 8.03 (d, $J$= 4.0 Hz, 1H), 7.46-7.48 (m, 1H), 7.14-7.21 (m, 2H), 3.50 (t, $J$= 8.0 Hz, 2H), 2.71 (t, $J$ = 8.0 Hz, 2H).

**Example 48 4-(4-(imidazo[1,2-a]pyridin-3-yl)furan-2-yl)-4-oxobutyric acid (I'-34)**

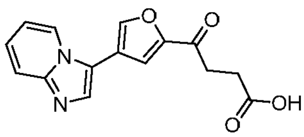

Step 1: 4-(imidazo[1,2-a]pyridin-3-yl)furan-2-carbaldehyde **(34'-1)**

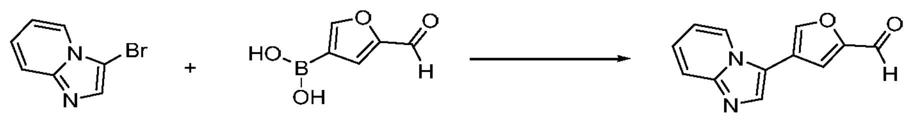

The preparation method of 4-(imidazo[1,2-a]pyridin-3-yl)furan-2-carbaldehyde **(34'-1)** was the same as that of compound **4'-1,** and a yellow solid was obtained with a yield of 60%. $^1$H NMR (400 MHz, DMSO-$d_6$) δ 9.72 (s, 1H), 8.74 (s, 1H), 8.59 (d, *J*= 8.0 Hz, 1H), 8.08 (s, 1H), 7.97 (s, 1H), 7.70 (d, *J* = 12.0 Hz, 1H), 7.32-7.37 (m, 1H), 7.04-7.07 (m, 1H).

Step 2: methyl 4-(4-(imidazo[1,2-a]pyridin-3-yl)furan-2-yl)-4-oxobutyrate **(34'-2)**

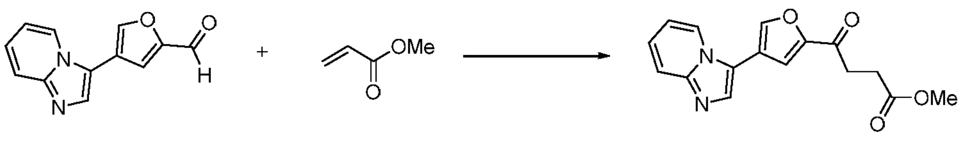

The preparation method of methyl 4-(4-(imidazo[1,2-a]pyridin-3-yl)furan-2-yl)-4-oxobutyrate **(34'-2)** was the same as that of compound **17'-2,** and a light brown oil was obtained with a yield of 66%. $^1$H NMR (400 MHz, $CDCl_3$) δ 8.16 (d, *J* = 8.0 Hz, 1H), 7.88 (s, 1H), 7.69-7.73 (m, 2H), 7.48 (s, 1H), 7.22-7.33 (m, 1H), 6.93 (t, *J* = 4.0 Hz, 1H), 3.72 (s, 3H), 3.27 (t, *J* = 8.0 Hz, 2H), 2.81 (t, *J* = 8.0 Hz, 2H).

Step 3: **4-(4-(imidazo[1,2-a]pyridin-3-yl)furan-2-yl)-4-oxobutyric** acid **(I'-34)**

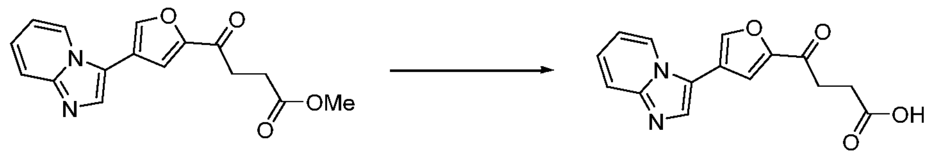

The preparation method of 4-(4-(imidazo[1,2-a]pyridin-3-yl)furan-2-yl)-4-oxobutyric acid **(I'-34)** was the same as that of compound **I'-4,** and a light brown solid was obtained with a yield of 50%. $^1$H NMR (400 MHz, DMSO-$d_6$) δ 12.22 (br s, 1H), 8.58-8.63 (m, 2H), 8.05 (s, 1H), 7.97 (s, 1H), 7.69 (d, *J* = 8.0 Hz, 1H), 7.36 (t, *J* = 8.0 Hz, 1H), 7.08 (t, *J* = 8.0 Hz, 1H), 3.17 (t, *J* = 8.0 Hz, 2H), 2.63 (t, *J* = 8.0 Hz, 2H).

**Example 49 4-(4-(1H-benzo[d]imidazol-1-yl)furan-2-yl)-4-oxobutyric acid (I'-35)**

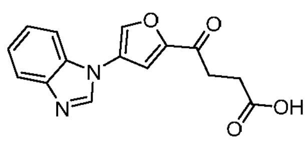

Step 1: 4-(1H-benzo[d]imidazol-1-yl)furan-2-carbaldehyde **(35'-1)**

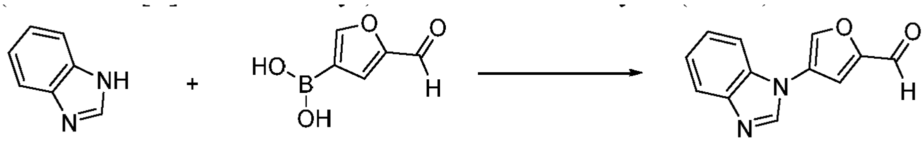

A mixture of 1H-benzo[d]imidazole (305 mg), (5-formylfuran-3-yl)boronic acid (300 mg), cuprous oxide (60 mg) and methanol (7 mL) was stirred openly at room temperature overnight, filtered. The filtrate was concentrated to dryness and subjected to column chromatography with dichloromethane : methanol=20:1 to obtain 179mg of 4-(1H-benzo[d]imidazol-1-yl)furan-2-carbaldehyde (35'-1) as a white solid with a yield of 39%. $^1$H NMR (400 MHz, DMSO-$d_6$) $\delta$ 9.73 (s, 1H), 8.85 (d, $J$ = 4.0 Hz, 1H), 8.66 (s, 1H), 8.18 (d, $J$ = 4.0 Hz, 1H), 7.80 (t, $J$= 8.0 Hz, 2H), 7.33-7.42 (m, 2H).

Step 2: methyl 4-(4-(1H-benzo[d]imidazol-1-yl)furan-2-yl)-4-oxobutyrate(35'-2)

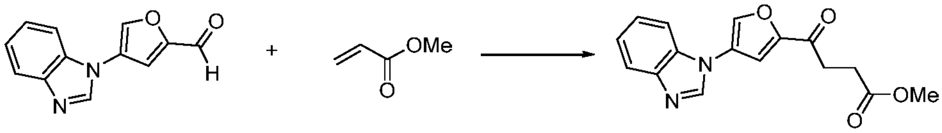

The preparation method of methyl 4-(4-(1H-benzo[d]imidazol-1-yl)furan-2-yl)-4-oxobutyrate (**35'-2**) was the same as that of compound **17'-2,** and a pale yellow oil was obtained with a yield of 40%. $^1$H NMR (400 MHz, $CDCl_3$) $\delta$ 8.05 (s, 1H), 7.95 (d, $J$= 0.8 Hz, 1H), 7.85-7.90 (m, 1H), 7.46-7.49 (m, 2H), 7.35-7.41 (m, 2H), 3.72 (s, 3H), 3.27 (t, $J$ = 6.8 Hz, 2H), 2.82 (t, $J$ = 6.8 Hz, 2H).

Step 3: 4-(4-(1H-benzo[d]imidazol-1-yl)furan-2-yl)-4-oxobutyric acid **(I'-35)**

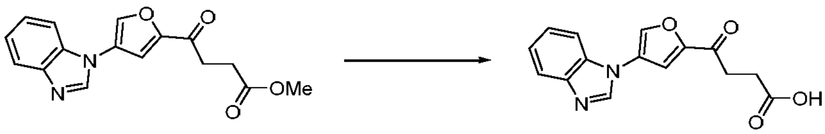

The preparation method of 4-(4-(1H-benzo[d]imidazol-1-yl)furan-2-yl)-4-oxobutyric acid **(I'-35)** was the same as that of compound **I'-4,** and a white solid was obtained with a yield of 71%. $^1$H NMR (400 MHz, DMSO-$d_6$) $\delta$ 12.26 (br s, 1H), 8.72 (d, $J$= 0.8 Hz, 1H), 8.63 (s, 1H), 8.13 (s, 1H), 7.56-7.78 (m, 2H), 7.30-7.40 (m, 2H), 3.15 (t, $J$= 6.4 Hz, 2H), 2.62 (t, $J$ = 6.4 Hz, 2H).

**Example 50 4-(4-(furan[2,3-c]pyridin-3-yl)furan-2-yl)-4-oxobutyric acid (I'-36)**

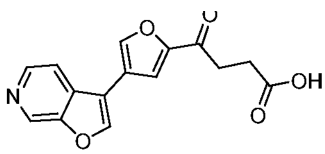

Step 1: 3-bromofuro[2,3-c]pyridine **(36'-1)**

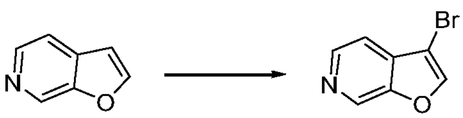

Under the protection of argon, furo[2,3-c]pyridine (180 mg) was dissolved in dichloromethane (2 mL), saturated aqueous sodium bicarbonate solution (12.3 mL) was added, and a solution of 1 M bromine in dichloromethane (10 mL) was slowly added under ice bath. The mixture was gradually warmed to room temperature and stirred for 4 hours, sodium

thiosulfate solution was added, extracted with ethyl acetate. The organic layer was concentrated to dryness, dissolved in 5.4 mL of methanol, and 20% NaOH aqueous solution (0.8 mL) was added. The reaction was stirred at room temperature for 1 hour, and the reaction solution was poured into water, and extracted with dichloromethane. The organic layer was concentrated to dryness, and subjected to column chromatography with ethyl acetate : petroleum ether=1:20 to obtain 40 mg of 3-bromofuro[2 ,3-c]pyridine (**36'-1**) as a yellow solid with a yield of 13%. $^1$H NMR (400 MHz, $CDCl_3$) $\delta$ 8.91 (s, 1H), 8.53 (d, *J*= 4.8 Hz, 1H), 7.79 (s, 1H), 7.53 (d, *J* = 5.3 Hz, 1H).

Step 2: 4-(4-(furo[2,3-c]pyridin-3-yl)furan-2-yl)-4-oxobutyric acid (**I'-36**)

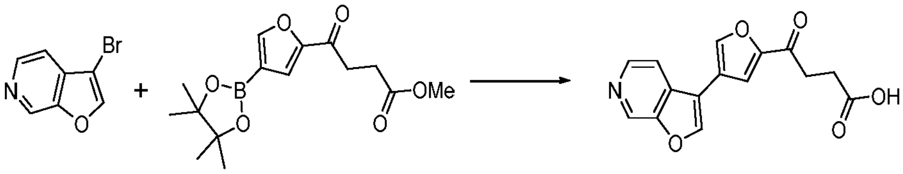

The preparation method of 4-(4-(furo[2,3-c]pyridin-3-yl)furan-2-yl)-4-oxobutyric acid (**I'-36**) was the same as that of compound **I'-16**, and a yellow solid was obtained with a yield of 42%. $^1$H NMR (400 MHz, DMSO-$d_6$) $\delta$ 9.04 (s, 1H), 8.71-8.78 (m, 2H), 8.52 (d, *J*= 5.5 Hz, 1H), 8.12 (d, *J* = 5.5 Hz, 1H), 8.03 (s, 1H), 3.11 (t, *J* = 6.4 Hz, 2H), 2.53 (t, *J* = 6.4 Hz, 2H).

**Example 51 4-(4-(benzofuran-3-yl)furan-2-yl)-2-methyl-4-oxobutyric** acid (**I'-37**)

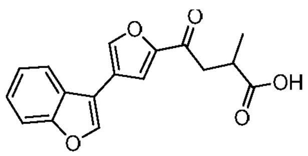

Step 1: butyl 4-(4-bromofuran-2-yl)-2-methyl-4-oxobutyrate (**37'-1**)

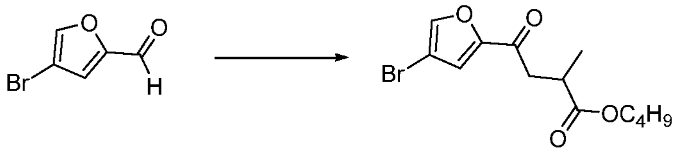

The preparation method of butyl 4-(4-bromofuran-2-yl)-2-methyl-4-oxobutyrate (**37'-1**) was the same as that of compound **17'-2**, and a yellow solid was obtained with a yield of 76 %. $^1$H NMR (400 MHz, $CDCl_3$) $\delta$ 7.57 (s, 1H), 7.20 (s, 1H), 4.07 (t, *J*= 6.6 Hz, 2H), 3.29 (dd, *J* = 8.2, 17.3 Hz, 1H), 3.15-3.03 (m, 1H), 2.89 (dd, *J* = 5.6, 17.3 Hz, 1H), 1.52-1.66 (m, 2H), 1.20-1.40 (m, 5H), 0.9 (t, *J* =7.9 Hz, 3H).

Step 2: butyl 4-(4-(benzofuran-3-yl)furan-2-yl)-2-methyl-4-oxobutyrate (**37'-2**)

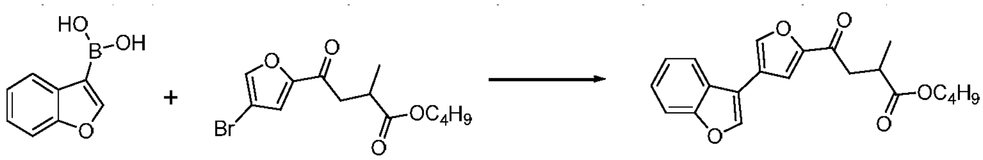

The preparation method of butyl 4-(4-(benzofuran-3-yl)furan-2-yl)-2-methyl-4-oxobutyrate (**37'-2**) was the same as that of compound **4'-1,** and a yellow solid was obtained with a yield of 55%. $^1$H NMR (400 MHz, $CDCl_3$) $\delta$ 7.94 (s, 1H), 7.81 (s, 1H), 7.56 (d, *J*= 8.1 Hz, 1H), 7.47 (s, 1H), 7.31-7.41 (m, 2H), 4.10 (t, *J* = 6.6 Hz, 2H), 3.40 (dd, *J* = 7.9, 17.2 Hz, 1H), 2.29 (dd, *J* = 5.9, 17.2 Hz,1H), 1.56-1.66 (m, 2H), 1.22-1.43 (m, 7H), 0.92 (t, *J* = 7.3 Hz, 3H).

Step 3: 4-(4-(benzofuran-3-yl)furan-2-yl)-2-methyl-4-oxobutyric acid (**I'-37**)

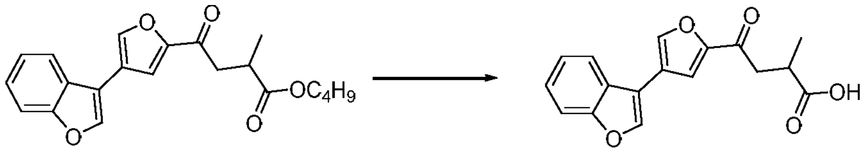

The preparation method of 4-(4-(benzofuran-3-yl)furan-2-yl)-2-methyl-4-oxobutyric acid (**I'-37**) was the same as that of compound **I'-4**, and a yellow solid was obtained with a yield of 55%. $^1$H NMR (400MHz, DMSO-$d_6$) $\delta$ 12.26 (br s, 1H), 8.66 (s, 1H), 8.50 (s, 1H), 7.99-8.06 (m, 2H), 7.67 (d, *J* = 7.3 Hz, 1H), 7.67 (d, *J* = 8.0 Hz, 1H), 7.35-7.46 (m, 2H), 3.23-3.33 (m, 2H), 2.85-3.01 (m, 2H), 1.19 (d, *J*= 6.7 Hz, 3H).

**Example 52 4-(4-(6-fluoro-1H-indol-3-yl)furan-2-yl)-4-oxobutyric acid** (**I'-38**)

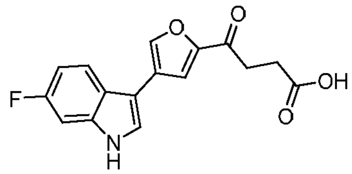

Step 1: 3-bromo-6-fluoro-1H-indole (**38'-1**)

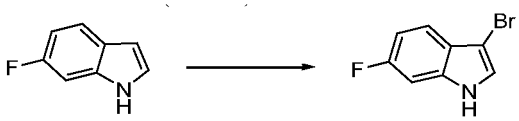

6-fluorindole (1 g) was dissolved in N,N-dimethylformamide (8 mL), N-bromosuccinimide (1.38 g) was added in batches, and the reaction solution was stirred at room temperature for 2 hours. The reaction solution was added to saturated sodium chloride solution in batches, and extracted with ethyl acetate. The organic phase was concentrated to dryness, and subjected to column chromatography with petroleum ether to obtain 1.6g of 3-bromo-6-fluoro-1H-indole (**38'-1**) as a pink oil with a yield of 100%. $^1$H NMR (400 MHz, $CDCl_3$) $\delta$ 8.73 (br s, 1H), 7.48 (dd, *J*= 5.3, 8.7 Hz, 1H), 7.20 (s, 1H), 7.05-7.11 (m, 1H), 6.91-6.99 (m, 1H).

Step 2: 1-Boc-3-bromo-6-fluoro-1H-indole (**38'-2**)

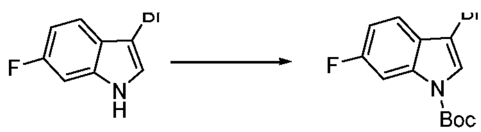

3-bromo-6-fluoro-1H-indole (1 g) and 4-dimethylaminopyridine (120 mg) were dissolved in tetrahydrofuran (8 mL), triethylamine (75 mg) and di-tert-butyl dicarbonate (1.2mL) were added, respectively, and stirred at room temperature

for 4 hours under the protection of argon. The reaction solution was concentrated to dryness, and subjected to column chromatography with ethyl acetate: petroleum ether = 1:100 to obtain 1.1 g of 1-Boc-3-bromo-6- fluoro-1H-indole (**38'-2**) as an off-white solid with a yield of 76%. $^1$H NMR (400 MHz, $CDCl_3$) $\delta$ 7.90 (s, 1H), 7.64 (s, 1H), 7.47 (dd, *J*= 5.3, 8.7 Hz, 1H), 7.09 (td, *J* = 1.9, 8.8 Hz, 1H), 1.69 (s, 9H).

Step 3: methyl 4-(4-(1H-indol-1-Boc-6-fluoro-3-yl)furan-2-yl)-4-oxobutyrate(38'-3)

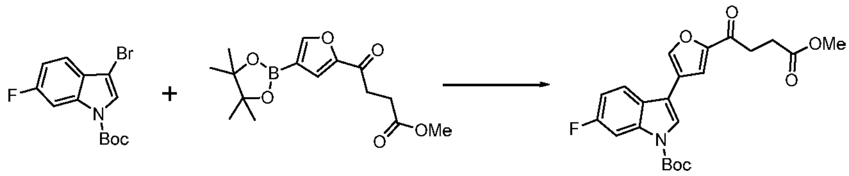

The preparation method of methyl 4-(4-(1H-indol-1-Boc-6-fluoro-3-yl)furan-2-yl)-4-oxobutyrate(**38'-3**) was the same as that of compound **4'-1,** and a yellow solid was obtained with a yield of 33%. $^1$H NMR (400MHz, $CDCl_3$) $\delta$ 7.87-7.99 (m, 2H), 7.70 (s, 1H), 7.59 (dd, *J*= 5.2, 8.7 Hz, 1H), 7.49 (s, 1H), 7.07 (td, *J* = 2.4, 9.0 Hz, 1H), 3.72 (s, 3H), 3.24 (t, *J* = 6.7 Hz, 2H), 2.79 (t, *J* = 6.7 Hz, 2H), 1.69 (s, 9H).

Step 4: 4-(4-(6-fluoro-1H-indol-3-yl)furan-2-yl)-4-oxobutyric acid (**I'-38**)

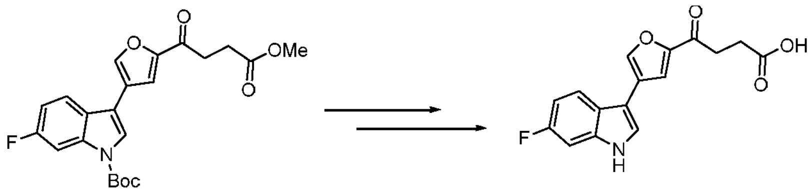

The mixture of methyl 4-(4-(1H-indol-1-Boc-6-fluoro-3-yl)furan-2-yl)-4-oxobutyrate (20 mg), dichloromethane (0.35 mL) and trifluoroacetic acid (0.07 mL) was stirred at room temperature for 6 hours. The reaction solution was adjusted to pH=7 with saturated sodium bicarbonate solution, and extracted with dichloromethane. The organic phase was concentrated to dryness, tetrahydrofuran (0.5 mL), water (0.5 mL) and 1M lithium hydroxide solution (0.11mL) were added, and stirred at room temperature for 2 hours. The reaction solution was adjusted to pH 1-3 with 2M hydrochloric acid, concentrated to dryness, and purified by preparative thin layer (dichloromethane: methanol = 30:1 (1% acetic acid) to obtain 11.4 mg of 4-(4-(6-fluoro-1H-indol-3-yl)furan-2-yl)-4-oxobutyric acid (**I'-38**) as a yellow solid with a yield of 78.6% in two steps. $^1$H NMR (400 MHz, DMSO-$d_6$) $\delta$ 12.2 (br s, 1H), 11.46 (s, 1H), 8.47 (s, 1H), 7.93 (s, 1H), 7.80-7.90 (m, 2H), 7.67 (d, *J* =10.0 Hz, 1H), 6.97 (d, *J* = 9.3 Hz, 1H), 3.13 (t, *J* = 6.4 Hz, 2H), 2.60 (t, *J* =6.4 Hz, 2H).

**Example 53 4-(4-(1H-pyrro[2,3-b]pyridin-3-yl)furan-2-yl)-2-methyl-4-oxobutyric acid (I'-39)**

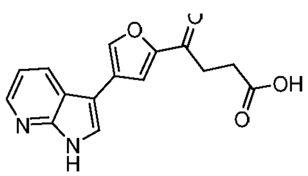

Step 1: 3-bromo-1H-1-Boc-pyrrolo[2,3-b]pyridine (**39'-1**)

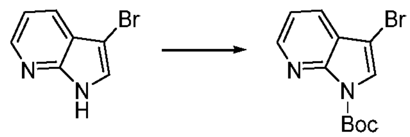

The preparation method of 3-bromo-1H-1-Boc-pyrrolo[2,3-b]pyridine (**39'-1**) was the same as that of compound **38'-2**, and a yellow oil was obtained with a yield of 95%. $^1$H NMR (400 MHz, $CDCl_3$) $\delta$ 8.55 (dd, *J* = 1.4, 4.8 Hz, 1H), 7.85(dd, *J*= 1.6, 7.9 Hz, 1H), 7.70 (s ,1H), 7.25-7.30 (m, 1H), 1.66 (s, 9H).

Step 2: 3-(4,4,5,5-tetramethyl-1,3,2-dioxaboran-2-yl)-1H-1-Boc-pyrrolo[2,3-b]pyridine (**39'-2**)

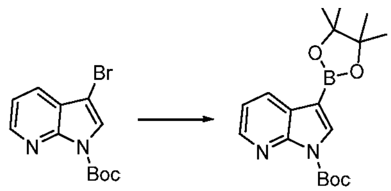

The preparation method of 3-(4,4,5,5-tetramethyl-1,3,2-dioxaboran-2-yl)-1H-1-Boc-pyrrolo[2,3-b]pyridine (**39'-2**) **was the same as that of compound** 10'-1, and an off-white solid was **obtained** with a yield of 37%. $^1$H NMR (400 MHz, $CDCl_3$) $\delta$ 8.52 (d, *J*= 4.7 Hz, 1H), 8.28 (d, *J*= 7.8 Hz, 1H), 8.07 (s ,1H), 7.24 (dd , *J* = 4.8, 7.8 Hz, 1H), 1.68 (s, 9H), 1.40 (s, 12H).

Step 4: methyl 4-(4-(1H-1-Boc-pyrrole[2,3-b]pyridin-3-yl)furan-2-yl)-2-methyl-4-oxobutyrate (**39'-3**)

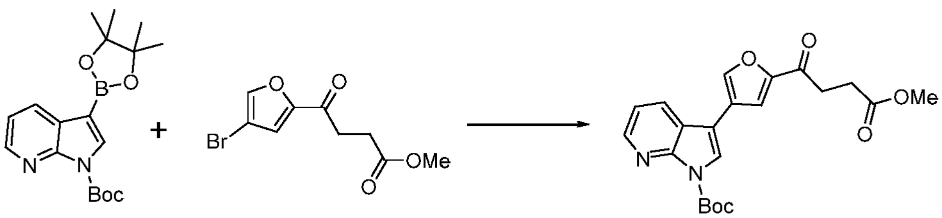

The preparation method of methyl 4-(4-(1H-1-Boc-pyrrole[2,3-b]pyridin-3-yl)furan-2-yl)-2-methyl-4-oxobutyrate (**39'-3**) was the same as that of compound **4'-1,** and a yellow oily liquid was obtained with a yield of 58%. $^1$H NMR (400 MHz, DMSO-$d_6$) $\delta$ 8.59 (d, *J* = 3.4 Hz, 1H), 8.01 (d, *J*= 6.4 Hz, 1H), 7.92 (s ,1H), 7.78 (s, 1H), 7.51 (s, 1H), 7.30 (dd, *J* = 4.8, 7.9 Hz, 1H), 3.72 (s, 3H), 3.72 (t, *J*= 6.7 Hz, 1H), 2.80 (t, *J* = 6.7 Hz, 1H), 1.70 (s, 9H).

Step 5: 4-(4-(1H-pyrro[2,3-b]pyridin-3-yl)furan-2-yl)-2-methyl-4-oxobutyric acid (**I'-39**)

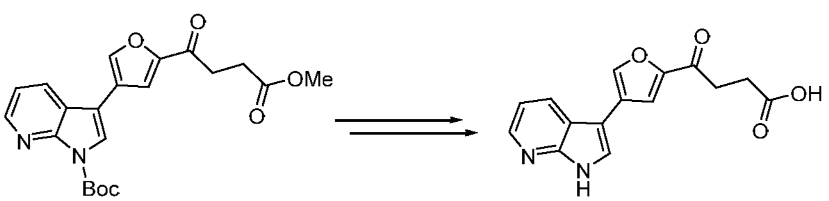

The preparation method of 4-(4-(1H-pyrro[2,3-b]pyridin-3-yl)furan-2-yl)-2-methyl-4-oxobutyric acid (**I'-39**) was the same as that of compound **I'-38**, and a yellow solid was obtained with a yield of 86%. LRMS (ESI) *m/z* [M+H]$^+$: 285.10.

**Example 54 4-(4-(5-methoxy-1H-indol-3-yl)furan-2-yl)-4-oxobutyric acid (I'-40)**

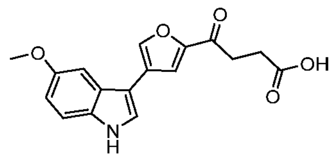

Step 1: 5-methoxy-3-(4,4,5,5-tetramethyl-1,3,2-dioxaboran-2-yl)-1H-1-Boc-indole (**40'-1**)

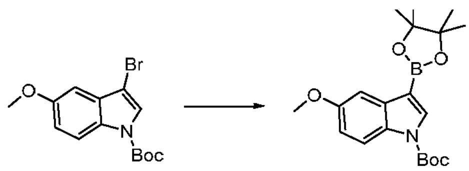

The preparation method of 5-methoxy-3-(4,4,5,5-tetramethyl-1,3,2-dioxaboran-2-yl)-1H-1-Boc-indole (**40'-1**) was the same as that of compound **10'-1**, and an off-white solid was obtained with a yield of 33%. $^1$H NMR (400MHz, $CDCl_3$) $\delta$ 8.03 (d, $J$ = 8.6 Hz, 1H), 7.97 (s, 1H),7.47 (d, $J$ =2.6 Hz, 1H), 6.92 (dd, $J$ = 2.6, 8.6 Hz, 1H), 3.89 (s, 3H), 1.66 (s, 9H), 1.37(s, 12H).

Step 2: methyl 4-(4-(5-methoxy-1H-1-Boc-indol-3-yl)furan-2-yl)-4-oxobutyrate (**40'-2**)

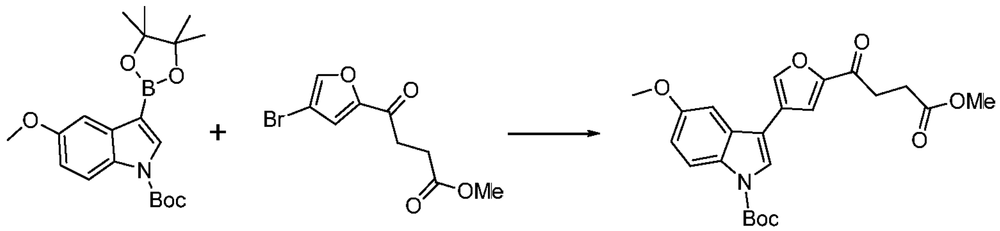

The preparation method of methyl 4-(4-(5-methoxy-1H-1-Boc-indol-3-yl)furan-2-yl)-4-oxobutyrate (**40'-2**) was the same as that of compound **4'-1,** and a yellow oil was obtained with a yield of 20%. $^1$H NMR (400 MHz, $CDCl_3$) $\delta$ 8.09 (s, 1H), 7.91(s, 1H), 7.70 (s, 1H), 7.50 (s, 1H), 7.10 (s, 1H), 7.00 (d, $J$= 9.2 Hz, 1H), 3.89 (s, 3H), 3.72(s, 3H),3.25 (t, $J$ = 6.6 Hz, 2H), 2.80 (t, $J$ = 6.6 Hz, 2H),1.69 (s, 9H).

Step 3: 4-(4-(5-methoxy-1H-indol-3-yl)furan-2-yl)-4-oxobutyric acid (**I'-40**)

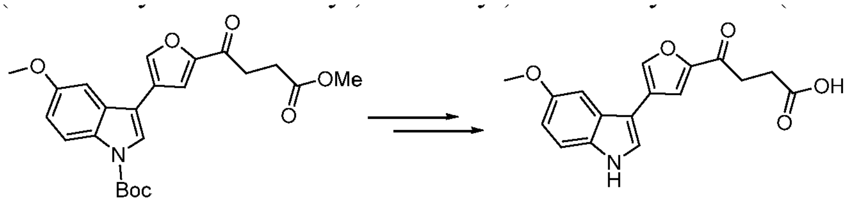

The preparation method of 4-(4-(5-methoxy-1H-indol-3-yl)furan-2-yl)-4-oxobutyric acid (**I'-40**) was the same as that of compound **I'-38**, and a yellow-brown solid was obtained with a yield of 79%. $^1$H NMR (400 MHz, DMSO-$d_6$) $\delta$

11.2 (br s, 1H), 8.47 (s, 1H), 7.90 (s, 1H), 7.77 (s, 1H) 7.33 (d, *J* = 8.7 Hz, 1H), 7.25 (s, 1H), 6.83 (s, 1H), 3.83 (s, 3H), 2.68 (t, *J* = 6.4 Hz, 2H), 2.34 (t, *J* = 6.4 Hz, 2H).

**Example 55 4-(4-(6-methyl-1H-indol-3-yl)furan-2-yl)-4-oxobutyric acid (I'-41)**

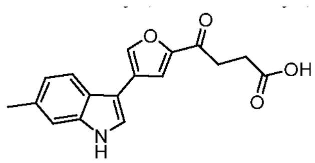

Step 1: 6-methyl-1H-1-Boc-indole (**41'-1**)

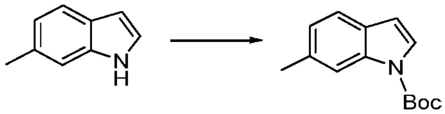

The preparation method of 6-methyl-1H-1-Boc-indole (**41'-1**) was the same as that of compound **38'-2**, and a colorless oil was obtained with a yield of 99%. $^1$H NMR (400 MHz, $CDCl_3$) $\delta$ 8.02 (s, 1H), 7.49 (t, *J* = 3.5 Hz, 1H), 7.44 (d, *J* = 7.9 Hz, 1H), 7.06 (d, *J* = 7.9 Hz, 1H),6.52(d, *J* = 3.7 Hz, 1H) 2.49 (s, 3H), 1.67(s, 9H).

Step 2: 3-bromo-6-methyl-1H-1-Boc-indole (**41'-2**)

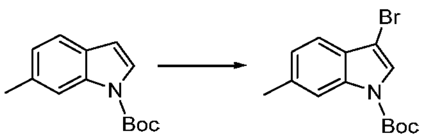

The preparation method of 3-bromo-6-methyl-1H-1-Boc-indole (**41'-2**) was the same as that of compound **38'-1**, and a pale yellow solid was obtained with a yield of 40%. $^1$H NMR (400 MHz, $CDCl_3$) $\delta$ 8.01 (s, 1H), 7.55 (s, 1H), 7.39 (d, *J* = 8.0 Hz, 1H), 7.14 (d, *J* = 8.0, 1H), 2.50 (s, 3H), 1.66 (s, 9H).

Step 3: methyl 4-(4-(6-methyl-1H-1-Boc-indol-3-yl)furan-2-yl)-4-oxobutyrate (**41'-3**)

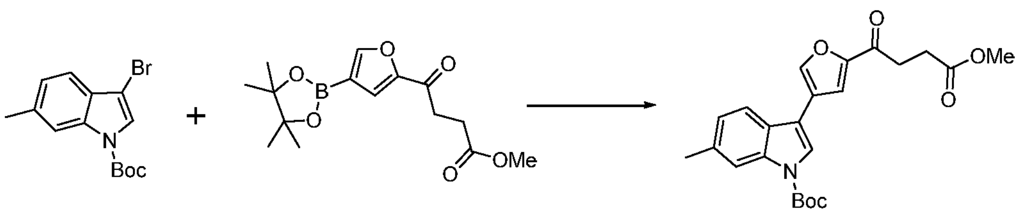

The preparation method of methyl

4-(4-(6-methyl-1H-1-Boc-indol-3-yl)furan-2-yl)-4-oxobutyrate (**41'-3**) was the same as that of compound **4'-1,** and a yellow solid was obtained with a yield of 44%. $^1$H NMR (400 MHz, $CDCl_3$) $\delta$ 8.07 (s, 1H), 7.93 (s, 1H), 7.64 (s,

1H), 7.55 (dd, *J* = 8.0 Hz, 1H), 7.51 (d, *J* = 0.7 Hz, 1H), 7.15 (d, *J* = 8.0 Hz, 1H), 3.72 (s, 3H), 3.24 (t, *J* = 6.7 Hz, 2H), 2.79 (t, *J* = 6.7 Hz, 2H), 2.51 (s, 3H), (s, 9H), 1.69 (s, 9H).

Step 4: 4-(4-(6-methyl-1H-indol-3-yl)furan-2-yl)-4-oxobutyric acid (**I'-41**)

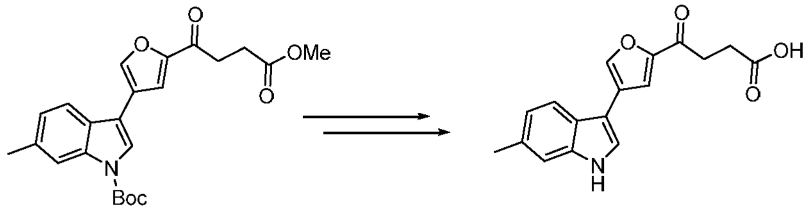

The preparation method of 4-(4-(6-methyl-1H-indol-3-yl)furan-2-yl)-4-oxobutyric acid (**I'-41**) was the same as that of compound **I'-38**, and a yellow solid was obtained with a yield of 76%. $^1$H NMR (400 MHz, DMSO-$d_6$) $\delta$ 11.20 (s, 1H), 8.41 (s, 1H), 7.89 (s, 1H), 7.69-7.76 (m, 2H), 7.21 (s, 1H), 6.94 (d, *J* = 7.9 Hz, 1H), 3.12 (t, *J* = 6.2 Hz, 2H), 2.59 (t, *J* = 6.2 Hz, 2H), 2.41 (s, 3H).

**Example 56 4-(4-(6-fluorobenzofuran-3-yl)furan-2-yl)-4-oxobutyric acid (I'-42)**

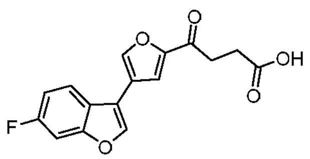

Step 1: 3-bromo-6-fluorobenzofuran (**42'-1**)

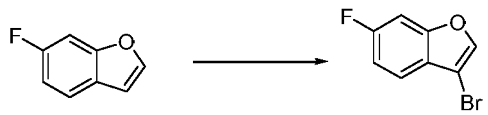

6-fluorobenzofuran (500 mg) was dissolved in dichloromethane (20 mL), 1 M bromine/dichloromethane solution (3.78 mL) was added dropwise under ice bath. The reaction solution was stirred at room temperature for 12 hours after addition, and concentrated to dryness. The residue was dissolved in 22 mL of tetrahydrofuran, a solution of potassium hydroxide in methanol (212 g of potassium hydroxide was dissolved in 22 mL of methanol) was added dropwise under an ice bath, stirred at room temperature for 1 hour after addition, anf the reaction solution was diluted with water, extracted with dichloromethane. The organic layers were combined, washed with water, washed with saturated brine, dried over anhydrous sodium sulfate, and concentrated to dryness to obtain 490.9 mg of 3-bromo-6-fluorobenzofuran (**42'-1**) as a brown-black crystalline solid with a yield of 60%. $^1$H NMR (400 MHz, DMSO-$d_6$) $\delta$ 8.33 (s, 1H), 7.68 (dd, *J* = 2.2, 9.3 Hz, 1H), 7.57 (dd, *J* = 5.4, 8.6 Hz, 1H), 7.28 (m, 1H).

Step 2: methyl 4-(4-bromofuran-2-yl)-4-oxobutyrate (**42'-2**)

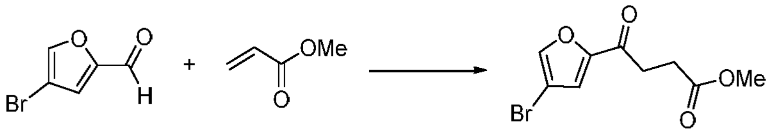

The mixture of 4-bromo-2-furancarboxaldehyde (2g), 3-benzylhydroxyethylmethylthiazole lithium chloride (618mg), 1-butyl-3-methylimidazol hexafluorophosphate (1.88g), methyl acrylate (2 mL) and triethylamine (952 μL) was heated and stirred at 80 °C for 12 hours under the protection of argon. The reaction solution was concentrated to dryness, and subjected to column chromatography with ethyl acetate: petroleum ether = 5:95 to obtian 1.57 g of methyl 4-(4-bromofuran-2-yl)-4-oxobutyrate (**42'-2**) as a yellow oily liquid with a yield of 53%. $^1$H NMR (400 MHz, $CDCl_3$) $\delta$7.57 (d, *J* = 0.7 Hz, 1H), 7.22 (d, *J* = 0.7 Hz, 1H), 3.69 (s, 3H), 3.14 (t, *J* = 6.7 Hz, 2H), 2.74 (t, *J* = 6.7 Hz, 2H).

Step 3: methyl 4-oxo-4-(4-(4,4,5,5-tetramethyl-1,3,2-dioxaboran-2-yl)furan-2-yl)butyrate (**42'-3**)

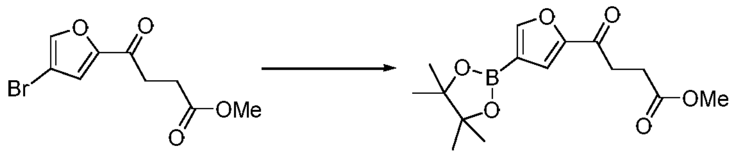

A mixture of methyl 4-(4-bromofuran-2-yl)-4-oxobutyric acid (1.2g), pinacol biboronate (1.4g), potassium acetate (1.35g), [1,1-bis(diphenylphosphino)ferrocene]palladium dichloride (100 mg) and 1,4-dioxane (20 mL) was heated and stirred at 80 °C overnight under the protection of argon. The reaction solution was concentrated to dryness, and subjected to column chromatography with ethyl acetate: petroleum ether = 15:85 to obtain 1.14 g of methyl 4-oxo-4-(4-(4,4,5,5-tetramethyl-1,3,2-dioxaboran-2-yl)furan-2-yl)butyrate (**42'-3**) as a yellow oily liquid with a yield of 81%. $^1$H NMR (400 MHz, $CDCl_3$) $\delta$7.88 (s, 1H), 7.42 (s, 1H), 3.69 (s, 3H), 3.15 (t, *J* = 6.7 Hz, 2H), 2.74 (t, *J* = 6.8 Hz, 2H), 1.32 (s, 12H).

Step 4: methyl 4-(4-(6-fluorobenzofuran-3-yl)furan-2-yl)-4-oxobutyrate (**42'-4**)

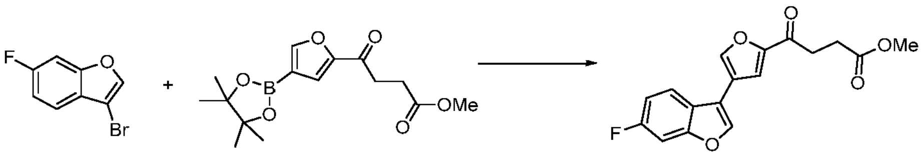

A mixture of 3-bromo-6-fluorobenzofuran (100 mg), methyl 4-oxo-4-(4-(4,4,5,5-tetramethyl-1,3,2-dioxaborane-2 -yl)furan-2-yl)butyrate(143 mg), sodium carbonate (197 mg), tetrakis(triphenylphosphine)palladium (53 mg), 1,4-dioxane (20 mL) and water (1 mL) was heated and stirred at 90°C for 12 hours under argon protection, and then cooled to room temperature. The reaction solution was concentrated to dryness and subjected to column chromatography with ethyl acetate: petroleum ether=1:9 to obtain 113 mg of methyl 4-(4-(6-fluorobenzofuran-3-yl)furan-2-yl)-4-oxobutyrate (**42'-4**) as a yellow foamy solid with a yield of 77%. $^1$H NMR (400 MHz, $CDCl_3$) $\delta$7.91 (s, 1H), 7.80 (s, 1H), 7.60 (dd, *J*= 5.3, 8.9 Hz, 1H), 7.46 (s, 1H), 7.28 (d, *J*= 1.7 Hz, 1H), 7.11 (td, *J*= 2.0, 9.3 Hz, 1H), 3.72 (s, 3H), 3.24 (t, *J* = 6.7 Hz, 2H), 2.79 (t, *J* = 6.7 Hz, 2H).

Step 5: 4-(4-(6-fluorobenzofuran-3-yl)furan-2-yl)-4-oxobutyric acid (**I'-42**)

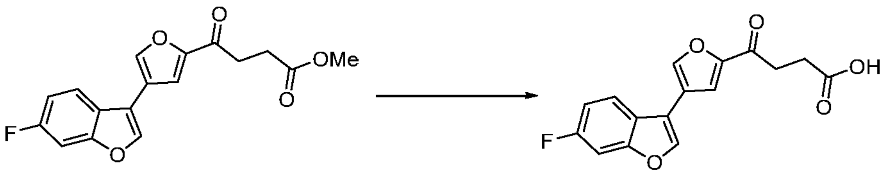

Methyl 4-(4-(6-fluorobenzofuran-3-yl)furan-2-yl)-4-oxobutyrate (80 mg) was dissolved in a mixed solution of tetrahydrofuran (7 mL) and water (3.5 mL), lithium hydroxide monohydrate (25 mg) was added to the solution, and stirred at room temperature for 2.5 hours. The reaction solution was adjusted to pH 1-3 with 6M hydrochloric acid, and extracted with ethyl acetate. The organic phase was concentrated to dryness, and purified by preparative thin layer chromatography (dichloromethane : methanol=25:1) to obtain 46 mg of 4-(4-(6-fluorobenzofuran-3-yl)furan-2-yl)-4-oxobutyric acid (**I'-42**) as a yellow-white solid with a yield of 60%. $^1H$ NMR (400 MHz, DMSO-$d_6$) $\delta$ 12.22 (s, 1H), 8.67 (s, 1H), 8.52 (s, 1H), 8.04 (dd, $J$= 8.4, 5.6 Hz, 1H), 7.98 (s, 1H), 7.65 (dd, $J$= 2.0, 9.0 Hz, 1H), 7.27 (t, $J$ = 8.5 Hz, 1H), 3.13 (t, $J$ = 6.3 Hz, 2H), 2.60 (t, $J$ = 6.5 Hz, 2H). LRMS (ESI) $m/z$ $[M+H]^+$: 303.10.

**Example 57 4-(4-(6-fluorobenzofuran-3-yl)thiophen-2-yl)-4-oxobutyric acid (I'-43)**

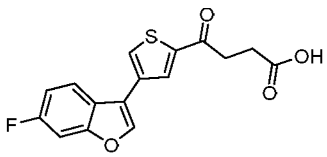

Step 1: methyl 4-4-6-fluorobenzofuran-3-lthiohen-2-1-4-oxobutrate (**43'-1**)

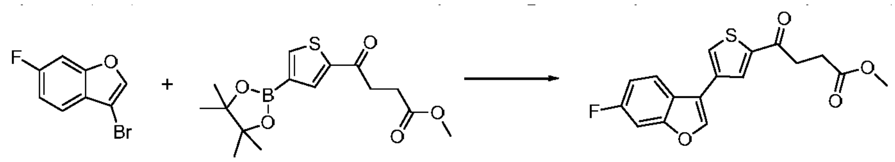

The preparation method of methyl 4-(4-(6-fluorobenzofuran-3-yl)thiophen-2-yl)-4-oxobutyrate (**43'-1**) was the same as that of compound **4'-1,** and a brown-yellow powder was obtained with a yield of 76.9%. $^1H$ NMR (400 MHz, $CDCl_3$) $\delta$ 7.97 (d, $J$ = 1.3 Hz, 1H), 7.84 (s, 1H), 7.79 (d, $J$ = 1.3 Hz, 1H), 7.70 (dd, $J$ = 5.3, 8.7 Hz, 1H), 7.29 (d, $J$ = 2.1 Hz, 1H), 7.11 (td, $J$ = 2.2, 9.2 Hz, 1H), 3.72 (s, 3H), 3.32 (t, $J$ = 6.7 Hz, 2H), 2.81 (t, $J$ = 6.7 Hz, 2H).

Step 2: 4-(4-(6-fluorobenzofuran-3-yl)thiohen-2-yl)-4-oxobutric acid (**I'-43)**

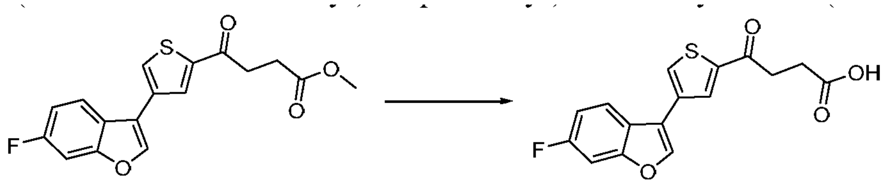

The preparation method of 4-(4-(6-fluorobenzofuran-3-yl)thiophen-2-yl)-4-oxobutyric acid (**I'-43**) was the same as that of compound **I'-4**, **and** a pale yellow solid was obtained with a yield of 86%. $^1H$ NMR (400 MHz, DMSO) $\delta$ 12.23 (s, 1H), 8.60 (s, 1H), 8.44 (d, $J$ = 1.3 Hz, 1H), 8.42 (d, $J$ = 1.2 Hz, 1H), 8.10 (dd, $J$ = 5.4, 8.7 Hz, 1H), 7.66 (dd, $J$ = 2.3, 9.2 Hz, 1H), 7.28 (td, $J$= 2.3, 9.3 Hz, 1H), 3.29 (t, $J$ = 6.6 Hz, 2H), 2.61 (t, $J$ = 6.4 Hz, 2H).

**Example 58 4-(4-(thieno[3,2-b]furan-3-yl)thiophen-2-yl)-4-oxobutyric acid** (**I'-44)**

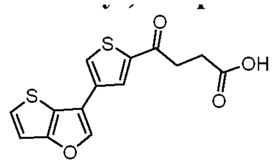

Step 1: 3,4-dibromofuran-2-carbaldehyde (**44'-1**)

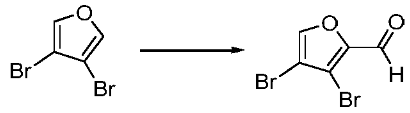

Under the protection of argon, phosphorus oxychloride (12.4 mL) was added dropwise to N,N-dimethylformamide (24 mL) cooled in an ice bath. The mixture was stirred for 1 hour under an ice bath, and 3,4-dibromofuran (2.5g) was added dropwise after addition. The mixture was stirred at room temperature for 1 hour after addition, and heated at 80°C for 48 hours. The reaction solution was poured into ice (200g), 2M sodium hydroxide was added to adjust pH = 8, and a solid was precipitated, filtered. The filter cake was subjected to column chromatography with ethyl acetate: petroleum ether = 1:99 to obtain 1.65 g of 3,4-dibromofuran-2-carbaldehyde (**44'-1**) as a yellow-green solid with a yield of 59%. $^1$H NMR (400 MHz, $CDCl_3$) $\delta$ 9.75 (s, 1H), 7.70 (s, 1H).

Step 2: Methyl 3-bromothieno3,2-bfuran-5-carboxlate (**44'-2**)

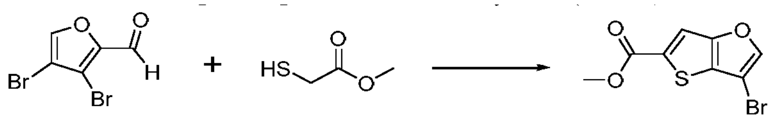

Sodium methoxide (426 mg) was dissolved in methanol (25 mL), methyl thioglycolate (70 $\mu$L) was added, stirred at room temperature for 15 minutes, and 3,4-dibromofuran-2-carbaldehyde (**44'-1**) (1 g) was added. The mixture was stirried at room temperature for 1 hour, and then stirred at 60 °C overnight. The reaction solution was diluted with water, and extracted with ethyl acetate. The organic phase was concentrated to dryness, and subjected to column chromatography with ethyl acetate: petroleum ether = 1:99 to obtain 802 mg of methyl 3-bromothieno[3,2-b]furan-5-carboxylate (**44'-2**) as a pale yellow solid with a yield of 78%. $^1$H NMR (400 MHz, $CDCl_3$) $\delta$ 7.76 (s, 1H), 7.67 (s, 1H), 3.92 (s, 3H).

Step 3: 3-bromothieno3,2-bfuran-5-carboxlic acid (**44'-3**)

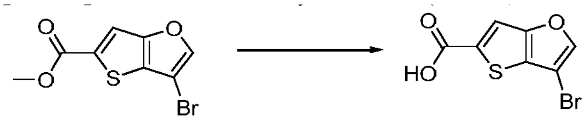

The preparation method of 3-bromothieno[3,2-b]furan-5-carboxylic acid (**44'-3**) was the same as that of compound **26'-2**, and a pale yellow solid was obtained with a yield of 99%. $^1$H NMR (400 MHz, DMSO-$d_6$) $\delta$ 13.46 (s, 1H), 8.36 (s, 1H), 7.96 (s, 1H).

Step 4: 3-bromothiophen[3,2-b]furan (**44'-4**)

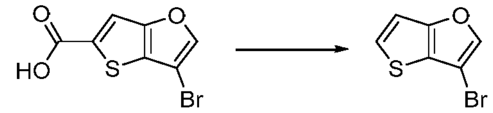

A mixture of 3-bromothieno[3,2-b]furan-5-carboxylic acid (**44'-3**) (150 mg), silver acetate (21 mg), potassium carbonate (26 mg) and N-methylpyrrolidone (1.5 mL) was stirred at 120 °C overnight under the protection of argon. The reaction solution was adjusted to pH=3 with 6M hydrochloric acid, and extracted with ethyl acetate. The organic phase was concentrated to dryness, and subjected to column chromatography with petroleum ether to obtain 61 mg of 3-bromothiophen[3,2-b]furan (**44'-4**) as a brown oil with a yield of 49%. $^1$H NMR (400 MHz, $CDCl_3$) $\delta$ 7.54 (d, *J*= 1.2 Hz, 1H), 7.25 (dd, *J*= 5.3, 1.3 Hz, 1H), 7.07 (d, *J* = 5.3 Hz, 1H).

Step 5: methyl 4-(4-(thieno[3,2-b]furan-3-yl)thiophen-2-yl)-4-oxobutyrate (**44'-5**)

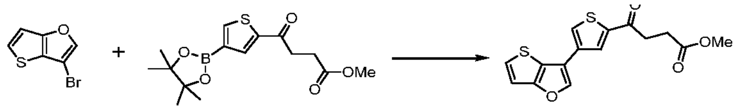

The preparation method of methyl 4-(4-(thieno[3,2-b]furan-3-yl)thiophen-2-yl)-4-oxobutyrate (**44'-5**) was the same as that of compound **4'-1,** and a brown solid was obtained with a yield of 59%. $^1$H NMR (400 MHz, $CDCl_3$) $\delta$ 7.95 (d, *J* = 1.2 Hz, 1H), 7.86 (d, *J* = 1.1 Hz, 1H), 7.69 (d, *J* = 1.2 Hz, 1H), 7.31 (dd, *J* = 1.2, 5.3 Hz, 1H), 7.12 (d, *J* = 5.3 Hz, 1H), 3.72 (s, 3H), 3.32 (t, *J* = 6.7 Hz, 2H), 2.80 (t, *J* = 6.7 Hz, 2H).

Step 6: 4-(4-(thieno[3,2-b]furan-3-yl)thiophen-2-yl)-4-oxobutyric acid (**I'-44**)

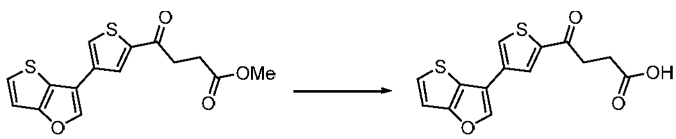

The preparation method of 4-(4-(thieno[3,2-b]furan-3-yl)thiophen-2-yl)-4-oxobutyric acid (**I'-44**) was the same as that of compound **I'-4**, and an orange-red solid was obtained with a yield of 53%. $^1$H NMR (400 MHz, DMSO-$d_6$) $\delta$ 12.21 (s, 1H), 8.51 (d, *J* = 1.2 Hz, 1H), 8.38 (d, *J* = 1.3 Hz, 1H), 8.04 (d, *J* = 1.3 Hz, 1H), 7.67 (dd, *J* = 1.2, 5.2 Hz, 1H), 7.34 (d, *J* = 5.3 Hz, 1H), 3.27 (t, *J* = 6.5 Hz, 2H), 2.62 (t, *J* = 6.4 Hz, 2H).

**Example 59 4-(4-(thieno[3,2-b]furan-3-yl)furan-2-yl)-4-oxobutric** acid (**I'-45**)

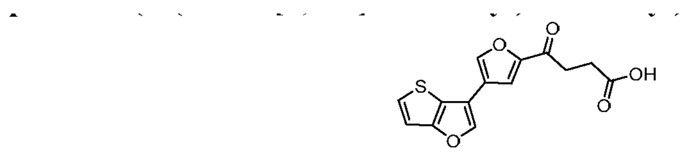

Step 1: 4-(4-(thieno[3,2-b]furan-3-yl)furan-2-yl)-4-oxobutyric acid (**45'-1**)

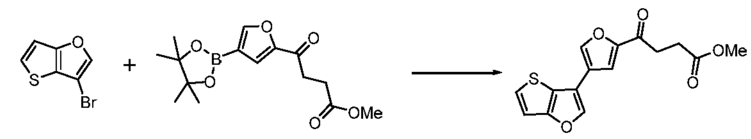

The preparation method of 4-(4-(thieno[3,2-b]furan-3-yl)furan-2-yl)-4-oxobutyric acid (**45'-1**) was the same as that of compound **4'-1,** and a yellow solid was obtained with a yield of 80%. $^1$H NMR (400 MHz, $CDCl_3$) $\delta$ 7.82 (s, 1H), 7.78 (s, 1H), 7.42 (s, 1H), 7.30 (d, *J* = 5.3 Hz, 1H), 7.11 (d, *J* = 5.3 Hz, 1H), 3.71 (s, 3H), 3.23 (t, *J* = 6.8 Hz, 2H), 2.78 (t, *J* = 6.8 Hz, 2H).

Step 2: 4-(4-(thieno[3,2-b]furan-3-yl)furan-2-yl)-4-oxobutric acid (**I'-45**)

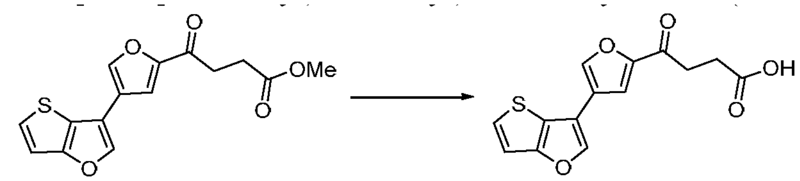

The preparation method of 4-(4-(thieno[3,2-b]furan-3-yl)furan-2-yl)-4-oxobutyric acid (**I'-45**) was the same as that of compound **I'-4**, and a pale yellow solid was obtained with a yield of 66%. $^1$H NMR (400 MHz, DMSO-$d_6$) $\delta$ 12.21 (s, 1H), 8.38 (s, 1H), 8.34 (s, 1H), 7.87 (s, 1H), 7.66 (d, *J*= 5.3 Hz, 1H), 7.33 (d, *J* = 5.2 Hz, 1H), 3.12 (t, *J* = 6.5 Hz, 2H), 2.60 (t, *J* = 6.4 Hz, 2H).

**Example 60 4-(4-(6-fluoro-1H-indol-3-yl)thiophen-2-yl)-4-oxobutyric acid (I'-46)**

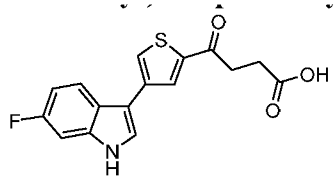

Step 1: methyl 4-(4-(6-fluoro-1-Boc-1H-indol-3-yl)thiophen-2-yl)-4-oxobutyrate (**46'-1**)

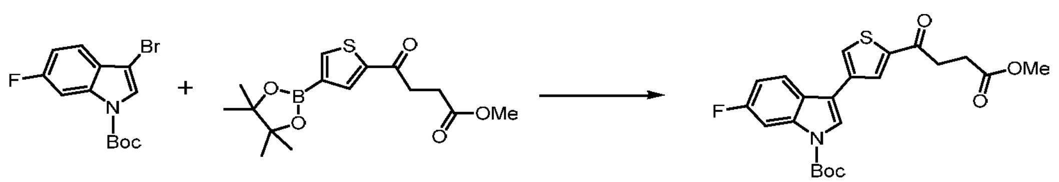

The preparation method of methyl 4-(4-(6-fluoro-1-Boc-1H-indol-3-yl)thiophen-2-yl)-4-oxobutyrate (**46'-1**) was the same as that of compound **4'-1,** and a yellow oily liquid was obtained with a yield of 84%. $^1$H NMR (400 MHz, $CDCl_3$) $\delta$ 7.90-8.05 (m, 2H), 7.65-7.80 (m, 3H), 7.08 (t, *J* = 7.9 Hz, 1H), 3.72 (s, 3H), 3.33 (t, *J* = 6.6 Hz, 2H), 2.81 (t, *J* = 6.6 Hz, 2H), 1.70 (s, 9H).

Step 2: 4-(4-(6-fluoro-1H-indol-3-yl)thiophen-2-yl)-4-oxobutyric acid (**I'-46**)

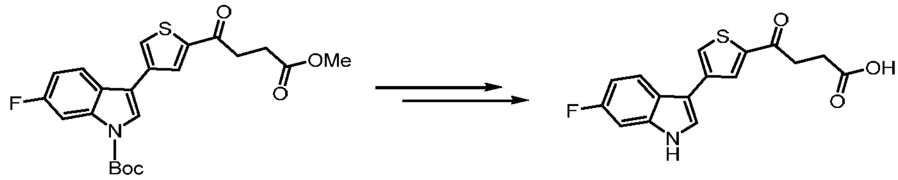

The preparation method of 4-(4-(6-fluoro-1H-indol-3-yl)thiophen-2-yl)-4-oxobutyric acid (**I'-46**) was the same as that of compound **I'-38**, and a yellow solid was obtained with a yield of 96%. $^1$H NMR (400 MHz, DMSO-$d_6$) $\delta$ 12.21 (br s, 1H), 11.49 (s, 1H), 8.38 (s, 1H), 8.14 (s, 1H), 7.96 (dd, $J$ = 5.4, 8.7 Hz, 1H), 7.91 (d, $J$ = 2.3 Hz, 1H), 7.23 (d, $J$ = 9.9 Hz, 1H), 6.98 (t, $J$ = 8.0 Hz, 1H), 3.30 (t, $J$ = 6.4 Hz, 2H), 2.60 (t, $J$ = 6.4 Hz, 2H).

**Example 61 4-(4-(6-chloro-1H-indol-3-yl)thiophen-2-yl)-4-oxobutyric acid** (**I'-47**)

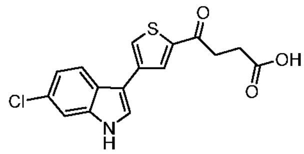

Step 1: 6-chloro-1-Boc-1H-indole (**47'-1**)

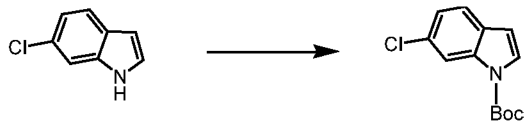

The preparation method of 6-chloro-1-Boc-1H-indole (**47'-1**) was the same as that of compound **38'-2**, and a white solid was obtained with a yield of 100%. $^1$H NMR (400 MHz, $CDCl_3$) $\delta$ 8.21 (s, 1H), 7.59 (d, $J$ = 2.8 Hz, 1H), 7.48 (d, $J$= 8.3 Hz, 1H), 7.22 (d, $J$= 8.3 Hz, 1H), 6.56 (d, $J$ = 3.6 Hz, 1H), 1.70 (s, 9H).

Step 2: 3-bromo-6-chloro-1-Boc-1H-indole (**47'-2**)

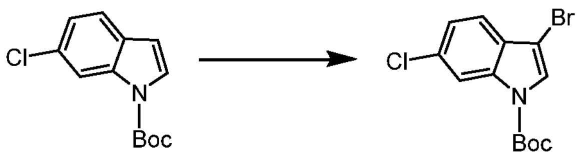

The preparation method of 3-bromo-6-chloro-1-Boc-1H-indole (**47'-2**) was the same as that of compound **38'-1**, and an off-white solid was obtained with a yield of 87%. $^1$H NMR (400 MHz, $CDCl_3$) $\delta$ 8.20 (s, 1H), 7.61 (s, 1H), 7.43 (d, $J$= 8.4 Hz, 1H), 7.27-7.31 (m, 1H), 1.67 (s, 9H).

Step 3: methyl 4-(4-(6-chloro-1-Boc-1H-indol-3-yl)thiophen-2-yl)-4-oxobutyrate (**47'-3**)

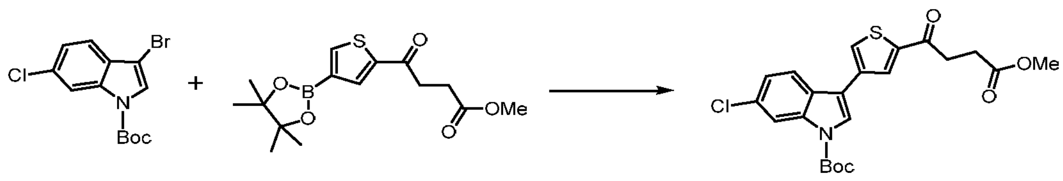

The preparation method of methyl 4-(4-(6-chloro-1-Boc-1H-indol-3-yl)thiophen-2-yl)-4-oxobutyrate (**47'-3**) was the same as that of compound **4'-1,** and a yellow oily liquid was obtained with a yield of 86%. $^1$H NMR (400 MHz, $CDCl_3$) $\delta$ 8.27 (s, 1H), 7.98 (s, 1H), 7.64-7.79 (m, 3H), 7.29 (dd, $J$ = 1.8, 8.5 Hz, 1H), 3.71 (s, 3H), 3.32 (t, $J$ = 6.6 Hz, 2H), 2.81(t, $J$ = 6.6 Hz, 2H), 1.70 (s, 9H).

Step 4: 4-(4-(6-chloro-1H-indol-3-yl)thiophen-2-yl)-4-oxobutyric acid (**I'-47**)

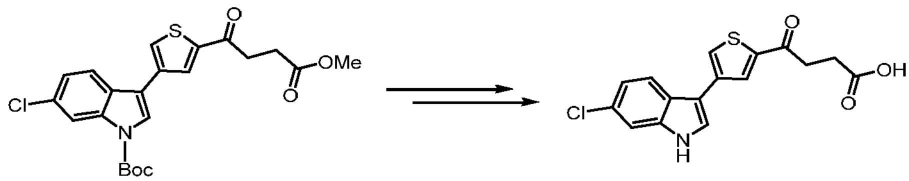

The preparation method of 4-(4-(6-chloro-1H-indol-3-yl)thiophen-2-yl)-4-oxobutyric acid (**I'-47**) was the same as that of compound **I'-38**, and a yellow solid was obtained with a yield of 90%. $^1$H NMR (400 MHz, DMSO-$d_6$) $\delta$ 12.22 (br s, 1H), 11.57 (s, 1H), 8.39 (d, $J$ = 1.3 Hz, 1H), 8.15 (d, $J$ = 1.3 Hz,1H), 7.97 (dd, $J$ = 5.6, 8.6 Hz, 1H), 7.50 (d, $J$ = 1.9 Hz, 1H), 7.13 (dd, $J$ = 1.9, 8.6 Hz, 1H), 3.30 (t, $J$ = 6.4 Hz, 2H), 2.61 (t, $J$ = 6.4 Hz, 2H).

**Example 62 4-(4-(5-fluoro-1H-indol-3-yl)thiophen-2-yl)-4-oxobutyric acid (I'-48)**

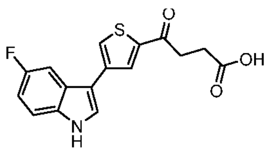

Step 1: 5-fluoro-1-Boc-1H-indole (**48'-1**)

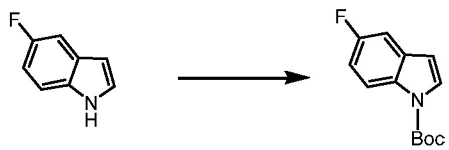

The preparation method of 5-fluoro-1-Boc-1H-indole (**48'-1**) was the same as that of compound **38'-2**, and a colorless oily liquid was obtained with a yield of 97%. $^1$H NMR (400 MHz, $CDCl_3$) $\delta$ 8.09 (s, 1H), 7.62 (d, $J$ = 3.0 Hz, 1H), 7.21 (dd, $J$ = 2.4, 8.9 Hz, 1H), 7.03 (td, $J$ = 2.5, 9.1 Hz, 1H), 6.52 (d, $J$ = 3.6 Hz, 1H), 1.67 (s, 9H).

Step 2: 3-bromo-5-fluoro-1-Boc-1H-indole (**48'-2**)

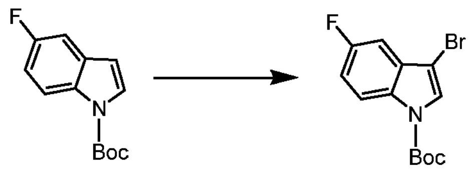

The preparation method of 3-bromo-5-fluoro-1-Boc-1H-indole (**48'-2**) was the same as that of compound **38'-1**, and an off-white solid was obtained with a yield of 80%. $^1$H NMR (400 MHz, $CDCl_3$) $\delta$ 8.11 (s, 1H), 7.67 (s, 1H), 7.18 (dt, $J$ = 6.0, 12.0 Hz, 1H), 7.09 (td, $J$ = 2.5, 9.1 Hz, 1H), 1.66 (s, 9H).

Step 3: methyl 4-(4-(5-fluoro-1-Boc-1H-indol-3-yl)thiophen-2-yl)-4-oxobutyrate (**48'-3**)

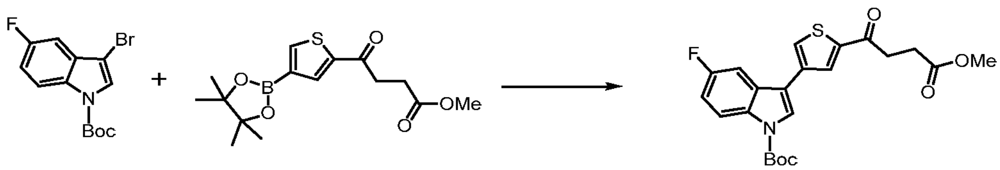

The preparation method of methyl 4-(4-(5-fluoro-1-Boc-1H-indol-3-yl)thiophen-2-yl)-4-oxobutyrate (**48'-3**) was the same as that of compound **4'-1,** and a yellow solid was obtained with a yield of 63%. $^1$H NMR (400 MHz, $CDCl_3$) $\delta$ 8.17 (s, 1H), 7.98 (d, $J$ = 1.3 Hz, 2H), 7.62-7.78 (m, 2H), 7.11 (td, $J$= 2.5, 9.0 Hz, 1H), 3.73 (s, 3H), 3.33 (t, $J$ = 6.7 Hz, 2H), 2.81 (t, $J$= 6.7 Hz, 2H), 1.70 (s, 9H).

Step 4: 4-(4-(5-fluoro-1H-indol-3-yl)thiophen-2-yl)-4-oxobutyric acid (**I'-48**)

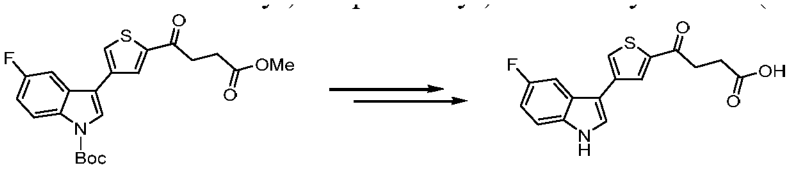

The preparation method of 4-(4-(5-fluoro-1H-indol-3-yl)thiophen-2-yl)-4-oxobutyric acid (**I'-48**) was the same as that of compound **I'-38**, and a yellow solid was obtained with a yield of 94%. $^1$H NMR (400 MHz, DMSO-$d_6$) $\delta$ 12.21 (br s, 1H), 11.54 (s, 1H), 8.40 (d, $J$ = 1.2 Hz, 1H), 8.16 (d, $J$= 1.2 Hz, 1H), 8.00 (d, $J$ = 2.6 Hz, 1H), 7.75 (dd, $J$ = 2.4, 10.3 Hz, 1H), 7.45 (dd, $J$= 4.7, 8.8 Hz, 1H), 7.03 (td, $J$= 2.4, 9.1 Hz, 1H), 6.98 (t, $J$ = 8.0 Hz, 1H), 3.31 (t, $J$ = 6.4 Hz, 2H), 2.60 (t, $J$ = 6.4 Hz, 2H).

**Example 63 4-(4-(7-fluoro-1H-indol-3-yl)thiophen-2-yl)-4-oxobutyric acid (I'-49)**

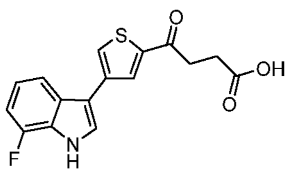

Step 1: 7-fluoro-1-Boc-1H-indole (**49'-1**)

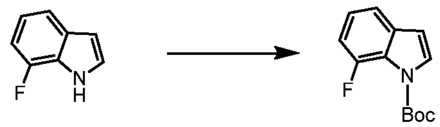

The preparation method of 7-fluoro-1-Boc-1H-indole (**49'-1**) was the same as that of compound **38'-2**, and a colorless oily liquid was obtained with a yield of 100%. $^1$H NMR (400 MHz, $CDCl_3$) $\delta$7.63 (d, *J*= 3.7 Hz, 1H), 7.32 (d, *J*= 7.7 Hz, 1H), 7.15 (td, *J*= 4.0, 7.8 Hz, 1H), 7.22 (dd, *J* = 8.0, 12.7 Hz, 1H) 6.58 (dd, *J*= 1.9, 3.6 Hz, 1H), 1.65 (s, 9H).

Step 2: 3-bromo-7-fluoro-1-Boc-1H-indole (**49'-2**)

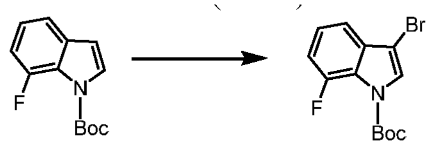

The preparation method of 3-bromo-7-fluoro-1-Boc-1H-indole (**49'-2**) was the same as that of compound **38'-1**, and an off-white solid was obtained with a yield of 80%. $^1$H NMR (400 MHz, $CDCl_3$) $\delta$7.70 (s, 1H), 7.33 (d, *J* = 7.8 Hz, 1H), 7.20-7.30 (m, 1H), 7.10 (dd, *J*= 7.9, 12.5 Hz, 1H), 1.65 (s, 9H).

Step 3: methyl 4-(4-(7-fluoro-1-Boc-1H-indol-3-yl)thiophen-2-yl)-4-oxobutyrate (**49'-3**)

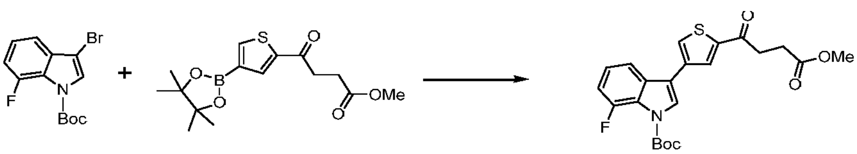

The preparation method of methyl 4-(4-(7-fluoro-1-Boc-1H-indol-3-yl)thiophen-2-yl)-4-oxobutyrate (**49'-3**) was the same as that of compound **4'-1,** and a yellow solid was obtained with a yield of 81%. $^1$H NMR (400 MHz, $CDCl_3$) $\delta$ 8.04 (d, *J* = 8.5 Hz, 1H), 8.00 (s, 1H), 7.81 (s, 1H), 7.68 (s, 1H), 7.27-7.36 (m, 1H), 6.94-7.02 (m, 1H), 3.72 (s, 3H), 3.32 (t, *J* = 6.8 Hz, 2H), 2.80 (t, *J* = 6.8 Hz, 2H), 1.70 (s, 9H).

Step 4: 4-(4-(7-fluoro-1H-indol-3-yl)thiophen-2-yl)-4-oxobutyric acid (**I'-49**)

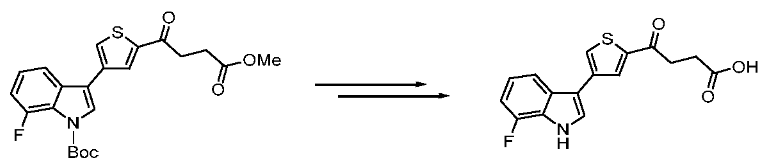

The preparation method of 4-(4-(7-fluoro-1H-indol-3-yl)thiophen-2-yl)-4-oxobutyric acid (**I'-49**) was the same as that of compound **I'-38**, and a pale yellow solid was obtained with a yield of 67%. $^1$H NMR (400 MHz, DMSO-$d_6$) $\delta$ 12.21 (br s, 1H), 11.93 (s, 1H), 8.43 (s, 1H), 8.17 (s, 1H), 8.00 (d, *J* = 2.5 Hz, 1H), 7.80 (d, *J* = 7.9 Hz, 1H), 6.97-7.14 (m, 2H), 3.29 (t, *J* = 6.4 Hz, 2H), 2.61 (t, *J* = 6.4 Hz, 2H).

**Example 64 Methyl 4-(4-(7-fluoro-1H-indol-3-yl)thiophen-2-yl)-4-oxobutyrate** (**I'-50**)

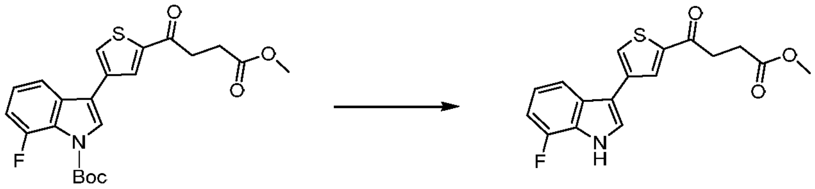

The preparation method of methyl 4-(4-(7-fluoro-1H-indol-3-yl)thiophen-2-yl)-4-oxobutyrate (**I'-50**) was the same as that of compound **31'-4**, and a pale yellow solid was obtained with a yield of 82%. $^1$H NMR (400 MHz, DMSO-$d_6$) $\delta$ 11.91 (s, 1H), 8.43 (d, $J$ = 1.6 Hz, 1H), 8.17 (d, $J$ = 1.6 Hz, 1H), 7.99 (d, $J$ = 2.8 Hz, 1H), 7.80 (d, $J$ = 8.0 Hz, 1H), 7.00-7.12 (m, 2H), 3.38 (t, $J$ = 6.4 Hz, 2H), 2.71 (t, $J$ = 6.4 Hz, 2H).

**Example 65 4-(4-(4-fluoro-1H-indol-3-yl)thiophen-2-yl)-4-oxobutyric acid (I'-51)**

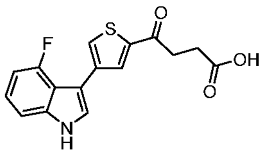

Step 1: 4-fluoro-1-Boc-1H-indole (**51'-1**)

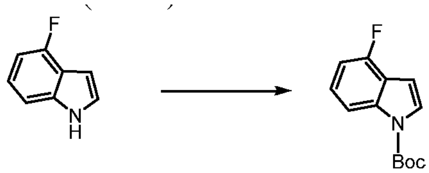

The preparation method of 4-fluoro-1-Boc-1H-indole (**51'-1**) was the same as that of compound **38'-2,** and a pale yellow solid was obtained with a yield of 95%. $^1$H NMR (400 MHz, $CDCl_3$) $\delta$ 7.93 (d, $J$ = 8.1 Hz, 1H), 7.56 (d, $J$= 3.5 Hz, 1H), 7.19-7.25 (m, 1H), 6.86-6.95 (m, 1H), 6.67 (d, $J$= 3.7 Hz, 1H), 1.67 (s, 9H).

Step 2: 3-bromo-4-fluoro-1-Boc-1H-indole (**51'-2**)

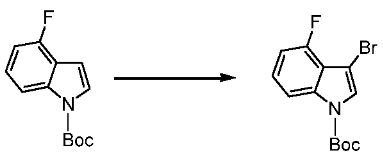

The preparation method of 3-bromo-4-fluoro-1-Boc-1H-indole (**51'-2**) was the same as that of compound **38'-1**, and a pale yellow solid was obtained with a yield of 82%. $^1$H NMR (400 MHz, $CDCl_3$) $\delta$ 7.98 (s, 1H), 7.58 (s, 1H), 7.28 (dd, $J$= 3.9, 7.0 Hz, 1H), 6.95 (dd, $J$ = 8.4, 10.2 Hz, 1H), 1.66 (s, 9H).

Step 3: methyl 4-(4-(4-fluoro-1-Boc-1H-indol-3-yl)thiophen-2-yl)-4-oxobutyrate (**51'-3**)

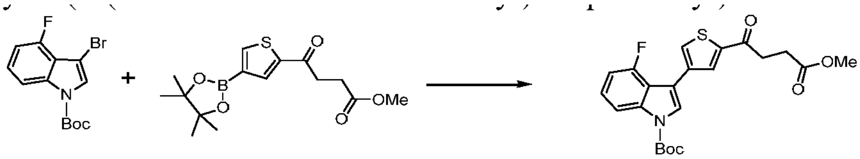

The preparation method of methyl 4-(4-(4-fluoro-1-Boc-1H-indol-3-yl)thiophen-2-yl)-4-oxobutyrate (**51'-3**) was the same as that of compound **4'-1,** and a yellow oil was obtained with a yield of 54%. $^1$H NMR (400 MHz, $CDCl_3$) $\delta$ 8.05 (d, $J$ = 8.6 Hz, 1H), 8.01 (t, $J$ = 1.5 Hz, 1H), 7.81 (s, 1H), 7.68 (s, 1H), 7.27-7.33 (m, 1H), 6.98 (dd, $J$ = 8.0, 11.1 Hz, 1H), 3.72 (s, 3H), 3.32 (t, $J$ = 6.8 Hz, 2H), 2.80 (t, $J$ = 6.8 Hz, 2H), 1.70 (s, 9H).

Step 4: 4-(4-(4-fluoro-1H-indol-3-yl)thiophen-2-yl)-4-oxobutyric acid (**I'-51**)

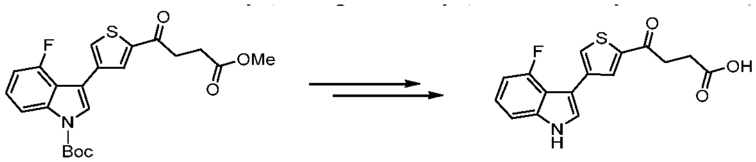

The preparation method of 4-(4-(4-fluoro-1H-indol-3-yl)thiophen-2-yl)-4-oxobutyric acid (**I'-51**) was the same as that of compound **I'-38**, and a pale yellow solid was obtained with a yield of 67%. $^1$H NMR (400 MHz, DMSO-$d_6$) $\delta$ 12.14 (br s, 1H), 11.77 (s, 1H), 8.29 (s, 1H), 7.95(s, 1H), 7.85 (d, $J$ = 2.5 Hz, 1H), 7.29 (d, $J$ = 8.1 Hz, 1H), 7.14 (td, $J$= 5.1, 8.0 Hz, 1H), 6.86 (dd, $J$ = 7.7, 12.2 Hz, 1H), 3.26 (t, $J$ = 6.4 Hz, 2H), 2.61 (t, $J$ = 6.4 Hz, 2H).

**Example 66 4-(4-(6-trifluoromethyl-1H-indol-3-yl)thiophen-2-yl)-4-oxobutyric acid (I'-52)**

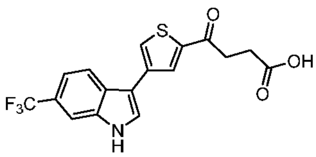

Step 1: 6-trifluoromethyl-1H-1-Boc-indole (**52'-1**)

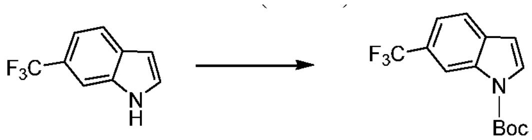

The preparation method of 6-trifluoromethyl-1H-1-Boc-indole (**52'-1**) was the same as that of compound **38'-2**, and a light brown oil was obtained with a yield of 100%. $^1$H NMR (400 MHz, $CDCl_3$) $\delta$ 8.48 (s, 1H), 7.73 (d, $J$ = 3.4 Hz, 1H), 7.65 (d, $J$ = 8.2 Hz, 1H), 7.47 (d, $J$ = 8.2 Hz, 1H), 6.62 (d, $J$ = 3.7 Hz, 1H), 1.69 (s, 9H).

Step 2: 3-bromo-6-trifluoromethyl-1H-1-Boc-indole (**52'-2**)

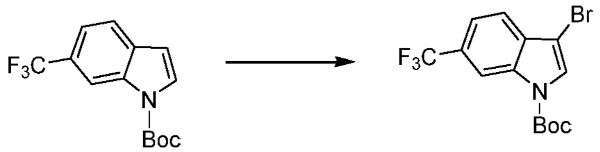

The preparation method of 3-romo-6-truoromety-1H-1-Boc-noe (**52'-2**) was the same as that of compound **38'-1**, and a pale yellow solid was obtained with a yield of 77%. $^1$H NMR (400 MHz, $CDCl_3$) $\delta$ 8.50 (s, 1H), 7.77 (s, 1H), 7.63 (d, $J$= 8.3 Hz, 1H), 7.56 (d, $J$ = 8.3 Hz, 1H), 1.68 (s, 9H).

Step 3: methyl 4-(4-(6-trifluoromethyl-1-Boc-1H-indol-3-yl)thiophen-2-yl)-4-oxobutyrate (**52'-3**)

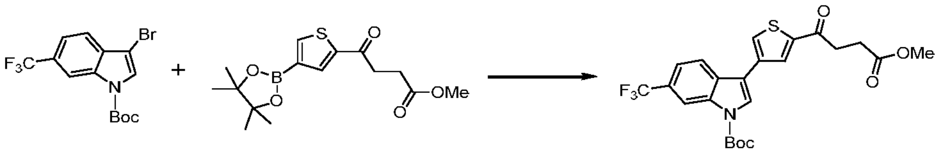

The preparation method of methyl 4-(4-(6-trifluoromethyl-1-Boc-1H-indol-3-yl)thiophen-2-yl)-4-oxobutyrate (**52'-3**) was same as that of compound **4'-1** , and a pale yellow oily liquid was obtained with a yield of 20%. $^1$H NMR (400 MHz, $CDCl_3$) $\delta$ 8.59 (s, 1H), 8.03 (s, 1H), 7.85-7.93 (m, 2H), 7.81 (s, 1H), 7.59 (d, $J$ = 8.3 Hz, 1H), 3.74 (s, 1H), 3.35 (t, $J$ = 6.8 Hz, 2H), 2.84 (t, $J$ = 6.8 Hz, 2H), 1.74 (s, 9H).

Step 4: 4-(4-(6-trifluoromethyl-1H-indol-3-yl)thiophen-2-yl)-4-oxobutyric acid (**I'-52**)

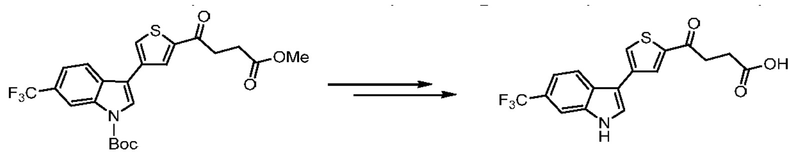

The preparation method of 4-(4-(6-trifluoromethyl-1H-indol-3-yl)thiophen-2-yl)-4-oxobutyric acid (**I'-52**) was the same as that of compound **I'-38**, and a pale yellow solid was obtained with a yield of 56%. $^1$H NMR (400 MHz, DMSO-$d_6$) $\delta$ 12.23 (br s, 1H), 11.88 (s, 1H), 8.43 (d, $J$ = 1.4 Hz, 1H), 8.14-8.24 (m, 3H), 7.80 (s, 1H), 7.41 (d, $J$ = 8.6 Hz, 1H), 3.31 (t, $J$ = 6.4 Hz, 2H), 2.61 (t, $J$ = 6.4 Hz, 2H).

**Example 67 4-(5-(1H-indol-3-yl)thiophen-3-yl)-4-oxobutyric acid** (**I'-53**)

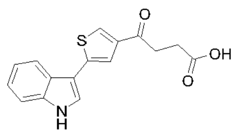

Step 1: 3-(4-formylthiophen-2-yl)-1-Boc-1H-indole (**53'-1**)

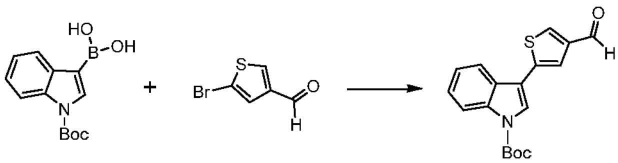

The preparation method of 3-(4-formylthiophen-2-yl)-1-Boc-1H-indole (**53'-1**) was the same as that of compound **4'-1,** and a light brown oil was obtained with a yield of 53% . $^1$H NMR (400 MHz, $CDCl_3$) $\delta$ 9.93 (s, 1H), 8.23 (d, *J* = 8.0 Hz, 1H), 8.08 (s, 1H), 7.91 (d, *J* = 8.0 Hz, 1H), 7.83 (s, 1H), 7.75 (s, 1H), 7.33-7.43 (m, 2H), 1.70 (s, 9H). LRMS (ESI) *m/z* $[M+H]^+$: 328.1.

Step 2: tert-butl 4-5-1H-indol-1-Boc-3-yl)thiohen-3-1-4-oxobutrate (**53'-2**)

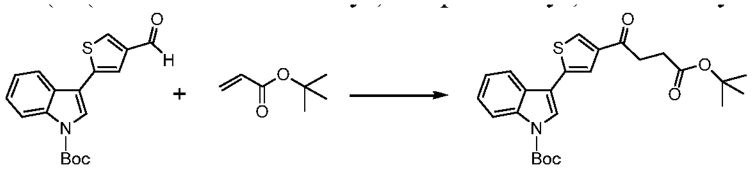

A mixture of compound **53'-1** (120 mg), tert-butyl acrylate (53 μL), tributylphosphine (92 μL), and anhydrous tetrahydrofuran (2 mL) was heated and stirred at 80° C for 5 hours under the protection of argon. The reaction solution was concentrated to dryness, and the residue was purified by preparative thin layer chromatography (ethyl acetate: petroleum ether = 1: 5) to obtain 40mg of tert-butyl 4-(5-(1H-indol-1-Boc-3-yl)thiophen-3-yl)-4-oxobutyrate (**53'-2**) as a colorless oil with a yield of 23%. $^1$H NMR (400 MHz, $CDCl_3$) $\delta$ 8.23 (d, *J* = 8.0 Hz, 1H), 8.04 (s, 1H), 7.91 (d, *J* = 8.0 Hz, 1H), 7.75-7.81 (m, 2H), 7.32-7.42 (m, 2H), 3.23 (t, *J* = 8.0 Hz, 2H), 2.71 (t, *J* = 8.0 Hz, 2H), 1.70 (s, 9H), 1.46 (s, 9H).

Step 3: 4-(5-(1H-indol-3-yl)thiophen-3-yl)-4-oxobutyric acid (**I'-53**)

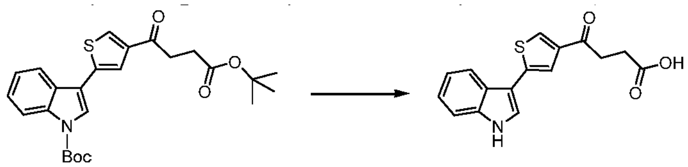

Compound **53'-2** (37 mg) was dissolved in 1 mL of dichloromethane, and a solution of trifluoroacetic acid in dichloromethane (120 μL trifluoroacetic acid/0.5 mL of dichloromethane) was added dropwise with stirring in an ice bath, and the ice bath was removed after the addition was completed, and stirred at room temperature for 17 hours. The reaction solution was concentrated to dryness, and the residue was purified by preparative thin layer chromatography (dichloromethane: methanol: acetic acid = 100:2:1) to obtain 9mg 4-(5-(1H-indol-3-yl)thiophen-3-yl)-4-oxobutyric acid ( **I'-53**) as a brown solid with a yield of 37%. $^1$H NMR (400 MHz, $CD_3OD$) $\delta$ 8.19 (d, *J* = 4.0 Hz, 1H), 7.89 (d, *J* = 8.0 Hz, 1H), 7.65 (s, 1H), 7.57 (s, 1H), 7.44 (d, *J* = 8.0 Hz, 1H), 7.12-7.21 (m, 2H), 3.30 (t, *J* = 8.0 Hz, 2H), 2.72 (t, *J* = 8.0 Hz, 2H). LRMS (ESI) *m/z* $[M-H]^-$: 298.0.

**Example 68 4-(4-(5-fluorobenzofuran-3-yl)thiohen-2-yl)-4-oxobutyric acid (I'-54)**

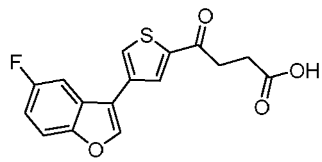

Step 1: 3-bromo-5-fluorobenzofuran (**54'-1**)

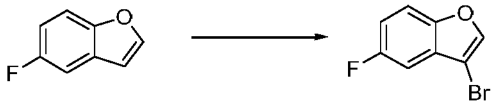

The preparation method of 3-bromo-5-fluorobenzofuran (**54'-1**) was the same as that of compound **42'-1,** and a brown oil was obtained with a yield of 52.5%. $^1$H NMR (400 MHz, $CDCl_3$) $\delta$7.59 (s, 1H), 7.34-7.27 (m, 2H), 6.97 (m, 1H).

Step 2: methyl 4-(4-(5-fluorobenzofuran-3-yl)thiophen-2-yl)-4-oxobutyrate (**54'-2**)

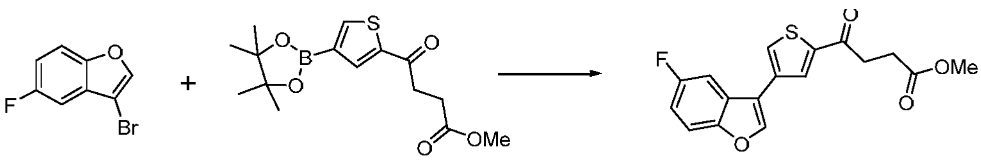

The preparation method of methyl 4-(4-(5-fluorobenzofuran-3-yl)thiophen-2-yl)-4-oxobutyrate (**54'-2**) was the same as that of compound **4'-1,** and a yellow oil was obtained. LRMS (ESI) *m/z* $[M+Na]^+$: 355.1.

Step 3: 4-4-5-fluorobenzofuran-3-yl)thiohen-2-yl)-4-oxobutyric acid (**I'-54**)

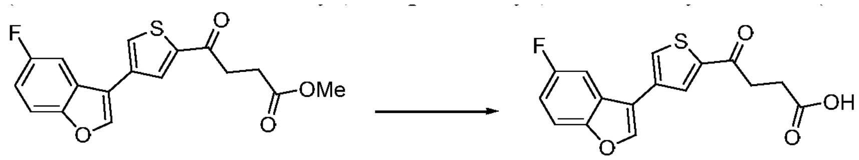

The preparation method of 4-(4-(5-fluorobenzofuran-3-yl)thiophen-2-yl)-4-oxobutyric acid (**I'-54**) was the same as that of compound **I'-4**, and a yellowish white powdery solid was obtained with a yield of 50.0%. $^1$H NMR (400 MHz, DMSO-$d_6$) $\delta$12.21 (s, 1H), 8.52 (s, 1H), 8.35 (s, 1H), 8.18 (s, 1H), 7.56 (d, *J* = 8.4 Hz, 1H), 7.43 (td, *J*= 5.4, 8.2 Hz, 1H), 7.20 (dd, *J* = 8.3, 11.0 Hz, 1H), 3.26 (t, *J* = 6.4 Hz, 2H), 2.61 (t, *J* = 6.3 Hz, 2H).

**Example 69 4-(4-(7-fluorobenzofuran-3-yl)thiophen-2-yl)-4-oxobutyric acid (I'-55)**

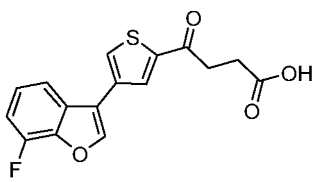

Step 1: 3-fluoro-2-((trimethylsilyl)methoxy)benzaldehyde (**55'-1**)

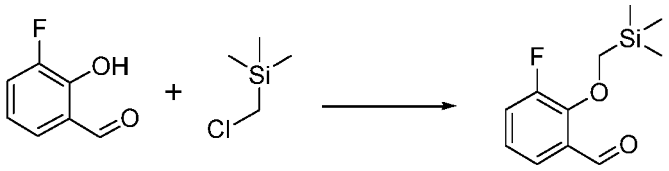

3-fluoro-2-hydroxybenzaldehyde (2g) and potassium carbonate (5.92g) were dissolved in N,N-dimethylformamide (39mL), (chloromethyl)trimethylsilane (2.21mL) and sodium iodide (2.37g) were added successively, heated and stirred at 65°C for 12 hours under argon protection. The reaction solution was cooled to room temperature, water was added, extracted with diethyl ether. The organic phase was concentrated to dryness, and the residue was subjected to column chromatography (petroleum ether) to obtain 3.25g 3-fluoro-2-((trimethylsilyl)methoxy)benzaldehyde (**55'-1**) as a colorless transparent oil with a yield of 100%. $^1$H NMR (400 MHz, $CDCl_3$) $\delta$ 10.40 (d, $J$ = 0.8 Hz, 1H), 7.65-7.51 (m, 1H), 7.31 (m, 1H), 7.06 (m, 1H), 4.00 (d, $J$ = 1.7 Hz, 2H), 0.17 (s, 9H).

Step 2: 7-fluoro-2,3-dihydrobenzofuran-3-ol (**55'-2**)

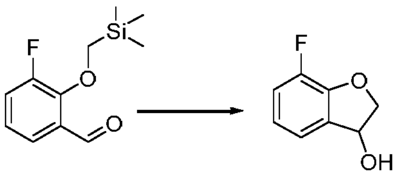

Compound **55'-1** (3 g) and cesium fluoride (6.04 g) were dissolved in N,N-dimethylformamide (40 mL), heated and stirred at 105°C for 36 hours under argon protection. The reaction solution was cooled to room temperature, saturated sodium bicarbonate and water were added successively, extracted with ethyl acetate, and the organic phase was concentrated to dryness. The residue was purified by column chromatography (ethyl acetate : petroleum ether = 15: 85) to obtain 1.88 g 7-fluoro-2,3-dihydrobenzofuran-3-ol (**55'-2**) as a yellow oil with a yield of 92.0%. $^1$H NMR (400 MHz, $CDCl_3$) $\delta$ 7.20 (d, $J$ = 7.4 Hz, 1H), 7.05 (m, 1H), 6.89 (m, 1H), 5.41 (d, $J$ = 5.8 Hz, 1H), 4.64 (dd, $J$ = 6.5, 10.8 Hz, 1H), 4.54 (dd, $J$ = 2.7, 10.8 Hz, 1H).

Step 3: 7-fluorobenzofuran (**55'-3**)

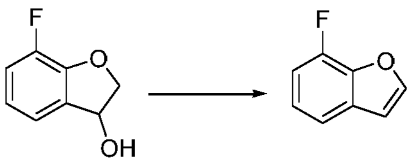

Compound **55'-2** (881 mg) was dissolved in methanol (21 mL), 4N hydrochloric acid (17.6 mL) was added dropwise under an ice bath, and the mixture was stirred at room temperature for 12 hours after addition. It was extracted with dichloromethane, and the organic phase was concentrated to dryness. The residue was purified by column chromatography (petroleum ether) to obtain 269.7 mg 7-fluorobenzofuran (**55'-3**) as a colorless oily liquid with a yield of 34.7%. $^1$H NMR (400 MHz, $CDCl_3$) $\delta$ 7.66 (d, $J$ = 2.1 Hz, 1H), 7.37 (dd, $J$ = 0.9, 7.8 Hz, 1H), 7.16 (td, $J$ = 4.4, 7.9 Hz, 1H), 7.04 (m, 1H), 6.82 (q, $J$ = 2.2, 2.9 Hz, 1H).

Step 4: 3-bromo-7-fluorobenzofuran (**55'-4**)

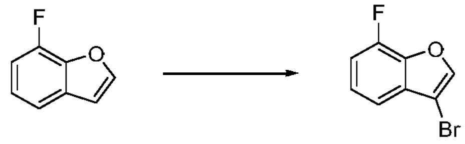

The preparation method of 3-bromo-7-fluorobenzofuran (**55'-4**) was the same as that of compound **42'-1**, and a colorless oily liquid was obtained with a yield of 22%. $^1$H NMR (400 MHz, $CDCl_3$) $\delta$ 7.69 (s, 1H), 7.33 (dd, $J$ = 1.1, 7.9 Hz, 1H), 7.26 (td, $J$ = 4.1, 8.1 Hz, 1H), 7.11 (m, 1H).

Step 5: methyl 4-(4-(7-fluorobenzofuran-3-yl)thiophen-2-yl)-4-oxobutyrate (**55'-5**)

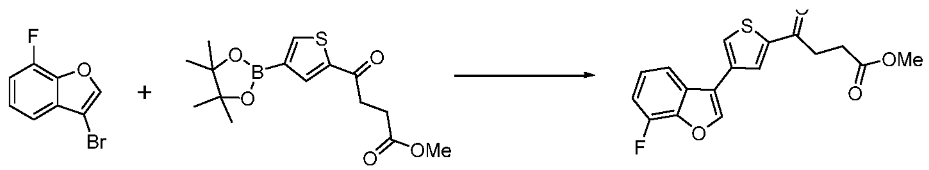

The preparation method of methyl 4-(4-(7-fluorobenzofuran-3-yl)thiophen-2-yl)-4-oxobutyrate(**55'-5**) was the same as that of compound **4'-1,** and a crude orange-yellow powdery solid was obtained, which was directly used in the next step.

Step 6: 4-(4-(7-fluorobenzofuran-3-yl)thiophen-2-yl)-4-oxobutyric acid (**I'-55**)

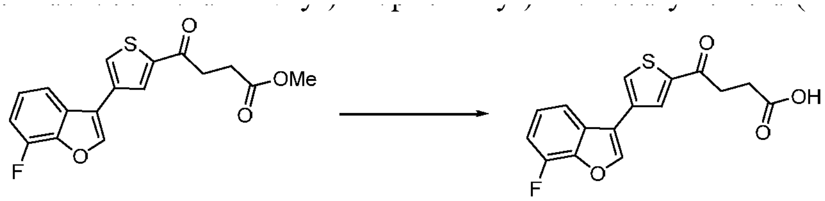

The preparation method of 4-(4-(7-fluorobenzofuran-3-yl)thiophen-2-yl)-4-oxobutyric acid (**I'-55**) was the same as that of compound **I'-4**, and a yellowish white powdery solid was obtained with a yield of 55.9%. $^1$H NMR (400 MHz, DMSO-$d_6$) $\delta$ 12.23 (s, 1H), 8.67 (s, 1H), 8.46 (d, $J$ = 1.0 Hz, 1H), 8.43 (s, 1H), 7.92 (d, $J$ = 7.5 Hz, 1H), 7.48-7.04 (m, 2H), 3.33-3.27 (t, $J$ = 6.4 Hz, 2H), 2.62 (t, $J$ = 6.4 Hz, 2H).

**Example 70 4-(3-fluoro-4-(1H-indol-3-yl)thiophen-2-yl)-4-oxobutyric acid (I'-56)**

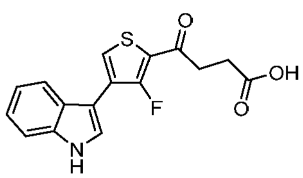

Step 1: methyl 4-bromo-3-fluorothiophene-2-carboxylate (**56'-1**)

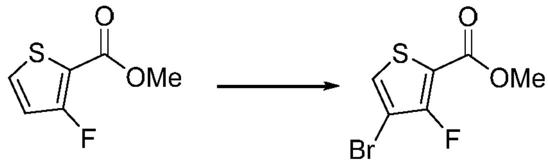

Methyl 3-fluorothiophen-2-carboxylate (1.18g), anhydrous aluminum trichloride (5.89g), and iron bromide (218mg) were dissolved in chloroform (35mL), and a solution of bromine (566μL) in chloroform (5 mL) was added dropwise under the protection of argon, and stirred at room temperature for 48 hours after addition. The reaction solution was poured into ice water, extracted with dichloromethane, and the organic phase was concentrated to dryness, and subjected to column chromatography with ethyl acetate: petroleum ether = 1: 9 to obtain 1.07 g of methyl 4-bromo-3-fluorothiophene-2-carboxylate (**56'-1**) as a yellow-white solid with a yield of 61%. $^1$H NMR (400 MHz, $CDCl_3$) $\delta$ 7.42 (d, *J* = 3.8 Hz, 1H), 3.91 (s, 3H).

Step 2: 4-bromo-3-fluorothiophen-2-carboxylic acid (**56'-2**)

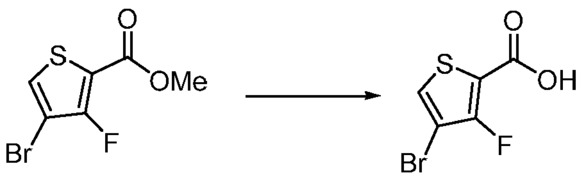

The preparation method of 4-bromo-3-fluorothiophen-2-carboxylic acid (**56'-2**) was the same as that of compound **I'-4**, and a brownish yellow solid was obtained with a yield of 100%. $^1$H NMR (400 MHz, DMSO-$d_6$) $\delta$ 13.73 (s, 1H), 8.11 (d, *J* = 4.2 Hz, 1H).

Step 3: tert-butyl 3-4-bromo-3-fluorothiophen-2-yl)-3-oxopropanoate **(56'-3)**

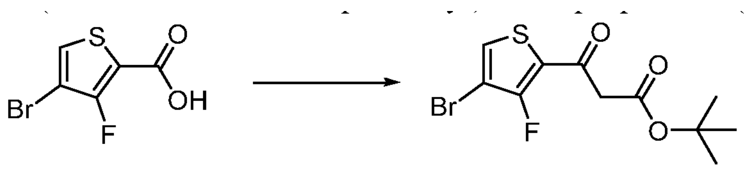

Compound **56'-2** (1.07 g) and N,N'-carbonyldiimidazole (729 mg) were dissolved in tetrahydrofuran (2.5 mL), and stirred at room temperature for 1 hour to obtain solution A.

Tert-butyl acetate (1.81 mL) was added dropwise into 2M lithium diisopropylamide (6.8 mL) at -78 °C, and stirred for 1 hour to obtain solution B. The solution A was added into solution B at -78 °C, and stirred for 1 hour after addition. The reaction solution was adjusted to pH≈4 with 1M hydrochloric acid, and the reaction solution was returned to room temperature, and extracted with ethyl acetate. The organic phase was concentrated to dryness, and subjected to column chromatography with ethyl acetate: petroleum ether = 2: 98 to obtain 530 mg of tert-butyl 3-(4-bromo-3-fluorothiophen-2-yl)-3-oxopropanoate (**56'-3**) as yellow oil with a yield of 66.7%. $^1$H NMR (400 MHz, $CDCl_3$) $\delta$ 7.55 (d, *J* = 3.8 Hz, 1H), 3.83 (d, *J* = 1.9 Hz, 2H), 1.46 (s, 9H).

Step 4: 1-tert-butl-4-methl-2-4-bromo-3-fluorothiohen-2-carbonlsuccinate **(56'-4)**

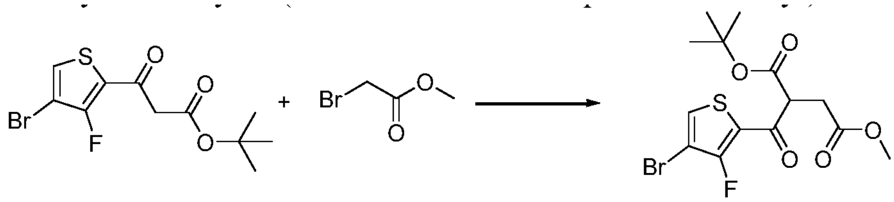

A solution of compound **56'-3** (330 mg) in tetrahydrofuran (1 mL) was slowly added dropwise to a mixture of 60% NaH (45 mg) and anhydrous tetrahydrofuran (3.5 mL) under an ice bath. The mixture was raised to room temperature after addition, and stirred for 1.5 hours until the reaction solution became clear and transparent, methyl bromoacetate (106 μL) was added dropwise, and the reaction was stirred at room temperature for 7 hours. The reaction solution was concentrated to dryness, and the residue was subjected to column chromatography with ethyl acetate: petroleum ether = 4: 96 to obtain 387 mg of 1-tert-butyl-4-methyl-2-(4-bromo-3-fluorothiophen-2-carbonyl)succinate (**56'-4**) as a colorless and transparent oil with a yield of 95.8%. $^1$H NMR (400 MHz, $CDCl_3$) $\delta$ 7.55 (d, $J$ = 3.8 Hz, 1H), 4.58 (dd, $J$ = 5.3, 8.9 Hz, 1H), 3.69 (s, 3H), 3.12 (dd, $J$ = 9.0, 17.3 Hz, 1H), 2.94 (dd, $J$ = 5.3, 17.4 Hz, 1H), 1.39 (s, 9H).

Step 5: methyl 4-4-bromo-3-fluorothiophen-2-yl)-4-oxobutyrate **(56'-5)**

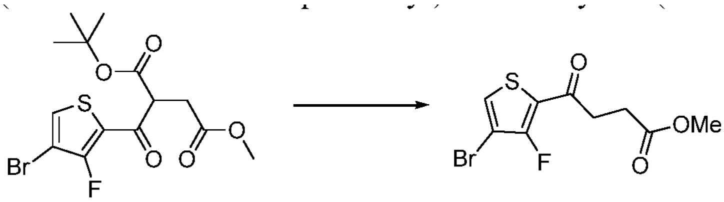

Trifluoroacetic acid (1.63 mL) was slowly added dropwise to a solution of compound 56'-4 (385 mg) in dichloromethane under an ice bath. The mixture was stirred for 5 hours under an ice bath after addition, and then returned to room temperature and stirred for 24 hours. The reaction solution was concentrated to dryness, and the residue was subjected to column chromatography with ethyl acetate: petroleum ether = 4: 96 to obtain 300 mg of methyl 4-(4-bromo-3-fluorothiophen-2-yl)-4-oxobutyrate (**56'-5**) as a pale yellow oil with a yield of 100%. $^1$H NMR (400 MHz, $CDCl_3$) $\delta$ 7.52 (d, $J$= 3.7 Hz, 1H), 3.71 (s, 3H), 3.26 (td, $J$ = 2.1, 6.5 Hz, 2H), 2.76 (t, $J$= 6.4 Hz, 2H).

Step 6: methyl 4-(3-fluoro-4-(1-Boc-1H-indol-3-yl)thiophen-2-yl)-4-oxobutyrate **(56'-6)**

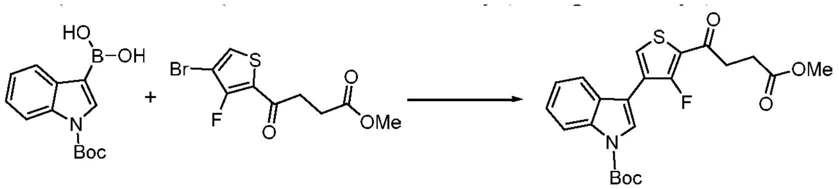

The preparation method of methyl 4-(3-fluoro-4-(1-Boc-1H-indol-3-yl)thiophen-2-yl)-4-oxobutyrate (**56'-6**) was the same as that of compound **4'-1,** 164 mg of pale yellow solid was obtained with a yield of 75%. $^1$H NMR (400 MHz, $CDCl_3$) $\delta$ 8.24 (d, $J$ = 7.4 Hz, 1H), 7.87 (s, 1H), 7.77 (d, $J$= 4.2 Hz, 1H), 7.74 (d, $J$ = 8.0 Hz, 1H), 7.44-7.37 (m, 1H), 7.33 (td, $J$ = 1.0, 7.7 Hz, 1H), 3.72 (s, 3H), 3.34 (td, $J$ = 2.1, 6.6 Hz, 2H), 2.78 (t, $J$ = 6.5 Hz, 2H), 1.70 (s, 9H).

Step 7: methyl 4-(3-fluoro-4-(1H-indol-3-yl)thiohen-2-yl)-4-oxobutyrate **(56'-7)**

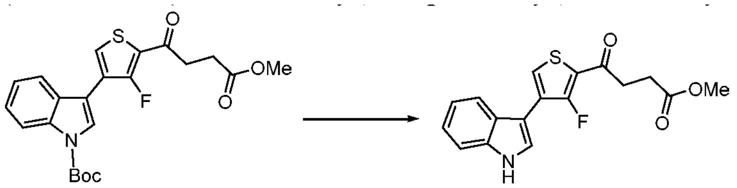

The preparation method of methyl 4-(3-fluoro-4-(1H-indol-3-yl)thiophen-2-yl)-4-oxobutyrate (**56'-7**) was the same as that of compound **31'-4**, 49mg of pale yellow solid was obtained with a yield of 40%. $^1$H NMR (400 MHz, $CDCl_3$) $\delta$8.37 (s, 1H), 7.84 (d, *J*= 7.9 Hz, 1H), 7.75 (d, *J* = 4.2 Hz, 1H), 7.57 (d, *J* = 1.9 Hz, 1H), 7.47 (d, *J* = 7.9 Hz, 1H), 7.34-7.27 (m, 1H), 7.23 (d, *J* = 7.8 Hz, 1H), 3.72 (s, 3H), 3.34 (t, *J* = 6.4 Hz, 2H), 2.78 (t, *J* = 6.5 Hz, 2H).

Step 8: 4-(3-fluoro-4-(1H-indol-3-yl)thiophen-2-yl)-4-oxobutyric acid **(I'-56)**

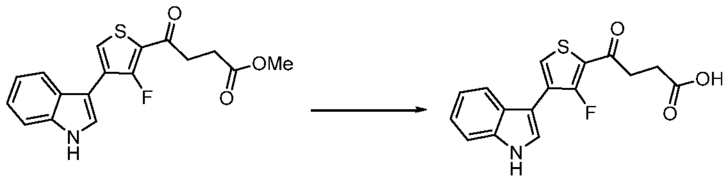

The preparation method of 4-(3-fluoro-4-(1H-indol-3-yl)thiophen-2-yl)-4-oxobutyric acid **(I'-56)** was the same as that of compound **I'-4**, 20mg of pale yellow solid was obtained with a yield of 43%. $^1$H NMR (400 MHz, DMSO-$d_6$) $\delta$ 12.21 (s, 1H), 11.53 (s, 1H), 8.19 (d, *J* = 4.5 Hz, 1H), 7.84 (d, *J* = 7.9 Hz, 1H), 7.73 (s, 1H), 7.48 (d, *J* = 8.0 Hz, 1H), 7.20 (t, *J* = 7.5 Hz, 1H), 7.13 (t, *J* = 7.5 Hz, 1H), 3.21 (t, *J* = 5.7 Hz, 2H), 2.60 (t, *J* = 6.3 Hz, 2H).

**Example 71 4-(4-(5-fluoro-1H-indol-3-yl)furan-2-yl)-4-oxobutyric acid (I'-57)**

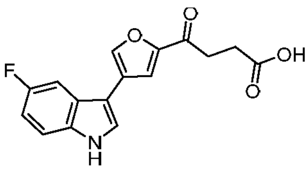

Step 1: methyl 4-(4-(5-fluoro-1-Boc-1H-indol-3-yl)furan-2-yl)-4-oxobutyrate **(57'-1)**

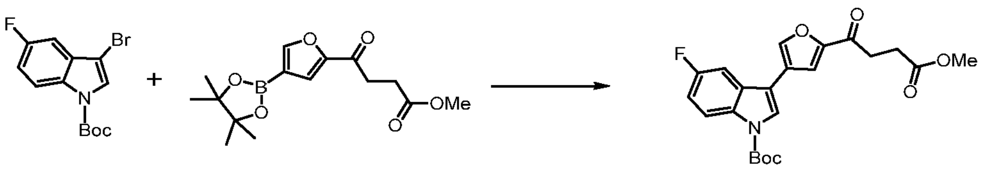

The preparation method of methyl 4-(4-(5-fluoro-1-Boc-1H-indol-3-yl)furan-2-yl)-4-oxobutyrate **(57'-1)** was the same as that of compound **4'-1,** and a pale yellow oily liquid was obtained with a yield of 50%. $^1$H NMR (400 MHz, $CDCl_3$) $\delta$8.16 (s, 1H), 7.89 (s, 1H), 7.75 (s, 1H), 7.48 (s, 1H), 7.29-7.35 (m, 1H), 7.11 (t, *J* = 9.0 Hz, 1H), 3.72 (s, 3H), 3.25 (t, *J* = 6.7 Hz, 2H), 2.79 (t, *J* = 6.7 Hz, 2H), 1.69 (s, 9H).

Step 2: 4-(4-(5-fluoro-1H-indol-3-yl)furan-2-yl)-4-oxobutyric acid **(I'-57)**

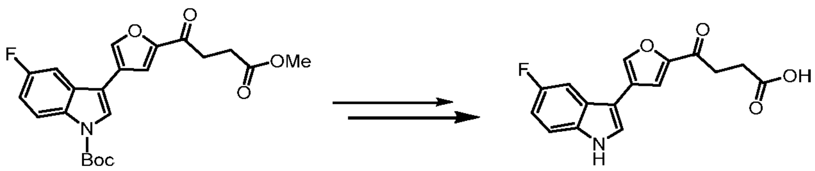

The preparation method of 4-(4-(5-fluoro-1H-indol-3-yl)furan-2-yl)-4-oxobutyric acid **(I'-57)** was the same as that of compound **I'-38**, and a yellow solid was obtained with a yield of 52%. $^1$H NMR (400 MHz, DMSO-$d_6$) $\delta$ 12.38 (br s, 1H), 11.51 (s, 1H), 8.49 (s, 1H), 7.94 (s, 1H), 7.91 (d, $J$ = 2.6 Hz, 1H), 7.67 (dd, $J$ = 2.3, 10.3 Hz, 1H), 7.67 (dd, $J$ = 4.7, 8.8 Hz, 1H), 7.02 (td, $J$ = 2.4, 9.2 Hz, 1H), 3.12 (t, $J$ = 6.5 Hz, 2H), 2.57 (t, $J$ = 6.5 Hz, 2H).

**Example 72 4-(4-(7-fluoro-1H-indol-3-yl)furan-2-yl)-4-oxobutyric acid (I'-58)**

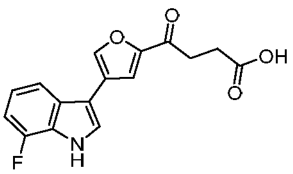

Step 1: methyl 4-(4-(7-fluoro-1-Boc-1H-indol-3-yl)furan-2-yl)-4-oxobutyrate **(58'-1)**

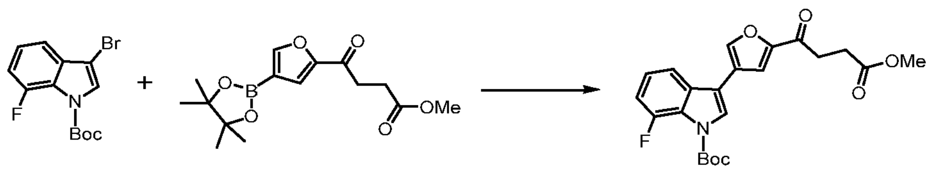

The preparation method of methyl 4-(4-(7-fluoro-1-Boc-1H-indol-3-yl)furan-2-yl)-4-oxobutyrate (58'-1) was the same as that of compound **4'-1,** and a pale yellow oily liquid was obtained with a yield of 54%. $^1$H NMR (400 MHz, $CDCl_3$) $\delta$ 7.90 (s, 1H), 7.77 (s, 1H), 7.49 (s, 1H), 7.44 (d, $J$ = 7.4 Hz, 1H), 7.21-7.29 (m, 1H), 7.10 (dd, $J$ = 8.0, 12.6 Hz, 1H), 3.72 (s, 3H), 3.24 (t, $J$ = 6.7 Hz, 2H), 2.79 (t, $J$ = 6.7 Hz, 2H), 1.67 (s, 9H).

Step 2: 4-(4-(7-fluoro-1H-indol-3-yl)furan-2-yl)-4-oxobutyric acid **(I'-58)**

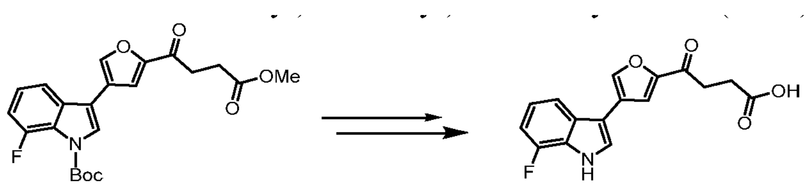

The preparation method of 4-(4-(7-fluoro-1H-indol-3-yl)furan-2-yl)-4-oxobutyric acid **(I'-58)** was the same as that of compound **I'-38**, **and** a pale yellow solid was obtained with a yield of 46%. $^1$H NMR (400 MHz, DMSO-$d_6$) $\delta$ 12.23 (br s, 1H), 11.89 (s, 1H), 8.48 (d, $J$ = 2.6 Hz, 1H), 7.88-8.02 (m, 2H), 7.69 (t, $J$ = 11.3 Hz, 1H), 6.94-7.13 (m, 3H), 3.13 (t, $J$ = 6.4 Hz, 2H), 2.60 (t, $J$ = 6.4 Hz, 2H).

**Example 73 4-(4-(4-fluoro-1H-indol-3-yl)furan-2-yl)-4-oxobutyric acid (I'-59)**

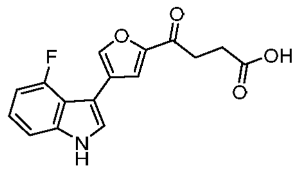

Step 1: methyl 4-(4-(4-fluoro-1-Boc-1H-indol-3-yl)furan-2-yl)-4-oxobutyrate (**59'-1**)

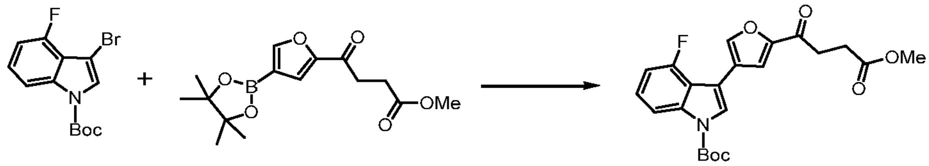

The preparation method of methyl 4-(4-(4-fluoro-1-Boc-1H-indol-3-yl)furan-2-yl)-4-oxobutyrate (**59'-1**) was the same as that of compound **4'-1**, and a pale yellow oil was obtained with a yield of 32%. $^1$H NMR (400 MHz, $CDCl_3$) $\delta$ 8.03 (d, *J* = 8.6 Hz, 1H), 7.96 (s, 1H), 7.68 (s, 1H), 7.52 (s, 1H), 7.21-7.34 (m, 1H), 6.94-7.03 (m, 1H), 3.72 (s, 3H), 3.24 (t, *J* = 6.8 Hz, 2H), 2.78 (t, *J* = 6.7 Hz, 2H), 1.69 (s, 9H).

Step 2: 4-(4-(4-fluoro-1H-indol-3-yl)furan-2-yl)-4-oxobutyric acid **(I'-59)**

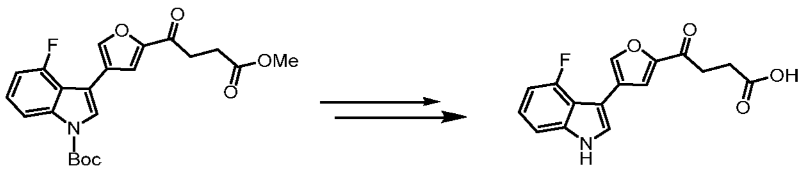

The preparation method of 4-(4-(4-fluoro-1H-indol-3-yl)furan-2-yl)-4-oxobutyric acid **(I'-59)** was the same as that of compound **I'-38**, and a yellow solid was obtained with a yield of 41%. $^1$H NMR (400 MHz, DMSO-$d_6$) $\delta$ 12.25 (br s, 1H), 11.74 (s, 1H), 8.13 (s, 1H), 7.79-7.88 (m, 2H), 7.28 (d, *J*= 8.2 Hz, 1H), 7.13 (dd, *J*= 8.1, 13.1 Hz, 1H), 6.85 (dd, *J*= 7.9, 11.8 Hz, 1H), 3.11 (t, *J* = 6.2 Hz, 2H), 2.60 (t, *J* = 6.4 Hz, 2H).

**Example 74 Methyl 4-(4-(1H-indol-4-yl)furan-2-yl)-4-oxobutyrate (I'-60)**

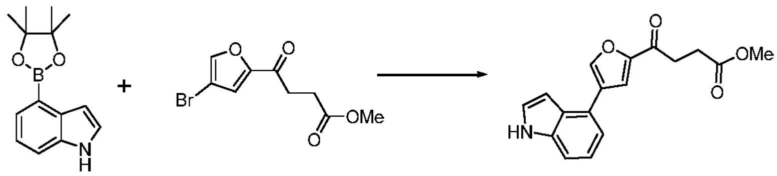

The preparation method of methyl 4-(4-(1H-indol-4-yl)furan-2-yl)-4-oxobutyrate (I'-60) was the same as that of compound **4'-1**, and a pale brown solid was obtained with a yield of 64%. $^1$H NMR (400 MHz, $CD_3OD$) $\delta$ 8.21 (s, 1H), 7.80 (s, 1H), 7.40 (d, *J*= 8.4 Hz, 1H), 7.33 (d, *J* = 3.2 Hz, 1H), 7.24 (d, *J* = 7.2 Hz, 1H), 7.17 (t, *J* = 7.6 Hz, 1H), 6.69 (d, *J* = 3.2 Hz, 1H), 3.69 (s, 3H), 3.29 (t, *J*= 6.4 Hz, 2H), 2.76 (t, *J* = 6.4 Hz, 2H). LRMS (ESI) *m/z* [M+H]$^+$: 298.1.

**Example 75 4-4-1H-indol-3-yl)thiophen-2-yl)-N-hydroxy-4-oxobutanamide (I'-61)**

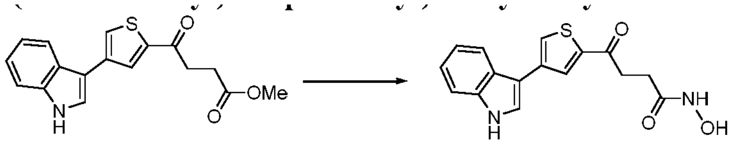

Compound **31'-4** (31 mg, 0.1 mmol) was dissolved in methanol (1 mL), and the mixture was dropped into a solution of 1.5N hydroxylamine/potassium hydroxide in methanol (23 mL/mmol), stirred at room temperature for 4 hours. The reaction solution was concentrated to dryness, and the residue was purified by preparative thin layer chromatography (dichloromethane : methanol = 10:1) to obtain 0.3 mg of 4-(4-(1H-indol-3-yl)thiophen-2-yl)-N-hydroxyl -4-oxobutanamide (**I'-61**) as a light brown solid with a yield of 0.9%. LRMS (ESI) *m/z* $[M+H]^+$: 315.1.

**Example 76 4-4-5-cano-1H-indol-3-yl)furan-2-yl)-4-oxobutric acid (I'-62)**

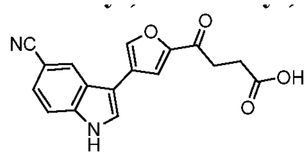

Step 1: 5-cyano-1H-1-Boc-indole (**62'-1**)

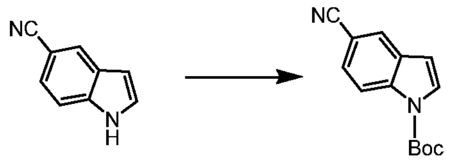

The preparation method of 5-cyano-1H-1-Boc-indole (**62'-1**) was the same as that of compound 38'-2, and a white solid was obtained with a yield of 90%. $^1H$ NMR (400 MHz, $CDCl_3$) $\delta$ 8.25 (d, *J* = 8.6 Hz, 1H), 7.90 (s, 1H), 7.70 (d, *J*= 3.7 Hz, 1H), 7.56 (dd, *J*= 1.6, 8.7 Hz, 1H), 6.63 (d, *J*= 3.7 Hz, 1H), 1.68 (s, 9H).

Step 2: 3-bromo-5-cyano-1-Boc-1H-indole (**62'-2**)

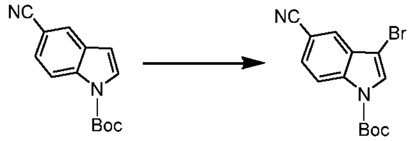

The preparation method of 3-bromo-5-cyano-1-Boc-1H-indole (**62'-2**) was the same as that of compound **38'-1**, and a pale yellow solid was obtained with a yield of 62%. $^1H$ NMR (400 MHz, $CDCl_3$) $\delta$ 8.27 (d, *J* = 8.5 Hz, 1H), 7.88 (d, *J* = 1.2 Hz, 1H), 7.75 (s, 1H), 7.62 (dd, *J*= 1.3, 8.7 Hz, 1H), 1.68 (s, 9H).

Step 3: methyl 4-(4-(5-cyano-1-Boc-1H-indol-3-yl)furan-2-yl)-4-oxobutyrate (**62'-3**)

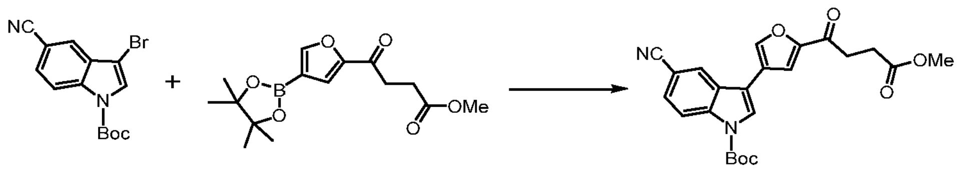

The preparation method of methyl 4-(4-(5-cyano-1-Boc-1H-indol-3-yl)furan-2-yl)-4-oxobutyrate (**62'-3**) was the same as that of compound **4'-1**, and an off-white solid was obtained with a yield of 32%. $^1$H NMR (400 MHz, $CDCl_3$) $\delta$ 8.33 (d, $J$ = 8.6 Hz, 1H), 8.00 (s, 1H), 7.92 (s, 1H), 7.82 (s, 1H), 7.64 (d, $J$ = 1.3, 8.7 Hz, 1H), 7.48 (s, 1H), 3.72 (s, 3H), 3.26 (t, $J$ = 6.7 Hz, 2H), 2.80 (t, $J$ = 6.6 Hz, 2H), 1.70 (s, 9H).

Step 4: 4-4-5-cano-1H-indol-3-yl)furan-2-yl)-4-oxobutric acid (**I'-62**)

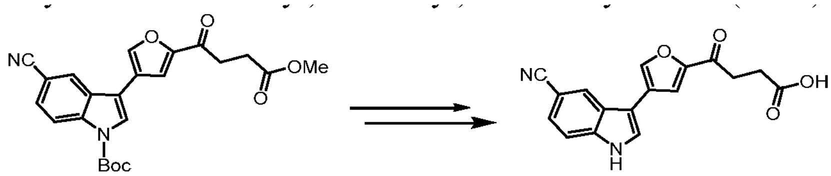

The preparation method of 4-(4-(5-cyano-1H-indol-3-yl)furan-2-yl)-4-oxobutyric acid (**I'-62**) was the same as that of compound **I'-38**, and a yellow solid was obtained with a yield of 75%. $^1$H NMR (400 MHz, DMSO-$d_6$) $\delta$ 12.21 (br s, 1H), 12.04 (s, 1H), 8.62 (s, 1H), 8.47 (s, 1H), 8.04 (s, 1H), 8.01 (s, 1H), 7.48-7.67(m, 2H), 3.14 (t, $J$ = 6.4 Hz, 2H), 2.60 (t, $J$ = 6.4 Hz, 2H).

**Example 77 Preparation of prodrugs isoamyl 4-(4-(7-fluoro-1H-indol-3-yl)thiophen-2-yl)-4-oxobutyrate (63-1)**

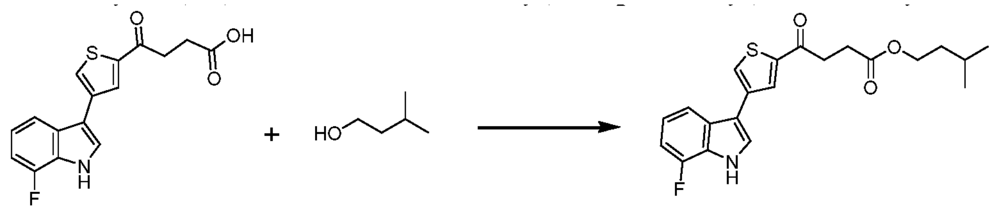

A mixture of compound **I'-49** (100 mg), isoamyl alcohol (33 mg), 4-dimethylaminopyridine (4 mg) and dichloromethane (4 mL) was stirred at room temperature for 10 minutes, and then dicyclohexylcarbodiimide (72 mg) was added and stirred at room temperature for 16 hours. The reaction solution was added with water, extracted with ethyl acetate, and the organic layer was concentrated to dryness. The residue was purified by preparative thin layer chromatography (petroleum ether: ethyl acetate = 5: 3) to obtain 10mg of isoamyl 4-(4-(7-fluoro-1H-indol-3-yl)thiophen-2-yl)-4-oxobutyrate (63-1) as a light brown solid with a yield of 8%. LRMS (ESI) $m/z$ $[M+H]^+$: 388.1.

**4-(4-(7-fluoro-1H-indol-3-yl)thiophen-2-yl)-4-oxobutyric acid (4-methoxy-4-oxo)butyrate (63-2)**

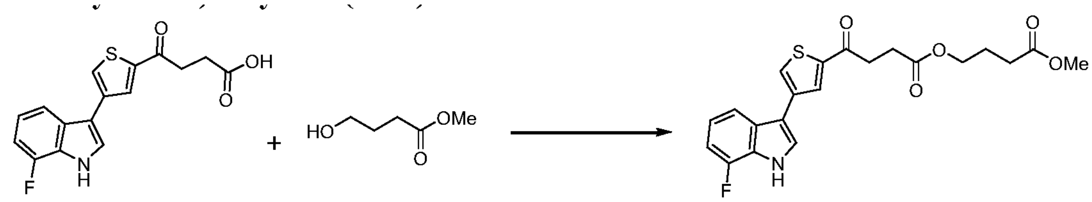

A mixture of compound **I'-49** (200 mg), methyl 4-hydroxybutyrate (149 mg), 4-dimethylaminopyridine (15 mg) and dichloromethane (5 mL) was stirred at room temperature for 10 minutes, and then dicyclohexylcarbodiimide (156 mg) was added and stirred at room temperature for 16 hours. The reaction solution was added with water, extracted with ethyl acetate, and the organic layer was concentrated to dryness. The residue was purified by preparative thin layer chromatography (petroleum ether: ethyl acetate = 5: 3) to obtain 22mg of 4-(4-(7-fluoro-1H-indol-3-yl)thiophen-2-yl)-4-oxobutyric acid (4-methoxy-4-oxo)butyrate (63'-2) as a light brown solid with a yield of 8%. LRMS (ESI) *m/z* [M+H]+: 418.0.

**4-(4-(7-fluoro-1H-indol-3-yl)thiophen-2-yl)-4-hydroxybutyric acid (63-3)**

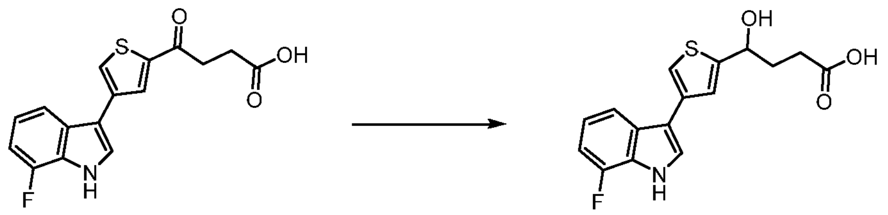

Compound **I'-49** (100 mg), sodium borohydride (12 mg), and methanol (3 mL) were mixed, fully replaced by argon, and stirred at room temperature for 12 hours. The reaction solution was diluted with water, extracted with ethyl acetate, and the organic layer was concentrated to dryness to obtain 72 mg of compound (63-3) with a yield of 72%. LRMS (ESI) *m/z* [M+H]+: 320.3.

**Methyl 4-4-7-fluoro-1H-indol-3-yl)thiophen-2-yl)-4-hyroxybutyrate (63-4)**

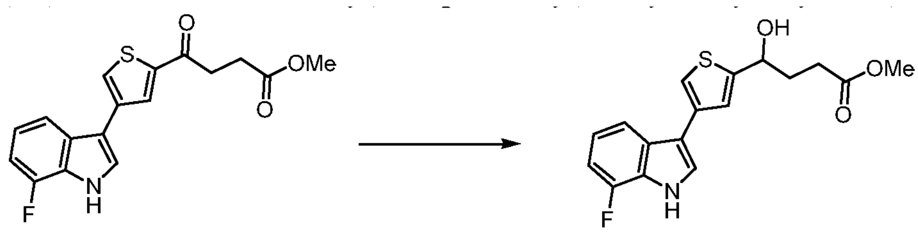

The preparation method of compound methyl 4-(4-(7-fluoro-1H-indol-3-yl)thiophen-2-yl)-4-hydroxybutyrate **(63-4)** was the same as that of compound **63-3**. LRMS (ESI) *m/z* [M+H]+: 333.1.

**(2-methoxy)ethyl 4-(4-(1H-indol-3-yl)furan-2-yl)-4-oxobutyrate (63-5)**

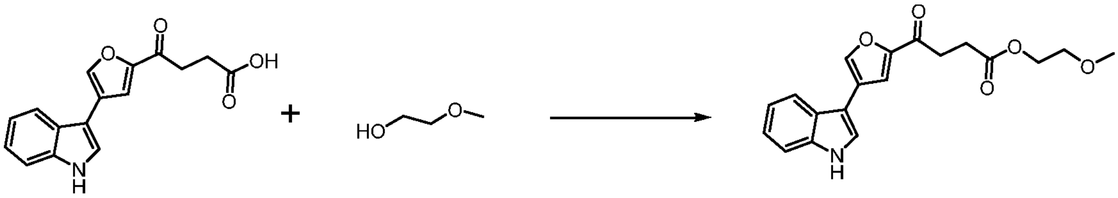

The preparation method of compound (2-methoxy)ethyl 4-(4-(1H-indol-3-yl)furan-2-yl)-4-oxobutyrate (63-5) was the same as that of compound 63-1. LRMS (ESI) *m/z* [M+H]+: 342.0.

**(2,2-dimethyl-1,3-dioxolan-4-yl) methyl 4-(4-(1H-indol-3-yl)furan-2-yl)-4-oxobutyrate (63-6)**

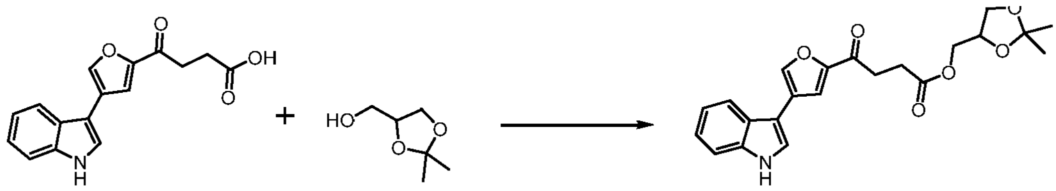

The preparation method of compound (2,2-dimethyl-1,3-dioxolan-4-yl) methyl 4-(4-(1H-indol-3-yl)furan-2-yl)-4-oxobutyrate (63-6) was the same as that of compound **63-1.** LRMS (ESI) *m/z* $[M+H]^+$: 398.0.

**(Tetrahydropyran-4-yl) 4-(4-(1H-indol-3-yl)furan-2-yl)-4-oxobutrate (63-7)**

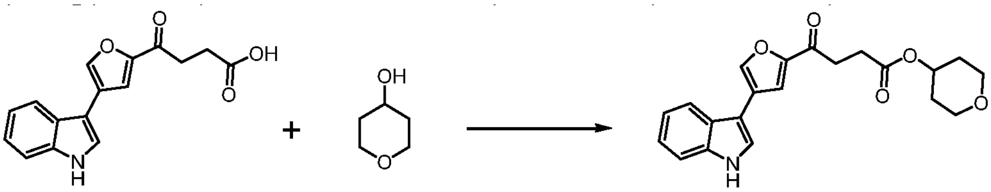

The preparation method of compound (tetrahydropyran-4-ol) 4-(4-(1H-indol-3-yl)furan-2-yl)-4-oxobutyrate (63-7) was the same as that of compound 63-1. LRMS (ESI) *m/z* $[M+H]^+$: 368.0.

**(3-Oxo-1,3-dihydroisobenzofuran-1-yl) 4-(4-(1H-indol-3-yl)furan-2-yl)-4-oxobutyrate (63 -8)**

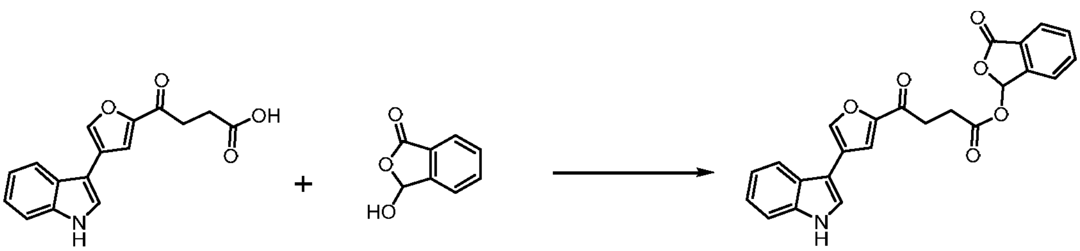

The preparation method of compound (3-oxo-1,3-dihydroisobenzofuran-1-ol) 4-(4-(1H-indol-3-yl)furan-2-yl)-4-oxobutyrate (63 -8) was the same as that of compound **63-1.** LRMS (ESI) *m/z* $[M+H]^+$: 417.0.

**(Pyridin-3-yl) 4-(4-(1H-indol-3-yl)thiophen-2-yl)-4-oxobutyrate (63-9)**

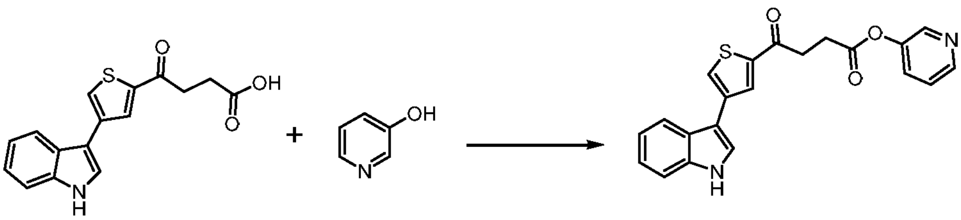

The preparation method of compound (pyridin-3-yl) 4-(4-(1H-indol-3-yl)thiophen-2-yl)-4-oxobutyrate **(63-9)** was the same as that of compound 63-1. LRMS (ESI) *m/z* $[M+H]^+$: 377.0.

**Benzyl 4-(4-(1H-indol-3-yl)thiophen-2-yl)-4-oxobutyrate(63-10)**

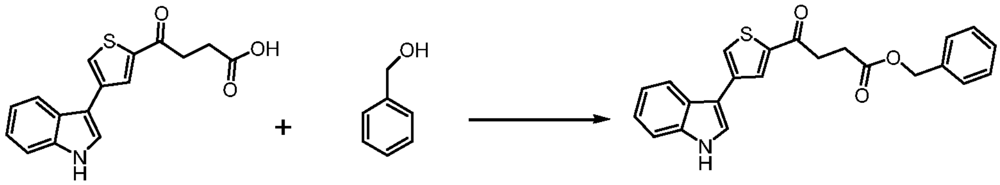

The preparation method of compound benzyl 4-(4-(1H-indol-3-yl)thiophen-2-yl)-4-oxobutyrate was the same as that of compound **63-1.** LRMS (ESI) *m/z* $[M+H]^+$: 390.0.

**Cyclohexyl 4-(4-(1H-indol-3-yl)thiophen-2-yl)-4-oxobutyrate (63-11)**

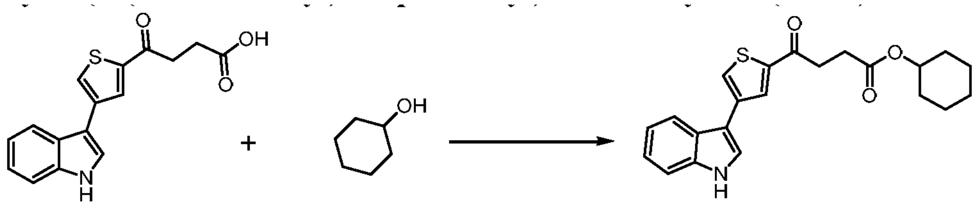

The preparation method of compound cyclohexyl 4-(4-(1H-indol-3-yl)thiophen-2-yl)-4-oxobutyrate **(63-11)** was the same as that of compound **63-1.** LRMS (ESI) *m/z* $[M+H]^+$: 382.0.

**(2-Morpholinyl)ethyl 4-(4-(1H-indol-3-yl)thiophen-2-yl)-4-oxobutyrate (63-12)**

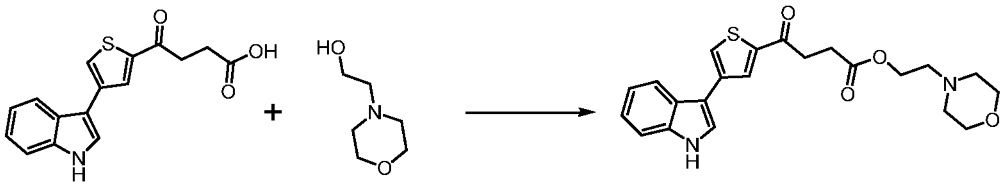

The preparation method of compound (2-morpholinyl)ethyl **4-(4-(1H-indol-3-yl)thiophen-2-yl)-4-oxobutyrate** (63-12) was the same as that of compound **63-1.** LRMS (ESI) *m/z* $[M+H]^+$: 413.0.

**(5,6,7,8- Tetrahydronaphthalen-2-yl) 4-(4-(1H-indol-3-yl)thiophen-2-yl)-4-oxobutyrate (63-13)**

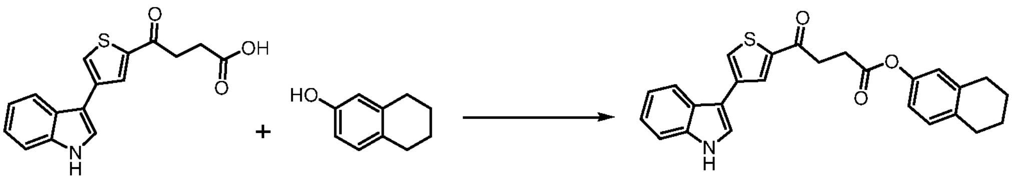

The preparation method of compound (5,6,7,8-tetrahydronaphthalen-2-yl) 4-(4-(1H-indol-3-yl)thiophen-2-yl)-4-oxobutyrate **(63-13)** was the same as that of compound **63-1.** LRMS (ESI) *m/z* $[M+H]^+$: 430.0.

**(2-Dimethylamino)ethyl 4-(4-(1H-indol-3-yl)thiophen-2-yl)-4-oxobutyrate (63-14)**

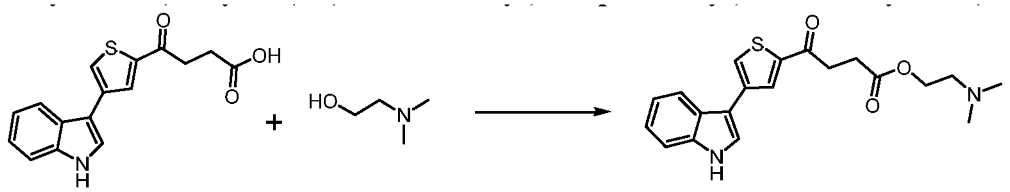

The preparation method of compound (2-dimethylamino)ethyl 4-(4-(1H-indol-3-yl)thiophen-2-yl)-4-oxobutyrate **(63-14)** was the same as that of compound **63-1.** LRMS (ESI) *m/z* $[M+H]^+$: 371.0.

**(5-Methyl-[1,3]dioxol-2-one-4-yl) methyl 4-(4-(1H-indol-3-yl)thiophen-2-yl)-4-oxobutyrate (63-15)**

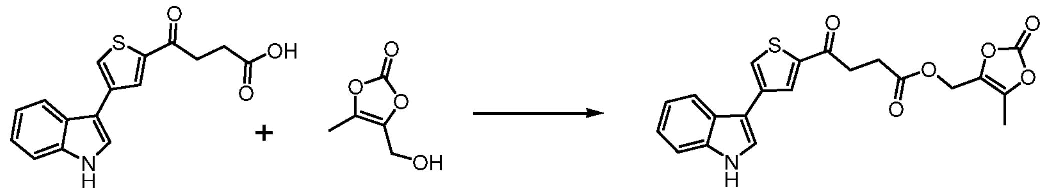

The preparation method of compound (5-methyl-[1,3]dioxol-2-one-4-yl) methyl 4-(4-(1H-indol-3-yl)thiophen-2-yl)-4-oxobutyrate(**63-15**) was the same as that of compound 63-1. LRMS (ESI) *m/z* $[M+H]^+$ : 412.0.

**(1-Acetoxy)ethyl 4-(4-(1H-indol-3-yl)furan-2-yl)-4-oxobutyrate (63-16)**

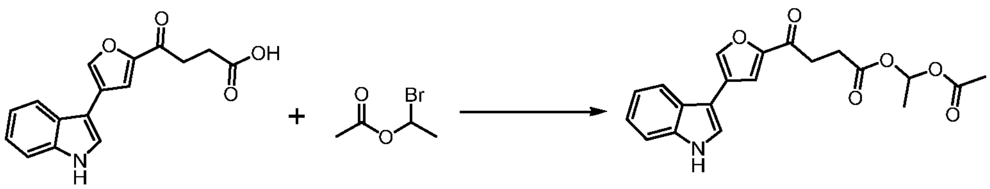

A mixture of compound **I'-30** (10 mg), 1-bromoethyl acetate (11.2 mg), potassium carbonate (5 mg) and dichloromethane (5 mL) was stirred at room temperature for 16 hours. The reaction solution was concentrated to dryness, and the residue was purified by preparative thin layer chromatography to obtain (1-acetoxy)ethyl 4-(4-(1H-indol-3-yl)furan-2-yl)-4-oxobutyrate **(63-16)**. LRMS (ESI) *m/z* $[M+H]^+$: 370.0.

**Example 78** 1-(4-(1H-indol-3-yl)thiophen-2-carbonyl)cyclopropan-1-carboxylic acid (**I-15**)

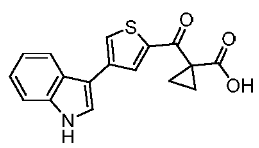

Step 1: methyl 1-(4-bromothiophen-2-carbonyl)cyclopropan-1-carboxylate (**15-1**)

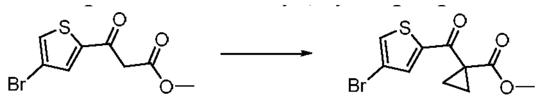

A mixture of compound **1-3** (225 mg), 1,2-dibromoethane (309 mg), potassium carbonate (355 mg) and N,N-dimethylformamide (2 mL) was °Cheated at 50 ° C for 24 hours. The reaction solution was diluted with water, extracted with ethyl acetate, and the organic phase was concentrated to dryness to obtain 112 mg of a colorless oil (**15-1**) with a yield of 45%. LRMS (EI) *m/z* $M^+$: 288.0.

Step 2: methyl 1-(4-(4,4,5,5-tetramethyl-1,3,2-dioxaboran-2-yl)thiophen-2-carbonyl)cyclopropan-1-formate (**15-2**)

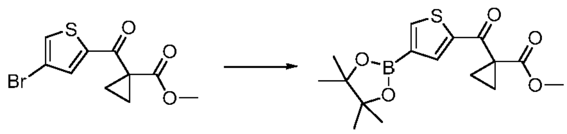

The preparation of methyl 1-(4-(4,4,5,5-tetramethyl-1,3,2-dioxaboran-2-yl)thiophen-2-carbonyl)cyclopropan-1-formate (**15-2**) **was the same as that of compound** 2-1, and a **yellow** oily liquid was obtained with a yield of 70%. LRMS (ESI) *m/z* $[M+H]^+$: 337.2.

Step 3: methyl 1-(4-(1H-indol-1-Boc-3-yl)thiophen-2-carbonyl)cyclopropan-1-formate (**15-3**)

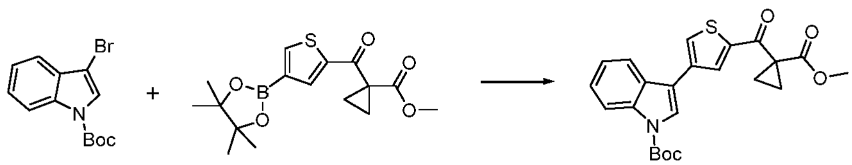

The preparation of methyl 1-(4-(1H-indol-1-Boc-3-yl)thiophen-2-carbonyl)cyclopropan-1-formate (**15-3**) was the same as that of compound **1-4** with a yield of 55%. LRMS (ESI) *m/z* $[M+H]^+$: 426.2.

Step 4: 1-(4-(1H-indol-3-yl)thiophen-2-carbonyl)cyclopropan-1-carboxylic acid (**I-15**)

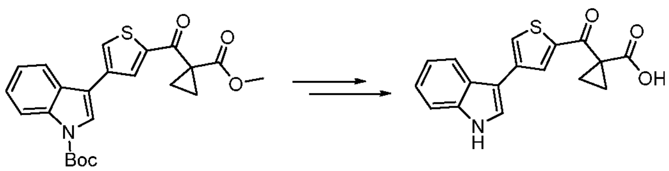

The preparation of 1-(4-(1H-indol-3-yl)thiophen-2-carbonyl)cyclopropan-1-carboxylic acid (**I-15**) was the same as that of compound **I-1** with a yield of 55%. LRMS (ESI) *m/z* $[M+H]^+$: 312.1.

**Example 79** methyl 3-(4-(1H-indol-3-yl)thiophen-2-yl)-3-oxopropionate (**I-16**)

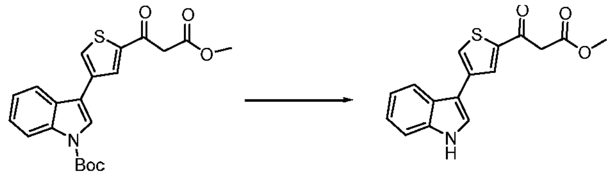

The preparation of methyl 3-(4-(1H-indol-3-yl)thiophen-2-yl)-3-oxopropionate (**I-16**) was the same as that of compound **30'-2**, and a yellow solid was obtained with a yield of 68%. $^1$H NMR (400 MHz, DMSO-$d_6$) $\delta$ 11.44 (s, 1H), 8.43 (d, $J$ = 1.4 Hz, 1H), 8.21 (d, $J$ = 1.4 Hz, 1H), 7.97 (d, $J$ = 7.5 Hz, $^1$H ), 7.85 (d, $J$ = 2.6 Hz, 1H), 7.46 (d, $J$ = 7.8 Hz, 1H),7.10-7.21 (m, 2H), 4.26 (s, 2H).

**Example 80** methyl 3-(4-(6-fluoro-1H-indol-3-yl)thiophen-2-yl)-3-oxopropionate (**I-17**)

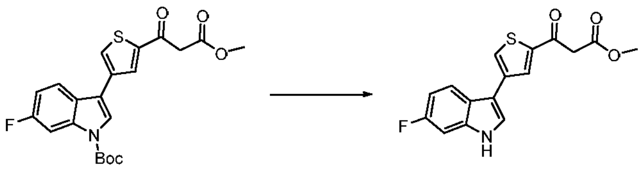

The preparation of methyl 3-(4-(6-fluoro-1H-indol-3-yl)thiophen-2-yl)-3-oxopropionate (**I-17**) was the same as that of compound **30**'-2, and a yellow solid was obtained with a yield of 84%. $^1$H NMR (400 MHz, DMSO-$d_6$) $\delta$ 11.50 (s, 1H), 8.42 (d, $J$ = 1.2 Hz, 1H), 8.22 (d, $J$ = 1.2 Hz, 1H), 7.96 (dd, $J$ = 5.3, 8.7 Hz, 1H), 7.85 (d, $J$ = 2.5 Hz, 1H), 7.24 (dd, $J$ = 2.3, 10.0 Hz, 1H), 6.99 (td, $J$ = 2.4, 9.5 Hz, 1H), 4.26 (s, 2H).

**Example 81** methyl 3-(4-(7-fluoro-1H-indol-3-yl)thiophen-2-yl)-3-oxopropanoate (**I-18**)

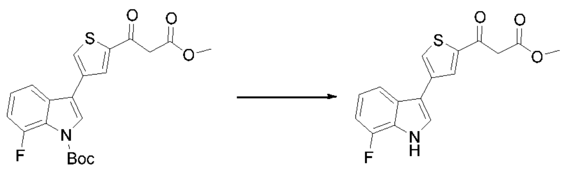

The preparation of methyl 3-(4-(7-fluoro-1H-indol-3-yl)thiophen-2-yl)-3-oxopropanoate (**I-18**) was the same as that of compound **30'-2,** and a yellow solid was obtained with a yield of 37%. $^1$H NMR (400 MHz, DMSO-$d_6$) $\delta$ 11.95(s, 1H), 8.45 (s, 1H), 8.25 (s, 1H), 7.93 (d, $J$ = 2.5 Hz, 1H), 7.79 (d, $J$ = 7.9 Hz, 1H), 6.96-7.16 (m, 2H), 4.26 (s, 2H).

**Example 82** methyl 3-(4-(5-fluoro-1H-indol-3-yl)thiophen-2-yl)-3-oxopropionate (**I-19**)

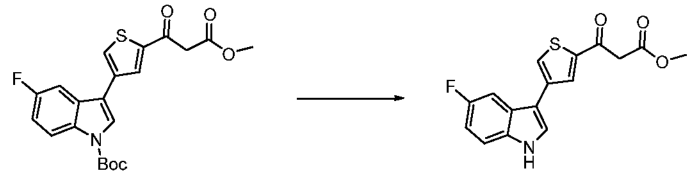

The preparation of methyl 3-(4-(5-fluoro-1H-indol-3-yl)thiophen-2-yl)-3-oxopropionate (I-19) was the same as that of compound **30'**-2, and a yellow solid was obtained with a yield of 78%. $^1H$ NMR (400 MHz, DMSO-$d_6$) $\delta$ 11.55 (s, 1H), 8.42 (s, 1H), 8.23 (s, 1H), 7.94 (d, $J$ = 2.3 Hz, 1H), 7.75 (d, $J$ = 8.6 Hz, 1H), 7.46 (dd, $J$ = 4.7, 8.8 Hz, 1H), 7.46 (td, $J$ = 2.1, 9.3 Hz, 1H), 4.27 (s, 2H).

**Example 83** methyl 3-(4-(4-fluoro-1H-indol-3-yl)thiophen-2-yl)-3-oxopropanoate (**I-20**)

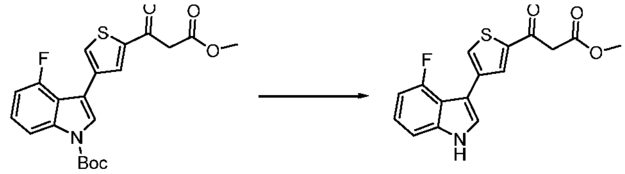

The preparation of methyl 3-(4-(4-fluoro-1H-indol-3-yl)thiophen-2-yl)-3-oxopropanoate (I-20) was the same as that of compound **30'-2**, and a yellow solid was obtained with a yield of 42%. $^1H$ NMR (400 MHz, DMSO-$d_6$) $\delta$ 11.78 (s, 1H), 8.25 (s, 1H), 7.95 (s, 1H), 7.81 (t, $J$ = 4.8 Hz, 1H), 7.29 (d, $J$ = 8.1 Hz, 1H), 7.07-7.19 (m, 1H), 6.86 (dd, $J$ = 7.8, 12.2 Hz, 1H), 4.28 (s, 2H).

**Example 84** methyl 3-(4-(dibenzo[b,d]thiophen-4-yl)thiophen-2-yl)-3-oxopropanoate (**I-21**)

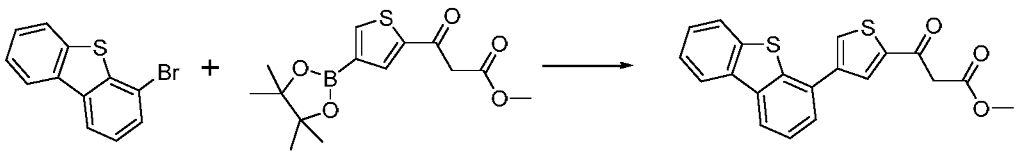

The preparation of methyl 3-(4-(dibenzo[b,d]thiophen-4-yl)thiophen-2-yl)-3-oxopropanoate (**I-21**) was the same as that of compound **1-4**, and a yellow oil was obtained with a yield of 59%. $^1H$ NMR (400 MHz, $CDCl_3$) $\delta$ 8.23-8.18 (m, 2H), 8.17-8.14 (m, 1H), 8.09 (d, $J$ = 1.4 Hz, 1H), 7.92-7.84 (m, 1H), 7.60-7.54 (m, 2H), 7.50 (dd, $J$= 5.6, 3.5 Hz, 2H), 4.02 (s, 2H), 3.80 (s, 3H).

**Example 85** methyl 3-(4-(benzorblthiophen-3-yl)thiophen-2-yl)-3-oxopropanoate (**I-22**)

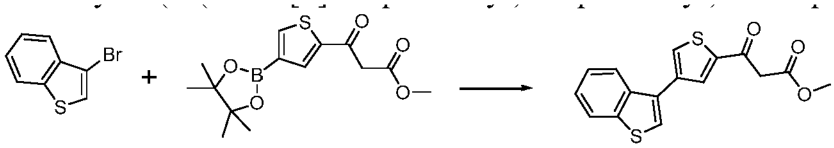

The preparation of methyl 3-(4-(benzo[b]thiophen-3-yl)thiophen-2-yl)-3-oxopropanoate (**I-22**) was the same as that of compound **1-4,** and a yellow oily liquid was obtained with a yield of 41%. $^1H$ NMR (400 MHz, $CDCl_3$) $\delta$ 7.98 (s, 1H), 7.96 - 7.87 (m, 2H), 7.84 (s, 1H), 7.49 (d, $J$ = 2.4 Hz, 1H), 7.44 (d, $J$= 1.9 Hz, 2H), 3.99 (s, 2H), 3.78 (s, 3H).

**Example 86** methyl 3-(4-(naphthalen-1-yl)thiophen-2-yl)-3-oxopropanoate (**I-23**)

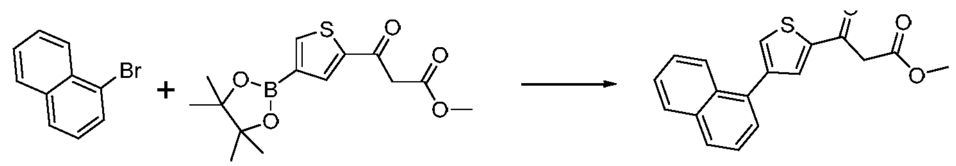

The preparation of methyl 3-(4-(naphthalen-1-yl)thiophen-2-yl)-3-oxopropanoate (**I-23**) was the same as that of compound **1-4, and** a yellow oily liquid was obtained with a yield of 56%. $^1$H NMR (400 MHz, $CDCl_3$) $\delta$ 7.96 - 7.86 (m, 4H), 7.76 (d, $J$ = 1.3 Hz, 1H), 7.58 - 7.44 (m, 4H), 3.98 (s, 2H), 3.78 (s, 3H).

**Example 87** methyl 3-9[3,3'-bithiohen]-5-yl)-3-oxoroanoate (**I-24**)

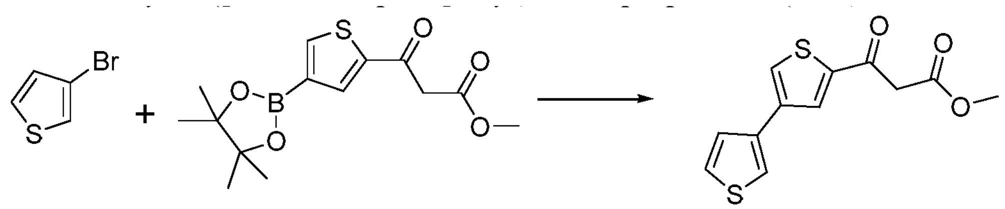

The preparation of methyl 3-([3,3'-bithiophen]-5-yl)-3-oxopropanoate (**I-24**) was the same as that of compound **1-4,** and a yellow oily liquid was obtained with a yield of 45%. $^1$H NMR (400 MHz, $CDCl_3$) $\delta$ 7.92 (d, $J$ = 0.9 Hz, 1H), 7.71 (d, $J$ = 0.8 Hz, 1H), 7.45 - 7.42 (m, 1H), 7.40 (dd, $J$ = 4.7, 2.7 Hz, 1H), 7.31 (d, $J$ = 5.0 Hz, 1H), 3.97 (s, 2H), 3.77 (s, 3H).

**Example 88** methyl (4-phenylthiophen-2-yl)-3-oxopropanoate (**I-25**)

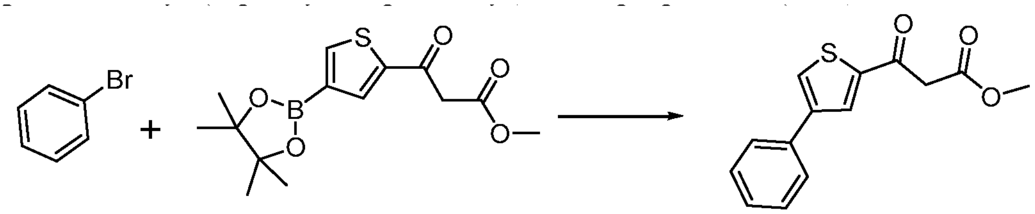

The preparation of methyl (4-phenylthiophen-2-yl)-3-oxopropanoate (1-25) was the same as that of compound **1-4,** and a yellow oily liquid was obtained with a yield of 61%. $^1$H NMR (400 MHz, $CDCl_3$) $\delta$ 7.99 (d, $J$ = 1.4 Hz, 1H), 7.80 (d, $J$ = 1.4 Hz, 1H), 7.60 - 7.55 (m, 2H), 7.44 (t, $J$ = 7.6 Hz, 2H), 7.35 (t, $J$ = 7.3 Hz, 1H), 3.98 (s, 2H), 3.77 (s, 3H).

**Example 89** methyl 3-(4-(benzofuran-3-yl)thiophen-2-yl)-3-oxopropanoate (**I-26**)

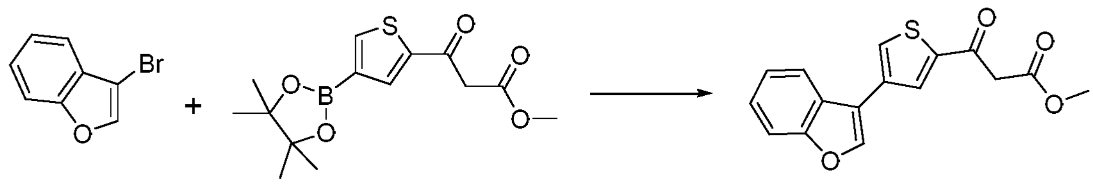

The preparation of methyl 3-(4-(benzofuran-3-yl)thiophen-2-yl)-3-oxopropanoate (**I-26**) was the same as that of compound **1-4, and** a yellow oily liquid was obtained with a yield of 34%. $^1$H NMR (400 MHz, $CDCl_3$) $\delta$ 7.99 (d, $J$ = 1.2 Hz, 1H), 7.87 (d, $J$ = 1.2 Hz, 1H), 7.86 (s, 1H), 7.80 - 7.75 (m, 1H), 7.57 (d, $J$ = 8.1 Hz, 1H), 7.43 - 7.32 (m, 2H), 3.99 (s, 2H), 3.79 (s, 3H).

**Example 90** 3-(4-(benzo[b]thiophen-4-yl)thiophen-2-yl)-3-oxopropionic acid (**I-27**)

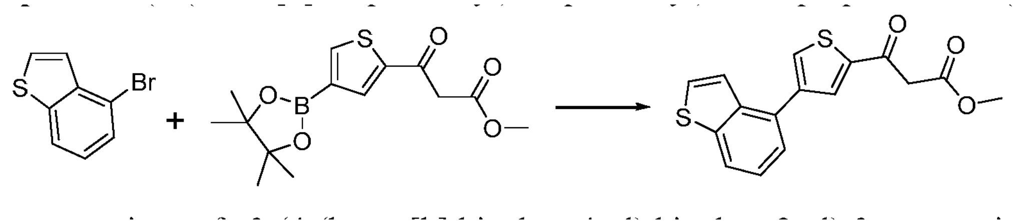

The preparation of 3-(4-(benzol[b]thiophen-4-yl)thiophen-2-yl)-3-oxopropionic acid (**I-27**) was the same as that of compound **1-4, and** a yellow oily liquid was obtained with a yield of 52%. $^1$H NMR (400 MHz, $CDCl_3$) $\delta$7.98 (d, *J* = 1.3 Hz, 1H), 7.91 (dd, *J* = 7.1, 1.3 Hz, 1H), 7.82 (d, *J* = 1.2 Hz, 1H), 7.52 (d, *J* = 5.6 Hz, 1H), 7.47 (d, *J* = 5.5 Hz, 1H), 7.44 - 7.37 (m, 2H), 3.99 (s, 2H), 3.78 (s, 3H).

**Example 91** methyl 3-(4-(benzofuran-7-yl)thiophen-2-yl)-3-oxopropanoate (**I-28**)

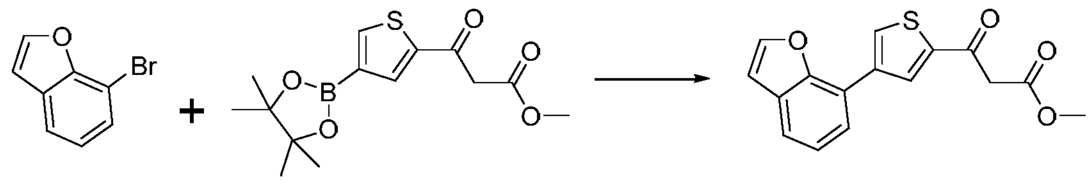

The preparation of methyl 3-(4-(benzofuran-7-yl)thiophen-2-yl)-3-oxopropanoate (**I-28**) was the same as that of compound **1-4, and** a yellow oily liquid was obtained with a yield of 47%. $^1$H NMR (400 MHz, $CDCl_3$) $\delta$8.32 (d, *J* = 1.4 Hz, 1H), 8.30 (d, *J* = 1.4 Hz, 1H), 7.74 (d, *J* = 2.2 Hz, 1H), 7.60 (dd, *J* = 7.7, 1.0 Hz, 1H), 7.55 (dd, *J* = 7.6, 0.8 Hz, 1H), 7.32 (t, *J* = 7.6 Hz, 1H), 6.86 (d, *J* = 2.2 Hz, 1H), 4.03 (s, 2H), 3.78 (s, 3H).

**Example 92** methyl 3-(5-(benzo[b]thiophen-3-yl)thiophen-2-yl)-3-oxopropanoate (**I-29**)

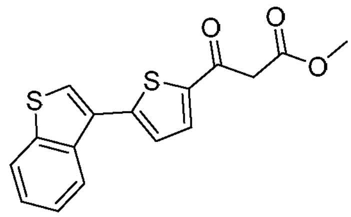

Step 1: 2-acetyl-5-bromothiophene (**29-1**)

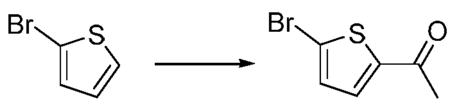

The preparation of 2-acetyl-5-bromothiophene (**29-1**) was the same as that of compound **1-1, and** an orange-yellow crystalline powder was obtained with a yield of 95%. $^1$H NMR (400 MHz, $CDCl_3$) $\delta$7.43 (d, *J*= 7.1 Hz, 1H), 7.10 (d, *J*= 3.8 Hz, 1H), 2.51 (s, 3H).

Step 2: methyl 3-(5-bromothiophen-2-yl)-3-oxopropanoate (29-2)

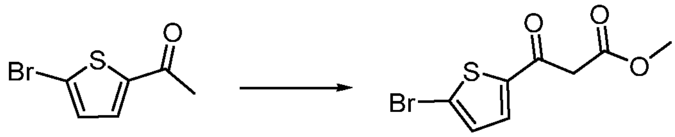

The preparation of methyl 3-(5-bromothiophen-2-yl)-3-oxopropanoate (**29-2**) was the same as that of compound **1-2, and** a brown-black oily liquid was obtained with a yield of 78%. $^{1}$H NMR (400 MHz, $CDCl_3$) $\delta$ 7.48 (d, $J$ = 4.1 Hz, 1H), 7.12 (d, $J$ = 4.1 Hz, 1H), 3.87 (s, 2H), 3.75 (s, 3H).

Step 3: methyl 3-(5-(4,4,5,5-tetramethyl-1,3,2-dioxaboran-2-yl)thiophen-2-yl)-3-oxopropanoate (**29-3**)

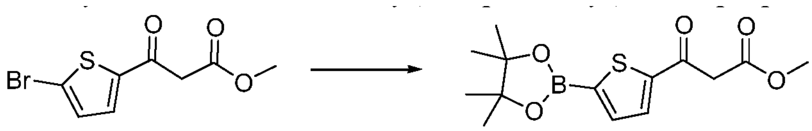

The preparation of methyl 3-(5-(4,4,5,5-tetramethyl-1,3,2-dioxaboran-2-yl)thiophen-2-yl)-3-oxopropanoate (**29-3**) **was the same as that of compound** 2-1, and **a** yellow oily liquid was obtained with a yield of 83%. $^{1}$H NMR (400 MHz, $CDCl_3$) $\delta$ 7.76 (d, $J$ = 3.7 Hz, 1H), 7.59 (d, $J$ = 3.7 Hz, 1H), 3.94 (s, 2H), 3.74 (s, 3H), 1.34 (s, 12H).

Step 4: methyl 3-(5-(benzo[b]thiophen-3-yl)thiophen-2-yl)-3-oxopropanoate (**I-29**)

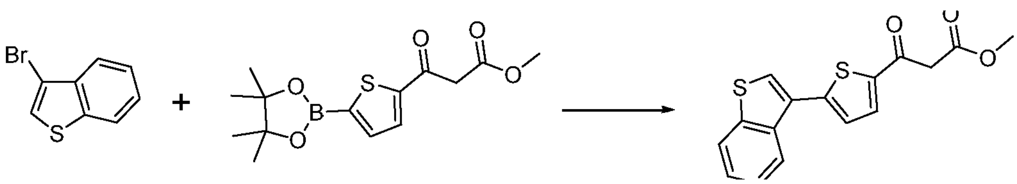

The preparation of methyl 3-(5-(benzo[b]thiophen-3-yl)thiophen-2-yl)-3-oxopropanoate (I-29) was the same as that of compound **1-4,** and a yellow oily liquid was obtained with a yield of 37%. $^{1}$H NMR (400 MHz, $CDCl_3$) $\delta$ 8.20-8.09 (m, 1H), 7.95 - 7.90 (m, 1H), 7.79 (d, $J$ = 4.0 Hz, 1H), 7.68 (s, 1H), 7.52-7.41 (m, 2H), 7.39 (d, $J$ = 4.0 Hz, 1H), 3.97 (s, 2H), 3.79 (s, 3H).

**Example 93** methyl 3-(5-(benzo[b]thiophen-4-yl)thiophen-2-yl)-3-oxopropanoate (**I-30**)

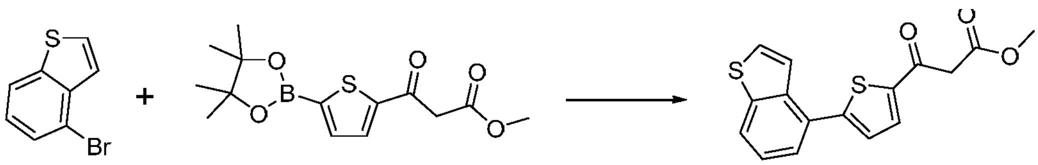

The preparation of methyl 3-(5-(benzo[b]thiophen-4-yl)thiophen-2-yl)-3-oxopropanoate (I-30) was the same as that of compound **1-4,** and a yellow oily liquid was obtained with a yield of 41%. $^{1}$H NMR (400 MHz, $CDCl_3$) $\delta$ 7.94 (d, $J$ = 8.1 Hz, 1H), 7.79 (d, $J$ = 4.0 Hz, 1H), 7.73 (dd, $J$ = 5.6, 0.7 Hz, 1H), 7.57 (d, $J$ = 5.5 Hz, 1H), 7.53 (dd, $J$ = 7.4, 0.9

Hz, 1H), 7.42 (d, *J* = 7.7 Hz, 1H), 7.38 (d, *J*= 4.0 Hz, 1H), 3.98 (s, 2H), 3.79 (s, 3H).

**Example 94** methyl 3-(4-(benzofuran-3-yl)furan-2-yl)-3-oxopropanoate (**I-31**)

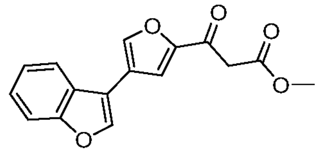

Step 1: methyl 3-(4-bromofuran-2-yl)-3-oxopropanoate (31-1)

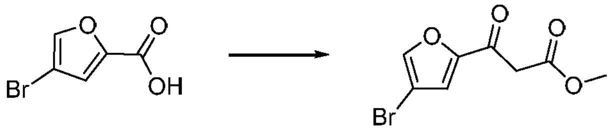

850 mg of 4-bromofuran-2-carboxylic acid was dissolved in 30 mL of anhydrous dichloromethane, 2 drops of N, *N*-dimethylformamide was added, and 565 μL of oxalyl chloride was added dropwise. The mixture was sttirred at room temperature for 1.5 hours after addition. The reaction mixture was spin-dried and dissolved in 10 mL of anhydrous tetrahydrofuran to obtain solution A. 460 μL of methyl acetate dissolved in 5 mL of anhydrous tetrahydrofuran was added dropwise to a solution of 2.9 mL of 2M lithium diisopropylamide in tetrahydrofuran at -78°C and stirred for 2 hours after addition to obtain solution B. The solution A was added dropwise to solution B at -78°C, and the stirring was continued for 2 hours. The reaction mixture was quenched by adding water, and the pH was adjusted to 5~ 6 with 1M hydrochloric acid, and extracted with ethyl acetate. The organic phase was concentrated to dryness, and subjected to column chromatography with 5% ethyl acetate/petroleum ether to obtain 554 mg of white powdery solid (**31-1**) with a yield of 50%. $^1$H NMR (400 MHz, $CDCl_3$) $\delta$ 7.60 (d, *J*= 0.6 Hz, 1H), 7.28 (d, *J*= 0.6 Hz, 1H), 3.84 (s, 2H), 3.75 (s, 3H).

Step 2: methyl 3-(4-(4,4,5,5-tetramethyl-1,3,2-dioxaboran-2-yl)furan-2-yl)-3-oxopropanoate (31-2)

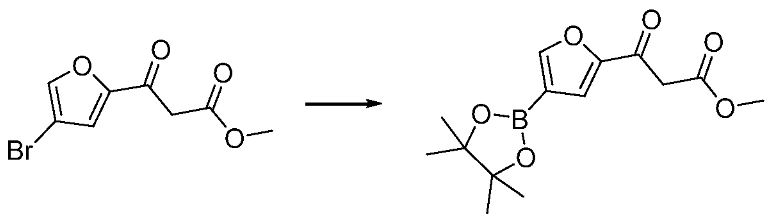

The preparation of methyl 3-(4-(4,4,5,5-tetramethyl-1,3,2-dioxaboran-2-yl)furan-2-yl)-3-oxopropanoate(**31-2**) **was the same as that of compound** 2-1, and a **colorless** transparent oily liquid was obtained with a yield of 50%. $^1$H NMR (400 MHz, $CDCl_3$) $\delta$ 7.90 (s, 1H), 7.45 (s, 1H), 3.84 (s, 2H), 3.74 (s, 3H), 1.32 (s, 12H).

Step 3: methyl 3-(4-(benzofuran-3-yl)furan-2-yl)-3-oxopropanoate (**I-31**)

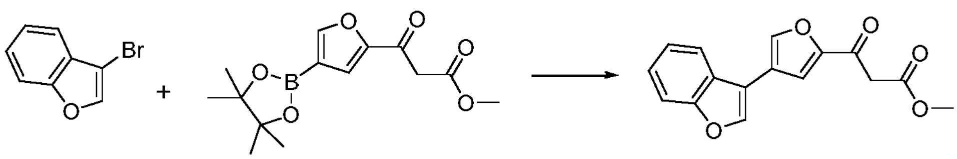

The preparation of methyl 3-(4-(benzofuran-3-yl)furan-2-yl)-3-oxopropanoate (**I-31**) was the same as that of compound **1-4**, and a pale yellow solid was obtained with a yield of 29%. $^1$H NMR (400 MHz, $CDCl_3$) $\delta$ 7.96 (s, 1H), 7.82 (s, 1H), 7.69 (d, *J*= 7.2 Hz, 1H), 7.56 (d, *J* = 8.1 Hz, 2H), 7.43 - 7.31 (m, 2H), 3.93 (s, 2H), 3.78 (s, 3H).

**Example 95** methyl 3-(4-(benzofuran-5-yl)furan-2-yl)-3-oxopropanoate (**I-32**)

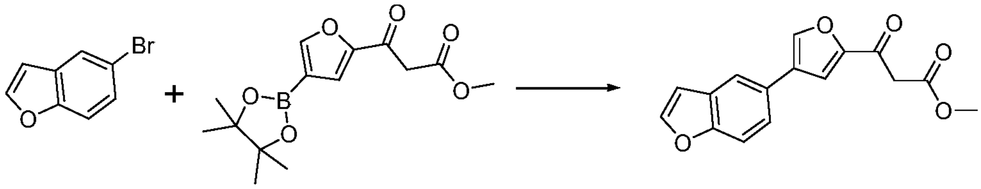

The preparation of methyl 3-(4-(benzofuran-5-yl)furan-2-yl)-3-oxopropanoate (**I-32**) was the same as that of compound **1-4, and** a colorless oily liquid was obtained with a yield of 29%. $^1$H NMR (400 MHz, $CDCl_3$) $\delta$ 7.87 (s, 1H), 7.72 (d, *J* = 1.3 Hz, 1H), 7.66 (d, *J*= 2.1 Hz, 1H), 7.58 (s, 1H), 7.54 (d, *J* = 8.5 Hz, 1H), 7.42 (dd, *J* = 8.5, 1.7 Hz, 1H), 6.80 (d, *J* = 1.3 Hz, 1H), 3.92 (s, 2H), 3.77 (s, 3H).

**Example 96** methyl 3-(4-(benzofuran-2-yl)furan-2-yl)-3-oxopropanoate (**I-33**)

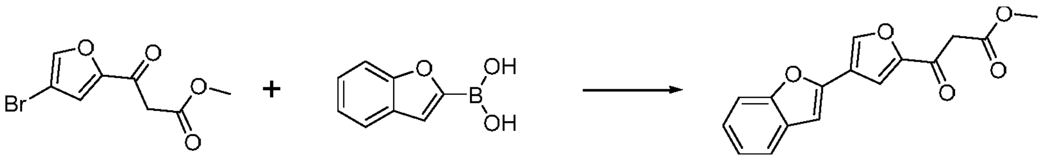

The preparation of methyl 3-(4-(benzofuran-2-yl)furan-2-yl)-3-oxopropanoate (**I-33**) was the same as that of compound **1-4,** and a pale yellow solid was obtained with a yield of 67%. $^1$H NMR (400 MHz, $CDCl_3$) $\delta$ 8.04 (d, *J* = 0.8 Hz, 1H), 7.63-7.53 (m, 2H), 7.53-7.47 (m, 1H), 7.31 (m, *J* = 8.2, 7.2, 1.4 Hz, 1H), 7.24 (dd, *J* = 7.4, 1.1 Hz, 1H), 6.86 (d, *J*= 0.9 Hz, 1H), 3.92 (s, 2H), 3.77 (s, 3H).

**Example 97** methyl 3-(4-(3,4-dimethoxyphenyl)furan-2-yl)-3-oxopropanoate (**I-34**)

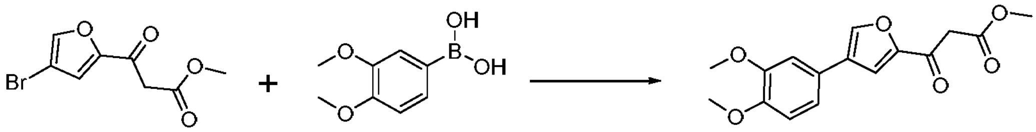

The preparation of methyl 3-(4-(3,4-dimethoxyphenyl)furan-2-yl)-3-oxopropanoate (**I-34**) was the same as that of compound **1-4,** and a yellow solid was obtained with a yield of 76%. $^1$H NMR (400 MHz, $CD_3OD$) $\delta$ 8.16 (d, *J* = 0.8 Hz, 1H), 7.78 (d, *J* = 0.9 Hz, 1H), 7.19 (s, 1H), 7.20 - 7.11 (m, 1H), 6.99 (d, *J*= 8.0 Hz, 1H), 3.97 (s, 2H), 3.89 (s, 3H), 3.85 (s, 3H), 3.74 (s, 3H).

**Example 98** methyl 3-(4-(benzo[d][1,3]dioxolan-5-yl)furan-2-yl)-3-oxopropanoate (**I-35** )

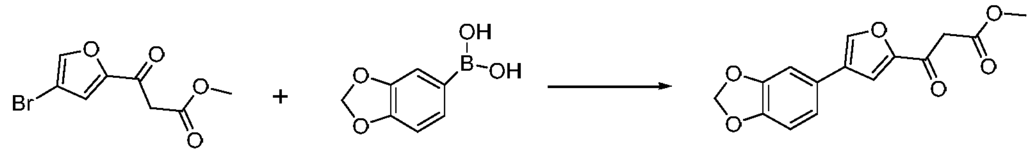

The preparation of methyl 3-(4-(benzo[d][1,3]dioxolan-5-yl)furan-2-yl)-3-oxopropanoate (**I-35** ) was the same as that of compound **1-4,** and a white solid was obtained with a yield of 74%. $^1$H NMR (400 MHz, DMSO-$d_6$) $\delta$ 8.47 (s, 1H), 8.03 (s, 1H), 7.32 (s, 1H), 7.20 (d, *J*= 8.1 Hz, 1H), 6.98 (d, *J*= 8.0 Hz, 1H), 6.05 (s, 2H), 4.01 (s, 2H), 3.66 (s, 3H).

**Example 99** methyl 3-(4-(benzofuran-6-yl)furan-2-yl)-3-oxopropanoate (**I-36**)

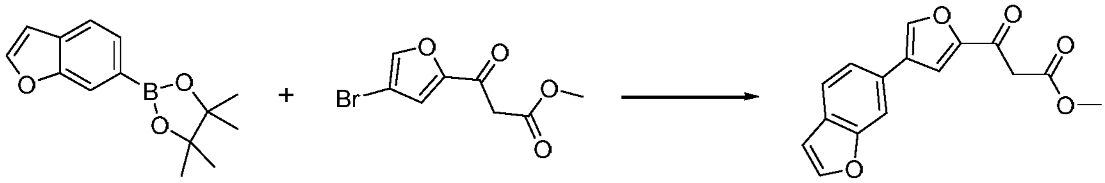

The preparation of methyl 3-(4-(benzofuran-6-yl)furan-2-yl)-3-oxopropanoate (**I-36**) was the same as that of compound **1-4,** and a pale yellow solid was obtained with a yield of 15%. $^1$H NMR (400 MHz, $CDCl_3$) $\delta$ 7.93 (d, *J* = 1.0 Hz, 1H), 7.71 (d, *J* = 2.2 Hz, 1H), 7.67-7.59 (m, 1H), 7.55-7.44 (m, 1H), 7.35-7.31 (m, 2H), 6.91 (dd, *J* = 2.2, 1.1 Hz, 1H), 3.94 (s, 2H), 3.78 (s, 3H).

**Example 100** methyl 3-(4-(6-fluoro-1H-indol-3-yl)furan-2-yl)-3-oxopropanoate (**I-37**)

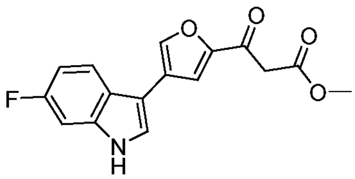

Step 1: methyl 3-(4-(6-fluoro-1H-1-Boc-indol-3-yl)furan-2-yl)-3-oxopropanoate (**37-1**)

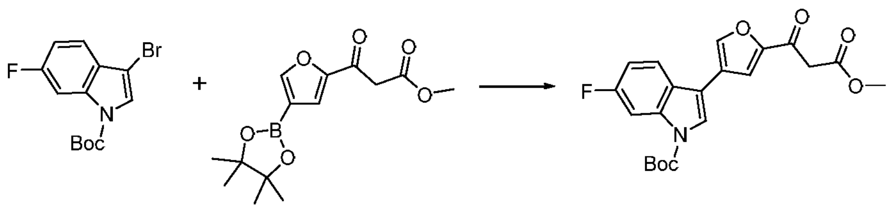

The preparation of methyl 3-(4-(6-fluoro-1H-1-Boc-indol-3-yl)furan-2-yl)-3-oxopropanoate (**37-1**) was the same as that of compound **1-4**, and a brown oil was obtained with a yield of 40%. $^1$H NMR (400 MHz, $CDCl_3$) $\delta$ 7.93 (s, 1H), 7.71 (s, 1H), 7.58 (dd, *J* = 8.7, 5.3 Hz, 1H), 7.54 (d, *J* = 0.9 Hz, 1H), 7.26 (s, 1H), 7.08 (dd, *J*= 8.9, 2.5 Hz, 1H), 3.93 (s, 2H), 3.78 (s, 3H), 1.69 (s, 9H).

Step 2: methyl 3-(4-(6-fluoro-1H-indol-3-yl)furan-2-yl)-3-oxopropanoate (**I-37**)

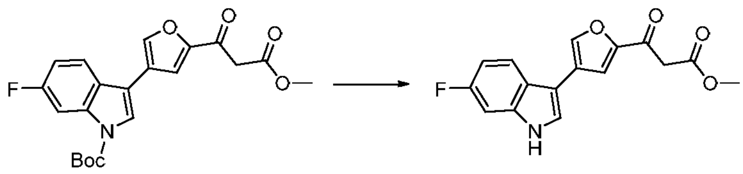

The preparation of methyl 3-(4-(6-fluoro-1H-indol-3-yl)furan-2-yl)-3-oxopropanoate (**I-37**) was the same as that of compound **30**'-2, and a light brown solid was obtained with a yield of 63%. $^1$H NMR (400 MHz, $CDCl_3$) $\delta$ 7.90 (q, *J* = 1.6, 0.8 Hz, 1H), 7.63 (dd, *J* = 8.7, 5.2 Hz, 1H), 7.54 (d, *J* = 0.8 Hz, 1H), 7.43 - 7.31 (m, 1H), 7.12 (dd, *J* = 9.3, 2.3 Hz, 1H), 6.99 (m, *J* = 11.0, 8.9, 2.4 Hz, 1H), 3.93 (d, *J*= 0.8 Hz, 2H), 3.78 (d, *J*= 0.9 Hz, 3H). LRMS (ESI) *m/z* $[M+H]^+$: 302.1.

**Example 101** methyl 3-(4-(7-fluoro-1H-indol-3-yl)furan-2-yl)-3-oxopropanoate (**I-38**)

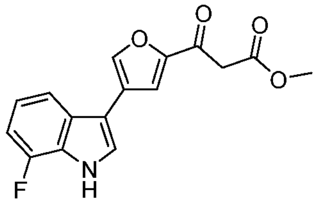

Step 1: methyl 3-(4-(7-fluoro-1H-1-Boc-indol-3-yl)furan-2-yl)-3-oxopropanoate (**38-1**)

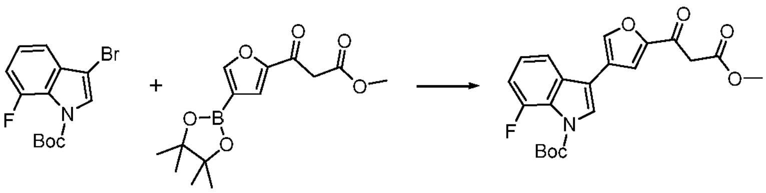

The preparation of methyl 3-(4-(7-fluoro-1H-1-Boc-indol-3-yl)furan-2-yl)-3-oxopropanoate (**38-1**) was the same as that of compound **1-4,** and a pale yellow oil was obtained with a yield of 25%. LRMS (ESI) *m/z* $[M+H]^+$: 402.2.

Step 2: methyl 3-(4-(7-fluoro-1H-indol-3-yl)furan-2-yl)-3-oxopropanoate (**I-38**)

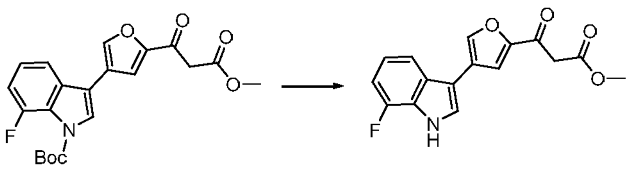

The preparation of methyl 3-(4-(7-fluoro-1H-indol-3-yl)furan-2-yl)-3-oxopropanoate (I-38) was the same as that of compound **30**'-2, and a light brown solid was obtained with a yield of 43%. $^1$H NMR (400 MHz, $CDCl_3$) $\delta$ 7.97 (dd, *J* = 1.6, 0.9 Hz, 1H), 7.55 (t, *J* = 1.0 Hz, 1H), 7.34 (d, *J*= 2.6 Hz, 1H), 7.26 (s, 1H), 7.21-7.16 (m, 1H), 6.85 (m, 1H), 3.92 (s, 2H), 3.77 (s, 3H). LRMS (ESI) *m/z* $[M+H]^+$: 302.1.

**Example 102** methyl 3-(4-(4-fluoro-1H-indol-3-yl)furan-2-yl)-3-oxopropanoate (**I-39**)

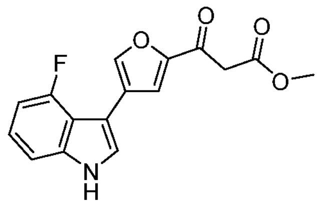

Step 1: methyl 3-(4-(4-fluoro-1H-1-Boc-indol-3-yl)furan-2-yl)-3-oxopropanoate (39-1)

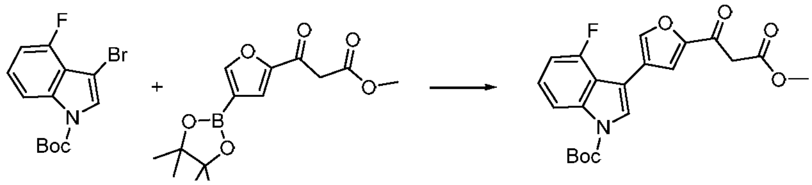

The preparation of methyl 3-(4-(4-fluoro-1H-1-Boc-indol-3-yl)furan-2-yl)-3-oxopropanoate (**39-1**) was the same as that of compound **1-4,** and a brown oil was obtained with a yield of 38%. LRMS (ESI) *m/z* $[M+H]^+$: 402.2.

Step 2: methyl 3-(4-(4-fluoro-1H-indol-3-yl)furan-2-yl)-3-oxopropanoate (**I-39**)

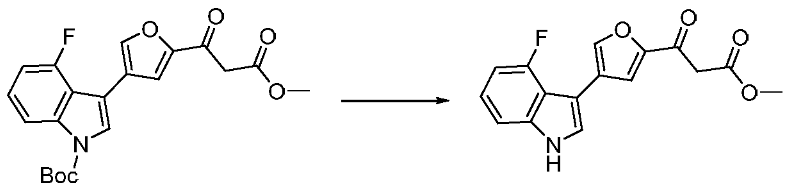

The preparation of methyl 3-(4-(4-fluoro-1H-indol-3-yl)furan-2-yl)-3-oxopropanoate (I-39) was the same as that of compound **30**'-2, and a light brown solid was obtained with a yield of 43%. $^1$H NMR (400 MHz, $CDCl_3$) $\delta$ 7.97 (dd, *J* = 1.6, 0.9 Hz, 1H), 7.55 (t, *J* = 1.0 Hz, 1H), 7.34 (d, *J* = 2.6 Hz, 1H), 7.26 (s, 1H), 7.21-7.16 (m, 1H), 6.85 (m, *J* = 11.7, 7.4, 1.2 Hz, 1H), 3.92 (s, 2H), 3.77 (s, 3H). LRMS (ESI) *m/z* $[M+H]^+$: 302.1.

**Example 103** methyl 3-(4-(5-fluoro-1H-indol-3-yl)furan-2-yl)-3-oxopropanoate (**I-40** )

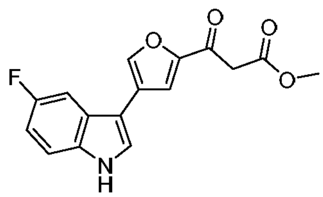

Step 1: methyl 3-(4-(5-fluoro-1H-1-Boc-indol-3-yl)furan-2-yl)-3-oxopropanoate (40-1)

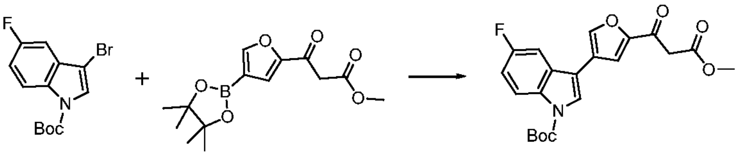

The preparation of methyl 3-(4-(5-fluoro-1H-1-Boc-indol-3-yl)furan-2-yl)-3-oxopropanoate (**40-1**) was the same as that of compound **1-4, and** a white solid was obtained with a yield of 83%. LRMS (ESI) *m/z* $[M+H]^+$: 402.2.

Step 2: methyl 3-(4-(5-fluoro-1H-indol-3-yl)furan-2-yl)-3-oxopropanoate (**I-40**)

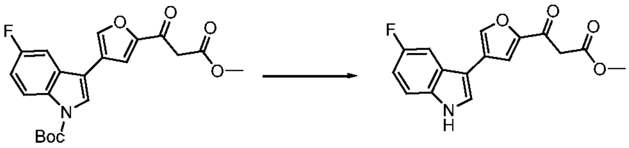

The preparation of methyl 3-(4-(5-fluoro-1H-indol-3-yl)furan-2-yl)-3-oxopropanoate (I-40) was the same as that of compound **30**'-2, and a light brown solid was obtained with a yield of 27%. LRMS (ESI) *m/z* $[M+H]^+$: 302.1.

**Example 104** methyl 3-(4-(benzofuran-4-yl)furan-2-yl)-3-oxopropanoate (**I-41**)

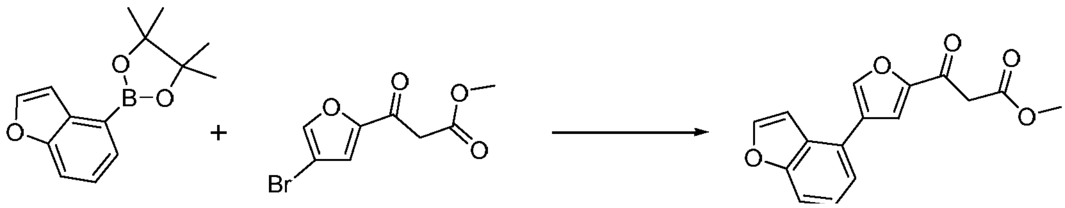

The preparation of methyl 3-(4-(benzofuran-4-yl)furan-2-yl)-3-oxopropanoate (**I-41**) was the same as that of compound **1-4**, and a pale yellow solid was obtained with a yield of 14%. $^1H$ NMR (400 MHz, $CDCl_3$) $\delta$ 7.90 (d, $J$ = 1.0 Hz, 1H), 7.66 (d, $J$ = 2.2 Hz, 1H), 7.65-7.58 (m, 3H), 7.39 (dd, $J$ = 8.1, 1.5 Hz, 1H), 6.79 (dd, $J$ = 2.2, 1.0 Hz, 1H), 3.92 (s, 2H), 3.77 (s, 3H).

**Example 105** methyl 3-(4-(5-methoxy-1H-indol-3-yl)thiophen-2-yl)-3-oxopropanoate (**I-42**)

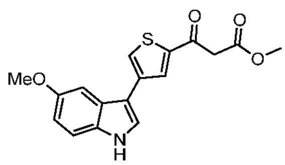

Step 1: methyl 3-(4-(5-methoxy-1H-1-Boc-indol-3-yl)thiophen-2-yl)-3-oxopropanoate (**42-1**)

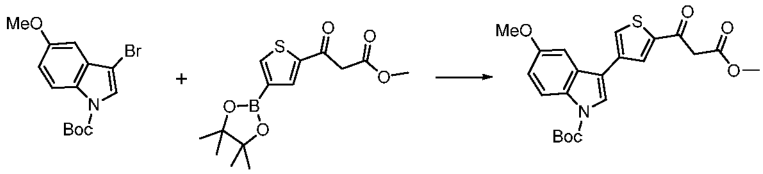

The preparation of methyl 3-(4-(5-methoxy-1H-1-Boc-indol-3-yl)thiophen-2-yl)-3-oxopropanoate (**42-1**) was the same as that of compound **1-4,** and a white solid was obtained with a yield of 75%. LRMS (ESI) *m/z* $[M+H]^+$: 430.2.

Step 2: methyl 3-(4-(5-methoxy-1H-indol-3-yl)thiophen-2-yl)-3-oxopropanoate(**I-42** )

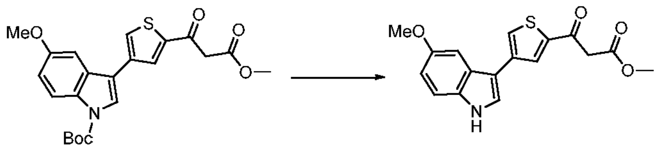

The preparation of methyl 3-(4-(5-methoxy-1H-indol-3-yl)thiophen-2-yl)-3-oxopropanoate (**I-42** ) was the same as that of compound **30'-2**, and a light brown solid was obtained with a yield of 60%. $^1H$ NMR (400 MHz, $CDCl_3$) $\delta$ 8.24 (br s, 1H), 8.03 (d, *J* = 1.2 Hz, 1H), 7.78 (d, *J* = 1.2 Hz, 1H), 7.41 (d, *J* = 2.4 Hz, 1H), 7.38 (d, *J* = 8.8 Hz, 1H), 7.30 (d, *J* = 2.4 Hz, 1H), 6.98 (dd, *J* = 2.4, 8.8 Hz, 1H), 4.02 (s, 2H), 3.92 (s, 3H), 3.80 (s, 3H).

**Example 106** methyl 3-(4-(5-methoxy-1H-indol-3-yl)furan-2-yl)-3-oxopropanoate (**I-43**)

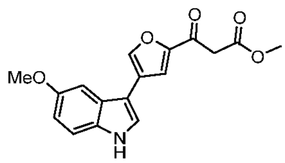

Step 1: methyl 3-(4-(5-methoxy-1H-1-Boc-indol-3-yl)furan-2-yl)-3-oxopropanoate (**43-1**)

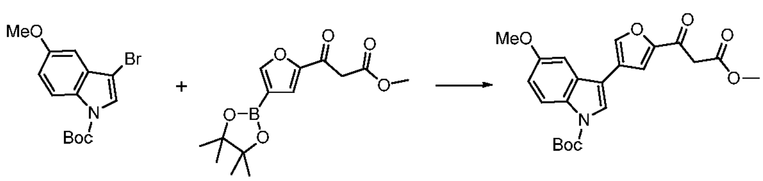

The preparation of methyl 3-(4-(5-methoxy-1H-1-Boc-indol-3-yl)furan-2-yl)-3-oxopropanoate (**43-1**) was the same as that of compound **1-4**, and a white solid was obtained with a yield of 70%. LRMS (ESI) *m/z* $[M+H]^+$: 413.1.

Step 2: methyl 3-(4-(5-methoxy-1H-indol-3-yl)furan-2-yl)-3-oxopropanoate (**I-43**)

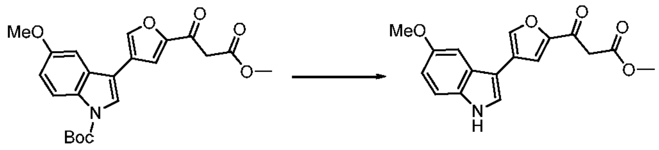

The preparation of methyl 3-(4-(5-methoxy-1H-indol-3-yl)furan-2-yl)-3-oxopropanoate (**I-43**) was the same as that of compound 30'-2, and a light brown solid was obtained with a yield of 76%. LRMS (ESI) *m/z* $[M+H]^+$: 314.1.

**Example 107** 5-(4-(4-fluoro-1H-indol-3-yl)thiophen-2-yl)-5-oxopentanoic acid (**I-44** )

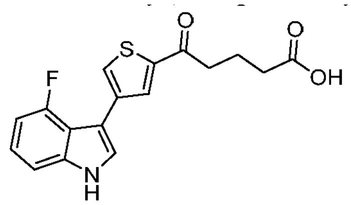

Step 1: methyl 5-(4-(4,4,5,5-tetramethyl-1,3,2-dioxaboran-2-yl)thiophen-2-yl)-5-oxopropanoate (**44-1**)

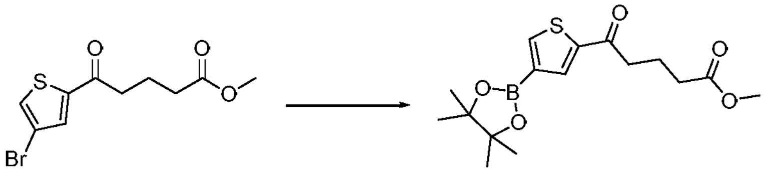

The preparation of methyl 5-(4-(4,4,5,5-tetramethyl-1,3,2-dioxaboran-2-yl)thiophen-2-yl)-5-oxopropanoate (**44-1**) was the same as that of compound 2-1, and a colorless transparent oily liquid was obtained with a yield of 86%. LRMS (ESI) *m/z* $[M+H]^+$:339.3.

Step 2: methyl 5-(4-(4-fluoro-1H-1-Boc-indol-3-yl)thiophen-2-yl)-5-oxopentanoate (**44-2**)

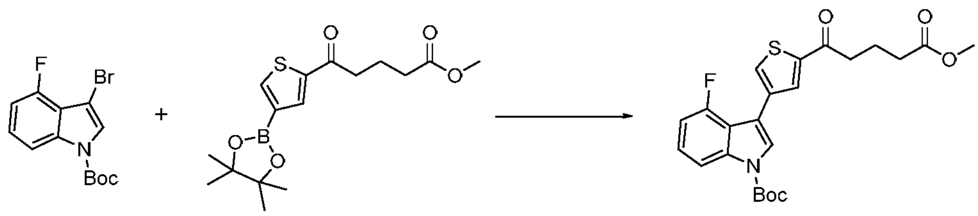

The preparation of methyl 5-(4-(4-fluoro-1H-1-Boc-indol-3-yl)thiophen-2-yl)-5-oxopentanoate (**44-2**) was the same as that of compound 1-4, and a light brown solid was obtained with a yield of 43%. LRMS (ESI) *m/z* $[M+H]^+$: 446.2.

Step 3: 5-(4-(4-fluoro-1H-indol-3-yl)thiophen-2-yl)-5-oxopentanoic acid (**I-44**)

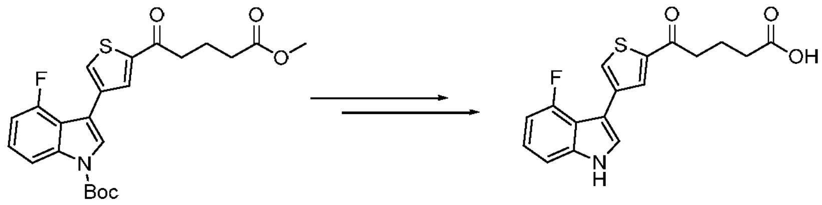

The preparation of 5-(4-(4-fluoro-1H-indol-3-yl)thiophen-2-yl)-5-oxopentanoic acid (**I-44**) was the same as that of compound **I-1,** and a brown solid was obtained as a yield of 68%. $^1$H NMR (400 MHz, DMSO-$d_6$) $\delta$ 12.11 (s, 1H), 11.72 (s, 1H), 8.28-8.18 (m, 1H), 7.97-7.89 (m, 1H), 7.81 (d, $J$ = 2.5 Hz, 1H), 7.28 (d, $J$ = 8.1 Hz, 1H), 7.13 (td, $J$ = 7.9, 5.0 Hz, 1H), 6.85 (dd, $J$ = 12.2, 7.8 Hz, 1H), 3.05 (t, $J$ = 7.3 Hz, 2H), 2.32 (t, $J$ = 7.4 Hz, 2H), 1.87 (p, $J$ = 7.5 Hz, 2H). LRMS (ESI) $m/z$ $[M+H]^+$: 332.1.

**Example 108** 5-(4-(5-fluoro-1H-indol-3-yl)thiophen-2-yl)-5-oxopentanoic acid (**I-45**)

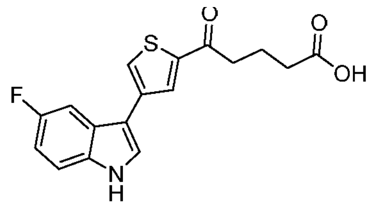

Step 1: methyl 5-(4-(5-flluoro-1H-1-Boc-indol-3-yl)thiophen-2-yl)-5-oxopentanoate (**45-1**)

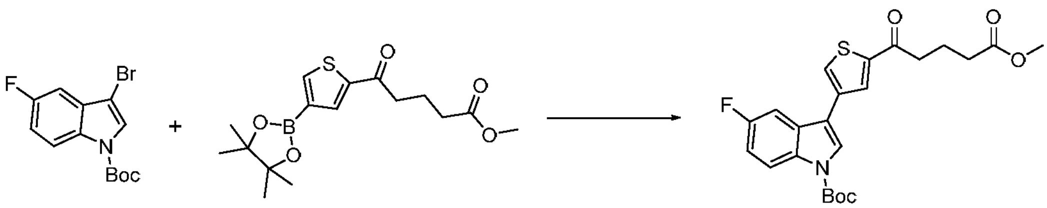

The preparation of methyl 5-(4-(5-fluoro-1H-1-Boc-indol-3-yl)thiophen-2-yl)-5-oxopentanoate (**45-1**) was same as that of compound **1-4**, and a light brown solid was obtained with a yield of 61%.$^1$H NMR (400 MHz, DMSO-$d_6$) $\delta$8.16 (s, 1H), 7.94 (d, $J$ = 1.4 Hz, 1H), 7.78 (s, 1H), 7.73 (d, $J$ = 1.4 Hz, 1H), 7.42 (dd, $J$ = 9.0, 2.5 Hz, 1H), 7.15-7.09 (m, 1H), 3.69 (s, 3H), 3.05 (t, $J$ = 7.2 Hz, 2H), 2.47 (t, $J$ = 7.2 Hz, 2H), 1.94 (p, $J$ = 7.3 Hz, 2H), 1.69 (s, 9H).

Step 2: 5-(4-(5-fluoro-1H-indol-3-yl)thiophen-2-yl)-5-oxopentanoic acid (**I-45**)

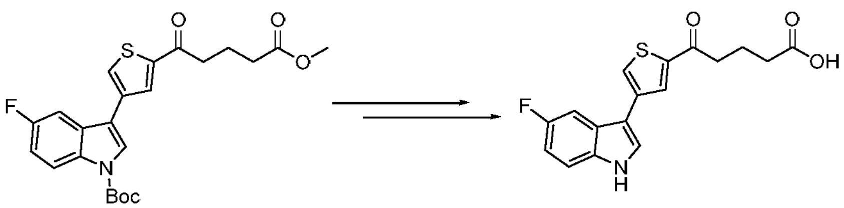

The preparation of 5-(4-(5-fluoro-1H-indol-3-yl)thiophen-2-yl)-5-oxopentanoic acid (**I-45**) was the same as that of compound **I-1,** and a light brown solid was obtained with a yield of 4%. $^1$H NMR (400 MHz, DMSO-$d_6$) $\delta$ 12.17 (s, 1H), 11.55 (s, 1H), 8.34 (d, $J$ = 1.5 Hz, 1H), 8.14 (d, $J$ = 1.5 Hz, 1H), 7.97 (d, $J$ = 2.6 Hz, 1H), 7.72 (dd, $J$ = 10.4, 2.5 Hz,

1H), 7.44 (dd, *J* = 8.9, 4.7 Hz, 1H), 7.02 (td, *J* = 9.1, 2.5 Hz, 1H), 3.09 (t, *J* = 7.3 Hz, 2H), 2.32 (t, *J*= 7.3 Hz, 2H), 1.86 (p, *J*= 7.4 Hz, 2H). LRMS (ESI) *m*/*z* $[M+H]^+$: 332.1.

**Example 109** 5-(4-(6-fluoro-1H-indol-3-yl)thiophen-2-yl)-5-oxopentanoic acid (**I-46**)

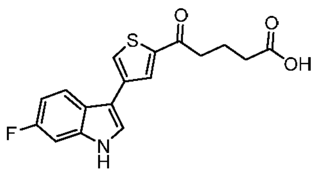

Step 1: methyl 5-(4-(6-fluoro-1H-1-Boc-indol-3-yl)thiophen-2-yl)-5-oxopentanoate (**46-1**)

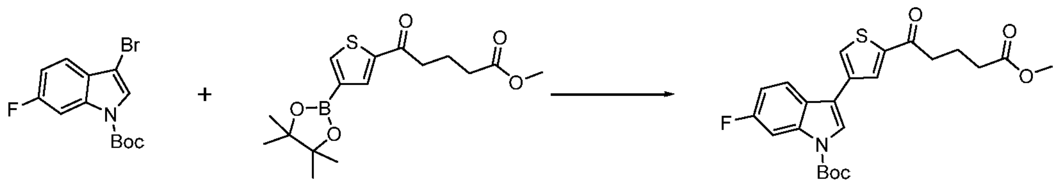

The preparation of methyl 5-(4-(6-fluoro-1H-1-Boc-indol-3-yl)thiophen-2-yl)-5-oxopentanoate (**46-1**) was same as that of compound **1-4**, and a light brown solid was obtained with a yield of 61%. LRMS (ESI) *m*/*z* $[M+H]^+$: 446.2.

Step 2: 5-(4-(6-fluoro-1H-indol-3-yl)thiophen-2-yl)-5-oxopentanoic acid (**I-46**)

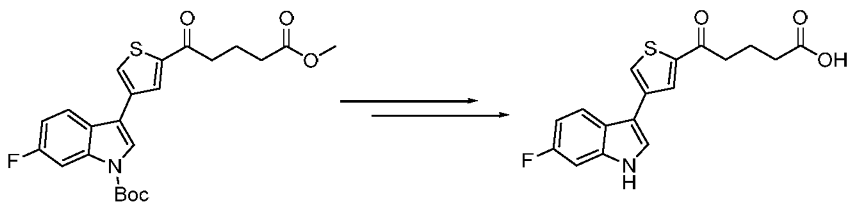

The preparation of 5-(4-(6-fluoro-1H-indol-3-yl)thiophen-2-yl)-5-oxopentanoic acid (**I-46**) was the same as that of compound **I-1**, and a light brown solid was obtained with a yield of 33%. $^1H$ NMR (400 MHz, DMSO-$d_6$) $\delta$ 11.52 (s, 1H), 8.35 (d, *J* = 1.4 Hz, 1H), 8.13 (d, *J* = 1.4 Hz, 1H), 7.95 (dd, *J* = 8.8, 5.2 Hz, 1H), 7.89 (d, *J* = 2.6 Hz, 1H), 7.23 (dd, *J* = 10.0, 2.4 Hz, 1H), 6.98 (td, *J* = 9.2, 2.3 Hz, 1H), 3.07 (t, *J* = 7.4 Hz, 2H), 2.26 (t, *J* = 7.3 Hz, 2H), 1.86-1.83 (m, 1H). LRMS (ESI) *m*/*z* $[M+H]^+$: 332.1.

**Example 110** 5-(4-(7-fluoro-1H-indol-3-yl)furan-2-yl)-5-oxopentanoic acid (**I-47**)

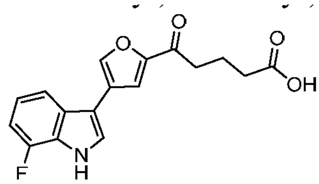

Step 1: Methyl 5-(4,5-dibromofuran-2-yl)-5-oxopentanoate (**47-1**)

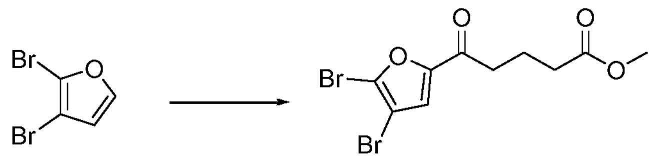

The preparation of methyl 5-(4,5-dibromofuran-2-yl)-5-oxopentanoate (**47-1**) was the same as that of compound **1-1** with a yield of 46%. $^1$H NMR (400 MHz, $CDCl_3$) $\delta$ 7.34 (s, 1H), 3.75 (s, 3H), 2.94 (t, *J* = 7.2 Hz, 2H), 2.54-2.47 (m, 2H), 2.10 (p, *J*= 7.3 Hz, 2H).

Step 2: methyl 5-(4-bromofuran-2-yl)-5-oxopentanoate (**47-2**)

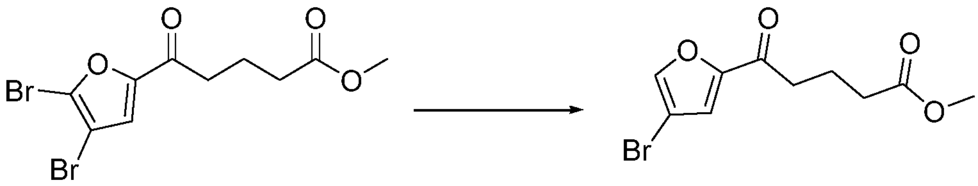

The preparation of methyl 5-(4-bromofuran-2-yl)-5-oxopentanoate (**47-2**) was the same as that of compound **1-3** with a yield of 100%. $^1$H NMR (400 MHz, $CDCl_3$) $\delta$ 7.56 (d, *J* = 0.9 Hz, 1H), 7.19 (d, *J* = 0.9 Hz, 1H), 3.67 (s, 3H), 2.88 (t, *J* = 7.2 Hz, 2H), 2.42 (d, *J* = 7.2 Hz, 2H), 2.03 (p, *J* = 7.2 Hz, 2H).

Step 3: methyl 5-(4-(4,4,5,5-tetramethyl-1,3,2-dioxaboran-2-yl)furan-2-yl)-5-oxopentanoate (**47-3**)

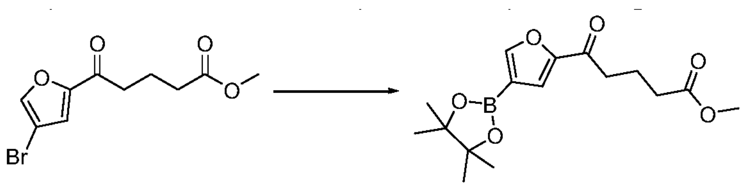

The preparation of methyl 5-(4-(4,4,5,5-tetramethyl-1,3,2-dioxaboran-2-yl)furan-2-yl)-5-oxopentanoate (**47-3**) was the same as that of compound **2-1,** and **a** yellow oily liquid was obtained with a yield of 23%. LRMS (ESI) *m/z* $[M+H]^+$: 323.2.

Step 4: methyl 5-(4-(7-fluoro-1H-1-Boc-indol-3-yl)furan-2-yl)-5-oxopentanoate (**47-4**)

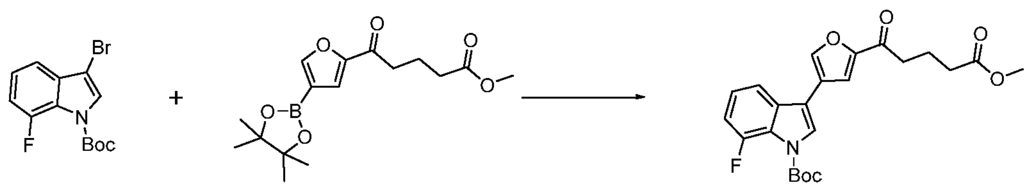

The preparation of methyl 5-(4-(7-fluoro-1H-1-Boc-indol-3-yl)furan-2-yl)-5-oxopentanoate (**47-4**) was same as that of compound **1-4**, and a light brown solid was obtained with a yield of 31%. $^1$H NMR (400 MHz, DMSO-$d_6$) $\delta$ 7.88 (d, *J*= 0.9 Hz, 1H), 7.76 (s, 1H), 7.50-7.40 (m, 2H), 7.29-7.22 (m, 1H), 7.10 (ddd, *J*= 12.5, 8.0, 1.0 Hz, 1H), 3.69 (s, 3H),

2.97 (t, $J$= 7.3 Hz, 2H), 2.46 (t, $J$ = 7.1 Hz, 2H), 2.16-2.06 (m, 2H).

Step 2: 5-(4-(7-fluoro-1H-indol-3-yl)furan-2-yl)-5-oxopentanoic acid (**I-47**)

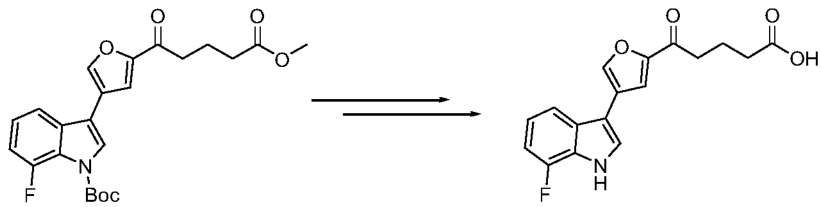

The preparation of 5-(4-(7-fluoro-1H-indol-3-yl)furan-2-yl)-5-oxopentanoic acid (**I-47**) was the same as that of compound **I-1**, and a light brown solid was obtained with a yield of 30%. $^1$H NMR (400 MHz, $CD_3OD$) δ 7.34 (s, 1H), 6.91 (s, 1H), 6.79 (s, 1H), 6.75 (d, $J$= 8.0 Hz, 1H), 6.26 (td, $J$ = 8.0, 4.7 Hz, 1H), 6.10 (dd, $J$ = 11.4, 7.8 Hz, 1H), 2.19 (t, $J$ = 7.3 Hz, 2H), 1.61 (t, $J$ = 7.3 Hz, 2H), 1.21 (p, $J$= 7.3 Hz, 2H). LRMS (ESI) $m/z$ $[M+H]^+$: 316.1.

**Example 111** 5-(4-(4-fluoro-1H-indol-3-yl)furan-2-yl)-5-oxopentanoic acid (**I-48**)

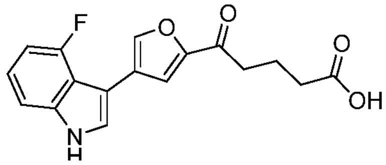

Step 1: methyl 5-(4-(4-fluoro-1H-1-Boc-indol-3-yl)furan-2-yl)-5-oxopentanoate (**48-1**)

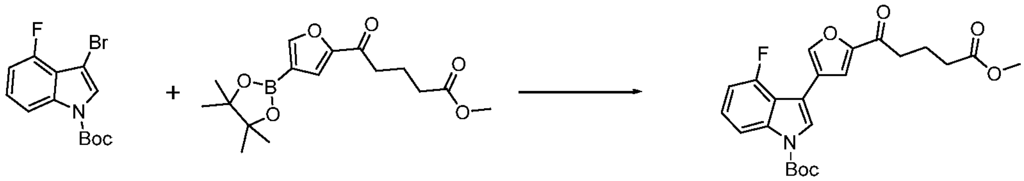

The preparation of methyl 5-(4-(4-fluoro-1H-1-Boc-indol-3-yl)furan-2-yl)-5-oxopentanoate (**48-1**) was the same as that of compound **1-4,** and a light brown solid was obtained with a yield of 34%.LRMS (ESI) $m/z$ $[M+H]^+$: 430.2.

Step 2: 5-(4-(4-fluoro-1H-indol-3-yl)furan-2-yl)-5-oxopentanoic acid (**I-48**)

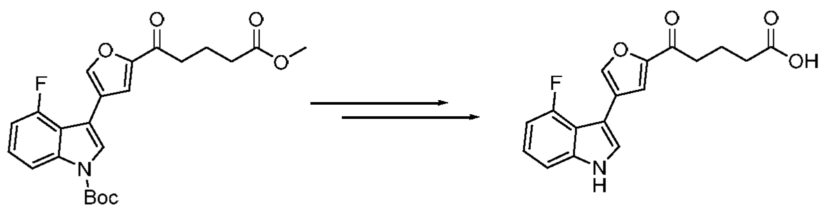

The preparation of 5-(4-(4-fluoro-1H-indol-3-yl)furan-2-yl)-5-oxopentanoic acid (**I-48**) was the same as that of compound **I-1,** and a light brown solid was obtained with a yield of 45%. LRMS (ESI) $m/z$ $[M+H]^+$: 316.1.

**Example 112** 5-(4-(5-fluoro-1H-indol-3-yl)furan-2-yl)-5-oxopentanoic acid (**I-49**)

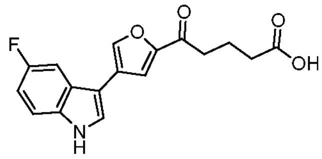

Step 1: methyl 5-(4-(5-fluoro-1H-1-Boc-indol-3-yl)furan-2-yl)-5-oxopentanoate (**49-1**)

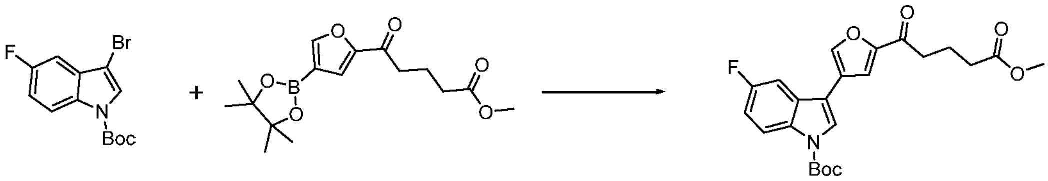

The preparation of methyl 5-(4-(5-fluoro-1H-1-Boc-indol-3-yl)furan-2-yl)-5-oxopentanoate (**49-1**) was the same as that of compound **1-4**, and a light brown solid was obtained with a yield of 45%. LRMS (ESI) *m/z* $[M+H]^+$: 430.2.

Step 2: 5-(4-(5-fluoro-1H-indol-3-yl)furan-2-yl)-5-oxopentanoic acid (**I-49**)

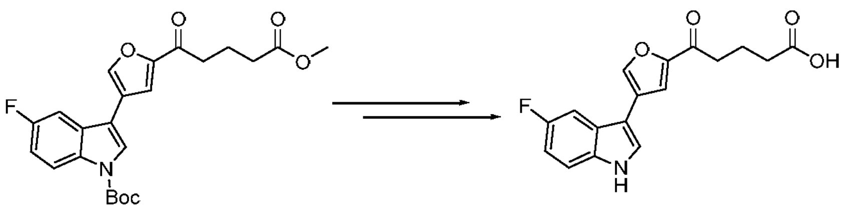

The preparation of 5-(4-(5-fluoro-1H-indol-3-yl)furan-2-yl)-5-oxopentanoic acid (**I-49**) was the same as that of compound **I-1,** and a light brown solid was obtained with a yield of 95%. LRMS (ESI) *m/z* $[M+H]^+$: 316.1.

**Example 113** 5-(4-(6-fluoro-1H-indol-3-yl)furan-2-yl)-5-oxopentanoic acid (**I-50**)

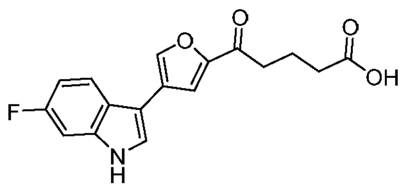

Step 1: methyl 5-(4-(6-fluoro-1H-1-Boc-indol-3-yl)furan-2-yl)-5-oxopentanoate (**50-1**)

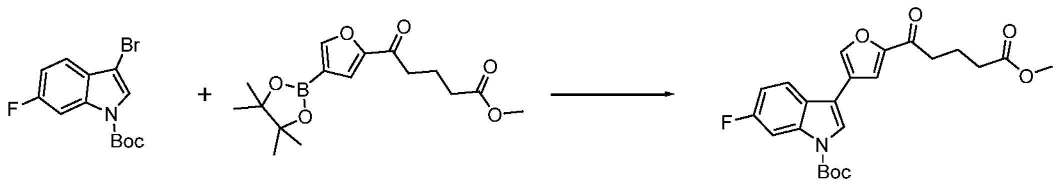

The preparation of methyl 5-(4-(6-fluoro-1H-1-Boc-indol-3-yl)furan-2-yl)-5-oxopentanoate (**50-1**) was the same as that of compound **1-4,** and a light brown solid was obtained with a yield of 45%. LRMS (ESI) *m*/*z* $[M+H]^+$: 430.2.

Step 2: 5-(4-(6-fluoro-1H-indol-3-yl)furan-2-yl)-5-oxopentanoic acid (**I-50**)

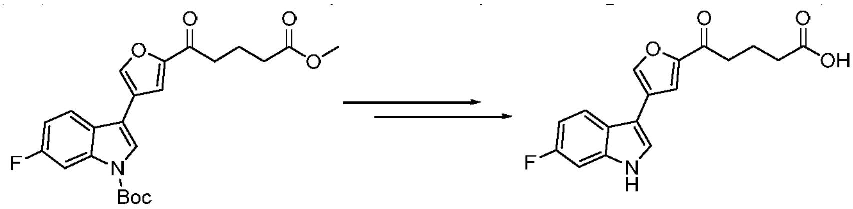

The preparation of 5-(4-(6-fluoro-1H-indol-3-yl)furan-2-yl)-5-oxopentanoic acid (**I-50**) was the same as that of compound **I-1**, and a light brown solid was obtained with a yield of 90%. LRMS (ESI) *m*/*z* $[M+H]^+$: 316.1.

**Example 114** 6-(4-(1H-indol-3-yl)thiophen-2-yl)-6-oxohexanoic acid (**I-51**)

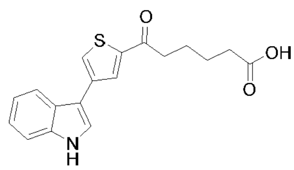

Step 1: methyl 6-(4,5-dibromothiophen-2-yl)-6-oxocaproate (51-1)

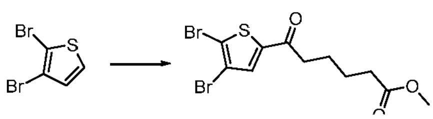

The preparation of methyl 6-(4,5-dibromothiophen-2-yl)-6-oxocaproate **(51-1)** was the same as that of compound **1-1** with a yield of 37%. $^1$H NMR (400 MHz, $CDCl_3$) $\delta$ 7.48 (s, 1H), 3.67 (s, 3H), 2.84 (t, *J* = 6.9 Hz, 2H), 2.36 (t, *J* = 7.0 Hz, 2H), 1.82-1.65 (m, 4H).

Step 2: methyl 6-4-bromothiohen-2-yl)-6-oxocaroate (**51-2**)

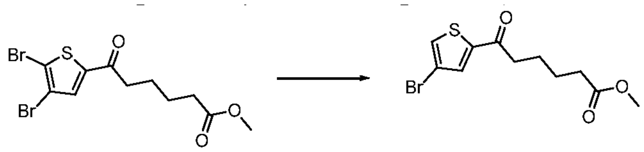

The preparation of methyl 6-(4-bromothiophen-2-yl)-6-oxocaproate (**51-2**) was the same as that of compound **1-3** with a yield of 87%. $^1$H NMR (400 MHz, $CDCl_3$) $\delta$ 7.59 (d, $J$ = 1.4 Hz, 1H), 7.52 (d, $J$ = 1.3 Hz, 1H), 3.67 (s, 3H), 2.89 (t, $J$ = 7.0 Hz, 2H), 2.36 (t, $J$ = 7.1 Hz, 2H), 1.85-1.64 (m, 4H).

Step 3: methyl 6-(4-(1H-1-Boc-indol-3-yl)thiophen-2-yl)-6-oxocaproate (51-3)

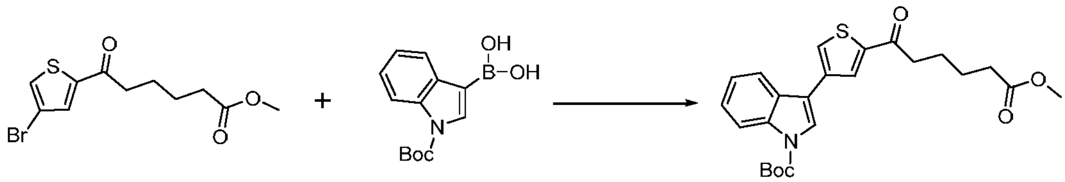

The preparation of methyl 6-(4-(1H-1-Boc-indol-3-yl)thiophen-2-yl)-6-oxocaproate (**51-3**) was same as that of compound **1-4**, and a light brown solid was obtained with a yield of 52%.$^1$H NMR (400 MHz, $CDCl_3$) $\delta$ 8.22 (d, $J$ = 8.3 Hz, 1H), 7.96 (d, $J$ = 1.4 Hz, 1H), 7.78 (tt, $J$= 3.4, 1.4 Hz, 3H), 7.36 (m, 2H), 3.67 (s, 3H), 2.99 (t, $J$= 7.1 Hz, 2H), 2.39 (t, $J$ = 7.2 Hz, 2H), 1.87-1.74 (m, 4H), 1.70 (s, 9H).

Step 4: 6-(4-(1H-indol-3-yl)thiophen-2-yl)-6-oxohexanoic acid (**I-51**)

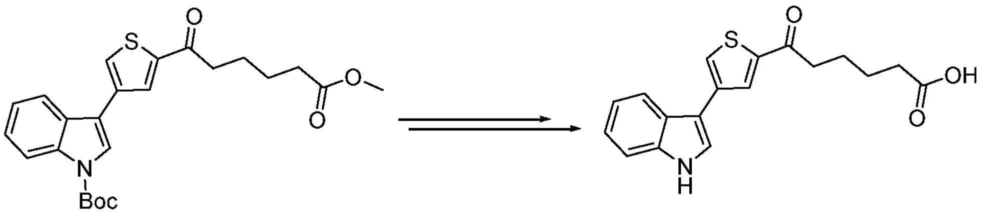

The preparation of 6-(4-(1H-indol-3-yl)thiophen-2-yl)-6-oxohexanoic acid (**I-51**) was the same as that of compound **I-1** with a yield of 73%. $^1$H NMR (400 MHz, DMSO-$d_6$) $\delta$ 12.02 (s, 1H), 11.40 (s, 1H), 8.35 (s, 1H), 8.10 (s, 1H), 7.95 (d, $J$ = 7.8 Hz, 1H), 7.88 (d, $J$ = 2.6 Hz, 1H), 7.44 (d, $J$ = 8.0 Hz, 1H), 7.14 (dt, $J$ = 20.4, 7.2 Hz, 2H), 3.06 (t, $J$ = 7.1 Hz, 2H), 2.28 (t, $J$ = 7.2 Hz, 2H), 1.64 (dp, $J$= 23.5, 7.4 Hz, 4H). LRMS (ESI) $m/z$ $[M+H]^+$: 328.1.

**Example 115** methyl 2,2-difluoro-3-(4-(7-fluoro-1H-indol-3-yl)thiophen-2-yl)-3-oxopropanoate (**I-52**)

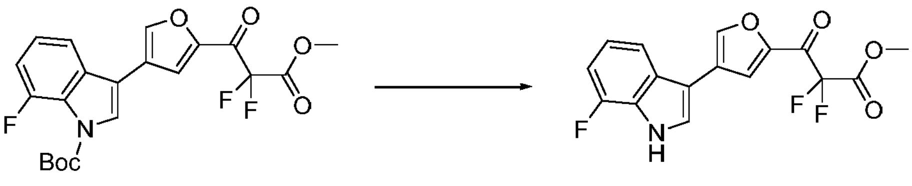

Preparation of methyl 2,2-difluoro-3-(4-(7-fluoro-1H-indol-3-yl)thiophen-2-yl)-3-oxopropanoate (**I-52**) was the same as that of compound **30'-2** with a yield of 59%. $^1$H NMR (400 MHz, DMSO-$d_6$) $\delta$ 8.54 (s, 1H), 8.28 (q, $J$ = 1.5 Hz,

1H), 7.95 (t, *J* = 1.3 Hz, 1H), 7.59 (d, *J* = 8.0 Hz, 1H), 7.48 (d, *J* = 2.6 Hz, 1H), 7.15 (td, *J* = 8.0, 4.8 Hz, 1H), 7.00 (dd, *J* = 11.0, 7.8 Hz, 1H), 3.96 (s, 3H). LRMS (ESI) *m/z* $[M+H]^+$: 354.1.

**Example 116** methyl 3-(2'-(2-amino-2-oxoethyl)-[3,3'-bithiophen]-5-yl)-3-oxopropanoate (**I-53**)

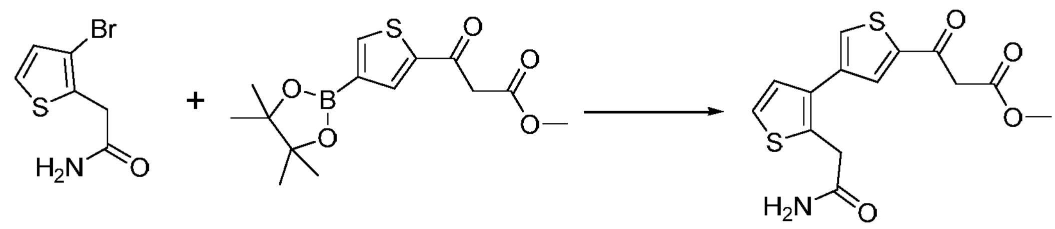

The preparation of methyl 3-(2'-(2-amino-2-oxoethyl)-[3,3'-bithiophen]-5-yl)-3-oxopropanoate (**I-53**) was the same as that of compound **1-4,** and a yellow oily liquid was obtained with a yield of 66%.LRMS (ESI) *m/z* $[M+H]^+$: 324.1.

**Example 117** methyl 3-(4-(3-(2-amino-2-oxoethyl)benzo[b]thiophen-2-yl)thiophen-2-yl)-3-oxopropanoate (**I-54**)

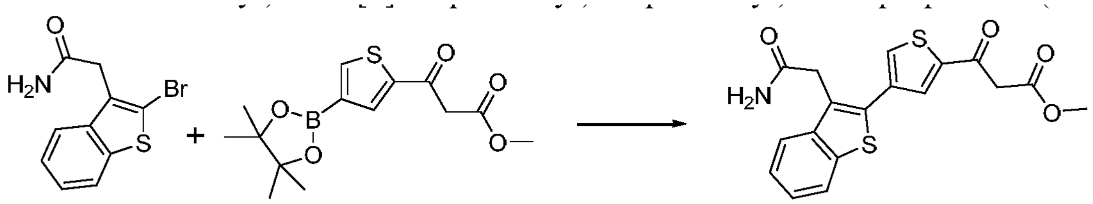

The preparation of methyl 3-(4-(3-(2-amino-2-oxoethyl)benzo[b]thiophen-2-yl)thiophen-2-yl)-3-oxopropanoate (**I-54**) was the same as that of compound **1-4,** and a yellow oily liquid was obtained with a yield of 63%.LRMS (ESI) *m/z* $[M+H]^+$: 374.0.

**Example 118 Effects of compounds on the activation of type I interferon pathway (ISG) in human THP1-Blue-ISG cells**

**1. Experimental method:**

20 μL of compound diluted with physiological saline was added to each well of a 96-well cell culture plate, the positive control compound was ADU-S100, and the solvent control group was added with 20 μL of physiological saline containing DMSO. THP1-Blue-ISG cells were counted, the cell concentration was adjusted to $5 \times 10^5$/mL, and 180 μL of cells were added to each well for incubation. Therefore, the final volume of each test well was 200 μL, the content of DMSO was 0.1%/0.5%, the test concentration of the compound was 10/50 μM, and the positive control compound was ADU-S100 at a final concentration of 10 μM, and incubated for 24 hours for detection.

After 24 hours, 20 μL of the culture solution was took out from each well to a new 96-well plate, 180 μL of chromogenic solution Quanti-Blue was added, placed in a 37 °C incubator, and the OD650 value was measured after 0.5-2 hours. The experiment was repeated twice.

The experimental results were used to evaluate the ability of compounds to induce the secretion of type I interferon pathway (ISG) by the Fold Change, which was calculated as follows:

Fold Change = Compound OD650/Control OD650, wherein the reaults of ≥ 2 times were considered valid.

**2. Experimental results**

The experimental results at the cellular level are shown in Table 2. The five-membered heterocyclic oxocarboxylic acid compounds of the present invention have the function of inducing the secretion of type I interferon pathway in human THP1-Blue-ISG cells, and the activity of some compounds at a concentration of 10 μM is comparable to that of the positive compound ADU-S100.

**Table 2: The effect of the compounds of the present invention on the activation of type I interferon pathway in human THP1-Blue-ISG cells**

| Compound number | Fold Change |
|---|---|
| **[a]ADU-S100** | 12.45@10μM |
| **I-1** | 14.29@10μM |
| **I-2** | 10.99@10μM |
| **I-3** | 9.82@10μM |
| **I-4** | 9.95@10μM |
| **I-13** | 12.59@30μM |
| **I-14** | 1.96@50μM |
| **I-16** | 14.3@10μM |
| **I-17** | 11.0@10μM |
| **I-18** | 9.8@10μM |
| **I-19** | 10.0@10μM |
| **I-26** | 14.59@20μM |
| **I-31** | 43.24@30μM |
| **I-34** | 23.68@30μM |
| **I-36** | 29.68@30μM |
| **I-37** | 71.47@10μM |
| **I-38** | 77.23@10μM |
| **I-39** | 59.47@10μM |
| **I-40** | 53.68@10μM |
| **I-42** | 61.77@10μM |
| **I-43** | 23.33@10μM |
| **I'-52** | 18.45@10μM |
| **I'-4** | 18.36@50 μM |
| **I'-10** | 15.12@50 μM |
| **I'-14** | 20.71@25 μM |
| **I'-17** | 21.65@25 μM |
| **I'-30** | 22.74@12.5 μM |
| **I'-31** | 13.81@10 μM |
| **I'-38** | 7.15@10 μM |
| **I'-42** | 16.81@30 μM |
| **I'-46** | 11.27@10μM |
| **I'-48** | 14.66@30μM |

(continued)

| Compound number | Fold Change |
|---|---|
| **I'-49** | 10.35@10μM |
| **I'-50** | 68.07@10μM |
| **I'-51** | 5.27@10μM |
| **I'-55** | 15.97@30μM |
| **I'-56** | 14.90@30μM |
| **I'-58** | 4.14@10μM |
| [a] ADU-S100 was the positive compound (Corrales, L.; Glickman, L. H.; McWhirter, S. M.; Kanne, D. B.; Sivick, K. E.; Katibah, G. E.; Woo, S. R.; Lemmens, E.; Banda, T.; Leong, J. J.; Metchette, K.; Dubensky, T. W. Jr.; Gajewski, T. F. Direct activation of STING in the tumor microenvironment leads to potent and systemic tumor regression and immunity. Cell Rep. 2015, 11(7), 1018-1030.) | |

**Example 119 Effects of compounds on the activation of type I interferon pathway in murine Raw-lucia cells**

**1. Experimental method:**

Raw-lucia cells were counted, the cell concentration was adjusted to 5 × $10^5$/mL, and 180 μL of cells were added to each well for incubation. 20 μL of compound diluted with physiological saline was added to each well of a 96-well cell culture plate after cells were adhered, the concentration of the compound was 50 μM/100 μM. The positive control compound was DMXAA at a concentration of 50 μM, and the control group was physiological saline free of DMSO, and 3 duplicate wells were set for each group, and incubated for 24 hours for detection.

After 24 hours, 20 μL of the culture medium was took out from each well to a new 96-well plate with bottom-transmitting, 50 μL of the luciferase detection reagent QUANTI-Luc™ was added, and the fluorescence value was measured immediately (in the dark). The experiment was repeated twice.

The experimental results were used to evaluate the ability of compounds to induce the secretion of type I interferon pathway by the Fold Change, which was calculated as follows:

Fold Change = Compound Luminescence/Control Luminescence, wherein the results of ≥2 times were considered valid.

**2. Experimental results**

The experimental results at the cellular level are shown in Table 3. The 4-oxobutyric acid compounds of the present invention have the function of inducing the secretion of type I interferon pathway in murine Raw-lucia cells. The activity of some compounds at a concentration of 50 μM was comparable to that of the positive compound DMXAA.

**Table 3: Effects of the compounds of the present invention on the activation** of **type I interferon pathway (ISG) in murine Raw-lucia cells**

| Compound number | Fold Change |
|---|---|
| [a]**DMXAA** | 27.3@50 μM |
| **I'-14** | 18.10@100 μM |
| **I'-17** | 12.51@50 μM |
| **I'-30** | 8.13@100 μM |
| **I'-31** | 21.79@50 μM |
| **I'-37** | 3.79@50 μM |
| **I'-38** | 7.10@50 μM |
| **I'-42** | 7.45@100 μM |

(continued)

| Compound number | Fold Change |
|---|---|
| **I'-46** | 31.38@100μM |
| **I'-48** | 7.63@100μM |
| **I'-49** | 36.19@50μM |
| **I'-51** | 9.75@100μM |
| **I'-54** | 4.50@100μM |
| **I'-55** | 12.16@50μM |
| **I'-56** | 12.92@50μM |
| **I'-58** | 14.20@100μM |
| [a]DMXAA was the positive compound (Shirey, K. A.; Nhu, Q. M.; Yim, K. C.; Roberts, Z. J.; Teijaro, J. R.; Farber, D. L.; Blanco, J. C.; Vogel, S. N. The anti-tumor agent, 5,6-dimethylxanthenone-4-acetic acid (DMXAA), induces IFN-beta-mediated antiviral activity in vitro and in vivo. J. Leukoc. Biol. 2011, 89(3):351-357.) | |

**Example 120 Preparation of pharmaceutical composition**

**Solvate**

Compound **1-5** (100mg ) was taken out, PEG400 (0.8mL) was added, 1% sodium bicarbonate aqueous solution (4.2mL) was added after 15 minutes of sonication, and stirred for 0.5 h at room temperature to prepare a pharmaceutical composition of 20 mg/mL.

**Tablet**

| | |
|---|---|
| Compound **1-5** | 150 g |
| Colloidal silicon dioxide | 0.5 g |
| Magnesium stearate | 4.6g |
| Modified starch | 14g |
| Microcrystalline cellulose | 212g |
| Lactose | 125g |

The above substances were mixed uniformly, and 1000 tablets were prepared by conventional technology. Appropriate aqueous or non-aqueous coatings can be used to enhance palatability, improve appearance and stability, or delay absorption.

**Capsule**

| | |
|---|---|
| Compound **1-5** | 150 g |
| Starch | 145g |
| Microcrystalline cellulose | 75g |

The above substances were mixed uniformly and packed into ordinary gelatin capsules to prepare 1000 capsules according to the conventional method.

**Example 121 Preparation of pharmaceutical composition**

**Solvate**

Compound **I'-31** (100mg ) was taken out, PEG400 (0.8mL) was added, 1% sodium bicarbonate aqueous solution (4.2mL) was added after 15 minutes of sonication, and stirred for 0.5 h at room temperature to prepare a pharmaceutical composition of 20 mg/mL.

| Tablet | |
|---|---|
| Compound **I'-31** | 150 g |
| Colloidal silicon dioxide | 0.6 g |
| Magnesium stearate | 4.5g |
| Modified starch | 15g |
| Microcrystalline cellulose | 210g |
| Lactose | 120g |

The above substances were mixed uniformly, and 1000 tablets were prepared by conventional technology. Appropriate aqueous or non-aqueous coatings can be used to enhance palatability, improve appearance and stability, or delay absorption.

| Capsule | |
|---|---|
| Compound **I'-31** | 150 g |
| Starch | 140g |
| Microcrystalline cellulose | 80g |

The above substances were mixed uniformly and packed into ordinary gelatin capsules to prepare 1000 capsules according to the conventional method.

**Example 122 Preparation of pharmaceutically acceptable salt**

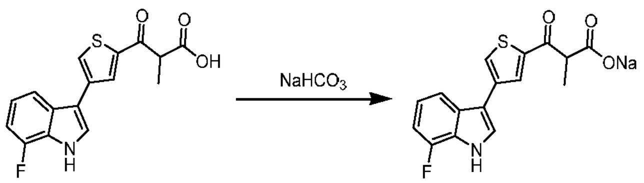


Compound **1-6** (100 mg) was taken out, and dissolved in tetrahydrofuran (2 mL), 1% aqueous sodium bicarbonate solution (2.65 mL) was added dropwise under ice bath, and stirred at room temperature for 2 hours after addition. The reaction solution was lyophilized to obtain 107 mg of foamy solid powder with a yield of 99.7%. $^1H$ NMR (400 MHz, DMSO-$d_6$+$D_2O$) δ 8.37(s, 1H), 8.15(s, 1H), 7.96(d, J= 2.4 Hz, 1H), 7.78 (d, *J* = 7.8 Hz, 1H), 6.96-7.14 (m, 2H), 3.09 (q, *J* = 7.1 Hz, 1H), 1.14 (d, *J* = 7.3 Hz, 3H).

**Example 123 Preparation of pharmaceutically acceptable salt**

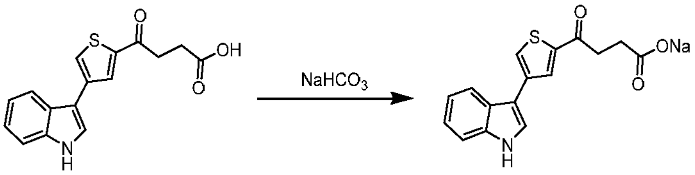


Compound **I'-31** (100 mg) was taken out, and dissolved in tetrahydrofuran (2 mL), 1% aqueous sodium bicarbonate solution (2.8 mL) was added dropwise under ice bath, and stirred at room temperature for 2 hours after addition. The reaction solution was lyophilized to obtain 107 mg of foamy solid powder with a yield of 99.7%. $^1H$ NMR (600 MHz, DMSO-$d_6$+$D_2O$) δ 8.32 (s, 1H), 8.05 (s, 1H), 7.96 (d, *J* = 6.0 Hz, 1H), 7.84 (s, 1H), 7.50 (d, *J* = 6.0 Hz, 1H), 7.17-7.24 (m, 2H), 3.25 (t, *J* = 6.0 Hz, 2H), 2.46 (t, *J* = 6.0 Hz, 2H).

**Example 124 Therapeutic effect of compound I'-31 on 4T1 breast cancer orthotopic tumor model in vivo**

**1. Experimental method:**

6-8 week old Balb/C mice were inoculated with $2.5 \times 10^5$ 4T1 mouse breast cancer cells in the mammary fat pad of each mouse. When the tumor volume grew to about 100 $mm^3$, the mice were randomly divided into 5 groups, 6 mice in each group. The mode of administration was intratumoral injection (IT), and DMXAA was used as a positive control, which was administered on days 1/2/3/4/7, respectively.

Experimental results were evaluated in the form of tumor volume growth curves.

**2. Experimental results**

The experimental results of the animal in vivo level are shown in Figure 1. The compound of the present invention **I'-31** has a significant anti-tumor effect in the 4T1 tumor model of the Balb/C strain, and tumor regression effect is achieved in some mouse tumor models.

**Example 125 Therapeutic effect of compound I'-31 on CT26 colon cancer subcutaneous xenograft model in vivo**

**1. Experimental method:**

6-8 week old Balb/C mice were inoculated with $2.5\times10^5$ CT26 mouse colon cancer cells in the mammary fat pad of each mouse. When the tumor volume grew to about 100$mm^3$, the mice were randomly divided into 5 groups, 6 mice in each group. The mode of administration was intratumoral injection (IT), and DMXAA was used as a positive control, which was administered on days 1/2/3/4/7, respectively.

Experimental results were evaluated in the form of tumor volume growth curves.

**2. Experimental results**

The experimental results of the animal in vivo level are shown in Figure 2. The compound of the present invention **I'-31** has a significant anti-tumor effect in the CT26 tumor model of the Balb/C strain, and tumor regression effect is achieved in some mouse tumor models.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula (I), or a pharmaceutically acceptable salt, prodrug, hydrate, solvate or crystal form thereof:

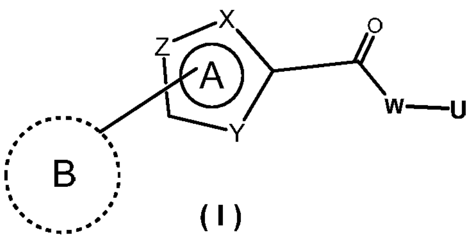

(I)

wherein,

W is selected from the group consisting of substituted or unsubstituted C3-C8 cycloalkyl, substituted or unsubstituted 3-8 membered heterocyclyl,

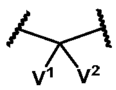, 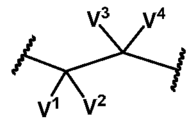,

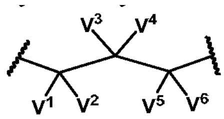 and 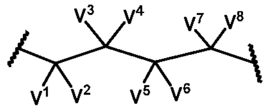,

wherein, $V^1$, $V^2$, $V^3$, $V^4$, $V^5$, $V^6$, $V^7$ and $V^8$ are each independently selected from the group consisting of hydrogen atom, halogen, deuterium, cyano, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl and substituted or unsubstituted 3-8-membered heterocyclyl;

or $V^1$ and $V^2$ together with the C atom to which they are attached form a substituted or unsubstituted C3-C8 cycloalkyl or substituted or unsubstituted 3-8 membered heterocyclyl;

or $V^3$ and $V^4$ together with the C atom to which they are attached form a substituted or unsubstituted C3-C8 cycloalkyl or substituted or unsubstituted 3-8 membered heterocyclyl;

or $V^5$ and $V^6$ together with the C atom to which they are attached form a substituted or unsubstituted C3-C8 cycloalkyl or substituted or unsubstituted 3-8 membered heterocyclyl;

or $V^7$ and $V^8$ together with the C atom to which they are attached form a substituted or unsubstituted C3-C8 cycloalkyl or substituted or unsubstituted 3-8 membered heterocyclyl;

or $V^1$ and $V^3$ together with the atoms to which they are attached form a substituted or unsubstituted C3-C8 cycloalkyl or a substituted or unsubstituted 3-8 membered heterocyclyl;

or $V^1$ and $V^5$ together with the atoms to which they are attached form a substituted or unsubstituted C3-C8 cycloalkyl or a substituted or unsubstituted 3-8 membered heterocyclyl;

or $V^3$ and $V^7$ together with the atoms to which they are attached form a substituted or unsubstituted C3-C8 cycloalkyl or a substituted or unsubstituted 3-8 membered heterocyclyl;

or $V^5$ and $V^7$ together with the atoms to which they are attached form a substituted or unsubstituted C3-C8 cycloalkyl or a substituted or unsubstituted 3-8 membered heterocyclyl;

or $V^1$ and $V^7$ together with the atoms to which they are attached form a substituted or unsubstituted C4-C8 cycloalkyl or a substituted or unsubstituted 4-8 membered heterocyclyl;

U is independently selected from the group consisting of $COOR^1$, COOH, CN, $CONH_2$ and

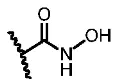,

wherein, $R^1$ is selected from the group consisting of substituted or unsubstituted C1-C6 alkyl, substituted or unsubstituted C3-C8 cycloalkyl and substituted or unsubstituted 3-8 membered heterocyclyl;

ring A is a five-membered heteroaromatic ring, wherein X, Y and Z are each independently selected from the group consisting of C, CH, C-$R^2$, NH, N, O and S, wherein, $R^2$ is selected from the group consisting of halogen, deuterium, hydroxyl, amino, cyano, carboxyl, formyl, substituted or unsubstituted formamido, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

ring B is independently selected from the group consisting of substituted or unsubstituted 5-14 membered heteroaromatic ring and substituted or unsubstituted C6-C14 aromatic ring;

wherein, unless otherwise specified, the "substituted" refers to being substituted by one or more groups selected from the group consisting of deuterium, halogen, hydroxyl, cyano, carboxyl, amino,

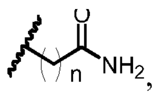,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

2. The compound of formula **(I),** or a pharmaceutically acceptable salt, prodrug, hydrate, solvate or crystal form thereof of claim 1 has a structure shown in formula (I'):

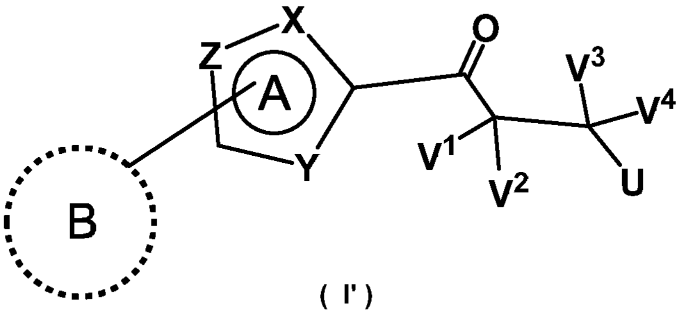

( I' )

wherein, $V^1$, $V^2$, $V^3$ and $V^4$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is independently selected from the group consisting of $COOR^m$, COOH, CN, $CONH_2$ and

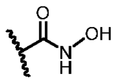,

wherein, $R^m$ is selected from substituted or unsubstituted C1-C6 alkyl, substituted or unsubstituted C3-C8 cycloalkyl or substituted or unsubstituted 3-8 membered heterocyclyl;
ring A is a five-membered heteroaromatic ring, wherein X, Y and Z are each independently selected from CH, $C\text{-}R^n$, NH, N, O or S, wherein, $R^n$ is selected from the group consisting of halogen, deuterium, hydroxyl, amino, cyano, carboxyl, formyl, substituted or unsubstituted formamido, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;
ring B is independently selected from the group consisting of substituted or unsubstituted 5-14 membered heteroaromatic ring and substituted or unsubstituted C6-C14 aromatic ring;
wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl.

3. The compound of formula **(I),** or a pharmaceutically acceptable salt, prodrug, hydrate, solvate or crystal form thereof of claim 1,

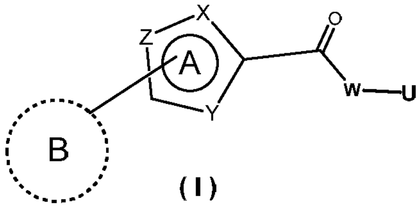

(I)

wherein W is selected from the group consisting of

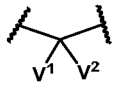, 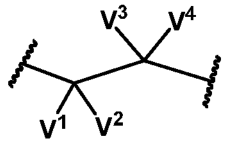,

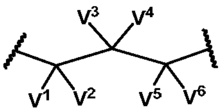 and 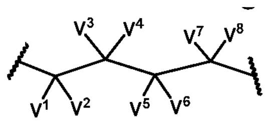,

wherein $V^1$, $V^2$, $V^3$, $V^4$, $V^5$, $V^6$, $V^7$ and $V^8$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is independently selected from the group consisting of $COOR^1$, COOH, CN, $CONH_2$ and

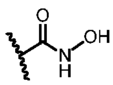,

wherein, $R^1$ is selected from the group consisting of substituted or unsubstituted C1-C6 alkyl, substituted or unsubstituted C3-C8 cycloalkyl and substituted or unsubstituted 3-8 membered heterocyclyl;
ring A is a five-membered heteroaromatic ring, wherein X, Y and Z are each independently selected from the group consisting of C, CH, C-$R^2$, NH, N, O and S, wherein $R^2$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
ring B and

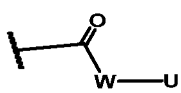

are located in the ortho or meta position of ring A;
wherein, the "substituted" refers to being substituted by one or more groups selected from the group consisting of deuterium, halogen, hydroxyl, cyano, carboxyl, amino,

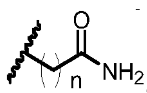,

amido, sulfonamido, C1-C6 alkylamino, substituted or unsubstituted C1-C6 alkyl, C2-C6 alkenyl, formyl, formamido or substituted formamido, substituted or unsubstituted C1-C6 alkoxy, substituted or unsubstituted C3-C8 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

**4.** The compound of formula **(I),** or a pharmaceutically acceptable salt, prodrug, hydrate, solvate or crystal form thereof of claim 1 has a structure shown in formula (I'):

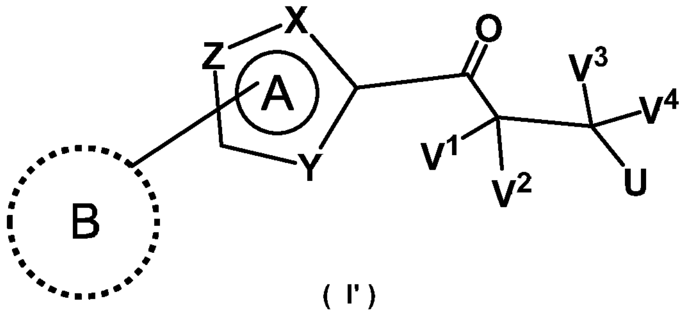

( I' )

wherein, $V^1$, $V^2$, $V^3$ and $V^4$ are each independently selected from the group consisting of hydrogen atom, halogen, substituted or unsubstituted C1-C3 alkyl, substituted or unsubstituted C1-C3 alkoxy, substituted or unsubstituted C3-C6 cycloalkyl and substituted or unsubstituted 3-8 membered heterocyclyl;

U is independently selected from the group consisting of $COOR^m$, COOH, CN, $CONH_2$ and

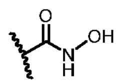

,

wherein, $R^m$ is selected from substituted or unsubstituted C1-C6 alkyl or substituted or unsubstituted C3-C6 cycloalkyl;
ring A is a five-membered heteroaromatic ring, wherein X, Y and Z are each independently selected from CH, C-$R^n$, NH, N, O or S, wherein $R^n$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
ring B and

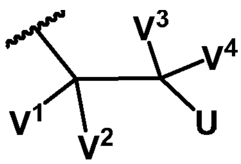

are located in the ortho or meta position of ring A;
wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, formyl, formamido or substituted formamido, substituted or unsubstituted C1-C3 alkoxy, substituted or unsubstituted C3-C6 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl,

C1-C3 alkoxy, C3-C6 cycloalkyl and 3-8 membered heterocyclyl.

**5.** The compound of formula **(I),** or a pharmaceutically acceptable salt, prodrug, hydrate, solvate or crystal form thereof of claim 1 has a structure shown in formula (II), (III), (IV), (V) or (VI):

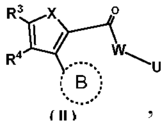 (II) , 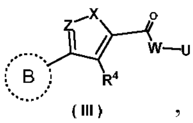 (III) , 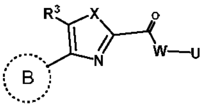 (IV) , 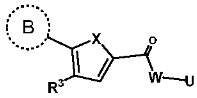 (V) or 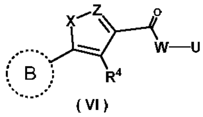 (VI)

wherein W is selected from the group consisting of

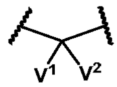 , 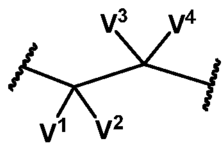 ,

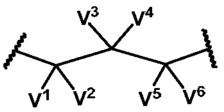 and 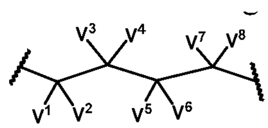 ,

wherein $V^1$, $V^2$, $V^3$, $V^4$, $V^5$, $V^6$, $V^7$ and $V^8$ are each independently selected from the group consisting of hydrogen atom, halogen, deuterium, substituted or unsubstituted C1-C6 alkyl, substituted or unsubstituted C1-C6 alkoxy, substituted or unsubstituted C3-C8 cycloalkyl and substituted or unsubstituted 3-8 membered heterocyclyl;

U is selected from the group consisting of $COOR^1$, COOH, CN, $CONH_2$ and

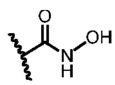 ,

wherein, $R^1$ is selected from the group consisting of substituted and unsubstituted C1-C6 alkyl and substituted or unsubstituted C3-C6 cycloalkyl;
wherein, X is selected form O, S or NH;
Z is selected from CH, N or C-$R^2$;
wherein, $R^2$ is selected from the group consisting of halogen, deuterium, hydroxyl, amino, cyano, carboxyl, formyl, substituted or unsubstituted C1-C3 alkyl, substituted or unsubstituted C1-C3 alkoxy and substituted or unsubstituted C3-C6 cycloalkyl;
$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C8 cycloalkyl and substituted or unsubstituted 3-8-membered heterocyclyl; wherein, the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
wherein, unless otherwise specified, the "substituted" refers to being substituted by one or more groups selected from the group consisting of deuterium, halogen, hydroxyl, cyano, carboxyl, amino,

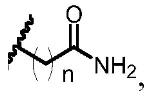

amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl, C1-C3 alkoxy, C3-C6 cycloalkyl and 3-8 membered heterocyclyl;
n is an integer of 1-6.

**6.** The compound of formula **(I),** or a pharmaceutically acceptable salt, prodrug, hydrate, solvate or crystal form thereof of claim 1 has a structure shown in formula **(II-1), (III-1), (IV-1), (V-1)** or **(VI-1):**

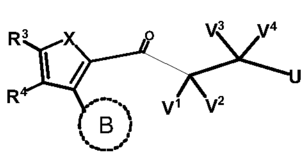
( II-1 ) ,

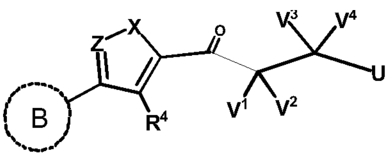
( III-1 ) ,

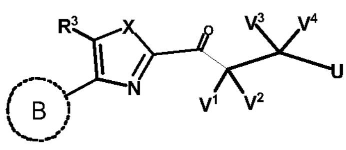
( IV-1 ) ,

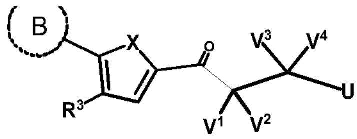
( V -1) or

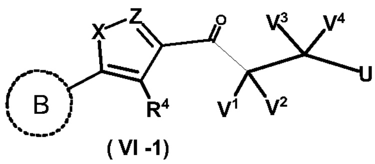
( VI -1)

wherein, X is selected form O, S or NH;

Z is selected from N or C-$R^3$;
$V^1$, $V^2$, $V^3$ and $V^4$ are each independently selected from hydrogen atom, halogen, C1-C3 alkyl, C1-C3 alkoxy or C3-C6 cycloalkyl;
U is selected from $COOR^m$, COOH, CN, $CONH_2$ or

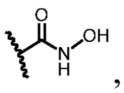

wherein, $R^m$ is selected from substituted or unsubstituted C1-C6 alkyl or substituted or unsubstituted C3-C6 cycloalkyl;
$R^3$ and $R^4$ are each independently selected from the group consisting of hydrogen atom, halogen, deuterium, amino, hydroxyl, cyano, carboxyl, substituted or unsubstituted C1-C3 alkyl, substituted or unsubstituted C1-C3 alkoxy, substituted or unsubstituted C3-C6 cycloalkyl and substituted or unsubstituted 3-8-membered heterocyclyl;
ring B is selected from the substituted or unsubstituted group consisting of five-membered heteroaromatic ring, six-membered aromatic heterocyclic ring, [5+5] aromatic heterocyclic ring or fused ring, [6+5] aromatic heterocyclic ring, [6+5+6] aromatic heterocyclic ring, [6+6] aromatic heterocyclic ring, six-membered aromatic ring and [6+6] aromatic ring;
wherein, the "substituted" refers to being substituted by one or more groups selected from the group consisting of deuterium, halogen, hydroxyl, cyano, carboxyl, amino, amido, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl and substituted or unsubstituted 3-8 membered heterocyclyl;
wherein, the substituted in the "substituted" refers to being substituted by one or more groups selected from the group consisting of deuterium, halogen, hydroxyl, cyano, carboxyl, amino, amido, sulfonamido, C1-C3 alkyl,

C1-C3 alkoxy, C3-C6 cycloalkyl and 3-8 membered heterocyclyl.

**7.** The compound of formula **(I),** or a pharmaceutically acceptable salt, prodrug, hydrate, solvate or crystal form thereof of claim 1, wherein ring B is selected from the following structures:

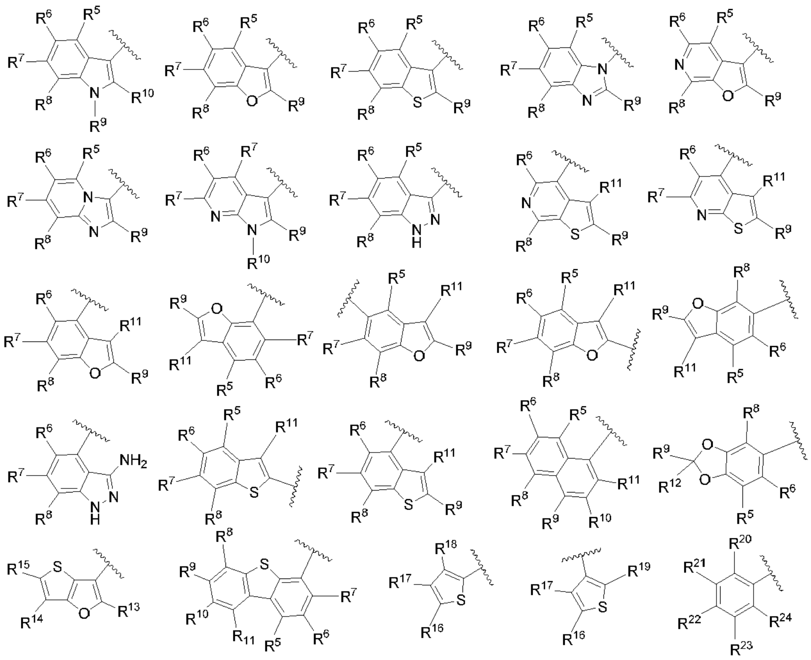

wherein, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$ and $R^{24}$ are each independently selected from the group consisting of hydrogen atom, halogen, deuterium, substituted or unsubstituted C1-C6 alkyl, C2-C6 alkenyl, substituted or unsubstituted C1-C6 alkoxy, substituted or unsubstituted C3-C6 cycloalkyl, substituted or unsubstituted 3-8 membered heterocyclyl, amino, amido, sulfonamido, hydroxyl,

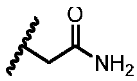,

cyano, carboxyl, formyl, formamido or substituted formamido and C1-C6 alkylamino;
wherein, the "substituted" refers to being substituted by one or more groups selected from the group consisting of deuterium, halogen, hydroxyl, cyano, carboxyl, amino, amido, sulfonamido, C1-C6 alkylamino, C1-C3 alkyl, C1-C3 alkoxy, C3-C6 cycloalkyl and 3-8 membered heterocyclyl.

**8.** A prodrug of the compound of formula I of claim 1, wherein it has a structure shown in formula (**I''**):

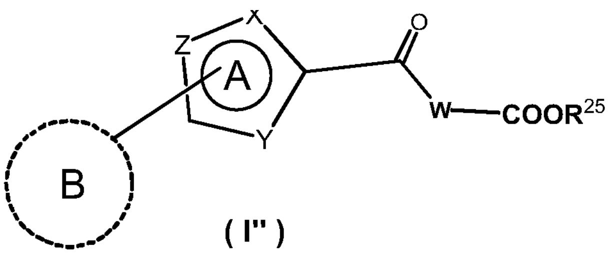

**( I'' )**

wherein,

$R^{25}$ is selected from substituted or unsubstituted C1-C6 alkyl, substituted or unsubstituted C2-C6 alkenyl, substituted or unsubstituted C1-C6 alkoxy, substituted or unsubstituted C3-C6 cycloalkyl, substituted or unsubstituted 3-8 membered heterocyclyl, substituted or unsubstituted C6-C12 aryl or substituted or unsubstituted 5-14 membered heteroaryl;
wherein, the "substituted" refers to being substituted by one or more groups selected from the group consisting of deuterium, halogen, hydroxyl, cyano, carboxyl, amino, amido, ester group, sulfonamido, C1-C6 alkylamino, substituted or unsubstituted C1-C3 alkyl, C2-C3 alkenyl, substituted or unsubstituted C1-C3 alkoxy, substituted or unsubstituted C3-C6 cycloalkyl, substituted or unsubstituted 3-8 membered heterocyclyl, substituted or unsubstituted C6-C12 aryl and substituted or unsubstituted 5-14-membered heteroaryl;
wherein, the substituted in the "substituted" refers to being substituted by one or more groups selected from the group consisting of deuterium, halogen, hydroxyl, cyano, carboxyl, amino, ester group, amido, sulfonamido, C1-C3 alkyl, C1-C3 alkoxy, C3-C6 cycloalkyl and 3-8 membered heterocyclyl;
W, U, ring A, X, Y, Z and ring B are as defined in claim 1.

**9.** The compound of formula **(I),** or a pharmaceutically acceptable salt, prodrug, hydrate, solvate or crystal form thereof of claim 1, wherein the compound is selected from the group consisting of:

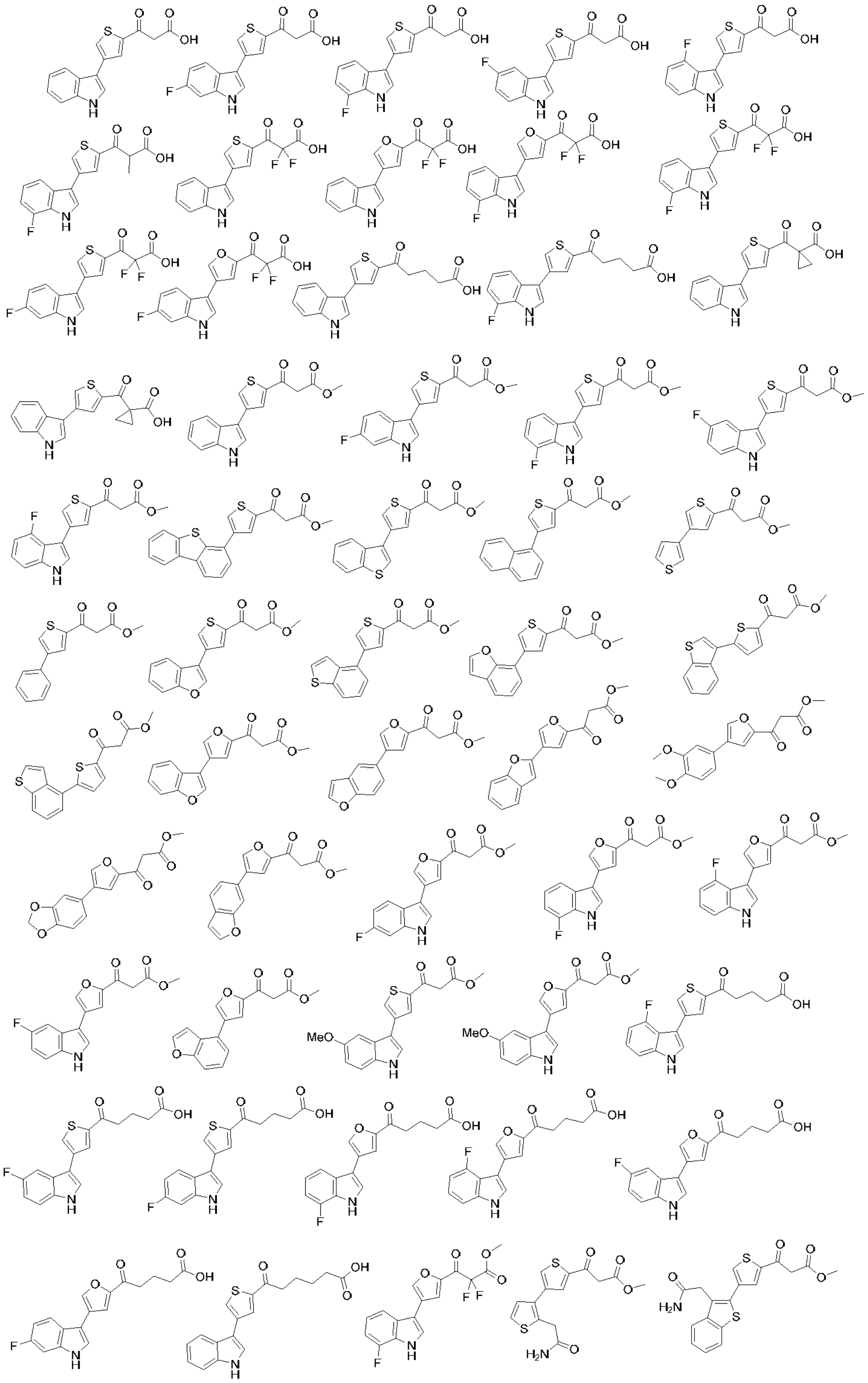

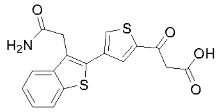 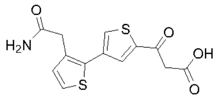 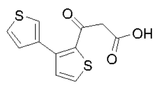 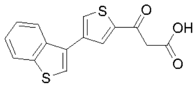 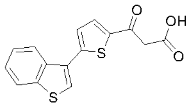

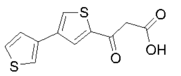 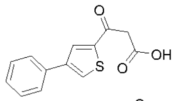 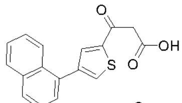 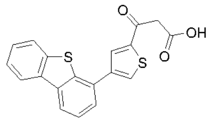 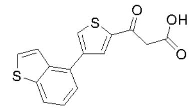

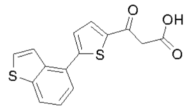 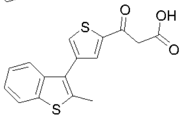 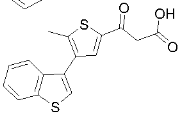 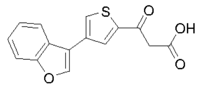 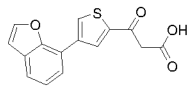

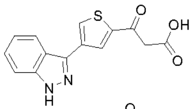 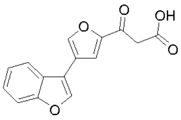 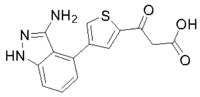 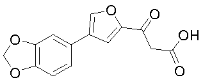 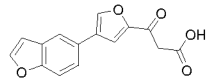

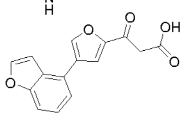 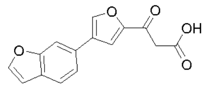 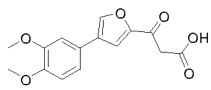 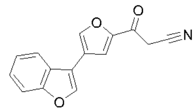 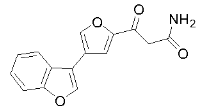

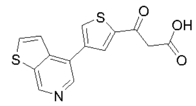 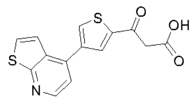 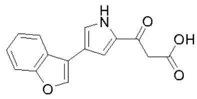 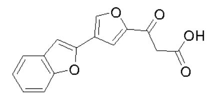 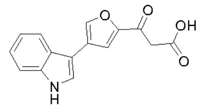

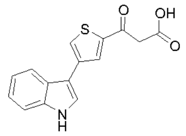 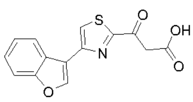 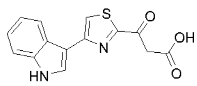 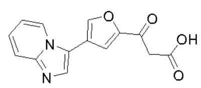 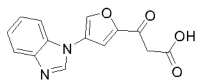

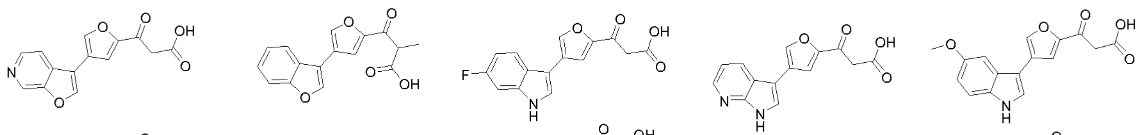

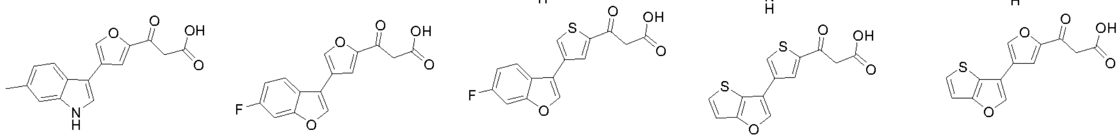

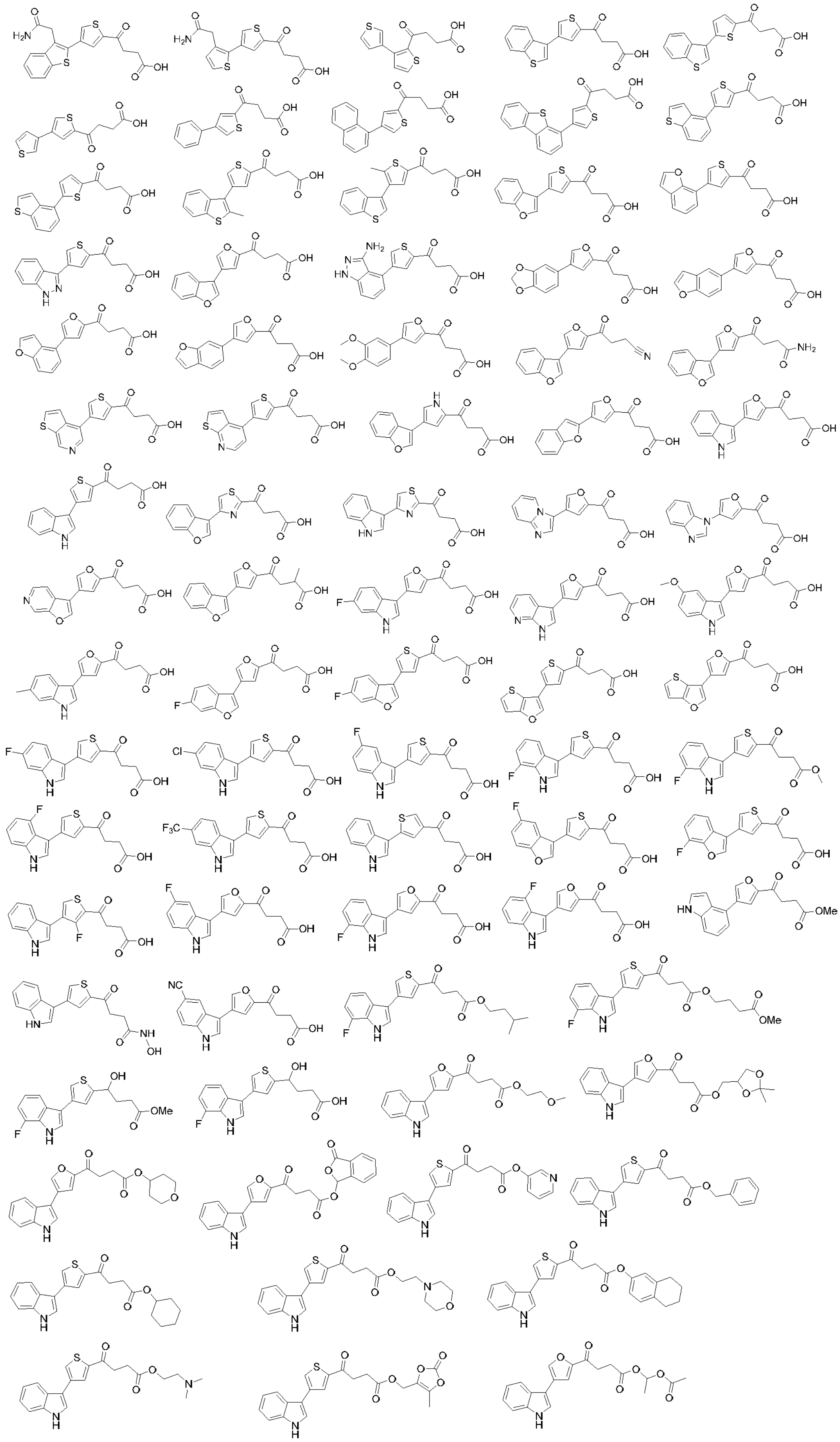

.

**10.** A pharmaceutical composition comprising a therapeutically effective amount of one or more of the compound of formula (I), or a pharmaceutically acceptable salt, prodrug, hydrate, solvate or crystal form thereof of any one of claims 1-5; and optionally a pharmaceutically acceptable carrier.

**11.** A method for the preparation of the compound of formula (I), or a pharmaceutically acceptable salt, prodrug, solvate or crystal form thereof of claim 1,

Method 1:
it comprises the following steps:

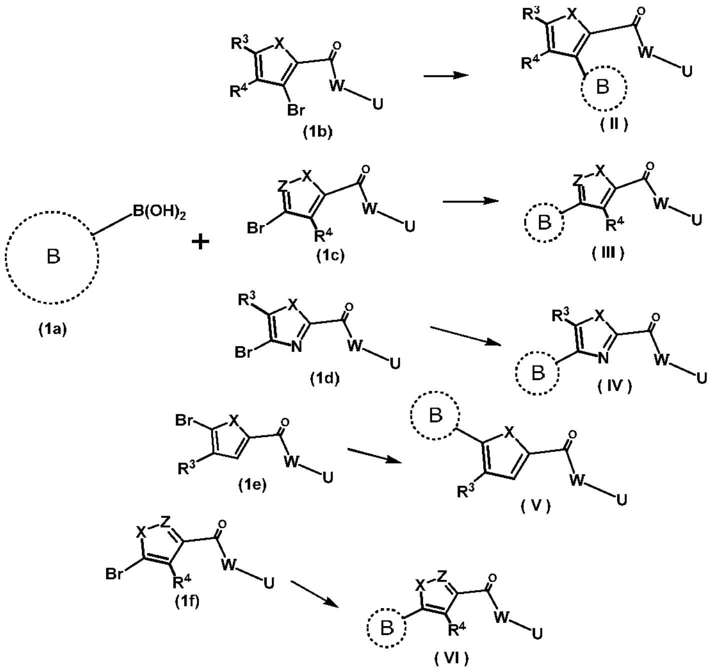

(1b) (II) (1a) + (1c) (III) (1d) (IV) (1e) (V) (1f) (VI)

in the presence of a catalyst, a boronic acid intermediate **(1a)** is reacted with an intermediate **(1b)** to obtain a compound of formula (II);
in the presence of a catalyst, a boronic acid intermediate **(1a)** is reacted with an intermediate **(1c)** to obtain a compound of formula (III);
in the presence of a catalyst, a boronic acid intermediate **(1a)** is reacted with an intermediate **(1d)** to obtain a compound of formula (IV);
in the presence of a catalyst, a boronic acid intermediate **(1a)** is reacted with an intermediate **(1e)** to obtain a compound of formula (V);
in the presence of a catalyst, a boronic acid intermediate **(1a)** is reacted with an intermediate **(1f)** to obtain a compound of formula (VI);
**Method 2:**
it comprises the following steps:

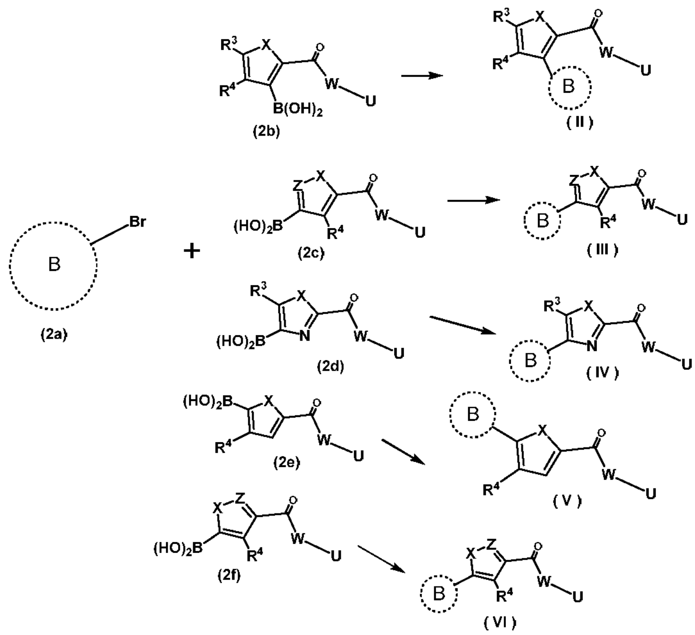

in the presence of a catalyst, the bromine-containing intermediate **(2a)** is reacted with a boronic acid intermediate **(2b)** to obtain a compound of formula (II);
in the presence of a catalyst, the bromine-containing intermediate **(2a)** is reacted with a boronic acid intermediate **(2c)** to obtain a compound of formula (III);
in the presence of a catalyst, the bromine-containing intermediate **(2a)** is reacted with a boronic acid intermediate **(2d)** to obtain a compound of formula (IV);
in the presence of a catalyst, the bromine-containing intermediate **(2a)** is reacted with a boronic acid intermediate **(2e)** to obtain a compound of formula (V);
in the presence of a catalyst, a bromine-containing intermediate **(2a)** is reacted with a boronic acid intermediate **(2f)** to obtain a compound of formula (VI);
wherein, $R^3$, $R^4$, W, X, Z, U, and ring B are as defined in claim 5.

**12.** Use of the compound, or a pharmaceutically acceptable salt, prodrug, hydrate, solvate or crystal form thereof of claim 1 for preparing a medicament, and the medicament is used for a use selected from the group consisting of:

1) for the prevention and/or treatment of immune response-related disease;
2) for the prevention and/or treatment of STING activity-related disease; and
3) for the prevention and/or treatment of immune response anti-infection-related disease.

**13.** The use of claim 12, wherein the immune response-related disease and/or STING activity-related disease is tumor or cancer; wherein the tumor or cancer is selected from the group consisting of colon cancer, breast cancer, lung cancer, melanoma, liver cancer, stomach cancer, cervical cancer, ovarian cancer, fibrosarcoma and squamous cell carcinoma, brain cancer, spine cancer, head and neck cancer, leukemia and blood cancer, skin cancer, genital system cancer, gastrointestinal system cancer, liver and bile duct cancer, kidney cancer and bladder cancer, bone cancer, lung cancer, malignant mesothelioma, sarcoma, lymphoma, adenocarcinoma, thyroid cancer, cardiac tumor, germ cell tumor, malignant neuroendocrinetumor, midline beam cancer, and unknown primary cancer.

**14.** The use of claim 12, wherein the immune response anti-infection-related disease is bacterial or viral infection, and the bacterial or viral infection is hepatitis B virus (HBV), hepatitis C virus (HCV), human papilloma virus (HPV), nasopharyngeal cancer-related EB virus (EBV) or human immunodeficiency virus (HIV) infection-related disease.

**15.** The use of claim 12, wherein the STING activity-related disease comprises an inflammatory disease, wherein the inflammatory disease is selected from acne vulgaris, asthma, celiaca, chronic prostatitis, glomerulonephritis, inflammatory bowel disease, pelvic inflammatory disease, reperfusion injury, rheumatoid arthritis, sarcoidosis, vasculitis, airway inflammation caused by house dust mites and interstitial cystitis.

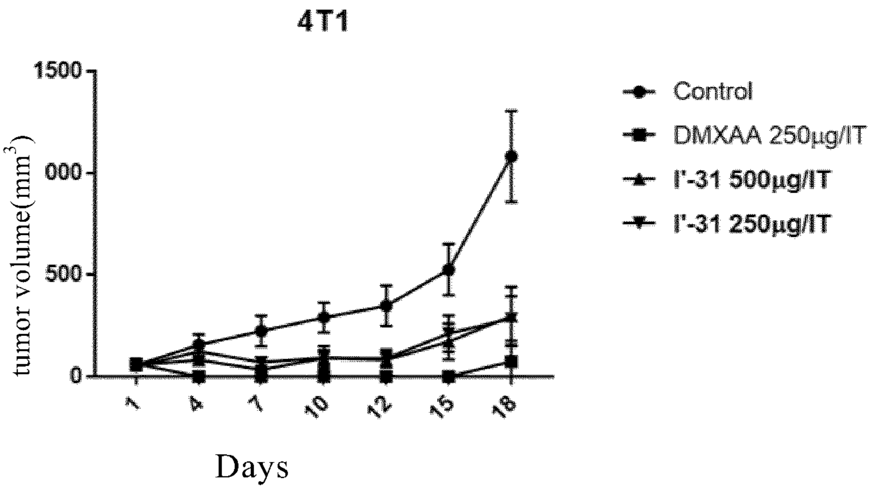


Figure 1

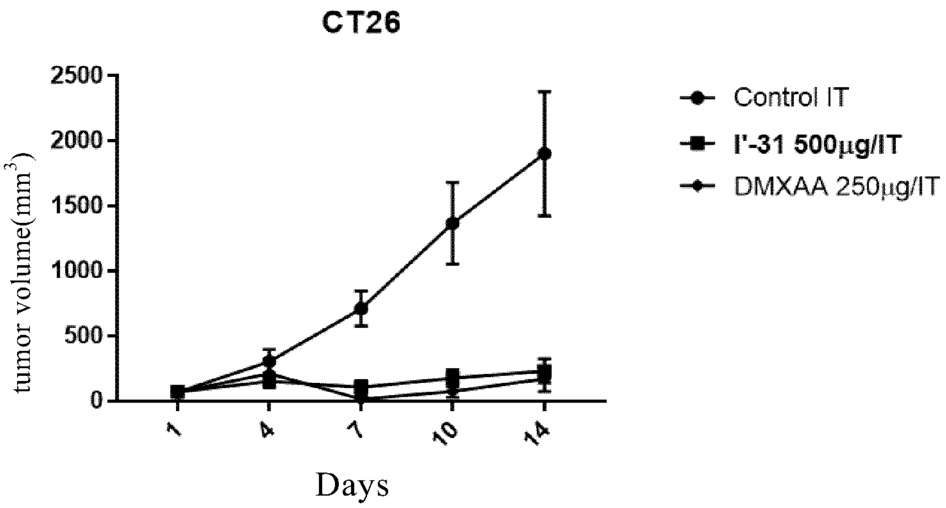


Figure 2

## INTERNATIONAL SEARCH REPORT

International application No.
**PCT/CN2020/123404**

**A. CLASSIFICATION OF SUBJECT MATTER**

C07D 409/04(2006.01)i; C07D 409/14(2006.01)i; C07D 407/04(2006.01)i; C07D 333/00(2006.01)i; C07D 231/56(2006.01)i; C07D 493/00(2006.01)i; C07D 487/00(2006.01)i; A61K 31/381(2006.01)i; A61K 31/403(2006.01)i; A61K 31/343(2006.01)i; A61K 31/357(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D409/-; C07D407/-; C07D333/-; C07D231/-; C07D493/-; C07D487/-; A61K31/-; A61P35/-; A61P37/-

Documentation searched other than minimum documentation to the extent that such documents are included in the fields searched

Electronic data base consulted during the international search (name of data base and, where practicable, search terms used)

CNKI, CNABS, SIPOBAS, DWPI, REGISTRY, CAPLUS(STN), 中国科学院上海药物研究所, 吲哚基, 苯并噻吩, 噻吩基, 呋喃基, 苯并咪唑基, 肿瘤, 癌症, 免疫应答, 干扰素, STING, indolyl+, Benzothiophen+, Thiophen+, Furanyl, Benzimidazolyl, tumor, cancer, immune response, structural formula search based on claim 9

**C. DOCUMENTS CONSIDERED TO BE RELEVANT**

| Category* | Citation of document, with indication, where appropriate, of the relevant passages | Relevant to claim No. |
|---|---|---|
| A | CN 110036001 A (MERCK SHARP & DOHME CORP.) 19 July 2019 (2019-07-19) entire document | 1-15 |
| A | CN 109563081 A (GLAXOSMITHKLINE INTELLECTUAL PROPERTY DEVELOPMENT LIMITED) 02 April 2019 (2019-04-02) entire document | 1-15 |
| A | WO 0039086 A1 (SHIONOGI & CO. et al.) 06 July 2000 (2000-07-06) entire document | 1-15 |
| A | US 5705525 A (ADIR) 06 January 1998 (1998-01-06) entire document | 1-15 |
| A | WO 2019165032 A1 (SCRIPPS RESEARCH INST.) 29 August 2019 (2019-08-29) entire document | 1-15 |
| A | JP 2007197324 A (TORAY INDUSTRIES) 09 August 2007 (2007-08-09) entire document | 1-15 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 January 2021** | **25 January 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/123404**

**C. DOCUMENTS CONSIDERED TO BE RELEVANT**

| Category* | Citation of document, with indication, where appropriate, of the relevant passages | Relevant to claim No. |
|---|---|---|
| A | WO 2019195063 A1 (MERCK SHARP & DOHME et al.) 10 October 2019 (2019-10-10)<br>entire document | 1-15 |
| A | Shichong Pang et al. "The effect of a "push-pull" structure on the turn-on fluorescence of photochromic thio-ketone type diarylethenes"<br>*Photochemical & Photobiological Sciences,* Vol. 14, No. 4, 16 January 2015 (2015-01-16), ISSN: 1474-905X,<br>pp. 765-774 | 1-15 |

Form PCT/ISA/210 (second sheet) (January 2015)

**INTERNATIONAL SEARCH REPORT**
**Information on patent family members**

International application No.
**PCT/CN2020/123404**

| Patent document cited in search report | Publication date (day/month/year) | Patent family member(s) | Publication date (day/month/year) |
|---|---|---|---|
| CN 110036001 A | 19 July 2019 | MX 2019003829 A | 01 July 2019 |
| | | US 10414747 B2 | 17 September 2019 |
| | | KR 20190056432 A | 24 May 2019 |
| | | JP 6636673 B2 | 29 January 2020 |
| | | CL 2019000902 A1 | 21 June 2019 |
| | | AU 2017339418 A1 | 18 April 2019 |
| | | BR 112019006816 A2 | 30 July 2019 |
| | | EP 3523287 A1 | 14 August 2019 |
| | | EA 201990846 A1 | 30 August 2019 |
| | | WO 2018067423 A1 | 12 April 2018 |
| | | US 2018093964 A1 | 05 April 2018 |
| | | JP 2019534876 A | 05 December 2019 |
| | | IL 265678 D0 | 30 May 2019 |
| | | CR 20190168 A | 17 May 2019 |
| | | AR 109788 A1 | 23 January 2019 |
| | | TW 201817723 A | 16 May 2018 |
| | | NI 201900029 A | 13 May 2019 |
| | | CA 3038903 A1 | 12 April 2018 |
| | | CO 2019003349 A2 | 12 April 2019 |
| | | EC SP19026680 A | 30 April 2019 |
| | | US 2019337917 A1 | 07 November 2019 |
| | | DO P2019000083 A | 15 July 2019 |
| | | US 2019337918 A1 | 07 November 2019 |
| | | US 10703738 B2 | 07 July 2020 |
| | | AU 2017339418 B2 | 16 April 2020 |
| | | PH 12019500712 A1 | 02 December 2019 |
| | | US 10730849 B2 | 04 August 2020 |
| | | PE 05 July 2019 A1 | 17 May 2019 |
| CN 109563081 A | 02 April 2019 | IL 262094 D0 | 29 November 2018 |
| | | MX 2018012266 A | 30 May 2019 |
| | | EA 201892128 A1 | 30 April 2019 |
| | | AU 2017247806 B2 | 14 November 2019 |
| | | RU 2018137389 A3 | 12 May 2020 |
| | | SG 11201808708 R A | 29 November 2018 |
| | | EP 3440072 B1 | 29 January 2020 |
| | | BR 112018070602 A2 | 05 February 2019 |
| | | KR 20180132783 A | 12 December 2018 |
| | | ES 2781474 T3 | 02 September 2020 |
| | | RU 2018137389 A | 12 May 2020 |
| | | AU 2017247806 A1 | 08 November 2018 |
| | | CA 3019628 A1 | 12 October 2017 |
| | | JP 2019510802 A | 18 April 2019 |
| | | EP 3440072 A1 | 13 February 2019 |
| | | WO 2017175156 A1 | 12 October 2017 |
| WO 0039086 A1 | 06 July 2000 | NO 20013179 L | 27 August 2001 |
| | | HU 0201472 A2 | 28 October 2003 |
| | | CN 1335834 A | 13 February 2002 |
| | | HU 0201472 A3 | 28 March 2006 |
| | | AP 200102169 A0 | 30 June 2001 |
| | | NO 20013179 A | 27 August 2001 |

Form PCT/ISA/210 (patent family annex) (January 2015)

**INTERNATIONAL SEARCH REPORT**
**Information on patent family members**

International application No.
**PCT/CN2020/123404**

| Patent document cited in search report | Publication date (day/month/year) | Patent family member(s) | Publication date (day/month/year) |
|---|---|---|---|
| | | TR 200101886 T2 | 21 December 2001 |
| | | ID 29027 A | 26 July 2001 |
| | | AP 2001002169 A0 | 30 June 2001 |
| | | EP 1142872 A4 | 17 April 2002 |
| | | CZ 20012160 A3 | 17 October 2001 |
| | | NO 20013179 D0 | 22 June 2001 |
| | | AU 1797900 A | 31 July 2000 |
| | | AP 200102169 D0 | 30 June 2001 |
| | | IL 143958 D0 | 21 April 2002 |
| | | BR 9916583 A | 25 September 2001 |
| | | EP 1142872 A1 | 10 October 2001 |
| | | US 6645956 B1 | 11 November 2003 |
| | | DE 69939749 D1 | 27 November 2008 |
| | | KR 20010089708 A | 08 October 2001 |
| | | RU 2001120016 A | 10 December 2003 |
| | | HK 1042701 A1 | 23 August 2002 |
| | | US 6620841 B1 | 16 September 2003 |
| | | CN 1178913 C | 08 December 2004 |
| | | AU 763040 B2 | 10 July 2003 |
| | | CA 2353961 A1 | 06 July 2000 |
| | | JP 3929244 B2 | 13 June 2007 |
| | | US 7098201 B2 | 29 August 2006 |
| | | RU 2225860 C2 | 20 March 2004 |
| | | NZ 512184 A | 29 August 2003 |
| | | US 2004002485 A1 | 01 January 2004 |
| | | PL 348596 A1 | 03 June 2002 |
| | | EP 1142872 B1 | 15 October 2008 |
| | | AT 411286 T | 15 October 2008 |
| US 5705525 A | 06 January 1998 | CN 1058965 C | 29 November 2000 |
| | | CA 2170097 A1 | 24 August 1996 |
| | | ZA 961472 B | 28 August 1996 |
| | | NO 960706 D0 | 22 February 1996 |
| | | NO 960706 L | 26 August 1996 |
| | | NO 314997 B1 | 23 June 2003 |
| | | NO 960706 A | 26 August 1996 |
| | | CY 2262 B1 | 04 July 2003 |
| | | DE 69612416 T2 | 22 November 2001 |
| | | FR 2730996 A1 | 30 August 1996 |
| | | NZ 286045 A | 28 October 1996 |
| | | EP 0728755 B1 | 11 April 2001 |
| | | FI 960795 A | 24 August 1996 |
| | | DK 0728755 T3 | 30 July 2001 |
| | | GR 3036192 T3 | 31 October 2001 |
| | | PT 728755 E | 28 September 2001 |
| | | ES 2158255 T3 | 01 September 2001 |
| | | CA 2170097 C | 26 February 2002 |
| | | ZA 9601472 A | 28 August 1996 |
| | | US RE37078 E1 | 27 February 2001 |
| | | US RE37078 E | 27 February 2001 |
| | | CN 1142496 A | 12 February 1997 |

Form PCT/ISA/210 (patent family annex) (January 2015)

**INTERNATIONAL SEARCH REPORT**
**Information on patent family members**

International application No.
**PCT/CN2020/123404**

| Patent document cited in search report | Publication date (day/month/year) | Patent family member(s) | Publication date (day/month/year) |
|---|---|---|---|
| | | FI 960795 A0 | 21 February 1996 |
| | | JP H08253470 A | 01 October 1996 |
| | | PT 728755 T | 28 September 2001 |
| | | EP 0728755 A1 | 28 August 1996 |
| | | AU 4567596 A | 29 August 1996 |
| | | AU 697832 B2 | 15 October 1998 |
| | | AT 200488 T | 15 April 2001 |
| | | DE 69612416 D1 | 17 May 2001 |
| | | FR 2730996 B1 | 20 June 1997 |
| WO 2019165032 A1 | 29 August 2019 | CA 3091670 A1 | 29 August 2019 |
| | | AU 2019225919 A1 | 24 September 2020 |
| JP 2007197324 A | 09 August 2007 | None | |
| WO 2019195063 A1 | 10 October 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7488802 B
- US 7521051 B
- US 8008449 B
- US 8354509 B
- US 8168757 B
- WO 2004004771 A
- WO 2004072286 A
- WO 2004056875 A
- WO 2013019906 A
- WO 2010077634 A1
- US 8383796 B
- WO 2010027827 A
- WO 2011066342 A
- WO 01002369 A
- WO 02068470 A
- WO 02010192 A


### Non-patent literature cited in the description


- Handbook of Pharmaceutical Salts. Properties, Selection and Use. Wiley-VCH, 2002
- **S. BERGE.** *Journal of Pharmaceutical Sciences,* 1977, vol. 66 (1), 1-19
- **P. GOULD.** *International J. of Pharmaceutics,* 1986, vol. 33, 201-217
- **ANDERSON.** The Practice of Medicinal Chemistry. Academic Press, 1996
- he Orange Book. Food & Drug Administration
- REMINGTON'S PHARMACEUTICAL SCIENCES. Mack Publishing Company
- THE HANDBOOK OF PHARMACEUTICAL ADDITIVES. Gower Publishing Limited
- THE HANDBOOK OF PHARMACEUTICAL EXCIPIENTS. American Pharmaceutical Association and the Pharmaceutical Press
- Remington's Pharmaceutical Sciences. Mack Publishing Company
- **CORRALES, L. ; GLICKMAN, L. H. ; MCWHIRTER, S. M. ; KANNE, D. B. ; SIVICK, K. E. ; KATIBAH, G. E. ; WOO, S. R. ; LEMMENS, E. ; BANDA, T. ; LEONG, J. J.** Direct activation of STING in the tumor microenvironment leads to potent and systemic tumor regression and immunity. *Cell Rep.,* 2015, vol. 11 (7), 1018-1030
- **SHIREY, K. A. ; NHU, Q. M. ; YIM, K. C. ; ROBERTS, Z. J. ; TEIJARO, J. R. ; FARBER, D. L. ; BLANCO, J. C. ; VOGEL, S. N.** The anti-tumor agent, 5,6-dimethylxanthenone-4-acetic acid (DMXAA), induces IFN-beta-mediated antiviral activity in vitro and in vivo. *J. Leukoc. Biol.,* 2011, vol. 89 (3), 351-357